# EUROPEAN PATENT APPLICATION

(11) **EP 4 461 355 A2**
(43) Date of publication of application: **13.11.2024**
(21) Application number: 24203388.4
(22) Date of filing: 03.05.2022
(51) Int. Cl.: A61P 3/00

(54) **BENZIMIDAZOYL GLP-1 RECEPTOR AGONISTS, PHARMACEUTICAL COMPOSITIONS COMPRISING THE SAME, AND METHODS FOR THEIR USE**

(30) Priority: 03.05.2021 US 202163183612 P; 24.11.2021 US 202163282686 P
(62) Divisional of application: 22725069.3
(71) Applicant: Carmot Therapeutics, Inc., Berkeley, California 94710 (US)
(72) Inventor: DU, Xiaohui, Berkeley, 94710 (US); FUCINI, Ray, Berkeley, 94710 (US); RAN, Xu, Berkeley, 94710 (US); YEH, Chien-Hung, Berkeley, 94710 (US); ZHOU, Xiang, Berkeley, 94710 (US); SAKYA, Subas Man, Berkeley, 94710 (US); WANG, Xiaofang, Berkeley, 94710 (US); KAWAI, Hiroyuki, Berkeley, 94710 (US); LEE, Craig, Berkeley, 94710 (US); GARAI, Sumanta, Berkeley, 94710 (US)
(74) Representative: Mathys & Squire

(57) **Abstract**

Provided herein are GLP-1 receptor modulator compounds, pharmaceutical compositions, methods of their preparation, and methods of their use in treatment, and/or diagnosis.

## Description

### CROSS-REFERENCES TO RELATED APPLICATIONS

This application is a PCT International Application, claiming the benefit of U.S. Provisional Application No. 63/282,686, filed on November 24, 2021, and U.S. Provisional Application No. 63/183,612, filed on May 3, 2021, the entire contents of each of which are herein incorporated by reference.

### FIELD

Provided herein are GLP-1 receptor modulator compounds; pharmaceutical compositions comprising the compounds; methods of producing the compounds; and methods of using the compounds, and compositions for therapy. The compounds, and compositions are useful, for instance, in methods of treatment, and prevention of metabolic diseases or conditions, methods of detection of metabolic diseases or conditions, and methods of diagnosis of metabolic diseases or conditions.

### BACKGROUND

Diabetes is a serious chronic disease that occurs when the pancreas does not produce enough insulin, or when the body cannot effectively use the insulin it produces. Complications of diabetes include, damage to the heart, blood vessels, eyes, kidneys, and nerves. Diabetes can increase risk of heart disease, and stroke. The results include serious effects on quality of life, health, and mortality. WHO Global Report on Diabetes, 2016, World Health Organization. As of 2017, approximately 462 million individuals worldwide, about 6.28% of the population was affected by type 2 diabetes, and this prevalence was increasing measurably. Khan et al., 2020, J. Epidemiol. Glob. Health 10(1):107-111. The global economic burden of diabetes in 2015 was estimated to be $1.3T, and estimated to increase to $2.1T by 2030. Bommer et al., 2018, Diabetes Care 41(5):963-970. Approximately 90-95% of all diabetes cases are type 2 diabetes. Tripathi & Srivastava, 2016, Med. Sci. Monit. 12(7):RA130-147.

The glucagon-like peptide-1 receptor (GLP-1 receptor, or GLP1R) has emerged as a potential target for treating type 2 diabetes. Its ligand, glucagon-like peptide-1 (GLP-1) enhances glucose-induced insulin secretion, and increases insulin synthesis among many other effects. Doyle and Egan, 2007, Pharmacol. Ther. 113(3):546-593. GLP-1 is known to delay gastric emptying, suppress food intake, increase satiety, and reduce weight in humans. Shah and Vella, 2014 Rev Endocr Metab Disord. 15(3): 181-187. Activating the GLP-1 receptor has been shown to have beneficial effects on insulin secretion and the maintenance of beta cell glucose sensing, transcription, synthesis, proliferation, and survival. Doyle and Egan, 2007, *supra.* While the GLP-1 receptor is a promising therapeutic target, only a handful of GLP-1 receptor drugs have been approved to date, and most, or all of these are peptide, or polypeptide drugs.

There is a need for additional therapies for treating metabolic diseases, and conditions, like type 2 diabetes. Small molecules targeting GLP-1 receptor should provide safe, stable, and easy to administer therapeutics for metabolic diseases, and conditions such as type 2 diabetes.

### SUMMARY

Provided herein are GLP-1 receptor modulator compounds of Formulas (I)-**(LVIII),** and sub-formulas thereof, compositions comprising the compounds, methods of producing the compounds, and methods of using the compounds, and compositions in treatment, and diagnosis. The compounds of Formula **(I)-(LVI),** and sub-formulas and embodiments thereof, are useful for modulating the activity of GLP-1 receptor. In certain embodiments, the compounds can be used to agonize the activity of GLP-1 receptor. In certain embodiments, the compounds can be used for treating diseases, or conditions modulated by GLP-1 receptor.

In one aspect, provided is a compound of Formula **(I),** or a pharmaceutically acceptable salt, or stereoisomer thereof: 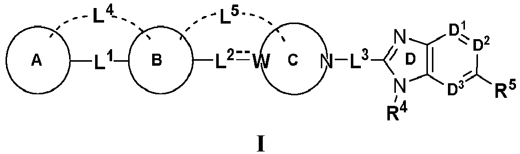
wherein, **A** is substituted or unsubstituted aryl, or substituted or unsubstituted heteroaryl; Ring **B** is substituted or unsubstituted aryl, substituted or unsubstituted heteroaryl, or substituted or unsubsuted bicyclic;
Ring C is substituted or unsubstituted C₄-C₆ heterocycloalkyl, or substituted or unsubstituted C₅ heterocycloalkenyl, or substituted or unsubstituted phenyl, wherein heterocycloalkyl and heterocycloalkenyl each comprises at least one N, linked to **L³** as depicted;
zero, one, or two of **D¹**, **D²**, and **D³** are N, and the rest are CH, or **CR⁶**;
**W** is **CR¹⁴**, or C; when **W** is **CR¹⁴**, the adjacent dashed line indicates a single bond; when **W** is C, the adjacent dashed line indicates a double bond or **L²** is -C(H)=;
**L¹** is selected from the group consisting of a bond, -O-, -CH₂-, and OCH₂-;
**L²** is selected from the group consisting of a bond, -CH₂-, -C(H)=, and -O-;
**L³** is -CH₂-
**L⁴** is absent, or **L⁴** together with **L¹** and Rings **A** and **B** form a fused tricycle, and **L⁵** is absent;
**L⁵** is absent, or **L⁵** together with **L²** and Rings **B** and **C** form a fused tricycle, and **L⁴** is absent;
**R⁴** is unsubstituted alkyl, substituted alkyl, unsubstituted arylalkylene, substituted arylalkylene, unsubstituted heteroalkyl, substituted heteroalkyl, unsubstituted heteroarylalkylene, substituted heteroarylalkylene, unsubstituted heterocycloalkylalkylene, or substituted heterocycloalkylalkylene;
**R⁵** is selected from the group consisting of -COOH, -COCF₃, -C(OH)CF₃, -CONHCN, - CONHOH, CONHOMe, -CONHSO₂N(Me)₂, -PO₃H₂, -PO(Me)(OH), -SO₃H, -SO₂NH₂, - SO₂NHMe, -B(OH)₂, 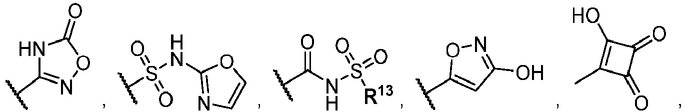 and tetrazolyl;
each **R⁶** is independently selected from the group consisting of alkyl, substituted alkyl, halo, hydroxyl, alkoxyl, **R¹¹R¹²**NCO-, and **R¹¹R¹²**N-;
**R¹¹** is hydrogen, or alkyl;
**R¹²** is hydrogen, or alkyl;
**R¹³** is hydrogen, or alkyl;
**R¹⁴** is hydrogen, cyano, halo, hydroxyl, alkyl, haloalklyl, or methyl;
wherein when the C ring is C₆ heterocycloalkyl, the **B** ring is pyrazole or

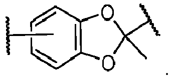

In one aspect, provided is a compound of Formula **(Ia),** or a pharmaceutically acceptable salt, or stereoisomer thereof: 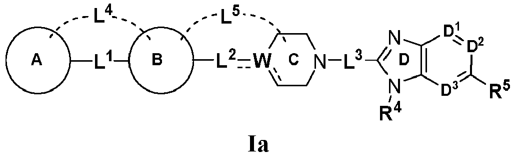
wherein, **A** is substituted or unsubstituted aryl, or substituted or unsubstituted heteroaryl;
Ring **B is** substituted or unsubstituted aryl, or substituted or unsubstituted heteroaryl;
Ring **C** is further unsubstituted or further substituted, and/or bridged;
zero, one, or two of **D¹**, **D²**, and **D³** are N, and the rest are CH or **CR⁶**;
**W** is CH or C; when **W** is CH the adjacent dashed lines indicate single bonds;
   when **W** is C, one adjacent dashed line indicates a double bond, for instance **L²** is - C(H)=;
**L¹** is selected from the group consisting of -O-, -CH₂-, and -OCH₂-;
**L²** is selected from the group consisting of a bond, -CH₂-, -C(H)=, and -O-;
**L³** is -CH₂-;
**L⁴** is absent, or **L⁴** together with **L¹** and Rings **A** and **B** form a fused tricycle, and **L⁵** is absent;
**L⁵** is absent, or **L⁵** together with L**²** and Rings **B** and **C** form a fused tricycle, and **L⁴** is absent;
**R⁴** is unsubstituted alkyl, substituted alkyl, unsubstituted arylalkylene, substituted arylalkylene, unsubstituted heteroalkyl, substituted heteroalkyl, unsubstituted heteroarylalkylene, substituted heteroarylalkylene, unsubstituted heterocycloalkylalkylene, or substituted heterocycloalkylalkylene;
**R⁵** is selected from the group consisting of -COOH, -COCF₃, -C(OH)CF₃, -CONHCN, - CONHOH, CONHOMe, -CONHSO₂N(Me)₂, -PO₃H₂, -PO(Me)(OH), -SO₃H, -SO₂NH₂, - SO₂NHMe, -B(OH)₂, 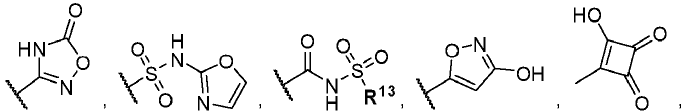 and tetrazolyl;
each **R⁶** is independently selected from the group consisting of alkyl, substituted alkyl, halo, hydroxyl, alkoxyl, **R¹¹R¹²**NCO-, and **R¹¹R¹²**N-;
**R¹¹** is hydrogen or alkyl;
**R¹²** is hydrogen or alkyl; and
**R¹³** is hydrogen or alkyl

In one aspect, provided is a compound of Formula (Ib), or a pharmaceutically acceptable salt, or stereoisomer thereof: 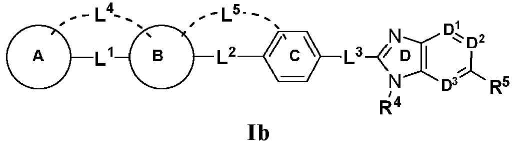
wherein, **A** is substituted or unsubstituted aryl, or substituted or unsubstituted heteroaryl; Ring **B** is substituted or unsubstituted aryl, or substituted or unsubstituted heteroaryl;
Ring **C** is further unsubstituted or further substituted, and/or fused;
zero, one, or two of **D¹**, **D²**, and **D³** are N, and the rest are CH or **CR⁶**;
**L¹** is selected from the group consisting of -O-, -CH₂-, and -OCH₂-;
**L²** is selected from the group consisting of a bond, -CH₂-, -C(H)=, and -O-;
**L³** is -CH₂-;
**L⁴** is absent, or **L⁴** together with **L¹** and Rings **A** and **B** form a fused tricycle, and **L⁵** is absent;
**L⁵** is absent, or **L⁵** together with **L²** and Rings **B** and **C** form a fused tricycle, and **L⁴** is absent;
**R⁴** is unsubstituted alkyl, substituted alkyl, unsubstituted arylalkylene, substituted arylalkylene, unsubstituted heteroalkyl, substituted heteroalkyl, unsubstituted heteroarylalkylene, substituted heteroarylalkylene, unsubstituted heterocycloalkylalkylene, or substituted heterocycloalkylalkylene;
**R⁵** is selected from the group consisting of -COOH, -COCF₃, -C(OH)CF₃, -CONHCN, - CONHOH, CONHOMe, -CONHSO₂N(Me)₂, -PO₃H₂, -PO(Me)(OH), -SO₃H, -SO₂NH₂, - SO₂NHMe, -B(OH)₂, 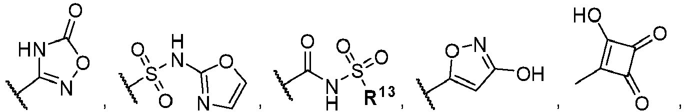 and tetrazolyl;
each **R⁶** is independently selected from the group consisting of alkyl, substituted alkyl, halo, hydroxyl, alkoxyl, **R¹¹R¹²**NCO-, and **R¹¹R¹²**N-;
**R¹¹** is hydrogen or alkyl;
**R¹²** is hydrogen or alkyl; and
**R¹³** is hydrogen or alkyl.

In one aspect, provided is a compound of Formula (Ic), or a pharmaceutically acceptable salt, or stereoisomer thereof: 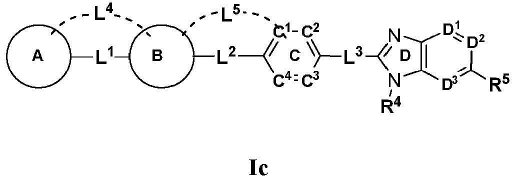
wherein, **A** is substituted or unsubstituted aryl, or substituted or unsubstituted heteroaryl; Ring **B** is substituted or unsubstituted aryl, or substituted or unsubstituted heteroaryl;
Ring **C** is further unsubstituted or further substituted, and/or fused;
zero, one, or two of **C¹**, **C²**, **C³**, and **C⁴** are N, and the rest are CH, or **CR⁶**;
zero, one, or two of **D¹**, **D²**, and **D³** are N, and the rest are CH or **CR⁶**;
**L¹** is selected from the group consisting of -O-, -CH₂-, and -OCH₂-;
**L²** is selected from the group consisting of a bond, -CH₂-, -C(H)=, and -O-;
**L³** is -CH₂-;
**L⁴** is absent, or **L⁴** together with **L¹** and Rings **A** and **B** form a fused tricycle, and **L⁵** is absent;
**L⁵** is absent, or **L⁵** together with L**²** and Rings **B** and **C** form a fused tricycle, and **L⁴** is absent;
**R⁴** is unsubstituted alkyl, substituted alkyl, unsubstituted arylalkylene, substituted arylalkylene, unsubstituted heteroalkyl, substituted heteroalkyl, unsubstituted heteroarylalkylene, substituted heteroarylalkylene, unsubstituted heterocycloalkylalkylene, or substituted heterocycloalkylalkylene;
**R⁵** is selected from the group consisting of -COOH, -COCF₃, -C(OH)CF₃, -CONHCN, - CONHOH, CONHOMe, -CONHSO₂N(Me)₂, -PO₃H₂, -PO(Me)(OH), -SO₃H, -SO₂NH₂, - SO₂NHMe, -B(OH)₂, 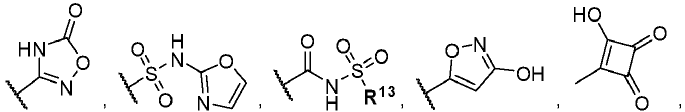 and tetrazolyl;
each **R⁶** is independently selected from the group consisting of alkyl, substituted alkyl, halo, hydroxyl, alkoxyl, **R¹¹R¹²**NCO-, and **R¹¹R¹²**N-;
**R¹¹** is hydrogen or alkyl;
**R¹²** is hydrogen or alkyl; and
**R¹³** is hydrogen or alkyl.

In one aspect provided is a compound selected from Compound 529, 530, 533, 534, 535, and 551, or a pharmaceutically salt, tautomer, stereoisomer, and/or mixture of stereoisomers thereof:

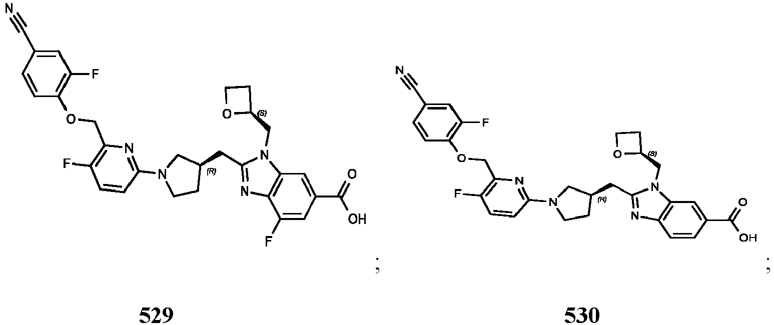

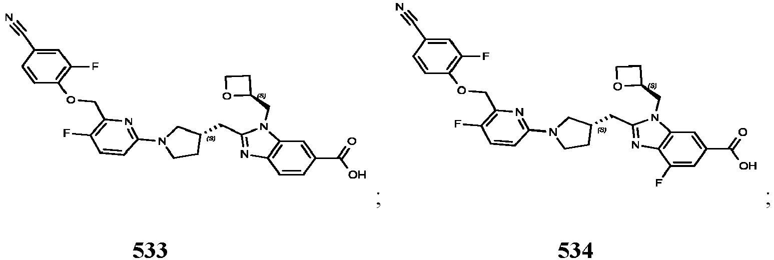

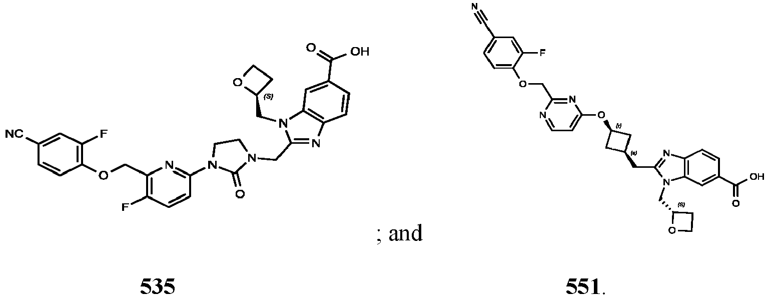

In certain aspects, the compounds are useful in methods of treatment and prevention of metabolic diseases, and conditions, methods of detection of metabolic diseases, and conditions, and methods of diagnosis of metabolic diseases, and conditions.

In another aspect, provided are compositions comprising a compound of Formula **(I), (Ia), (Ib),** or **(Ic).** In some embodiments, the compositions are pharmaceutical compositions. Any suitable pharmaceutical composition may be used. In a further aspect, provided herein is a kit comprising the compound of Formula **(I), (Ia), (Ib),** or **(Ic),** or embodiments thereof, or a pharmaceutical composition thereof.

In another aspect, provided herein are methods of using the compounds or the compositions described herein. In some embodiments, the methods are for treatment. In some embodiments, the methods are diagnostic methods. In some embodiments, the methods are analytical methods. In some embodiments, the compounds, or compositions described herein are used to treat a disease, or condition. In some aspects, the disease, or condition is selected from metabolic diseases, or conditions. In certain embodiments, the disease is type 2 diabetes.

Also provided herein is the use of compounds described herein, and compositions thereof, for the treatment of a metabolic disease, or condition. Also provided herein is the use of compounds described herein, and compositions thereof, for the treatment of type 2 diabetes.

### DESCRIPTION OF EXEMPLARY EMBODIMENTS

Described herein are GLP-1 receptor compounds useful for treating metabolic diseases or conditions, such as type 2 diabetes.

### Definitions

Unless otherwise defined, all terms of art, notations and other scientific terminology used herein are intended to have the meanings commonly understood by those of skill in the art to which this disclosure pertains. In some cases, terms with commonly understood meanings are defined herein for clarity, and/or ready reference. The techniques and procedures described or referenced herein are generally well understood, and are commonly employed using conventional methodologies by those skilled in the art. As appropriate, procedures involving the use of commercially available kits, and reagents are generally carried out in accordance with manufacturer-defined protocols, and conditions unless otherwise noted.

As used herein, the singular forms "a," "an," and "the" include the plural referents unless the context clearly indicates otherwise.

The term "about" indicates and encompasses an indicated value, and a range above and below that value. In certain embodiments, the term "about" indicates the designated value ± 10%, ± 5%, or ± 1%. In certain embodiments, the term "about" indicates the designated value ± one standard deviation of that value. In certain embodiments, for example, logarithmic scales (e.g., pH), the term "about" indicates the designated value ± 0.3, ± 0.2, or ± 0.1.

When referring to the compounds provided herein, the following terms have the following meanings unless indicated otherwise. Unless defined otherwise, all technical and scientific terms used herein have the same meaning as is commonly understood by one of ordinary skill in the art. In the event that there is a plurality of definitions for a term herein, those in this section prevail unless stated otherwise.

"Alkoxy" and "alkoxyl," refer to the group -O**R"** where **R"** is alkyl or cycloalkyl. Alkoxy groups include, in certain embodiments, methoxy, ethoxy, n-propoxy, isopropoxy, n-butoxy, tert-butoxy, sec-butoxy, n-pentoxy, n-hexoxy, 1,2-dimethylbutoxy, and the like.

The term "alkoxyamine," as used herein, refers to the group -alkylene-O-NH₂, wherein alkylene is as defined herein. In some embodiments, alkoxyamine groups can react with aldehydes to form oxime residues. Examples of alkoxyamine groups include -CH₂CH₂-O-NH₂ and -CH₂-O-NH₂.

The term "alkyl," as used herein, unless otherwise specified, refers to a saturated straight, or branched hydrocarbon. In certain embodiments, the alkyl group is a primary, secondary, or tertiary hydrocarbon. In certain embodiments, the alkyl group includes one to ten carbon atoms (i.e., C₁ to C₁₀ alkyl). In certain embodiments, the alkyl is a lower alkyl, for example, C₁₋₆ alkyl, and the like. In certain embodiments, the alkyl group is selected from the group consisting of methyl, ethyl, propyl, isopropyl, butyl, isobutyl, secbutyl, t-butyl, pentyl, isopentyl, neopentyl, hexyl, isohexyl, 3-methylpentyl, 2,2-dimethylbutyl, and 2,3-dimethylbutyl. In certain embodiments, "substituted alkyl" refers to an alkyl substituted with one, two, or three groups independently selected from a halogen (e.g., fluoro (F), chloro (Cl), bromo (Br), or iodo (I)), alkyl, haloalkyl, hydroxyl, amino, alkylamino, alkoxy, aryl, heteroaryl, cycloalkyl, and heterocycloalkylalkylene. In some embodiments, alkyl is unsubstituted.

The term "alkylene," as used herein, unless otherwise specified, refers to a divalent alkyl group, as defined herein. "Substituted alkylene" refers to an alkylene group substituted as described herein for alkyl. In some embodiments, alkylene is unsubstituted.

"Alkenyl" refers to an olefinically unsaturated hydrocarbon group, in certain embodiments, having up to about eleven carbon atoms, or from two to six carbon atoms (e.g., "lower alkenyl"), which can be straight-chained or branched, and having at least one, or from one to two sites of olefinic unsaturation. "Substituted alkenyl" refers to an alkenyl group substituted as described herein for alkyl.

"Alkenylene" refers to a divalent alkenyl as defined herein. Lower alkenylene is, for example, C₂-C₆-alkenylene.

"Alkynyl" refers to acetylenically unsaturated hydrocarbon groups, in certain embodiments, having up to about eleven carbon atoms, or from two to six carbon atoms (e.g., "lower alkynyl"), which can be straight-chained or branched, and having at least one, or from one to two sites of acetylenic unsaturation. Non-limiting examples of alkynyl groups include acetylene (-C=CH), propargyl (-CH₂C≡CH), and the like. "Substituted alkynyl" refers to an alkynyl group substituted as described herein for alkyl.

"Alkynylene" refers to a divalent alkynyl as defined herein. Lower alkynylene is, for example, C₂-C₆-alkynylene.

"Amino" refers to -NH₂.

The term "alkylamino," as used herein, and unless otherwise specified, refers to the group -NH**R"** where **R"** is, for example, C₁₋₁₀alkyl, as defined herein. In certain embodiments, alkylamino is C₁₋₆alkylamino.

The term "dialkylamino," as used herein, and unless otherwise specified, refers to the group -N**R"R"** where, each **R"** is independently C₁₋₁₀alkyl, as defined herein. In certain embodiments, dialkylamino is di-C₁₋₆alkylamino.

The term "aryl," as used herein, and unless otherwise specified, refers to phenyl, biphenyl, or naphthyl. The term includes both substituted and unsubstituted moieties. An aryl group can be substituted with any described moiety including, but not limited to, one or more moieties (e.g., in some embodiments one, two, or three moieties) selected from the group consisting of halogen (e.g., fluoro (F), chloro (Cl), bromo (Br), or iodo (I)), alkyl, haloalkyl, hydroxyl, amino, alkylamino, arylamino, alkoxy, aryloxy, nitro, cyano, sulfonic acid, sulfate, phosphonic acid, phosphate, and phosphonate, wherein each moiety is independently either unprotected, or protected as necessary, as would be appreciated by those skilled in the art (e.g., Greene, et al., Protective Groups in Organic Synthesis, John Wiley and Sons, Second Edition, 1991); and wherein the aryl in the arylamino and aryloxy substituents are not further substituted.

The term "arylamino," as used herein, and unless otherwise specified, refers to an -N**R'R**" group where **R' is** hydrogen, or C₁-C₆-alkyl; and **R"** is aryl, as defined herein.

The term "arylene," as used herein, and unless otherwise specified, refers to a divalent aryl group, as defined herein.

The term "aryloxy," as used herein, and unless otherwise specified, refers to an -O**R** group where **R** is aryl, as defined herein.

"Alkarylene" refers to an arylene group, as defined herein, wherein the aryl ring is substituted with one or two alkyl groups. "Substituted alkarylene" refers to an alkarylene, as defined herein, where the arylene group is further substituted, as defined herein for aryl.

"Aralkylene" refers to an -alkyl-arylene- or arylene-alkyl, for instance, a -C₁-C₂ alkyl-arylene-, -arylene-C₁-C₂ alkyl-, or - C₁-C₂ alkyl-arylene-C₁-C₂ alkyl- group, where arylene is as defined herein. "Substituted aralkylene" refers to an aralkylene, as defined herein, where the aralkylene group is substituted, as defined herein for aryl. "Substituted C₁-C₂ alkyl" refers to a C₁-C₂ alkyl group that is substituted as defined herein for an alkyl.

"Arylalkylene" refers to -alkyl-arylene- or arylene-alkyl, for instance, a -C₁-C₂ alkyl-arylene-, -arylene-C₁-C₂ alkyl -, or - C₁-C₂ alkyl-arylene-C₁-C₂ alkyl- group, where arylene is as defined herein. "Substituted arylalkylene" refers to an arylalkylene, as defined herein, where the arylalkylene group is substituted, as defined herein for aryl. "Substituted C₁-C₂ alkyl" refers to a C₁-C₂ alkyl group that is substituted as defined herein for an alkyl.

"Carboxyl" or "carboxy" refers to -C(O)OH or -COOH.

The term "cycloalkyl," as used herein, unless otherwise specified, refers to a saturated cyclic hydrocarbon. In certain embodiments, the cycloalkyl group may be a saturated, and/or bridged, and/or non-bridged, and/or a fused bicyclic group. In certain embodiments, the cycloalkyl group includes three to ten carbon atoms (i.e., C₃ to C₁₀ cycloalkyl). In some embodiments, the cycloalkyl has from three to fifteen carbons (C₃₋₁₅), from three to ten carbons (C₃₋₁₀), from three to seven carbons (C₃₋₇), or from three to six carbons (C₃-C₆) (i.e., "lower cycloalkyl"). In certain embodiments, the cycloalkyl group is cyclopropyl, cyclobutyl, cyclopentyl, cyclohexyl, cyclohexylmethyl, cycloheptyl, bicyclo[2.1.1]hexyl, bicyclo[2.2.1]heptyl, decalinyl, or adamantyl.

The term "cycloalkylene," as used herein refers to a divalent cycloalkyl group, as defined herein. In certain embodiments, the cycloalkylene group is cyclopropylene 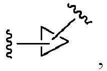 cyclobutylene 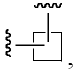 cyclopentylene 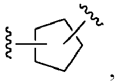 cyclohexylene 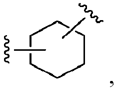 cycloheptylene 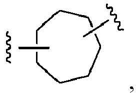 and the like. Lower cycloalkylene refers to a C₃-C₆-cycloalkylene.

The term "cycloalkylalkyl," as used herein, unless otherwise specified, refers to an alkyl group, as defined herein, substituted with one or two cycloalkyl, as defined herein.

The term "ester," as used herein, refers to -C(O)O**R**, or -COO**R**, where **R** is alkyl, as defined herein.

The term "haloalkyl" refers to an alkyl group, as defined herein, substituted with one, or more halogen atoms (e.g., in some embodiments one, two, three, four, or five) which are independently selected.

The term "heteroalkyl" refers to an alkyl, as defined herein, in which one or more carbon atoms are replaced by heteroatoms. As used herein, "heteroalkenyl" refers to an alkenyl, as defined herein, in which one, or more carbon atoms are replaced by heteroatoms. As used herein, "heteroalkynyl" refers to an alkynyl, as defined herein, in which one, or more carbon atoms are replaced by heteroatoms. Suitable heteroatoms include, but are not limited to, nitrogen (N), oxygen (O), and sulfur (S) atoms. Heteroalkyl, heteroalkenyl, and heteroalkynyl are optionally substituted. Examples of heteroalkyl moieties include, but are not limited to, aminoalkyl, sulfonylalkyl, and sulfinylalkyl. Examples of heteroalkyl moieties also include, but are not limited to, methylamino, methylsulfonyl, and methylsulfinyl. "Substituted heteroalkyl" refers to heteroalkyl substituted with one, two, or three groups independently selected from halogen (e.g., fluoro (F), chloro (Cl), bromo (Br), or iodo (I)), alkyl, haloalkyl, hydroxyl, amino, alkylamino, and alkoxy. In some embodiments, a heteroalkyl group may comprise one, two, three, or four heteroatoms. Those of skill in the art will recognize that a 4-membered heteroalkyl may generally comprise one, or two heteroatoms, a 5- or 6-membered heteroalkyl may generally comprise one, two, or three heteroatoms, and a 7- to 10-membered heteroalkyl may generally comprise one, two, three, or four heteroatoms.

The term "heteroalkylene," as used herein, refers to a divalent heteroalkyl, as defined herein. "Substituted heteroalkylene" refers to a divalent heteroalkyl, as defined herein, substituted as described for heteroalkyl.

The term "heterocycloalkyl" refers to a monovalent, monocyclic, or multicyclic non-aromatic ring system, wherein one, or more of the ring atoms are heteroatoms independently selected from oxygen (O), sulfur (S), and nitrogen (N) (e.g., where the nitrogen, or sulfur atoms may be optionally oxidized, and the nitrogen atoms may be optionally quatemized) and the remaining ring atoms of the non-aromatic ring are carbon atoms. In certain embodiments, heterocycloalkyl is a monovalent, monocyclic, or multicyclic fully-saturated ring system. In certain embodiments, the heterocycloalkyl group has from three to twenty, from three to fifteen, from three to ten, from three to eight, from four to seven, from four to eleven, or from five to six ring atoms. The heterocycloalkyl may be attached to a core structure at any heteroatom or carbon atom which results in the creation of a stable compound. In certain embodiments, the heterocycloalkyl is a monocyclic, bicyclic, tricyclic, or tetracyclic ring system, which may include a fused or bridged ring system and in which the nitrogen or sulfur atoms may be optionally oxidized, and/or the nitrogen atoms may be optionally quatemized. In some embodiments, heterocycloalkyl radicals include, but are not limited to, 2,5-diazabicyclo[2.2.2]octanyl, decahydroisoquinolinyl, dihydrobenzisoxazinyl, dihydrofuryl, dihydroisoindolyl, dihydropyranyl, dihydropyrazolyl, dihydropyrazinyl, dihydropyridinyl, dihydropyrimidinyl, dihydropyrrolyl, dioxolanyl, 1,4-dithianyl, furanonyl, imidazolidinyl, imidazolinyl, indolinyl, isothiazolidinyl, isoxazolidinyl, morpholinyl, octahydroindolyl, octahydroisoindolyl, oxazolidinonyl, oxazolidinyl, oxiranyl, piperazinyl, piperidinyl, 4-piperidonyl, pyrazolidinyl, pyrazolinyl, pyrrolidinyl, pyrrolinyl, quinuclidinyl, tetrahydrofuryl, tetrahydroisoquinolinyl, tetrahydropyranyl, tetrahydrothienyl, thiamorpholinyl, thiazolidinyl, tetrahydroquinolinyl, and 1,3,5-trithianyl. In certain embodiments, heterocycloalkyl may also be optionally substituted as described herein. In certain embodiments, heterocycloalkyl is substituted with one, two, or three groups independently selected from halogen (e.g., fluoro (F), chloro (Cl), bromo (Br), or iodo (I)), alkyl, haloalkyl, hydroxyl, amino, alkylamino, and alkoxy. In some embodiments, a heterocycloalkyl group may comprise one, two, three, or four heteroatoms. Those of skill in the art will recognize that a 4-membered heterocycloalkyl may generally comprise one or two heteroatoms, a 5 or 6-membered heterocycloalkyl may generally comprise one, two, or three heteroatoms, and a 7- to 10-membered heterocycloalkyl may generally comprise one, two, three, or four heteroatoms.

"Heterocycloalkylene" refers to a divalent heterocycloalkyl as defined herein.

A "heterocycloalkenyl" group, as used herein, refers to a mono- or bicylic (e.g., 5-to 10-membered mono- or bicyclic) non-aromatic ring structure having one or more double bonds, and wherein one or more of the ring atoms is a heteroatom (e.g., N, O, or S). Monocyclic and bicyclic heterocycloaliphatics are numbered according to standard chemical nomenclature.

The term "heteroaryl" refers to a monovalent monocyclic aromatic group, and/or multicyclic aromatic group, wherein at least one aromatic ring contains one, or more heteroatoms independently selected from oxygen, sulfur, and nitrogen in the ring. Each ring of a heteroaryl group can contain one, or two oxygen atoms, one, or two sulfur atoms, and/or one to four nitrogen atoms, provided that the total number of heteroatoms in each ring is four, or less and each ring contains at least one carbon atom. In certain embodiments, the heteroaryl has from five to twenty, from five to fifteen, or from five to ten ring atoms. A heteroaryl may be attached to the rest of the molecule via a nitrogen or a carbon atom. In some embodiments, monocyclic heteroaryl groups include, but are not limited to, furanyl, imidazolyl, isothiazolyl, isoxazolyl, oxadiazolyl, oxazolyl, pyrazinyl, pyrazolyl, pyridazinyl, pyridyl, pyrimidinyl, pyrrolyl, triazolyl, thiadiazolyl, thiazolyl, thienyl, tetrazolyl, and triazinyl. Examples of bicyclic heteroaryl groups include, but are not limited to, benzofuranyl, benzimidazolyl, benzoisoxazolyl, benzopyranyl, benzothiadiazolyl, benzothiazolyl, benzothienyl, benzotriazolyl, benzoxazolyl, furopyridyl, imidazopyridinyl, imidazothiazolyl, indolizinyl, indolyl, indazolyl, isobenzofuranyl, isobenzothienyl, isoindolyl, isoquinolinyl, naphthyridinyl, oxazolopyridinyl, phthalazinyl, pteridinyl, purinyl, pyridopyridyl, pyrrolopyridyl, quinolinyl, quinoxalinyl, quinazolinyl, thiadiazolopyrimidyl, and thienopyridyl. Examples of tricyclic heteroaryl groups include, but are not limited to, acridinyl, benzindolyl, carbazolyl, dibenzofuranyl, perimidinyl, phenanthrolinyl, phenanthridinyl, phenarsazinyl, phenazinyl, phenothiazinyl, phenoxazinyl, and xanthenyl. In certain embodiments, heteroaryl may also be optionally substituted as described herein. "Substituted heteroaryl" is a heteroaryl substituted as defined for aryl.

The term "heteroarylene" refers to a divalent heteroaryl group, as defined herein. "Substituted heteroarylene" is a heteroarylene substituted as defined for aryl.

The term "heteroarylalkylene" refers to -alkyl-heteroarylene- or -heteroarylene-alkyl, for instance, a a -C₁-C₂ alkyl-heteroarylene-, -heteroarylene-C₁-C₂ alkyl-, or -C₁-C₂ alkyl-heteroarylene-C₁-C₂ alkyl- group, where heteroarylene is as defined herein. "Substituted heteroarylalkylene" refers to a heteroarylalkylene, as defined herein, where the heteroarylalkylene group is substituted, as defined herein for aryl. "Substituted C₁-C₂ alkyl" refers to a C₁-C₂ alkyl group that is substituted as defined herein for an alkyl.

The term "protecting group," as used herein, and unless otherwise specified, refers to a group that is added to an oxygen, nitrogen, or phosphorus atom to prevent its further reaction, or for other purposes. A wide variety of oxygen, and nitrogen protecting groups are known to those skilled in the art of organic synthesis. (See, e.g., Greene, et al., Protective Groups in Organic Synthesis, John Wiley and Sons, Fourth Edition, 2006, which is incorporated herein by reference).

"Pharmaceutically acceptable salt" refers to any salt of a compound provided herein which retains its biological properties, and which is not toxic or otherwise undesirable for pharmaceutical use. Such salts may be derived from a variety of organic, and inorganic counter-ions well known in the art. Such salts include, but are not limited to (1) acid addition salts formed with organic, or inorganic acids such as hydrochloric, hydrobromic, sulfuric, nitric, phosphoric, sulfamic, acetic, trifluoroacetic, trichloroacetic, propionic, hexanoic, cyclopentylpropionic, glycolic, glutaric, pyruvic, lactic, malonic, succinic, sorbic, ascorbic, malic, maleic, fumaric, tartaric, citric, benzoic, 3-(4-hydroxybenzoyl)benzoic, picric, cinnamic, mandelic, phthalic, lauric, methanesulfonic, ethanesulfonic, 1,2-ethane-disulfonic, 2-hydroxyethanesulfonic, benzenesulfonic, 4-chlorobenzenesulfonic, 2-naphthalenesulfonic, 4-toluenesulfonic, camphoric, camphorsulfonic, 4-methylbicyclo[2.2.2]-oct-2-ene-1-carboxylic, glucoheptonic, 3-phenylpropionic, trimethylacetic, tert-butylacetic, lauryl sulfuric, gluconic, glutamic, hydroxynaphthoic, salicylic, stearic, cyclohexylsulfamic, quinic, muconic acid, and the like; or (2) salts formed when an acidic proton present in the parent compound either (a) is replaced by a metal ion, for example, an alkali metal ion, an alkaline earth ion, or an aluminum ion, or alkali metal, or alkaline earth metal hydroxides, such as sodium, potassium, calcium, magnesium, aluminum, lithium, zinc, and barium hydroxide, or ammonia; or (b) coordinates with an organic base, such as aliphatic, alicyclic, or aromatic organic amines, including, without limitation, ammonia, methylamine, dimethylamine, diethylamine, picoline, ethanolamine, diethanolamine, triethanolamine, ethylenediamine, lysine, arginine, ornithine, choline, *N,N'*-dibenzylethylene-diamine, chloroprocaine, procaine, *N*-benzylphenethylamine, TV-methylglucamine piperazine, tris(hydroxymethyl)-aminomethane, tetramethylammonium hydroxide, and the like.

Pharmaceutically acceptable salts further include, by way of example and without limitation, sodium, potassium, calcium, magnesium, ammonium, and tetraalkylammonium salts, and the like, and when the compound contains a basic functionality, salts of non-toxic organic, or inorganic acids, such as hydrohalides, for example, hydrochloride and hydrobromide, sulfate, phosphate, sulfamate, nitrate, acetate, trifluoroacetate, trichloroacetate, propionate, hexanoate, cyclopentylpropionate, glycolate, glutarate, pyruvate, lactate, malonate, succinate, sorbate, ascorbate, malate, maleate, fumarate, tartarate, citrate, benzoate, 3-(4-hydroxybenzoyl)benzoate, picrate, cinnamate, mandelate, phthalate, laurate, methanesulfonate (mesylate), ethanesulfonate, 1,2-ethane-disulfonate, 2-hydroxyethanesulfonate, benzenesulfonate (besylate), 4-chlorobenzenesulfonate, 2-naphthalenesulfonate, 4-toluenesulfonate, camphorate, camphorsulfonate, 4-methylbicyclo[2.2.2]-oct-2-ene-1-carboxylate, glucoheptonate, 3-phenylpropionate, trimethylacetate, *ter*t-butylacetate, lauryl sulfate, gluconate, glutamate, hydroxynaphthoate, salicylate, stearate, cyclohexylsulfamate, quinate, muconate, and the like.

The term "substantially free of" or "substantially in the absence of" with respect to a composition refers to a composition that includes at least 85%, or 90% by weight, in certain embodiments 95%, 98 %, 99%, or 100% by weight; or in certain embodiments, 95%, 98%, 99%, or 100% of the designated enantiomer or diastereomer of a compound. In certain embodiments, in the methods and compounds provided herein, the compounds are substantially free of one of two enantiomers. In certain embodiments, in the methods and compounds provided herein, the compounds are substantially free of one of two diastereomers. In certain embodiments, in the methods, and compounds provided herein, the compounds are substantially free of enantiomers (i.e., a racemic, or 50:50 mixture of compounds).

Similarly, the term "isolated" with respect to a composition refers to a composition that includes at least 85%, 90%, 95%, 98%, or 99% to 100% by weight, of the compound, the remainder comprising other chemical species, enantiomers or diastereomers.

"Solvate" refers to a compound provided herein, or a salt thereof, that further includes a stoichiometric, or non-stoichiometric amount of solvent bound by non-covalent intermolecular forces. Where the solvent is water, the solvate is a hydrate.

"Isotopic composition" refers to the amount of each isotope present for a given atom, and "natural isotopic composition" refers to the naturally occurring isotopic composition, or abundance for a given atom. Atoms containing their natural isotopic composition may also be referred to herein as "non-enriched" atoms. Unless otherwise designated, the atoms of the compounds recited herein are meant to represent any stable isotope of that atom. For example, unless otherwise stated, when a position is designated specifically as hydrogen (H), the position is understood to have hydrogen at its natural isotopic composition.

"Isotopic enrichment" refers to the percentage of incorporation of an amount of a specific isotope at a given atom in a molecule in the place of that atom's natural isotopic abundance. For example, deuterium (D) enrichment of 1% at a given position means that 1% of the molecules in a given sample contain deuterium at the specified position. Because the naturally occurring distribution of deuterium is about 0.0156%, deuterium enrichment at any position in a compound synthesized using non-enriched starting materials is about 0.0156%. The isotopic enrichment of the compounds provided herein can be determined using conventional analytical methods known to one of ordinary skill in the art, including mass spectrometry and nuclear magnetic resonance spectroscopy.

"Isotopically enriched" refers to an atom having an isotopic composition other than the natural isotopic composition of that atom. "Isotopically enriched" may also refer to a compound containing at least one atom having an isotopic composition other than the natural isotopic composition of that atom.

As used herein, "alkyl," "alkylene," "alkylamino," "dialkylamino," "cycloalkyl," "aryl," "arylene," "alkoxy," "amino," "carboxyl," "heterocycloalkyl," "heteroaryl," "heteroarylene," "carboxyl," and "amino acid" groups optionally comprise deuterium (D) at one, or more positions where hydrogen (H) atoms are present, and wherein the deuterium composition of the atom or atoms is other than the natural isotopic composition.

Also as used herein, "alkyl," "alkylene," "alkylamino," "dialkylamino," "cycloalkyl," "aryl," "arylene," "alkoxy," "amino," "carboxyl," "heterocycloalkyl," "heteroaryl," "heteroarylene," "carboxyl," and "amino acid" groups optionally comprise carbon-13 (¹³C) at an amount other than the natural isotopic composition.

As used herein, term "EC₅₀" refers to a dosage, concentration, or amount of a particular test compound that elicits a dose-dependent response at 50% of maximal expression of a particular response that is induced, provoked, or potentiated by the particular test compound.

As used herein, and unless otherwise specified, the term "IC₅₀" refers to an amount, concentration, or dosage of a particular test compound that achieves a 50% inhibition of a maximal response in an assay that measures such response.

As used herein, the terms "subject", and "patient" are used interchangeably. The terms "subject", and "subjects" refer to an animal, such as a mammal including a non-primate (e.g., a cow, pig, horse, cat, dog, rat, and mouse), and a primate (e.g., a monkey, such as a cynomolgous monkey, a chimpanzee, and a human), and in certain embodiments, a human. In certain embodiments, the subject is a farm animal (e.g., a horse, a cow, a pig, etc.), or a pet (e.g., a dog or a cat). In certain embodiments, the subject is a human.

As used herein, the terms "therapeutic agent", and "therapeutic agents" refer to any agent(s) which can be used in the treatment, or prevention of a disorder, or one or more symptoms thereof. In certain embodiments, the term "therapeutic agent" includes a compound provided herein. In certain embodiments, a therapeutic agent is an agent which is known to be useful for, or has been, or is currently being used for the treatment, or prevention of a disorder, or one, or more symptoms thereof.

"Therapeutically effective amount" refers to an amount of a compound, or composition that, when administered to a subject for treating a condition, is sufficient to effect such treatment for the condition. A "therapeutically effective amount" can vary depending on, *inter alia,* the compound, the disease or disorder, and its severity, and the age, weight, *etc.,* of the subject to be treated.

"Treating" or "treatment" of any disease, or disorder refers, in certain embodiments, to ameliorating a disease, or disorder that exists in a subject. In another embodiment, "treating", or "treatment" includes ameliorating at least one physical parameter, which may be indiscernible by the subject. In yet another embodiment, "treating", or "treatment" includes modulating the disease, or disorder, either physically (e.g., stabilization of a discernible symptom), or physiologically (e.g., stabilization of a physical parameter), or both. In yet another embodiment, "treating", or "treatment" includes delaying, or preventing the onset of the disease, or disorder, or delaying, or preventing recurrence of the disease, or disorder. In yet another embodiment, "treating", or "treatment" includes the reduction or elimination of either the disease, or disorder, or retarding the progression of the disease, or disorder, or of one, or more symptoms of the disease, or disorder, or reducing the severity of the disease, or disorder, or of one, or more symptoms of the disease, or disorder.

As used herein, the terms "prophylactic agent", and "prophylactic agents" as used refer to any agent(s) which can be used in the prevention of a disorder, or one or more symptoms thereof. In certain embodiments, the term "prophylactic agent" includes a compound provided herein. In certain other embodiments, the term "prophylactic agent" does not refer a compound provided herein. For example, a prophylactic agent is an agent which is known to be useful for, or has been or is currently being used to prevent, or impede the onset, development, progression, and/or severity of a disorder.

As used herein, the phrase "prophylactically effective amount" refers to the amount of a therapy (e.g., prophylactic agent) which is sufficient to result in the prevention, or reduction of the development, recurrence, or onset of one or more symptoms associated with a disorder, or to enhance or improve the prophylactic effect(s) of another therapy (e.g., another prophylactic agent).
*Compounds of Formulae **(1),** (**Ia-Ic**), (**IIe**), (**IIf**), (**IIi- IIo**), (**XIX**)-(**XXIV**), (**XXVIII**), (**XXIX**), (**XXXII-XXXVIII**), and (**XLVIII-LVI**)*

Provided herein are GLP-1 receptor compounds useful for modulating one, or more properties of GLP-1 receptor. The compounds can be prepared as described herein and used for therapy, or diagnosis. In certain embodiments, the therapy is the treatment of a metabolic disease or condition. In certain embodiments, the therapy is the treatment of type 2 diabetes.

The embodiments described herein include the recited compounds as well as pharmaceutically acceptable salts, hydrates, solvates, stereoisomers, tautomers, and/or mixtures thereof.

In certain embodiments, provided is a compound of Formula (I), or a pharmaceutically acceptable salt, tautomer, stereoisomer, and/or mixture of stereoisomers thereof: 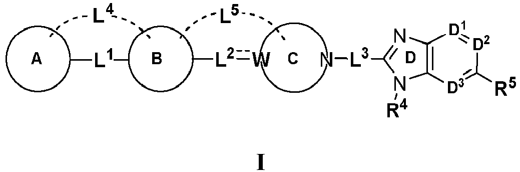 wherein **A** is substituted or unsubstituted aryl, substituted or unsubstituted heteroaryl; Ring **B** is substituted or unsubstituted aryl, or substituted or unsubstituted heteroaryl; Ring **C** is further unsubstituted or further substituted, and/or bridged; zero, one, or two of **D¹**, **D²**, and **D³** are N, and the rest are CH, or **CR⁶**; **L¹** is selected from the group consisting of a bond, -O-, -CH₂-, and -OCH₂-; **L²** is selected from the group consisting of a bond, -CH₂-, -C(H)=, and -O-; **L³** is -CH₂- ; **L⁴** is absent, or **L⁴** together with **L¹** and Rings **A** and **B** form a fused tricycle, and **L⁵** is absent; **L⁵** is absent, or **L⁵** together with **L²** and Rings **B** and **C** form a fused tricycle, and **L⁴** is absent; **R⁴** is unsubstituted alkyl, substituted alkyl, unsubstituted arylalkylene, substituted arylalkylene, unsubstituted heteroalkyl, substituted heteroalkyl, unsubstituted heteroarylalkylene, or substituted heteroarylalkylene; **R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, -SO₂NH₂, -SO₂NHMe, -B(OH)₂, 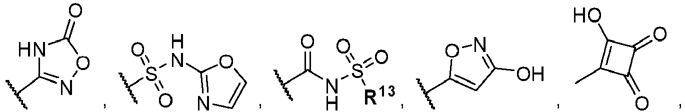 and tetrazolyl; each **R⁶** is independently selected from the group consisting of alkyl, substituted alkyl, halo, hydroxyl, alkoxyl, **R¹¹R¹²**NCO-, and **R¹¹R¹²**N-; **R¹¹** is hydrogen, or alkyl; **R¹²** is hydrogen, or alkyl; and **R¹³** is hydrogen, or alkyl.

In certain embodiments of Formula **(I),** when **L¹** is -OCH₂-, **L⁴** is absent, and **L⁵** is absent, then either **L²** is -CH₂-, -C(H)=, or -O-, or **L³** is -CH₂- , or both. In certain embodiments, when **L¹** is -OCH₂-, **L⁴** is absent, and **L⁵** is absent, then **L²** is -CH₂-, -C(H)=, or -O-. In certain embodiments, when **L¹** is -OCH₂-, **L⁴** together with **L¹** and Rings **A** and **B** form a fused tricycle. In certain embodiments, when **L¹** is -OCH₂-, **L⁵** together with **L²** and Rings **B and C** form a fused tricycle. In certain embodiments, when **L¹** is -OCH₂-, Ring **C** is substituted or unsubstituted azetidinyl, substituted or unsubstituted pyrrolidinyl, or substituted or unsubstituted piperidinyl.

In certain embodiments, **A** is a ring selected from phenyl. In certain embodiments, Ring **C** is selected from piperidine and piperazine. In certain embodiments, Ring **B** is selected from phenyl, furan, pyridine, pyrimidine, thiophene, oxazole, and benzofuran.In..

In certain embodiments, **A** is phenyl. In certain embodiments, Ring **C** is selected from substituted piperidine, azetidine, pyrrolidine, phenyl, and pyridine. In certain embodiments, Ring **B** is selected from phenyl, furan, pyridine, pyrimidine, thiophene, oxazole, and benzofuran. In certain embodiments, **A** is phenyl, and Ring **C** is azetidine. In certain embodiments, **A** is phenyl, and Ring **C** is pyrrolidine. In certain embodiments, **A** is phenyl, Ring C is azetidine, and Ring **B** is phenyl. In certain embodiments, **A** is phenyl, Ring **C** is azetidine, and Ring **B** is furan. In certain embodiments, **A** is phenyl, Ring **C** is pyrrolidine, and Ring **B** is phenyl. In certain embodiments, **A** is phenyl, Ring C is pyrrolidine, and Ring **B** is furan. In certain embodiments, **A** is phenyl, Ring **C** is phenyl, and Ring **B** is pyrimidine. In certain embodiments, **A** is phenyl, Ring **C** is pyridine, and Ring **B** is pyrimidine.

In certain embodiments, provided is a compound of Formula **(Ia),** or a pharmaceutically acceptable salt, tautomer, stereoisomer, and/or mixture of stereoisomers thereof: 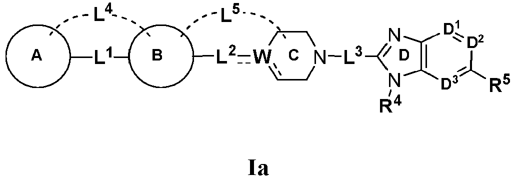 wherein **A** is substituted or unsubstituted aryl, or substituted or unsubstituted heteroaryl; Ring **B** is substituted or unsubstituted aryl, or substituted or unsubstituted heteroaryl; Ring C is further unsubstituted or further substituted, and/or bridged; zero, one, or two of **D¹**, **D²**, and **D³** are N, and the rest are CH, or **CR⁶**; **W** is CH or C; when **W** is CH, the adjacent dashed lines indicate single bonds; when **W** is C, one adjacent dashed line indicates a double bond, for instance **L²** is -C(H)=; **L¹** is selected from the group consisting of -O-, -CH₂-, and -OCH₂-; **L²** is selected from the group consisting of a bond, -CH₂-, -C(H)=, and -O-; **L³** is -CH₂-; **L⁴** is absent, or **L⁴** together with **L¹** and Rings **A** and **B** form a fused tricycle, and **L⁵** is absent; **L⁵** is absent, or **L⁵** together with **L²** and Rings **B** and **C** form a fused tricycle, and **L⁴** is absent; **R⁴** is unsubstituted alkyl, substituted alkyl, unsubstituted arylalkylene, substituted arylalkylene, unsubstituted heteroalkyl, substituted heteroalkyl, unsubstituted heteroarylalkylene, or substituted heteroarylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 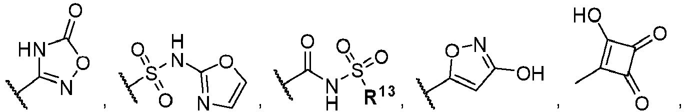 and tetrazolyl; each **R⁶** is independently selected from the group consisting of alkyl, substituted alkyl, halo, hydroxyl, alkoxyl, **R¹¹R¹²**NCO-, and **R¹¹R¹²**N-; **R¹¹** is hydrogen, or alkyl; **R¹²** is hydrogen, or alkyl; and **R¹³** is hydrogen, or alkyl.

In certain embodiments of Formula **(Ia),** when **L¹** is -OCH₂-, **L⁴** is absent, and **L⁵** is absent, then either **L²** is -CH₂-, -C(H)=, or -O-, or **L³** is -OCH₂, or both. In certain embodiments, when **L¹** is -OCH₂, **L⁴** is absent, and **L⁵** is absent, then **L²** is -CH₂-, -C(H)=, or -O-. In certain embodiments, when **L¹** is -OCH₂-, **L⁴** is absent, and **L⁵** is absent, then **L³** is - CH₂-. In certain embodiments, when **L¹** is -OCH₂-, **L⁴** together with **L¹** and Rings **A** and **B** form a fused tricycle. In certain embodiments, when **L¹** is -OCH₂-, **L⁵** together with **L²** and Rings **B** and **C** form a fused tricycle.

In certain embodiments, provided is a compound of Formula **(Ib),** or a pharmaceutically acceptable salt, tautomer, stereoisomer, and/or mixture of stereoisomers thereof: 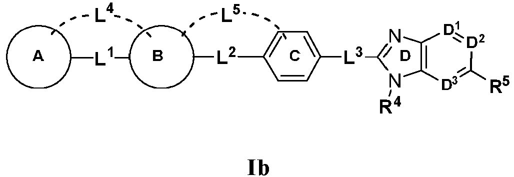 wherein **A** is substituted or unsubstituted aryl, or substituted or unsubstituted heteroaryl; Ring **B** is substituted or unsubstituted aryl, or substituted or unsubstituted heteroaryl; Ring **C** is further unsubstituted or further substituted, and/or fused; zero, one, or two of **D¹, D²**, and **D³** are N, and the rest are CH, or **CR⁶**; **W** is CH or C; when **W** is CH, the adjacent dashed lines indicate single bonds; when **W** is C, one adjacent dashed line indicates a double bond, for instance **L²** is -C(H)=; **L¹** is selected from the group consisting of -O-, -CH₂-, and -OCH₂-; **L²** is selected from the group consisting of a bond, -CH₂-, -C(H)=, and -O-; **L³** is -CH₂-; **L⁴** is absent, or **L⁴** together with **L¹** and Rings **A** and **B** form a fused tricycle, and **L⁵** is absent; **L⁵** is absent, or **L⁵** together with **L²** and Rings **B** and **C** form a fused tricycle, and **L⁴** is absent; **R⁴** is unsubstituted alkyl, substituted alkyl, unsubstituted arylalkylene, substituted arylalkylene, unsubstituted heteroalkyl, substituted heteroalkyl, unsubstituted heteroarylalkylene, or substituted heteroarylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 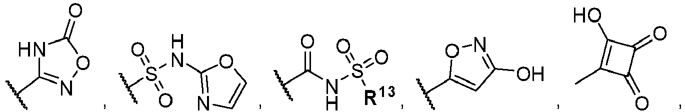 and tetrazolyl; each **R⁶** is independently selected from the group consisting of alkyl, substituted alkyl, halo, hydroxyl, alkoxyl, **R¹¹R¹²**NCO-, and **R¹¹R¹²**N-; **R¹¹** is hydrogen, or alkyl; **R¹²** is hydrogen, or alkyl; and **R¹³** is hydrogen, or alkyl.

In certain embodiments of Formula **(Ib),** when **L¹** is -OCH₂-, **L⁴** is absent, and **L⁵** is absent, then either **L²** is -CH₂-, -C(H)=, or -O-, or **L³** is -OCH₂, or both. In certain embodiments, when **L¹** is -OCH₂, **L⁴** is absent, and **L⁵** is absent, then **L²** is -CH₂-, -C(H)=, or -O-. In certain embodiments, when **L¹** is -OCH₂-, **L⁴** is absent, and **L⁵** is absent, then **L³** is - CH₂-. In certain embodiments, when **L¹** is -OCH₂-, **L⁴** together with **L¹** and Rings **A** and **B** form a fused tricycle. In certain embodiments, when **L¹** is -OCH₂-, **L⁵** together with **L²** and Rings **B** and **C** form a fused tricycle.

In certain embodiments, provided is a compound of Formula (Ic), or a pharmaceutically acceptable salt, tautomer, stereoisomer, and/or mixture of stereoisomers thereof: 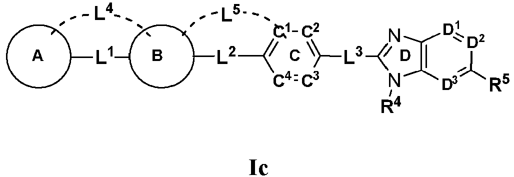 wherein **A** is substituted or unsubstituted aryl, or substituted or unsubstituted heteroaryl; Ring **B** is substituted or unsubstituted aryl, or substituted or unsubstituted heteroaryl; Ring **C** is further unsubstituted or further substituted, and/or fused; zero, one, or two of **C¹**, **C²**, **C³**, and **C⁴** are N, and the rest are CH, or **CR⁶**; zero, one, or two of **D¹, D²**, and **D³** are N, and the rest are CH, or **CR⁶**; **W** is CH or C; when **W** is CH, the adjacent dashed lines indicate single bonds; when **W** is C, one adjacent dashed line indicates a double bond, for instance **L²** is -C(H)=; **L¹** is selected from the group consisting of -O-, -CH₂-, and -OCH₂-; **L²** is selected from the group consisting of a bond, -CH₂-, -C(H)=, and -O-; **L³** is -CH₂-; **L⁴** is absent, or **L⁴** together with **L¹** and Rings **A** and **B** form a fused tricycle, and **L⁵** is absent; **L⁵** is absent, or **L⁵** together with **L²** and Rings **B** and **C** form a fused tricycle, and **L⁴** is absent; **R⁴** is unsubstituted alkyl, substituted alkyl, unsubstituted arylalkylene, substituted arylalkylene, unsubstituted heteroalkyl, substituted heteroalkyl, unsubstituted heteroarylalkylene, or substituted heteroarylalkylene; **R⁵** is selected from the group consisting of -COOH, -COCF₃, -C(OH)CF₃, -CONHCN, - CONHOH, CONHOMe, -CONHSO₂N(Me)₂, -PO₃H₂, -PO(Me)(OH), -SO₃H, -SO₂NH₂, - SO₂NHMe, -B(OH)₂, 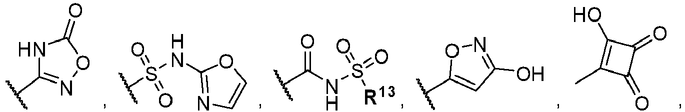 and tetrazolyl; each **R⁶** is independently selected from the group consisting of alkyl, substituted alkyl, halo, hydroxyl, alkoxyl, **R¹¹R¹²**NCO-, and **R¹¹R¹²**N-; **R¹¹** is hydrogen, or alkyl; **R¹²** is hydrogen, or alkyl; and **R¹³** is hydrogen, or alkyl.

In certain embodiments of Formula **(Ic),** when **L¹** is -OCH₂-, **L⁴** is absent, and **L⁵** is absent, then either **L²** is -CH₂-, -C(H)=, or -O-, or **L³** is -OCH₂, or both. In certain embodiments, when **L¹** is -OCH₂, **L⁴** is absent, and **L⁵** is absent, then **L²** is -CH₂-, -C(H)=, or -O-. In certain embodiments, when **L¹** is -OCH₂-, **L⁴** is absent, and **L⁵** is absent, then **L³** is - CH₂-. In certain embodiments, when **L¹** is -OCH₂-, **L⁴** together with **L¹** and Rings **A** and **B** form a fused tricycle. In certain embodiments, when **L¹** is -OCH₂-, **L⁵** together with **L²** and Rings **B** and **C** form a fused tricycle.

In certain embodiments, **A** is a ring selected from phenyl. In certain embodiments, Ring **C** is selected from piperidine, and piperazine. In certain embodiments, Ring **B** is selected from phenyl, furan, pyridine, pyrimidine, thiophene, oxazole, and benzofuran...

In certain embodiments, the compound of Formula **(I)** is according to Formula (**IIe**), or a pharmaceutically acceptable salt, tautomer, stereoisomer, and/or mixture of stereoisomers thereof: 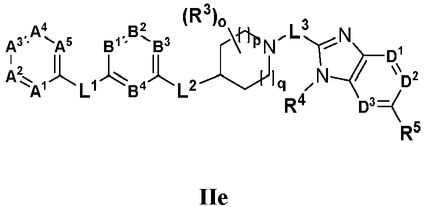 wherein zero, one, or two of **A¹**, **A²**, **A³**, **A⁴**, and **A⁵** are N, and the rest are CH, or **CR¹**; zero, one, or two of **B¹**, **B²**, **B³**, and **B⁴** are N, and the rest are CH or C**R²**, wherein, in Formula **IIe**, **B⁴** is absent; zero, one, or two of **D¹**, **D²**, and **D³** are N, and the rest are CH, or **CR⁶**; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkyl, haloalkoxy, and **R¹¹R¹²**NCO-; **R⁴** is unsubstituted alkyl, substituted alkyl, unsubstituted arylalkylene, substituted arylalkylene, heteroalkyl, substituted heteroalkyl, unsubstituted heteroarylalkylene, or substituted heteroarylalkylene; **R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, -SO₂NH₂, -SO₂NHMe, - B(OH)₂, 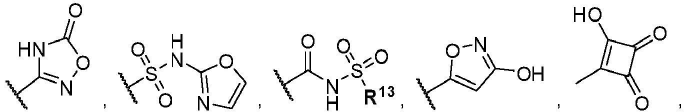 and tetrazolyl; each **R⁶** is independently selected from the group consisting of F, and methyl; each **R¹¹** is hydrogen, or alkyl; each **R¹²** is hydrogen, or alkyl; each **R¹³** is hydrogen, or alkyl; o is 1, 2, 3, or 4; **ρ is** 0 or 1; and **q** is 0 or 1.

In certain embodiments, the compound of Formula **(I)** is according to Formula **(IIf),** or a pharmaceutically acceptable salt, tautomer, stereoisomer, and/or mixture of stereoisomers thereof: 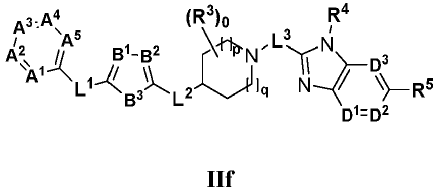 wherein zero, one, or two of **A¹**, **A²**, **A³**, **A⁴**, and **A⁵** are N, and the rest are CH, or **CR¹**; zero, one, or two of **B¹**, **B²**, **B³**, and **B⁴** are N, and the rest are CH or C**R²**, wherein, in Formula **IIf, B⁴** is absent; zero, one, or two of **D¹**, **D²**, and **D³** are N, and the rest are CH, or **CR⁶**; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkyl, haloalkoxy, and **R¹¹R¹²**NCO-; **R⁴** is unsubstituted alkyl, substituted alkyl, unsubstituted arylalkylene, substituted arylalkylene, heteroalkyl, substituted heteroalkyl, unsubstituted heteroarylalkylene, or substituted heteroarylalkylene; **R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, -SO₂NH₂, -SO₂NHMe, - B(OH)₂, 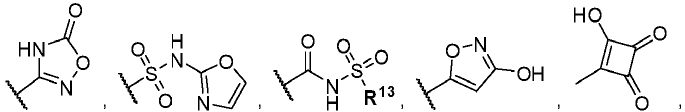 and tetrazolyl; each **R⁶** is independently selected from the group consisting of F, and methyl; each **R¹¹** is hydrogen, or alkyl; each **R¹²** is hydrogen, or alkyl; each **R¹³** is hydrogen, or alkyl; **o** is 1, 2, 3, or 4, ; **p** is 0 or 1; and **q** is 0 or 1.

In certain embodiments, the compound of Formula **(I)** is according to Formula **(IIi),** or a pharmaceutically acceptable salt, tautomer, stereoisomer, and/or mixture of stereoisomers thereof: 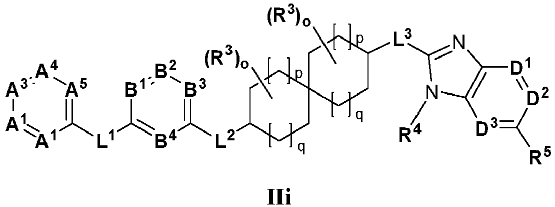 wherein zero, one, or two of **A¹**, **A²**, **A³**, **A⁴**, and **A⁵** are N, and the rest are CH, or **CR¹**; zero, one, or two of **B¹**, **B²**, **B³**, and B**⁴** are N, and the rest are CH or C**R²**, wherein, in Formula **IIi, B⁴** is absent; zero, one, or two of **D¹**, **D²**, and **D³** are N, and the rest are CH, or **CR⁶**; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkyl, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, or substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 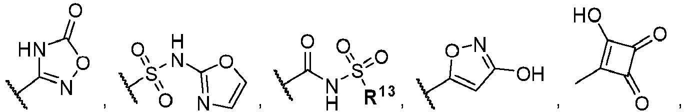 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; **o** is an integer from 0 to 4; **p is** 0 or 1; **q** is 0 or 1. In certain embodiments, **p is** 0 and **q** is 0. In certain embodiments, **p** is 1 and **q** is 0. In certain embodiments, **p** is 0 and **q** is **1.** In certain embodiments, **p** is 0 and **q** is 0.

In certain embodiments, the compound of Formula **(I)** is according to Formula **(IIj),** or a pharmaceutically acceptable salt, tautomer, stereoisomer, and/or mixture of stereoisomers thereof: 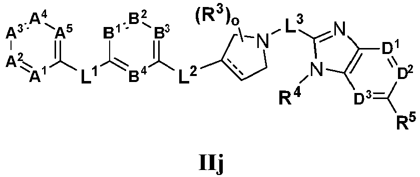 wherein zero, one, or two of **A¹**, **A²**, **A³**, **A⁴**, and **A⁵** are N, and the rest are CH, or **CR¹**; zero, one, or two of **B¹**, **B²**, **B³**, and **B**⁴ are N, and the rest are CH or C**R²**, wherein, in Formula **IIj, B⁴** is absent; zero, one, or two of **D¹**, **D²**, and **D³** are N, and the rest are CH, or **CR⁶**; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkyl, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, or substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 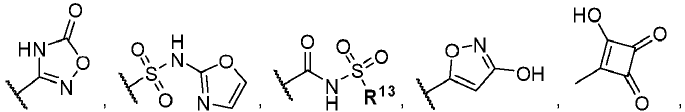 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; **o** is an integer from 0 to 4.

In certain embodiments, the compound of Formula **(I)** is according to Formula (**IIk**), or a pharmaceutically acceptable salt, tautomer, stereoisomer, and/or mixture of stereoisomers thereof: 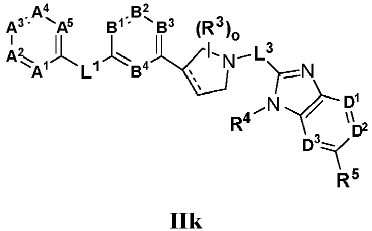 wherein zero, one, or two of **A¹**, **A²**, **A³**, **A⁴**, and **A⁵** are N, and the rest are CH, or **CR¹**; zero, one, or two of **B¹**, **B²**, **B³**, and **B⁴** are N, and the rest are CH or C**R²**, wherein, in Formula **IIk, B⁴** is absent; zero, one, or two of **D¹**, **D²**, and D**³** are N, and the rest are CH, or **CR⁶**; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkyl, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, or substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 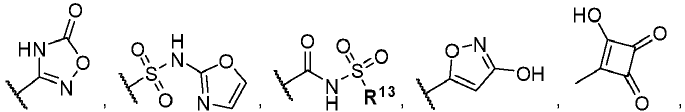 and tetrazolyl; each **R¹¹** is hydrogen or alkyl; each **R¹²** is hydrogen or alkyl; each **R¹³** is hydrogen or alkyl; **n** is an integer from 0 to 5; m is an integer from 0 to 4; **o** is an integer from 0 to 4.

In certain embodiments, the compound of Formula **(I)** is according to Formula (**III**), or a pharmaceutically acceptable salt, tautomer, stereoisomer, and/or mixture of stereoisomers thereof: 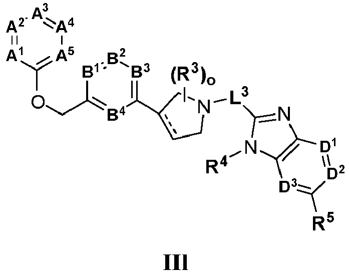 wherein zero, one, or two of **A¹**, **A²**, **A³**, **A⁴**, and **A⁵** are N, and the rest are CH, or **CR¹**; zero, one, or two of **B¹**, **B²**, **B³**, and **B⁴** are N, and the rest are CH or C**R²**, wherein, in Formula **III, B⁴** is absent; zero, one, or two of **D¹**, **D²**, and **D³** are N, and the rest are CH, or **CR⁶**; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkyl, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, or substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 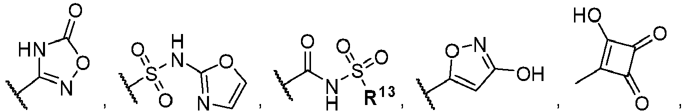 and tetrazolyl; each **R¹¹** is hydrogen or alkyl; each **R¹²** is hydrogen or alkyl; each **R¹³** is hydrogen or alkyl; n is an integer from 0 to 5; **m** is an integer from 0 to 4; **o** is an integer from 0 to 4.

In certain embodiments, the compound of Formula **(I)** is according to Formula (**IIm**), or a pharmaceutically acceptable salt, tautomer, stereoisomer, and/or mixture of stereoisomers thereof: 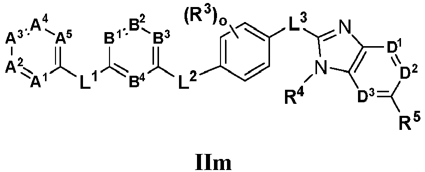 wherein zero, one, or two of **A¹**, **A²**, **A³**, **A⁴**, and **A⁵** are N, and the rest are CH, or **CR¹**; zero, one, or two of **B¹**, **B²**, **B³**, and **B⁴** are N, and the rest are CH or C**R²**, wherein, in Formula **IIm, B⁴** is absent; zero, one, or two of **D¹**, **D²**, and **D³** are N, and the rest are CH, or **CR⁶**; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkyl, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, or substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 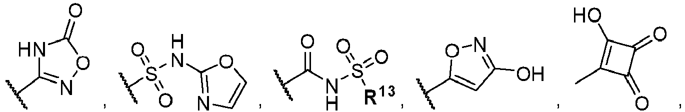 and tetrazolyl; each **R¹¹** is hydrogen or alkyl; each **R¹²** is hydrogen or alkyl; each **R¹³** is hydrogen or alkyl; n is an integer from 0 to 5; **m** is an integer from 0 to 4; **o** is an integer from 0 to 4. In certain embodiments, each **R³** is independently selected from the group consisting of F, alkyl, substituted alkyl, CH₂F, and CN.

In certain embodiments, the compound of Formula (I) is according to Formula (IIn), or a pharmaceutically acceptable salt, tautomer, stereoisomer, and/or mixture of stereoisomers thereof: 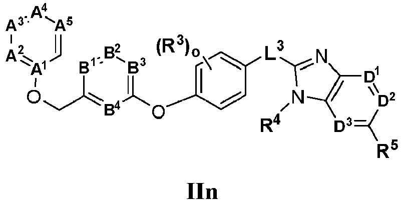 wherein zero, one, or two of **A¹**, **A²**, **A³**, **A⁴**, and **A⁵** are N, and the rest are CH, or **CR¹**; zero, one, or two of **B¹**, **B²**, **B³**, and **B⁴** are N, and the rest are CH or C**R²**, wherein, in Formula **IIn, B⁴** is absent; zero, one, or two of **D¹**, **D²**, and **D³** are N, and the rest are CH, or **CR⁶**; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkyl, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, or substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 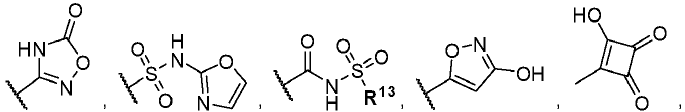 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; **o** is an integer from 0 to 4. In certain embodiments, each **R³** is independently selected from the group consisting of F, alkyl, substituted alkyl, CH₂F, and CN.

In certain embodiments, the compound of Formula **(I)** is according to Formula (**IIm**), or a pharmaceutically acceptable salt, tautomer, stereoisomer, and/or mixture of stereoisomers thereof: 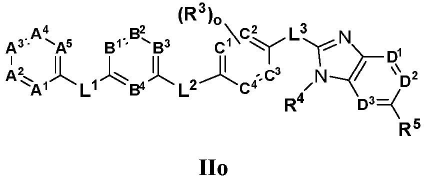 wherein zero, one, or two of **A¹**, **A²**, **A³**, **A⁴**, and **A⁵** are N, and the rest are CH, or **CR¹**; zero, one, or two of **B¹**, **B²**, **B³**, and **B⁴** are N, and the rest are CH or C**R²**, wherein, in Formula **IIo**, **B⁴** is absent; zero, one, or two of **C¹**, **C²**, **C³**, and **C⁴** are N, and the rest are CH, or **CR⁶**; zero, one, or two of **D¹, D²**, and **D**³ are N, and the rest are CH, or **CR⁶**; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkyl, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, or substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 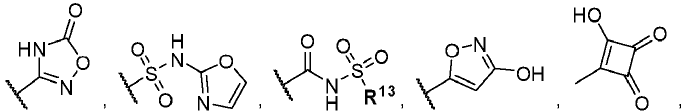 and tetrazolyl; each **R¹¹** is hydrogen or alkyl; each **R¹²** is hydrogen or alkyl; each **R¹³** is hydrogen or alkyl; n is an integer from 0 to 5; **m** is an integer from 0 to 4; **o** is an integer from 0 to 4. In certain embodiments, each **R³** is independently selected from the group consisting of F, alkyl, substituted alkyl, CH₂F, and CN.

In certain embodiments, provided is a compound of Formula **(XIX),** or a pharmaceutically salt, tautomer, stereoisomer, and/or mixture of stereoisomers thereof: 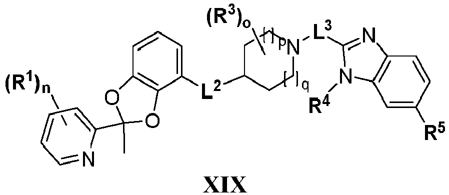 wherein **L²** is selected from the group consisting of a bond, -CH₂-, -C(H)=, and -O-; **L³** is - CH₂-; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 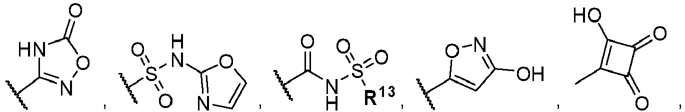 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; **o** is an integer from 0 to 4; **p is** 0 or 1; **q** is 0 or 1. In certain embodiments, **p is** 0 and **q** is 0. In certain embodiments, **p** is 1 and **q** is 0. In certain embodiments, **p** is 0 and **q** is 1. In certain embodiments, **p** is 1 and **q** is 1. In certain embodiments, the compound of Formula **(XIX)** is selected from compounds 172, 173, 184, 185, 201, and 209 in Table 1.

In certain embodiments, provided is a compound of Formula **(XX),** or a pharmaceutically salt, tautomer, stereoisomer, and/or mixture of stereoisomers thereof: 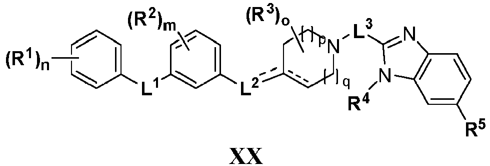 wherein **L¹** is selected from the group consisting of -O-, -CH₂-, and -OCH₂-; **L²** is selected from the group consisting of a bond, -CH₂-, -C(H)=, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, or substituted heteroarylalkylene; **R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, - SO₂NH₂, -SO₂NHMe, -B(OH)₂, 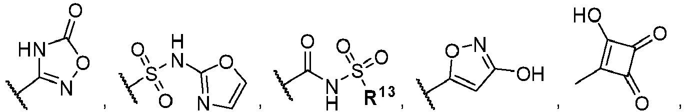 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; **o** is an integer from 0 to 4; **p is** 0 or 1; **q** is 0 or 1. In certain embodiments, **p is** 0 and **q** is 0. In certain embodiments, **p** is 1 and **q** is 0. In certain embodiments, **p** is 0 and **q** is 1. In certain embodiments, **p** is 1 and **q** is 1. In certain embodiments, the compound of Formula **(XX)** is selected from compounds 119, 134, 192, 194-197, 210, 212, 222, 223, 228, 229, 233, 234, 237, 238, 350, 383, 387-389, 400, 401, 405, 406, 411, 412, 414, 415, 417-420, 426, 439, 443, and 446-448 in Table 1.

In certain embodiments, provided is a compound of Formula **(XXI),** or a pharmaceutically salt, tautomer, stereoisomer, and/or mixture of stereoisomers thereof: 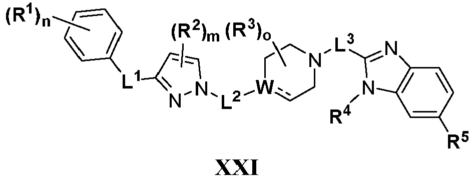 wherein **W** is **CR¹⁴** or C; when W is **CR¹⁴**, the adjacent dashed line indicates a single bond; when **W** is C, the adjacent dashed line indicates a double bond or **L²** is -C(H)=; **L¹** is selected from the group consisting of -O-, -CH₂-, and -OCH₂-; **L²** is selected from the group consisting of a bond, -CH₂-, -C(H)=, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 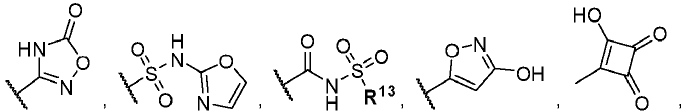 and tetrazolyl; each **R¹¹** is hydrogen or alkyl; each **R¹²** is hydrogen or alkyl; each **R¹³** is hydrogen or alkyl; n is an integer from 0 to 5; **m** is an integer from 0 to 4; **o** is an integer from 0 to 4. In certain embodiments, the compound of Formula **(XXI)** is selected from compounds 127, 281, and 282 in Table 1.

In certain embodiments, provided is a compound of Formula **(XXII),** or a pharmaceutically salt, tautomer, stereoisomer, and/or mixture of stereoisomers thereof: 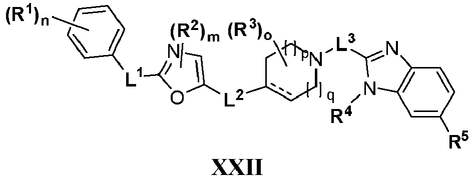 wherein **L¹** is selected from the group consisting of -O-, -CH₂-, and -OCH₂- ; **L²** is selected from the group consisting of a bond, - -CH₂-, -C(H)=, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 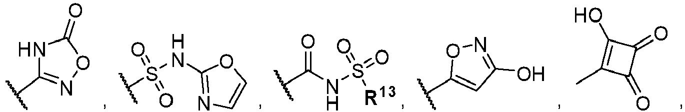 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; **o** is an integer from 0 to 4; **p is** 0 or 1; **q** is 0 or 1. In certain embodiments, **p is** 0 and **q** is 0. In certain embodiments, **p** is 1 and **q** is 0. In certain embodiments, **p** is 0 and **q** is 1. In certain embodiments, **p** is 1 and **q** is 1. In certain embodiments, the compound of Formula **(XXII)** is selected from compounds 147, 167, 191, 213, 225, and 474 in Table 1.

In certain embodiments, provided is a compound of Formula **(XXIV),** or a pharmaceutically salt, tautomer, stereoisomer, and/or mixture of stereoisomers thereof: 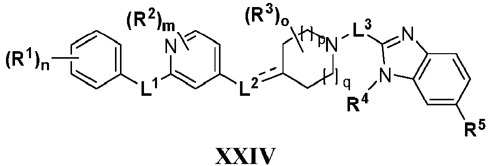 wherein **L¹** is selected from the group consisting of -O-, -CH₂-, and -OCH₂-, ; **L²** is selected from the group consisting of a bond, -CH₂-, -C(H)=, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 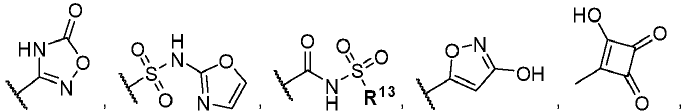 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; **o** is an integer from 0 to 4; **p is** 0 or 1; **q** is 0 or 1. In certain embodiments, **p is** 0 and **q** is 0. In certain embodiments, **p** is 1 and **q** is 0. In certain embodiments, **p** is 0 and **q** is 1. In certain embodiments, **p** is 1 and **q** is 1. In certain embodiments, the compound of Formula **(XXIV)** is selected from compounds 175-177, 180, 181, 193, 211, 218, 219, 226, 236, 244, 245, 273, 289, 293, 298, 392, 399, 402-404, 431, 433,, and 440in Table 1.

In certain embodiments, provided is a compound of Formula **(XXVIII),** or a pharmaceutically salt, tautomer, stereoisomer, and/or mixture of stereoisomers thereof: 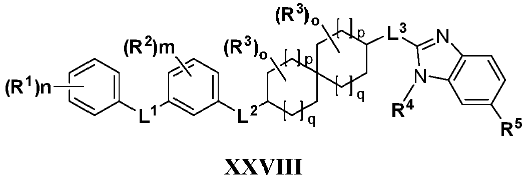 wherein **L¹** is selected from the group consisting of -O-, -CH₂-, and -OCH₂-; **L²** is selected from the group consisting of a bond, -CH₂-, -C(H)=, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 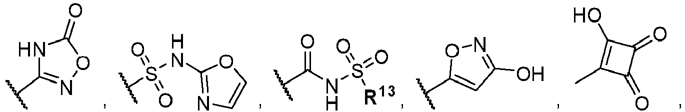 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; **o** is an integer from 0 to 4; **p is** 0 or 1; **q** is 0 or 1. In certain embodiments, **p is** 0 and **q** is 0. In certain embodiments, **p** is 1 and **q** is 0. In certain embodiments, **p** is 0 and **q** is 1. In certain embodiments, **p** is 0 and **q** is 0. In certain embodiments, **p is** 0 and **q** is 0. In certain embodiments, the compound of Formula **(XXVIII)** is compound 235 in Table 1.

In certain embodiments, provided is a compound of Formula **(XXIX),** or a pharmaceutically salt, tautomer, stereoisomer, and/or mixture of stereoisomers thereof: 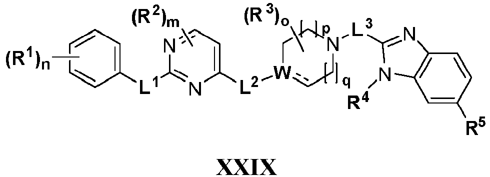 wherein **W** is C**R¹⁴** or C; when W is **CR¹⁴**, the adjacent dashed line indicates a single bond; when **W** is C, the adjacent dashed line indicates a double bond or **L²** is -C(H)=; **L¹** is selected from the group consisting of bond, -O-, -CH₂-, and -OCH₂-; **L²** is selected from the group consisting of a bond, -CH₂-, -C(H)=, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 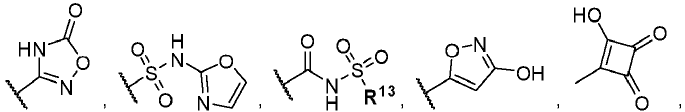 and tetrazolyl; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; and **o** is an integer from 0 to 4; **p is** 0 or 1; **q** is 0 or 1. In certain embodiments, **p** is 0 and q is 0. In certain embodiments, **p** is 1 and **q** is 0. In certain embodiments, **p is** 0 and **q** is 1. In certain embodiments, **p** is 0 and **q** is 0. In certain embodiments, **p** is 0 and **q** is 0. In certain embodiments, **L²** is selected from the group consisting of a bond, -C(O)-, -CH₂-, -C(H)=, -SO₂-, -O-, -CF₂-. -CHF-, or -CH(OH)-, and 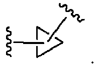 In certain embodiments, the compound of Formula **(XXIX)** is selected from compounds 360 and 395 in Table 1.

In certain embodiments, provided is a compound of Formula (**XXXII**), or a pharmaceutically salt, tautomer, stereoisomer, and/or mixture of stereoisomers thereof: 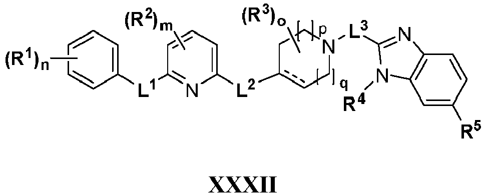 wherein **L¹** is selected from the group consisting of -O-, -CH₂-, and -OCH₂-; **L²** is selected from the group consisting of a bond, -CH₂-, -C(H)=, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 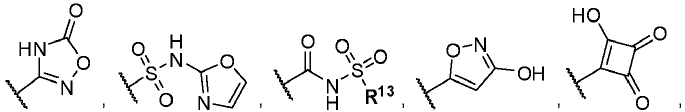 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; and **o** is an integer from 0 to 4; **p is** 0 or 1; **q** is 0 or 1. In certain embodiments, **p is** 0 and **q** is 0. In certain embodiments, **p is** 1 and **q** is 0. In certain embodiments, **p** is 0 and **q** is 1. In certain embodiments, **p** is 0 and **q** is 0. In certain embodiments, **p** is 0 and **q** is 0. In certain embodiments, **L²** is selected from the group consisting of a bond, -C(O)-, -CH₂-, -C(H)=, -SO₂-, -O-, -CF₂-. -CHF-, or -CH(OH)-, and 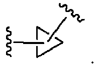 In certain embodiments, the compound of Formula **(XXXII)** is selected from compounds 384, 385, 407-410, 422, 423, 429, 436, 437, 438, , and 456, and 458 in Table 1.

In certain embodiments, provided is a compound of Formula **(XXXIII),** or a pharmaceutically salt, tautomer, stereoisomer, and/or mixture of stereoisomers thereof: 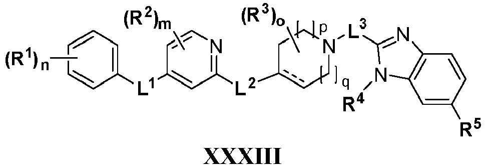 wherein **L¹** is selected from the group consisting of -O-, -CH₂-, and -OCH₂-; **L²** is selected from the group consisting of a bond, -CH₂-, -C(H)=, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 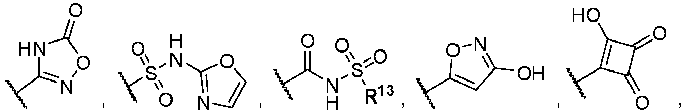 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; and **o** is an integer from 0 to 4; **p** is 0 or 1; **q** is 0 or 1. In certain embodiments, **p is** 0 and **q** is 0. In certain embodiments, **p** is 1 and **q** is 0. In certain embodiments, **p** is 0 and **q** is 1. In certain embodiments, **p** is 0 and **q** is 0. In certain embodiments, **p** is 0 and **q** is 0. In certain embodiments, **L²** is selected from the group consisting of a bond, -C(O)-, -CH₂-, -C(H)=, -SO₂-, -O-, -CF₂-. -CHF-, or -CH(OH)-, and 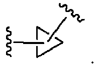 In certain embodiments, the compound of Formula **(XXXIII)** is selected from compounds 390, 391, 393, 394, 397, and 398 in Table 1.

In certain embodiments, provided is a compound of Formula **(XXXIV),** or a pharmaceutically salt, tautomer, stereoisomer, and/or mixture of stereoisomers thereof: 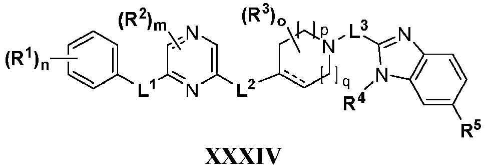 wherein **L¹** is selected from the group consisting of -O-, -CH₂-, and -OCH₂-; **L²** is selected from the group consisting of a bond, -CH₂-, -C(H)=, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 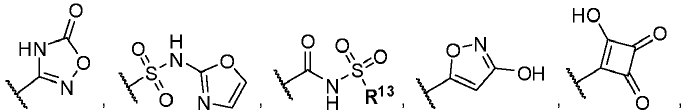 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; and **o** is an integer from 0 to 4; **p** is 0 or 1; **q** is 0 or 1. In certain embodiments, **p is** 0 and **q** is 0. In certain embodiments, **p** is 1 and **q** is 0. In certain embodiments, **p** is 0 and **q** is 1. In certain embodiments, **p** is 0 and **q** is 0. In certain embodiments, **p** is 0 and **q** is 0. In certain embodiments, **L²** is selected from the group consisting of a bond, -C(O)-, -CH₂-, -C(H)=, -SO₂-, -O-, -CF₂-. -CHF-, or -CH(OH)-, and 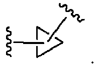 In certain embodiments, the compound of Formula **(XXXIV)** is compound 396 in Table 1.

In certain embodiments, provided is a compound of Formula **(XXXV),** or a pharmaceutically salt, tautomer, stereoisomer, and/or mixture of stereoisomers thereof: 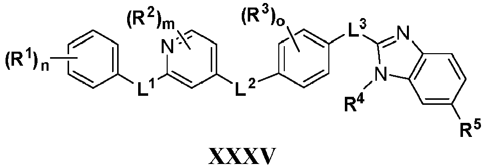 wherein **L¹** is selected from the group consisting of -O-, -CH₂-, and -OCH₂-; **L²** is selected from the group consisting of a bond, -CH₂-, -C(H)=, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 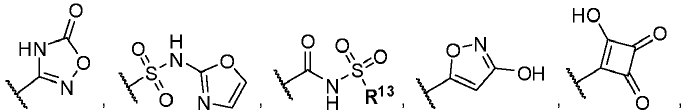 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; and **o** is an integer from 0 to 4. In certain embodiments, **L²** is selected from the group consisting of a bond, -C(O)-, -CH₂-, -C(H)=,-SO₂-, -O-, -CF₂-. -CHF-, or -CH(OH)-, and 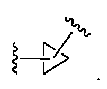 In certain embodiments, each **R³** is independently selected from the group consisting of F, alkyl, substituted alkyl, CH₂F, and CN. In certain embodiments, the compound of Formula **(XXXV)** is selected from compounds 462 and 466in Table 1.

In certain embodiments, provided is a compound of Formula **(XXXVI),** or a pharmaceutically salt, tautomer, stereoisomer, and/or mixture of stereoisomers thereof: 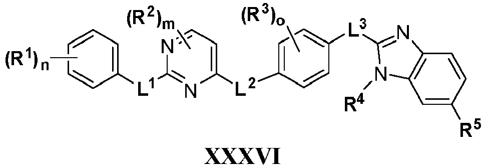 wherein **L¹** is selected from the group consisting of -O-, -CH₂-, and -OCH₂-; **L²** is selected from the group consisting of a bond, -CH₂-, -C(H)=, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 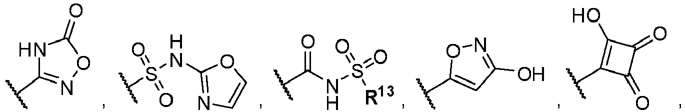 and tetrazolyl; each **R¹¹** is hydrogen or alkyl; each **R¹²** is hydrogen or alkyl; each **R¹³** is hydrogen or alkyl; n is an integer from 0 to 5; m is an integer from 0 to 4; and **o** is an integer from 0 to 4. In certain embodiments, **L²** is selected from the group consisting of a bond, -C(O)-, -CH₂-, -C(H)=, - SO₂-, -O-, -CF₂-. -CHF-, or -CH(OH)-, and 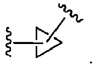 In certain embodiments, each **R³** is independently selected from the group consisting of F, alkyl, substituted alkyl, CH₂F, and CN. In certain embodiments, the compound of Formula **(XXXVI)** is selected from compounds 460, 465, and 468in Table 1.

In certain embodiments, provided is a compound of Formula **(XXXVII)**, or a pharmaceutically salt, tautomer, stereoisomer, and/or mixture of stereoisomers thereof: 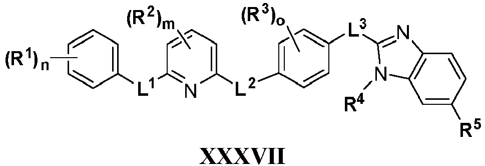 wherein **L¹** is selected from the group consisting of -O-, -CH₂-, and -OCH₂-; **L²** is selected from the group consisting of a bond, -CH₂-, -C(H)=, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene; **R⁵** is selected from the group consisting of -COOH, -COCF₃, -C(OH)CF₃, -CONHCN, -CONHOH, CONHOMe, - CONHSO₂N(Me)₂, -PO₃H₂, -PO(Me)(OH), -SO₃H, -SO₂NH₂, -SO₂NHMe, -B(OH)₂, 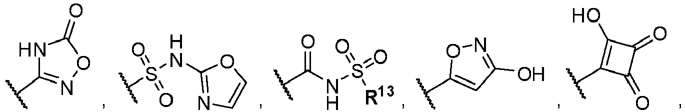 and tetrazolyl; each **R¹¹** is hydrogen or alkyl; each **R¹²** is hydrogen or alkyl; each **R¹³** is hydrogen or alkyl; **n** is an integer from 0 to 5; **m** is an integer from 0 to 4; and **o** is an integer from 0 to 4. In certain embodiments, **L²** is selected from the group consisting of a bond, -C(O)-, -CH₂-, -C(H)=, - SO₂-, -O-, -CF₂-. -CHF-, or -CH(OH)-, and 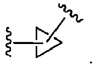 In certain embodiments, each **R³** is independently selected from the group consisting of F, alkyl, substituted alkyl, CH₂F, and CN. In certain embodiments, the compound of Formula **(XXXVII)** is selected from compounds 386, 461, 463, and 464in Table 1.

In certain embodiments, provided is a compound of Formula **(XXXVIII),** or a pharmaceutically salt, tautomer, stereoisomer, and/or mixture of stereoisomers thereof: 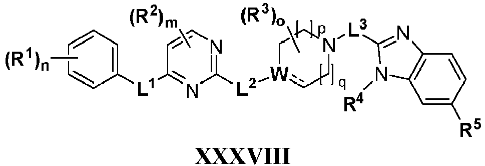 wherein **W** is **CR¹⁴** or C; when **W** is **CR¹⁴,** the adjacent dashed line indicates a single bond; when **W** is C, the adjacent dashed line indicates a double bond or **L²** is -C(H)=; **L¹** is selected from the group consisting of -O-, -CH₂-, and -OCH₂-; **L²** is selected from the group consisting of a bond, -CH₂-, -C(H)=, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 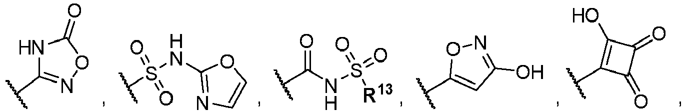 and tetrazolyl; each **R¹¹** is hydrogen, or alkyl; each **R¹²** is hydrogen, or alkyl; each **R¹³** is hydrogen, or alkyl; n is an integer from 0 to 5; **m** is an integer from 0 to 4; and **o** is an integer from 0 to 4; **p** is 0 or 1; **q** is 0 or 1. In certain embodiments, **p** is 0 and **q** is 0. In certain embodiments, **p** is 1 and **q** is 0. In certain embodiments, **p** is 0 and **q** is 1. In certain embodiments, **p** is 0 and **q** is 0. In certain embodiments, **p** is 0 and **q** is 0. In certain embodiments, **L²** is selected from the group consisting of a bond, -C(O)-, -CH₂-, -C(H)=, -SO₂-, -O-, -CF₂-. -CHF-, or -CH(OH)-, and 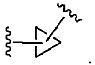 In certain embodiments, the compound of Formula **(XXXVIII)** is compound 435 in Table 1.

In certain embodiments, provided is a compound of Formula **(XLVIII),** or a pharmaceutically salt, tautomer, stereoisomer, and/or mixture of stereoisomers thereof: 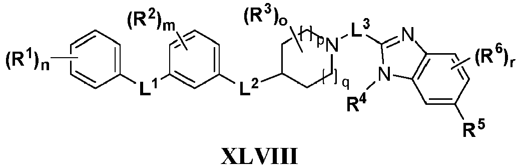 wherein **L¹** is selected from the group consisting of -O-, -CH₂-, and -OCH₂-; **L²** is selected from the group consisting of a bond, -CH₂-, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 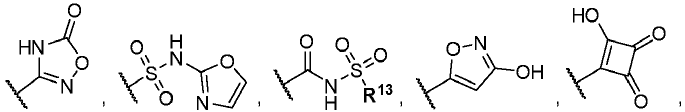 and tetrazolyl; each **R⁶** is independently selected from the group consisting of F, and methyl; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; and **o** is an integer from 0 to 4; **p** is 0 or 1; **q** is 0 or 1. In certain embodiments, **p is** 0 and **q** is 0. In certain embodiments, **p** is 1 and **q** is 0. In certain embodiments, **p** is 0 and **q** is 1. In certain embodiments, **p** is 0 and **q** is 0. In certain embodiments, **p** is 0 and **q** is 0. In certain embodiments, **L²** is -CH₂-. **R⁶** is F. In certain embodiments, the compound of Formula **(XLVIII)** is selected from compounds 351, 444, 445, 449, and 450 in Table 1.

In certain embodiments, provided is a compound of Formula **(XLIX),** or a pharmaceutically salt, tautomer, stereoisomer, and/or mixture of stereoisomers thereof: 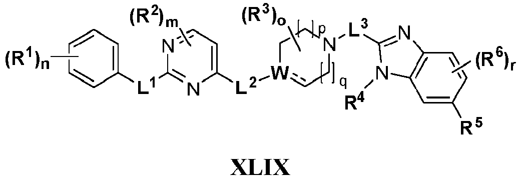 wherein **W** is **CR¹⁴** or C; when **W** is **CR¹⁴**, the adjacent dashed line indicates a single bond; when **W** is C, the adjacent dashed line indicates a double bond or **L²** is -C(H)=; **L¹** is selected from the group consisting of -O-, -CH₂-, and -OCH₂-; **L²** is selected from the group consisting of a bond, -CH₂-, -C(H)=, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 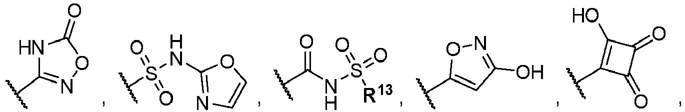 and tetrazolyl; each **R⁶** is independently selected from the group consisting of F, and methyl; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; and **o** is an integer from 0 to 4; **p** is 0 or 1; **q** is 0 or 1. In certain embodiments, **p** is 0 and **q** is 0. In certain embodiments, **p** is 1 and **q** is 0. In certain embodiments, **p** is 0 and **q** is 1. In certain embodiments, **p** is 0 and **q** is 0. In certain embodiments, **p** is 0 and **q** is 0. In certain embodiments, **L²** is -CH₂-. In certain embodiments **R⁶** is F. In certain embodiments, the compound of Formula **(XLIX)** is selected from compounds 361 and 546 in Table 1.

In certain embodiments, provided is a compound of Formula **(L),** or a pharmaceutically salt, tautomer, stereoisomer, and/or mixture of stereoisomers thereof: 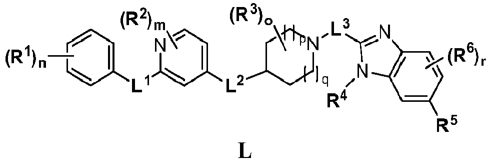 wherein **L¹** is selected from the group consisting of -O-, -CH₂-, and -OCH₂-; **L²** is selected from the group consisting of a bond, -CH₂-, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 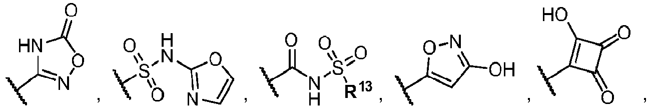 and tetrazolyl; **R⁶** is independently selected from the group consisting of F, and methyl; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; and **o** is an integer from 0 to 4; **p** is 0 or 1; **q** is 0 or 1. In certain embodiments, **p** is 0 and **q** is 0. In certain embodiments, **p** is 1 and **q** is 0. In certain embodiments, **p** is 0 and **q** is 1. In certain embodiments, **p** is 0 and **q** is 0. In certain embodiments, **p** is 0 and **q** is 0. In certain embodiments, **L²** is -CH₂-. In certain embodiments **R⁶** is F. In certain embodiments, the compound of Formula (L) is a compound selected from compounds 427 and 428 in Table 1.

In certain embodiments, provided is a compound of Formula **(LI),** or a pharmaceutically salt, tautomer, stereoisomer, and/or mixture of stereoisomers thereof: 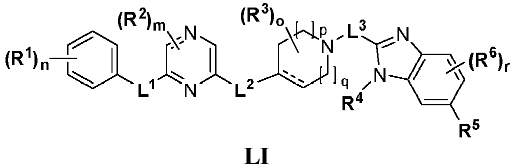 wherein **L¹** is selected from the group consisting of -O-, -CH₂-, and -OCH₂-; **L²** is selected from the group consisting of a bond, -CH₂-, -C(H)=, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R**¹¹**R**¹²NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of - COOH, -COCF₃, -C(OH)CF₃, -CONHCN, -CONHOH, CONHOMe, -CONHSO₂N(Me)₂, - PO₃H₂, -PO(Me)(OH), -SO₃H, -SO₂NH₂, -SO₂NHMe, -B(OH)₂, 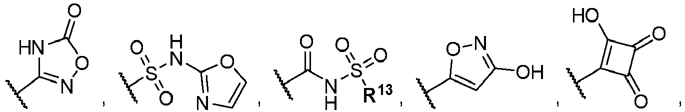 and tetrazolyl; **R⁶** is independently selected from the group consisting of F, and methyl; each **R¹¹** is hydrogen, or alkyl; each **R¹²** is hydrogen, or alkyl; each **R¹³** is hydrogen, or alkyl; **n** is an integer from 0 to 5; **m** is an integer from 0 to 4; and **o** is an integer from 0 to 4; **p** is 0 or 1; **q** is 0 or 1. In certain embodiments, **p** is 0 and **q** is 0. In certain embodiments, **p** is 1 and **q** is 0. In certain embodiments, **p** is 0 and **q** is 1. In certain embodiments, **p** is 0 and **q** is 0. In certain embodiments, **p** is 0 and **q** is 0. In certain embodiments, **L²** is -CH₂-. In certain embodiments **R⁶** is F. In certain embodiments, the compound of Formula **(LI)** is a compound selected from compounds 459, 471, 537, 538, and 543 in Table 1.

In certain embodiments, provided is a compound of Formula **(LII),** or a pharmaceutically salt, tautomer, stereoisomer, and/or mixture of stereoisomers thereof: 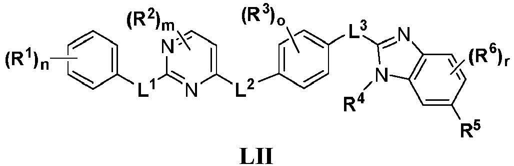 wherein **L¹** is selected from the group consisting of -O-, -CH₂-, and -OCH₂-; **L²** is selected from the group consisting of a bond, -CH₂-, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 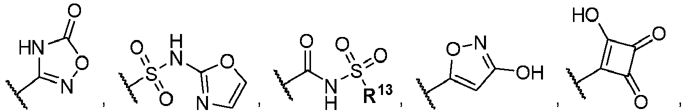 and tetrazolyl; **R⁶** is independently selected from the group consisting of F, and methyl; each **R¹¹** is hydrogen, or alkyl; each **R¹²** is hydrogen, or alkyl; each **R¹³** is hydrogen, or alkyl. In certain embodiments, **L²** is -CH₂-. In certain embodiments **R⁶** is F. In certain embodiments, the compound of Formula **(LII)** is a compound selected from compounds 467, 470, 539, and 540 in Table 1.

In certain embodiments, provided is a compound of Formula **(LIII),** or a pharmaceutically salt, tautomer, stereoisomer, and/or mixture of stereoisomers thereof: 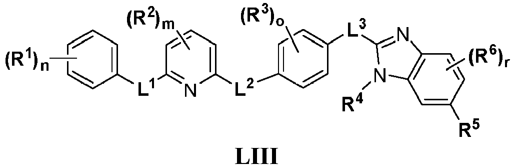 wherein **L¹** is selected from the group consisting of -O-, -CH₂-, and -OCH₂-; **L²** is selected from the group consisting of a bond, -CH₂-, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 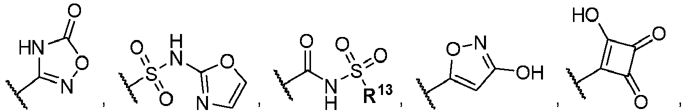 and tetrazolyl; **R⁶** is independently selected from the group consisting of F, and methyl; each **R¹¹** is hydrogen, or alkyl; each **R¹²** is hydrogen, or alkyl; each **R¹³** is hydrogen, or alkyl. In certain embodiments, **L²** is -CH₂-. In certain embodiments **R⁶** is F. In certain embodiments, the compound of Formula **(LIII)** is compound 469 in Table 1.

In certain embodiments, provided is a compound of Formula **(LIV),** or a pharmaceutically salt, tautomer, stereoisomer, and/or mixture of stereoisomers thereof: 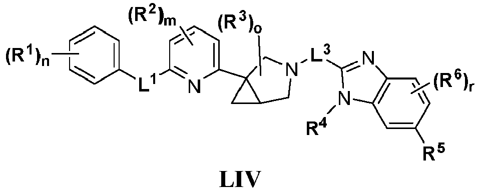 wherein **L¹** is selected from the group consisting of -O-, -CH₂-, and -OCH₂-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of - COOH, -COCF₃, -C(OH)CF₃, -CONHCN, -CONHOH, CONHOMe, -CONHSO₂N(Me)₂, - PO₃H₂, -PO(Me)(OH), -SO₃H, -SO₂NH₂, -SO₂NHMe, -B(OH)₂, 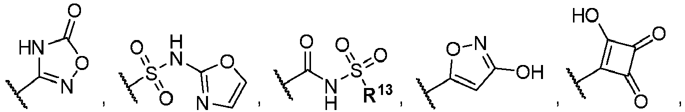 and tetrazolyl; **R⁶** is independently selected from the group consisting of F, and methyl; each **R¹¹** is hydrogen, or alkyl; each **R¹²** is hydrogen, or alkyl; each **R¹³** is hydrogen, or alkyl. In certain embodiments **R⁶** is F. In certain embodiments, the compound of Formula **(LIV)** is a compound selected from compounds 522-525, 531, and 532 in Table 1.

In certain embodiments, provided is a compound of Formula **(LV),** or a pharmaceutically salt, tautomer, stereoisomer, and/or mixture of stereoisomers thereof: 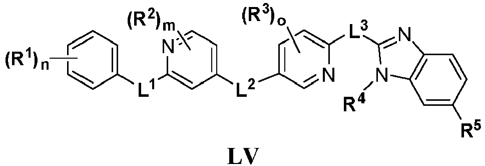 wherein **L¹** is selected from the group consisting of -O-, -CH₂-, and -OCH₂-; **L²** is selected from the group consisting of a bond, -CH₂-, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of -COOH, -COCF₃, - C(OH)CF₃, -CONHCN, -CONHOH, CONHOMe, -CONHSO₂N(Me)₂, -PO₃H₂, - PO(Me)(OH), -SO₃H, -SO₂NH₂, -SO₂NHMe, -B(OH)₂, 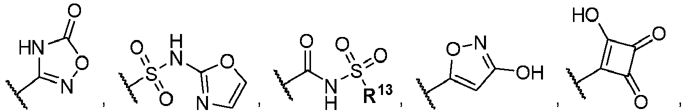 and tetrazolyl; each **R¹¹** is hydrogen, or alkyl; each **R¹²** is hydrogen, or alkyl; each **R¹³** is hydrogen, or alkyl. In certain embodiments, the compound of Formula **(LV)** is compound 542 in Table 1.

In certain embodiments, provided is a compound of Formula **(LVI),** or a pharmaceutically salt, tautomer, stereoisomer, and/or mixture of stereoisomers thereof:

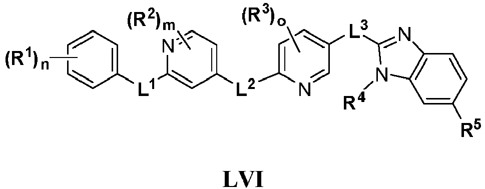

wherein **L¹** is selected from the group consisting of -O-, -CH₂-, and -OCH₂-; **L²** is selected from the group consisting of a bond, -CH₂-, and -O-; **L³** is -CH₂-; each **R¹** is independently selected from the group consisting of alkyl, substituted alkyl, halo, cyano, azido, alkoxy, haloalkoxy, and **R¹¹R¹²**NCO-; each **R²** is independently selected from the group consisting of alkyl, substituted alkyl, halo, cyano, azido, alkoxy, haloalkoxy, and **R¹¹R¹²**NCO-; each **R³** is independently selected from the group consisting of alkyl, substituted alkyl, halo, cyano, azido, alkoxy, haloalkoxy, and **R¹¹R¹²**NCO-; **R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkyl, or substituted heterocycloalkyl; **R⁵** is selected from the group consisting of - COOH, -COCF₃, -C(OH)CF₃, -CONHCN, -CONHOH, CONHOMe, -CONHSO₂N(Me)₂, - PO₃H₂, -PO(Me)(OH), -SO₃H, -SO₂NH₂, -SO₂NHMe, -B(OH)₂, 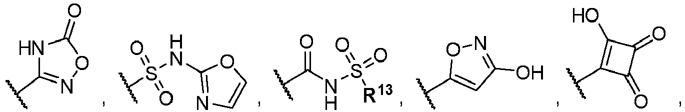 and tetrazolyl; each **R¹¹** is hydrogen, or alkyl; each **R¹²** is hydrogen, or alkyl; each **R¹³** is hydrogen, or alkyl. In certain embodiments, the compound of Formula **(LVI)** is compound 544 in Table 1.

In certain embodiments, provided is a compound of any of Formulas **(I) - (LVI),** or a pharmaceutically salt, tautomer, stereoisomer, and/or mixture of stereoisomers thereof, wherein each **R²** and **R³** is H, or F.

In certain embodiments, provided is a compound of any of Formulas **(I) - (LVI),** or a pharmaceutically salt, tautomer, stereoisomer, and/or mixture of stereoisomers thereof, wherein m is 0; and **o** is 0.

In certain embodiments, provided is a compound of any of Formulas **(I) - (LVI),** or a pharmaceutically salt, tautomer, stereoisomer, and/or mixture of stereoisomers thereof, wherein **W** is N, or CH.

In certain embodiments, provided is a compound of any of Formulas **(I) - (LVI)** or a pharmaceutically salt, tautomer, stereoisomer, and/or mixture of stereoisomers thereof, wherein **W** is C.

In certain embodiments, provided is a compound of any of Formulas **(I) - (LVI)** or a pharmaceutically salt, tautomer, stereoisomer, and/or mixture of stereoisomers thereof, wherein **W** is CH

In certain embodiments, provided is a compound of any of Formulas **(I) - (LVI)** or a pharmaceutically salt, tautomer, stereoisomer, and/or mixture of stereoisomers thereof, wherein **W** is **CR¹⁴**.

In certain embodiments, provided is a compound of any of Formulas **(I) - (LVI),** or a pharmaceutically salt, tautomer, stereoisomer, and/or mixture of stereoisomers thereof, wherein **R⁴** is selected from the group consisting of1-(cyanomethyl)-cycloprop-1-yl-methyl, (1-ethyl-1H-imidazol-5-yl)-methyl, 1-(Fluoromethyl)-cycloprop-1-yl-methyl, isoxazol-5-yl-methyl, oxetan-2-yl-methyl, (2*S*)-oxetan-2-yl-methyl, oxelan-3-yl-methyl, (3*R*)-oxelan-3-yl-methyl, 1,3-oxazol-2-yl-methyl, and tetrahydrofura-2-yl-methyl.

In certain embodiments, provided is a compound of any of Formulas **(I) - (LVI),** or a pharmaceutically salt, tautomer, stereoisomer, and/or mixture of stereoisomers thereof, wherein **R⁴** is selected from the group consisting of(1-ethyl-1H-imidazol-5-yl)-methyl, oxetan-2-yl-methyl, (2*S*)-oxetan-2-yl-methyl, oxelan-3-yl-methyl, (3*R*)-oxelan-3-yl-methyl, and 1,3-oxazol-2-yl-methyl.

In certain embodiments, provided is a compound of any of Formulas **(I) - (LVI),** or a pharmaceutically salt, tautomer, stereoisomer, and/or mixture of stereoisomers thereof, wherein **R⁴** is selected from the group consisting of1-(cyanomethyl)-cycloprop-1-yl-methyl, 1-(Fluoromethyl)-cycloprop-1-yl-methyl, isoxazol-5-yl-methyl, and tetrahydrofura-2-yl-methyl.

In certain embodiments, provided is a compound of any of Formulas **(I) - (LVI),** or a pharmaceutically salt, tautomer, stereoisomer, and/or mixture of stereoisomers thereof, wherein **R⁴** is selected from the group consisting of oxetan-2-yl-methyl, (1-ethyl-1H-imidazol-5-yl)-methyl, oxelan-3-yl-methyl, and 1,3-oxazol-2-yl-methyl.

In certain embodiments, provided is a compound of any of Formulas **(I) - (LVI),** or a pharmaceutically salt, tautomer, stereoisomer, and/or mixture of stereoisomers thereof, wherein **R⁴** is selected from the group consisting of oxetan-2-yl-methyl, and (1-ethyl-1H-imidazol-5-yl)-methyl.

In certain embodiments, provided is a compound of any of Formulas **(I)** - **(LVI),** or a pharmaceutically salt, tautomer, stereoisomer, and/or mixture of stereoisomers thereof, wherein **R⁴** is oxetan-2-yl-methyl.

In certain embodiments, provided is a compound of any of Formulas **(I)** - **(LVI),** or a pharmaceutically salt, tautomer, stereoisomer, and/or mixture of stereoisomers thereof, wherein **R⁴** is (2S)-oxetan-2-yl-methyl.

In certain embodiments, provided is a compound of any of Formulas **(I)** - **(LVI),** or a pharmaceutically salt, tautomer, stereoisomer, and/or mixture of stereoisomers thereof, wherein **R⁴** is (1-ethyl-1H-imidazol-5-yl)-methyl.

In certain embodiments, provided is a compound of Formula **(LVI),** or a pharmaceutically salt, tautomer, stereoisomer, and/or mixture of stereoisomers thereof, wherein **R⁴** is H.

In certain embodiments, provided is a compound of Formula **(LVI),** or a pharmaceutically salt, tautomer, stereoisomer, and/or mixture of stereoisomers thereof, wherein **R⁴** is selected from the group consisting of oxelan-3-yl-methyl and 1,3-oxazol-2-yl-methyl.

In certain embodiments, provided is a compound of Formula **(LVI),** or a pharmaceutically salt, tautomer, stereoisomer, and/or mixture of stereoisomers thereof, wherein **R⁴** is (3*R*)-oxelan-3-yl-methyl.

In certain embodiments, provided is a compound of any of Formulas **(I)** - **(LVI),** or a pharmaceutically salt, tautomer, stereoisomer, and/or mixture of stereoisomers thereof, wherein **R⁵** is -COOH, or -COOMe.

In certain embodiments, provided is a compound of any of Formulas **(I)** - **(LVI),** or a pharmaceutically salt, tautomer, stereoisomer, and/or mixture of stereoisomers thereof, wherein **R⁵** is -COOH

In certain embodiments, provided is a compound of any of Formulas **(I)** - **(LVI),** or a pharmaceutically salt, tautomer, stereoisomer, and/or mixture of stereoisomers thereof, wherein **R⁵** is tetrazolyl.

In certain embodiments, provided is a compound of any of Formulas **(I)** - **(LVI),** or a pharmaceutically salt, tautomer, stereoisomer, and/or mixture of stereoisomers thereof, wherein **R⁵** is 1H-1,2,3,4-tetrazol-5-yl.

In certain embodiments, provided is a compound of any of Formulas **(I)** - **(LVI),** or a pharmaceutically salt, tautomer, stereoisomer, and/or mixture of stereoisomers thereof, wherein **L²** is -CH₂-.

In certain embodiments, provided is a compound of any of Formulas **(I)** - **(LVI),** or a pharmaceutically salt, tautomer, stereoisomer, and/or mixture of stereoisomers thereof, wherein **L²** is a bond, -CH₂-, or -C(H)=-O-.

In certain embodiments, provided is a compound of any of Formulas **(I)** - **(LVI),** or a pharmaceutically salt, tautomer, stereoisomer, and/or mixture of stereoisomers thereof, wherein **L²** is -CH= or -O-.

In certain embodiments, provided is a compound of Formula **(LVI),** or a pharmaceutically salt, tautomer, stereoisomer, and/or mixture of stereoisomers thereof, wherein **L²** is a bond.

In certain embodiments, provided is a compound of any of Formulas **(I)** - **(LVI),** or a pharmaceutically salt, tautomer, stereoisomer, and/or mixture of stereoisomers thereof, wherein **L³** is -CH₂-.

In certain embodiments, provided is a compound of Formulas **(LIX),** or a pharmaceutically salt, tautomer, stereoisomer, and/or mixture of stereoisomers thereof, wherein **L¹** is -OCH₂-.

In certain embodiments, provided is a compound of any of Formulas **(I)** - **(LVI),** or a pharmaceutically salt, tautomer, stereoisomer, and/or mixture of stereoisomers thereof, wherein **L¹** is -OCH₂-; and **L²** is -CH₂-.

In certain embodiments, provided is a compound of any of Formulas **(I)** - **(LVI),** or a pharmaceutically salt, tautomer, stereoisomer, and/or mixture of stereoisomers thereof, wherein **L¹** is -O-.

In certain embodiments, provided is a compound of any of Formulas **(I) - (LVI),** or a pharmaceutically salt, tautomer, stereoisomer, and/or mixture of stereoisomers thereof, wherein **L¹** is -CH₂-.

In certain embodiments, provided is a compound of any of Formulas **(I) - (LVI),** or a pharmaceutically salt, tautomer, stereoisomer, and/or mixture of stereoisomers thereof, wherein **L¹** is -OCH₂-.

In certain embodiments of any of Formulas **(I)-(LVI), L¹** is -O-; and **L²** is -CH₂-. In certain embodiments of any of Formulas **(I)-(LVI), L¹** is -O-; and **L²** is -C(H)=. In certain embodiments of any of Formulas **(I)-(LVI), L¹** is -O-; **L²** is -CH₂-; and **L³** is -CH₂-. In certain embodiments of any of Formulas **(I)-(LVI), L¹** is -O-; **L²** is -C(H)=; and **L³** is -CH₂-. In certain embodiments of any of Formulas **(I)-(LVI), L¹** is -O-; **L²** is -CH₂-; **L³** is -CH₂-; and **R²** and **R³** are each H. In certain embodiments of any of Formulas **(I)-(LVI), L¹** is -O-; **L²** is -C(H)=; **L³** is -CH₂-; and **R²** and **R³** are each H. In certain embodiments of any of Formulas **(I)-(LVI), L¹** is -O-; **L²** is -CH₂-; **L³** is -CH₂-; **R²** and **R³** are each H; and **R⁴** is (2*S*)-oxetan-2-yl-methyl. In certain embodiments of any of Formulas **(I)-(LVI), L¹** is -O-; **L²** is -C(H)=; **L³** is -CH₂-; **R²** and **R³** are each H; and **R⁴** is (2S)-oxetan-2-yl-methyl. IIn certain embodiments of any of Formulas **(I)-(LVI), L¹** is -O-; **L²** is -CH₂-; **L³** is -CH₂-; **R**² and **R³** are each H; and **R⁴** is (1-ethyl-1H-imidazol-5-yl)-methyl. In certain embodiments of any of Formulas **(I)-(LVI), L¹** is - O-; **L²** is -C(H)=; **L³** is -CH₂-; **R**² and **R³** are each H; and **R⁴** is (1-ethyl-1H-imidazol-5-yl)-methyl. In certain embodiments of any of Formulas **(I)-(LVI), L¹** is -O-; **L²** is -CH₂-; **L³** is - CH₂-; **R²** and **R³** are each H; **R⁴** is (2*S*)-oxetan-2-yl-methyl; and **R⁵** is -COOH. In certain embodiments of any of Formulas **(I)-(LVI), L¹** is -O-; **L²** is -C(H)=; **L³** is -CH₂-; **R²** and **R³** are each H; **R⁴** is (2*S*)-oxetan-2-yl-methyl; and **R⁵** is -COOH. In certain embodiments of any of Formulas **(I)-(LVI), L¹** is -O-; **L²** is -CH₂-; **L³** is -CH₂-; **R²** and R**³** are each H; **R⁴** is (1-ethyl-1H-imidazol-5-yl)-methyl; and **R⁵** is -COOH. In certain embodiments of any of Formulas **(I)-(LVI), L¹** is -O-; **L²** is -C(H)=; **L³** is -CH₂-; **R**² and **R³** are each H; **R⁴** is (1-ethyl-1H-imidazol-5-yl)-methyl; and **R⁵** is -COOH. In certain embodiments of any of Formulas **(I)-(LVI), L¹** is -O-; **L²** is -CH₂-; **L³** is -CH₂-; **R²** and **R³** are each H; **R⁴** is (2S)-oxetan-2-yl-methyl; and **R⁵** is tetrazolyl, for instance 1H-1,2,3,4-tetrazol-5-yl. In certain embodiments of any of Formulas **(I)-(LVI), L¹** is -O-; **L²** is -C(H)=; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain embodiments of any of Formulas **(I)-(LVI), L¹** is -O-; **L²** is -CH₂-; **L³** is -CH₂-; **R**² and **R³** are each H; **R⁴** is (1-ethyl-1H-imidazol-5-yl)-methyl; and **R⁵** is tetrazolyl, for instance 1H-1,2,3,4-tetrazol-5-yl. In certain embodiments of any of Formulas **(I)-(LVI), L¹** is -O-; **L²** is -C(H)=; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In any embodiment according to this paragraph, **R¹** can be selected from H, Cl, F, Me, -CF₃, -OMe, -CN, -C(O)NMe₂, alkyl, propyl, isopropyl, and cyclopropyl. In certain embodiments of any of Formulas **(I)-(LVI), R⁴** is 1-(cyanomethyl)-cycloprop-1-yl-methyl. In certain embodiments of any of Formulas **(I)-(LVI), R⁴** is 1-(Fluoromethyl)-cycloprop-1-yl-methyl. In certain embodiments of any of Formulas **(I)-(LVI), R⁴** is isoxazol-5-yl-methyl. In certain embodiments of any of Formulas **(I)-(LVI), R⁴** is tetrahydrofura-2-yl-methyl.

In certain embodiments of any of Formulas **(I)-(LVI), L¹** is -OCH₂-; and **L²** is - CH₂-. In certain embodiments of any of Formulas **(I)-(LVI), L¹** is -OCH₂-; and **L²** is -C(H)=. In certain embodiments of any of Formulas **(I)-(LVI), L¹** is -OCH₂-; **L²** is -CH₂-; and **L³** is - CH₂-. In certain embodiments of any of Formulas **(I)-(LVI), L¹** is -OCH₂-; **L²** is -C(H)=; and **L³** is -CH₂-. In certain embodiments of any of Formulas **(I)-(LVI) L¹** is -OCH₂-; **L²** is -CH₂-; **L³** is -CH₂-; and **R**² and **R³** are each H. In certain embodiments of any of Formulas **(I)-(LVI), L¹** is -OCH₂-; **L²** is -C(H)=; **L³** is -CH₂-; and **R**² and **R³** are each H. In certain embodiments of any of Formulas **(I)-(LVI), L¹** is -OCH₂-; **L²** is -CH₂-; **L³** is -CH₂-; **R²** and **R³** are each H; and **R⁴** is (2S)-oxetan-2-yl-methyl. In certain embodiments of any of Formulas **(I)-(LIX) L¹** is - OCH₂-; **L²** is -C(H)=; **L³** is -CH₂-; **R²** and **R³** are each H; and **R⁴** is (2S)-oxetan-2-yl-methyl. In certain embodiments of any of Formulas **(I)-(LVI), L¹** is -OCH₂-; **L²** is -SO₂-; **L³** is -CH₂-; **R²** and **R³** are each H; and **R⁴** is (2*S*)-oxetan-2-yl-methyl. In certain embodiments of any of Formulas **(I)-(LVI), L¹** is -OCH₂-; **L²** is -CH₂-; **L³** is -CH₂-; **R**² and **R³** are each H; and **R⁴** is (1-ethyl-1H-imidazol-5-yl)-methyl. In certain embodiments of any of Formulas **(I)-(LVI), L¹** is -OCH₂-; **L²** is -C(H)=; **L³** is -CH₂-; **R²** and **R³** are each H; and **R⁴** is (1-ethyl-1H-imidazol-5-yl)-methyl. In certain embodiments of any of Formulas **(I)-(LVI), L¹** is -OCH₂-; **L²** is -CH₂-; **L³** is -CH₂-; **R²** and **R³** are each H; **R⁴** is (2S)-oxetan-2-yl-methyl; and **R⁵** is -COOH. In certain embodiments of any of Formulas **(I)-(LVI), L¹** is -OCH₂-; **L²** is -C(H)=; **L³** is -CH₂-; **R²** and **R³** are each H; **R⁴** is (2*S*)-oxetan-2-yl-methyl; and **R⁵** is -COOH. In certain embodiments of any of Formulas **(I)-(LVI), L¹** is -OCH₂-; **L²** is -CH₂-; **L³** is -CH₂-; **R**² and **R³** are each H; **R⁴** is (1-ethyl-1H-imidazol-5-yl)-methyl; and **R⁵** is -COOH. In certain embodiments of any of Formulas **(I)-(LVI), L¹** is -OCH₂-; **L²** is -C(H)=; **L³** is -CH₂-; **R²** and **R³** are each H; **R⁴** is (1-ethyl-1H-imidazol-5-yl)-methyl; and **R⁵** is -COOH. In certain embodiments of any of Formulas **(I)-(LVI), L¹** is -OCH₂-; **L²** is -CH₂-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain embodiments of any of Formulas **(I)-(LVI), L¹** is -OCH₂-; **L²** is - C(H)=; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain embodiments of any of Formulas **(I)-(LVI), L¹** is -OCH₂-; **L²** is -CH₂-; **L³** is -CH₂-; **R²** and **R³** are each H; **R⁴** is (1-ethyl-1H-imidazol-5-yl)-methyl; and **R⁵** is tetrazolyl, for instance 1H-1,2,3,4-tetrazol-5-yl. In certain embodiments of any of Formulas **(I)-(LVI), L¹** is -OCH₂-; **L²** is -C(H)=; **L³** is -CH₂-; **R**² and **R³** are each H; **R⁴** is (1-ethyl-1H-imidazol-5-yl)-methyl; and **R⁵** is tetrazolyl, for instance 1H-1,2,3,4-tetrazol-5-yl. In any embodiment according to this paragraph, **R¹** can be selected from H, Cl, F, Me, - CF₃, -OMe, -CN, -C(O)NMe₂, alkyl, propyl, isopropyl, and cyclopropyl. In certain embodiments of any of Formulas **(I)-(LVI), R⁴** is 1-(cyanomethyl)-cycloprop-1-yl-methyl. In certain embodiments of any of Formulas **(I)-(LVI), R⁴** is 1-(Fluoromethyl)-cycloprop-1-yl-methyl. In certain embodiments of any of Formulas **(I)-(LVI), R⁴** is isoxazol-5-yl-methyl. In certain embodiments of any of Formulas **(I)-(LVI), R⁴** is tetrahydrofura-2-yl-methyl.

In certain embodiments of any of Formulas **(I)-(LVI), L¹** is -O-; **L²** is -CH₂-; **L³** is -CH₂-; **R²** and **R³** are each H; and **R⁴** is oxelan-3-yl-methyl. In certain embodiments of any of Formulas **(I)-(LVI), L¹** is -O-; **L²** is -C(H)=; **L³** is -CH₂-; **R**² and **R³** are each H; and **R⁴** is oxelan-3-yl-methyl. In certain embodiments of any of Formulas **(I)-(LVI), L¹** is -O-; **L²** is - CH₂-; **L³** is -CH₂-; **R²** and **R³** are each H; and **R⁴** is 1,3-oxazol-2-yl-methyl. In certain embodiments of any of Formulas **(I)-(LIX), L¹** is -O-; **L²** is -C(H)=; **L³** is -CH₂-; **R²** and **R³** are each H; and **R⁴** is 1,3-oxazol-2-yl-methyl. In certain embodiments of any of Formulas **(I)-(LVI), L¹** is -O-; **L²** is -C(O)-; **L³** is -CH₂-; **R**² and **R³** are each H; **R⁴** is oxelan-3-yl-methyl; and **R⁵** is -COOH. In certain embodiments of any of Formulas **(I)-(LVI), L¹** is -O-; **L²** is -CH₂-; **L³** is -CH₂-; **R²** and **R³** are each H; **R⁴** is oxelan-3-yl-methyl; and **R⁵** is -COOH. In certain embodiments of any of Formulas **(I)-(LVI), L¹** is -O-; **L²** is -C(H)=; **L³** is -CH₂-; **R²** and **R³** are each H; **R⁴** is oxelan-3-yl-methyl; and **R⁵** is -COOH. In certain embodiments of any of Formulas **(I)-(LVI), L¹** is -O-; **L²** is -CH₂-; **L³** is -CH₂-; **R**² and **R³** are each H; **R⁴** is 1,3-oxazol-2-yl-methyl; and **R⁵** is -COOH. In certain embodiments of any of Formulas (I)-(LVI), **L¹** is -O-; **L²** is -C(H)=; **L³** is -CH₂-; **R²** and R**³** are each H; **R⁴** is 1,3-oxazol-2-yl-methyl; and **R⁵** is -COOH. In certain embodiments of any of Formulas **(I)-(LVI), L¹** is -O-; **L²** is -CH₂-; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain embodiments of any of Formulas **(I)-(LVI), L¹** is -O-; **L²** is -C(H)=; **L³** is -CH₂-; **R²** and **R³** are each H; **R⁴** is oxelan-3-yl-methyl; and **R⁵** is tetrazolyl, for instance 1H-1,2,3,4-tetrazol-5-yl. In certain embodiments of any of Formulas **(I)-(LVI), L¹** is -O-; **L²** is -CH₂-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain embodiments of any of Formulas **(I)-(LVI), L¹** is -O-; **L²** is -C(H)=; L³ is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any embodiment according to this paragraph, **R¹** can be selected from H, Cl, F, Me, -CF₃, -OMe, -CN, - C(O)NMe₂, alkyl, propyl, isopropyl, and cyclopropyl. In certain embodiments of any of Formulas **(I)-(LVI), R⁴** is 1-(cyanomethyl)-cycloprop-1-yl-methyl. In certain embodiments of any of Formulas **(I)-(LVI), R⁴** is 1-(Fluoromethyl)-cycloprop-1-yl-methyl. In certain embodiments of any of Formulas **(I)-(LVI), R⁴** is isoxazol-5-yl-methyl. In certain embodiments of any of Formulas **(I)-(LVI), R⁴** is tetrahydrofura-2-yl-methyl.

In certain embodiments of any of Formulas **(I)-(LVI), L¹** is -OCH₂-; **L²** is -CH₂-; **L³** is -CH₂-; **R**² and **R³** are each H; and **R⁴** is oxelan-3-yl-methyl. In certain embodiments of any of Formulas **(I)-(LVI), L¹** is -OCH₂-; **L²** is -C(H)=; **L³** is -CH₂-; **R²** and **R³** are each H; and **R⁴** is oxelan-3-yl-methyl. In certain embodiments of any of Formulas **(I)-(LVI), L¹** is - OCH₂-; **L²** is -CH₂-; **L³** is -CH₂-; **R²** and **R³** are each H; and **R⁴** is 1,3-oxazol-2-yl-methyl. In certain embodiments of any of Formulas **(I)-(LVI), L¹** is -OCH₂-; **L²** is -C(H)=; **L³** is -CH₂-; **R**² and **R³** are each H; and **R⁴** is 1,3-oxazol-2-yl-methyl. In certain embodiments of any of Formulas **(I)-(LVI), L¹** is -OCH₂-; **L²** is -CH₂-; **L³** is -CH₂-; **R²** and **R³** are each H; **R⁴** is oxelan-3-yl-methyl; and **R⁵** is -COOH. In certain embodiments of any of Formulas **(I)-(LVI), L¹** is -OCH₂-; **L²** is -C(H)=; **L³** is -CH₂-; **R²** and **R³** are each H; **R⁴** is oxelan-3-yl-methyl; and **R⁵** is -COOH. In certain embodiments of any of Formulas (I)-(LVI), **L¹** is -OCH₂-; **L²** is -CH₂-; **L³** is -CH₂-; **R²** and **R³** are each H; **R⁴** is 1,3-oxazol-2-yl-methyl; and **R⁵** is -COOH. In certain embodiments of any of Formulas **(I)-(LVI), L¹** is -OCH₂-; **L²** is -C(H)=; **L³** is -CH₂-; **R²** and **R³** are each H; **R⁴** is 1,3-oxazol-2-yl-methyl; and **R⁵** is -COOH. In certain embodiments of any of Formulas **(I)-(LVI), L¹** is -OCH₂-; **L²** is -CH₂-; **L³** is -CH₂-; **R**² and **R³** are each H; **R⁴** is oxelan-3-yl-methyl; and **R⁵** is tetrazolyl, for instance 1H-1,2,3,4-tetrazol-5-yl. In certain embodiments of any of Formulas (I)-(LVI), **L¹** is -OCH₂-; **L²** is -C(H)=; **L³** is -CH₂-; **R²** and **R³** are each H; **R⁴ is** oxelan-3-yl-methyl; and **R⁵** is tetrazolyl, for instance 1H-1,2,3,4-tetrazol-5-yl. In certain embodiments of any of Formulas **(I)-(LVI), L¹** is -OCH₂-; **L²** is -CH₂-; **L³** is - CH₂-; **R²** and **R³** are each H; **R⁴** is 1,3-oxazol-2-yl-methyl; and **R⁵** is tetrazolyl, for instance 1H-1,2,3,4-tetrazol-5-yl. In certain embodiments of any of Formulas **(I)-(LVI), L¹** is -OCH₂-; **L²** is -C(H)=; **L³** is -CH₂-; **R²** and **R³** are each H; **R⁴** is 1,3-oxazol-2-yl-methyl; and **R⁵** is tetrazolyl, for instance 1-1,2,3,4-tetrazol-5-yl. In any embodiment according to this paragraph, **R¹** can be selected from H, Cl, F, Me, -CF₃, -OMe, -CN, -C(O)NMe₂, alkyl, propyl, isopropyl, and cyclopropyl. In certain embodiments of any of Formulas **(I)-(LVI), R⁴** is 1-(cyanomethyl)-cycloprop-1-yl-methyl. In certain embodiments of any of Formulas **(I)-(LVI), R⁴** is 1-(Fluoromethyl)-cycloprop-1-yl-methyl. In certain embodiments of any of Formulas **(I)-(LVI), R⁴** is isoxazol-5-yl-methyl. In certain embodiments of any of Formulas **(I)-(LVI), R⁴** is tetrahydrofura-2-yl-methyl.

In certain embodiments of any of Formulas **(I)-(LVI), L¹** is -O-; and **L²** is -O-. In certain embodiments of any of Formulas **(I)-(LVI), L¹** is -O-; **L²** is -O-; and **L³** is -CH₂-. In certain embodiments of any of Formulas **(I)-(LVI), L¹** is -O-; **L²** is -O-; **L³** is -CH₂-; and **R²** and **R³** are each H. In certain embodiments of any of Formulas (I)-(LVI), **L¹** is -O-; **L²** is -O-; **L³** is -CH₂-; **R²** and **R³** are each H; and **R⁴** is (2S)-oxetan-2-yl-methyl. In certain embodiments of any of Formulas **(I)-(LVI), L¹** is -O-; **L²** is -O-; **L³** is -CH₂-; **R²** and **R³** are each H; and **R⁴** is (1-ethyl-1H-imidazol-5-yl)-methyl. In certain embodiments of any of Formulas **(I)-(LVI), L¹** is -O-; **L²** is -O-; **L³** is -CH₂-; **R²** and **R³** are each H; **R⁴** is (2*S*)-oxetan-2-yl-methyl; and **R⁵** is -COOH. In certain embodiments of any of Formulas **(I)-(LVI), L¹** is -O-; **L²** is -O-; **L³** is - CH₂-; **R²** and **R³** are each H; **R⁴** is (1-ethyl-1H-imidazol-5-yl)-methyl; ; and **R⁵** is -COOH. In certain embodiments of any of Formulas **(I)-(LVI), L¹** is -O-; **L²** is -O-; **L³** is -CH₂-; **R²** and **R³** are each H; **R⁴** is (2*S*)-oxetan-2-yl-methyl; and **R⁵** is tetrazolyl, for instance 1H-1,2,3,4-tetrazol-5-yl. In certain embodiments of any of Formulas (I)-(LVI), **L¹** is -O-; **L²** is -O-; **L³** is -CH₂-; **R²** and **R³** are each H; **R⁴** is (1-ethyl-1H-imidazol-5-yl)-methyl; and **R⁵** is tetrazolyl, for instance 1-1,2,3,4-tetrazol-5-yl. In any embodiment according to this paragraph, **R¹** can be selected from H, Cl, F, Me, -CF₃, -OMe, -CN, -C(O)NMe₂, alkyl, propyl, isopropyl, and cyclopropyl. In certain embodiments of any of Formulas **(I)-(LVI), R⁴** is 1-(cyanomethyl)-cycloprop-1-yl-methyl. In certain embodiments of any of Formulas **(I)-(LVI), R⁴** is 1-(Fluoromethyl)-cycloprop-1-yl-methyl. In certain embodiments of any of Formulas **(I)-(LVI), R⁴** is isoxazol-5-yl-methyl. In certain embodiments of any of Formulas **(I)-(LVI), R⁴** is tetrahydrofura-2-yl-methyl.

In certain embodiments of any of Formulas **(I)-(LVI) L¹** is -OCH₂-; and **L²** is -O-. In certain embodiments of any of Formulas **(I)-(LVI) L¹** is -OCH₂-; **L²** is -O-; and **L³** is -CH₂-. In certain embodiments of any of Formulas **(I)-(LVI), L¹** is -OCH₂-; **L²** is -O-; **L³** is -CH₂-; and **R²** and **R³** are each H. In certain embodiments of any of Formulas **(I)-(LVI) L¹** is -OCH₂-; **L²** is -O-; **L³** is -CH₂-; **R²** and **R³** are each H; and **R⁴** is (2*S*)-oxetan-2-yl-methyl. In certain embodiments of any of Formulas **(I)-(LVI), L¹** is -OCH₂-; **L²** is -O-; **L³** is -CH₂-; **R²** and **R³** are each H; and **R⁴** is (1-ethyl-1H-imidazol-5-yl)-methyl. In certain embodiments of any of Formulas **(I)-(LVI), L¹** is -OCH₂-; **L²** is -O-; **L³** is -CH₂-; **R²** and **R³** are each H; **R⁴** is (2*S*)-oxetan-2-yl-methyl; and **R⁵** is -COOH. In certain embodiments of any of Formulas **(I)-(LVI), L¹** is -OCH₂-; **L²** is -O-; **L³** is -CH₂-; R**²** and **R³** are each H; **R⁴** is (1-ethyl-1H-imidazol-5-yl)-methyl; and **R⁵** is -COOH. In certain embodiments of any of Formulas **(I)-(LVI), L¹** is -OCH₂-; **L²** is -O-; **L³** is -CH₂-; **R²** and **R³** are each H; **R⁴** is (2*S*)-oxetan-2-yl-methyl; and **R⁵** is tetrazolyl, for instance 1-1,2,3,4-tetrazol-5-yl. In certain embodiments of any of Formulas **(I)-(LVI), L¹** is -OCH₂-; **L²** is -O-; **L³** is -CH₂-; **R²** and **R³** are each H; **R⁴** is (1-ethyl-1H-imidazol-5-yl)-methyl; and **R⁵** is tetrazolyl, for instance 1-1,2,3,4-tetrazol-5-yl. In any embodiment according to this paragraph, **R¹** can be selected from H, Cl, F, Me, -CF₃, -OMe, - CN, -C(O)NMe₂, alkyl, propyl, isopropyl, and cyclopropyl. In certain embodiments of any of Formulas **(I)-(LVI), R⁴** is 1-(cyanomethyl)-cycloprop-1-yl-methyl. In certain embodiments of any of Formulas **(I)-(LVI), R⁴** is 1-(Fluoromethyl)-cycloprop-1-yl-methyl. In certain embodiments of any of Formulas **(I)-(LVI), R⁴** is isoxazol-5-yl-methyl. In certain embodiments of any of Formulas **(I)-(LVI), R⁴** is tetrahydrofura-2-yl-methyl.

In certain embodiments of any of Formulas **(I)-(LVI), L¹** is -O-; **L²** is -O-; **L³** is - CH₂-; **R²** and **R³** are each H; and **R⁴** is oxelan-3-yl-methyl. In certain embodiments of any of Formulas **(I)-(LVI), L¹** is -O-; **L²** is -O-; **L³** is -CH₂-; **R²** and **R³** are each H; and **R⁴** is 1,3-oxazol-2-yl-methyl. In certain embodiments of any of Formulas **(I)-(LVI), L¹** is -O-; **L²** is -O-; **L³** is -CH₂-; **R²** and **R³** are each H; **R⁴** is oxelan-3-yl-methyl; and **R⁵** is -COOH. In certain embodiments of any of Formulas **(I)-(LVI), L¹** is -O-; **L²** is -O-; **L³** is -CH₂-; **R²** and **R³** are each H; **R⁴** is 1,3-oxazol-2-yl-methyl; ; and **R⁵** is -COOH. In certain embodiments of any of Formulas **(I)-(LVI), L¹** is -O-; **L²** is -O-; **L³** is -CH₂-; R**²** and **R³** are each H; **R⁴** is oxelan-3-yl-methyl; and **R⁵** is tetrazolyl, for instance 1H-1,2,3,4-tetrazol-5-yl. In certain embodiments of any of Formulas **(I)-(LVI), L¹** is -O-; **L²** is -O-; **L³** is -CH₂-; **R²** and **R³** are each H; **R⁴** is 1,3-oxazol-2-yl-methyl; and **R⁵** is tetrazolyl, for instance 1-1,2,3,4-tetrazol-5-yl. IIn any embodiment according to this paragraph, **R¹** can be selected from H, Cl, F, Me, -CF₃, -OMe, - CN, -C(O)NMe₂, alkyl, propyl, isopropyl, and cyclopropyl. In certain embodiments of any of Formulas **(I)-(LVI), R⁴** is 1-(cyanomethyl)-cycloprop-1-yl-methyl. In certain embodiments of any of Formulas **(I)-(LVI), R⁴** is 1-(Fluoromethyl)-cycloprop-1-yl-methyl. In certain embodiments of any of Formulas **(I)-(LVI), R⁴** is isoxazol-5-yl-methyl. In certain embodiments of any of Formulas **(I)-(LVI), R⁴** is tetrahydrofura-2-yl-methyl.

In certain embodiments of any of Formulas **(I)-(LVI), L¹** is -OCH₂-; **L²** is -O-; **L³** is -CH₂-; **R²** and **R³** are each H; and **R⁴** is oxelan-3-yl-methyl. In certain embodiments of any of Formulas **(I)-(LVI), L¹** is -OCH₂-; **L²** is -O-; **L³** is -CH₂-; **R²** and **R³** are each H; and **R⁴** is 1,3-oxazol-2-yl-methyl. In certain embodiments of any of Formulas **(I)-(LVI), L¹** is -OCH₂-; **L²** is -O-; **L³** is -CH₂-; **R²** and **R³** are each H; **R⁴** is oxelan-3-yl-methyl; and **R⁵** is -COOH. In certain embodiments of any of Formulas **(I)-(LVI), L¹** is -OCH₂-; **L²** is -O-; **L³** is -CH₂-; **R²** and **R³** are each H; **R⁴** is 1,3-oxazol-2-yl-methyl; ; and **R⁵** is -COOH. In certain embodiments of any of Formulas **(I)-(LVI), L¹** is -OCH₂-; **L²** is -O-; **L³** is -CH₂-; **R²** and **R³** are each H; **R⁴** is oxelan-3-yl-methyl; and **R⁵** is tetrazolyl, for instance 1H-1,2,3,4-tetrazol-5-ylIn certain embodiments of any of Formulas **(I)-(LVI), L¹** is -OCH₂-; **L²** is -O-; **L³** is -CH₂-; **R²** and **R³** are each H; **R⁴** is 1,3-oxazol-2-yl-methyl; and **R⁵** is tetrazolyl, for instance 1H-1,2,3,4-tetrazol-5-yl. In any embodiment according to this paragraph, **R¹** can be selected from H, Cl, F, Me, - CF₃, -OMe, -CN, -C(O)NMe₂, alkyl, propyl, isopropyl, and cyclopropyl. In certain embodiments of any of Formulas **(I)-(LVI), R⁴** is 1-(cyanomethyl)-cycloprop-1-yl-methyl. In certain embodiments of any of Formulas **(I)-(LVI), R⁴** is 1-(Fluoromethyl)-cycloprop-1-yl-methyl. In certain embodiments of any of Formulas **(I)-(LVI), R⁴** is isoxazol-5-yl-methyl. In certain embodiments of any of Formulas **(I)-(LVI), R⁴** is tetrahydrofura-2-yl-methyl.

In certain embodiments, provided is a compound of Table 1 below, or a pharmaceutically salt, tautomer, stereoisomer, and/or mixture of stereoisomers thereof.

| **Compound** | **Structure** | **IUPAC** |
|---|---|---|
| 119 | 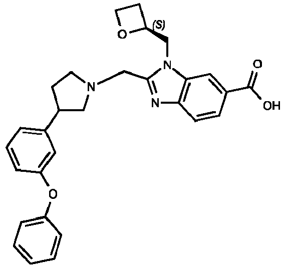 | 1-{[(2S)-oxetan-2-yl]methyl}-2-{[3-(3-phenoxyphenyl)pyrrolidin-1-yl]methyl}-1H-1,3-benzodiazole-6-carboxylic acid |
| 127 | 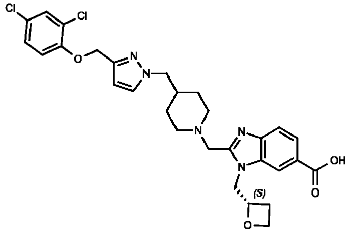 | 2-{[4-({3-[(2,4-dichlorophenoxy)methyl]-1H-pyrazol-1-yl}methyl)piperidin-1-yl]methyl}-1-{[(2S)-oxetan-2-yl]methyl}-1H-1,3-benzodiazole-6-carboxylic acid |
| 134 | 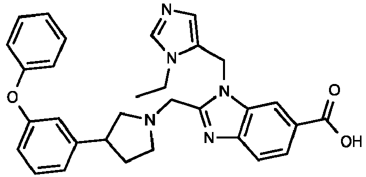 | 1-[(1-ethyl-1H-imidazol-5-yl)methyl]-2-{[3-(3-phenoxyphenyl)pyrrolidin-1-yl]methyl}-1H-1,3-benzodiazole-6-carboxylic acid |
| 147 | 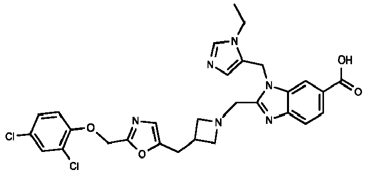 | 2-{[3-({2-[(2,4-dichlorophenoxy)methyl]-1,3-oxazol-5-yl}methyl)azetidin-1-yl]methyl}-1-[(1-ethyl-1H-imidazol-5-yl)methyl]-1H-1,3-benzodiazole-6-carboxylic acid |
| 167 | 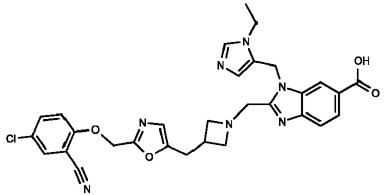 | 2-{[3-({2-[(4-chloro-2-cyanophenoxy)methyl]-1,3-oxazol-5-yl}methyl)azetidin-1-yl]methyl}-1-[(1-ethyl-1H-imidazol-5-yl)methyl]-1H-1,3-benzodiazole-6-carboxylic acid |
| 172 | 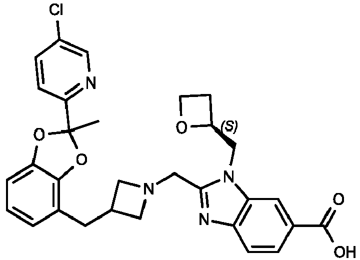 | 2-[(3-{[2-(5-chloropyridin-2-yl)-2-methyl-2H-1,3-benzodioxol-4-yl]methyl}azetidin-1-yl)methyl]-1-{[(2S)-oxetan-2-yl]methyl1-1H-1,3-benzodiazole-6-carboxylic acid |
| 173 | 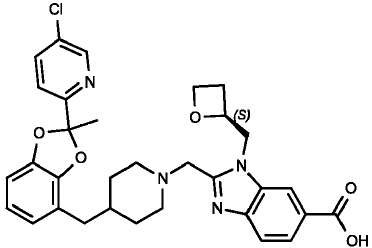 | 2-[(4-{[2-(5-chloropyridin-2-yl)-2-methyl-2H-1,3-benzodioxol-4-yl]methyl}piperidin-1-yl)methyl]-1-{[(2S)-oxetan-2-yl] methyl}-1H-1,3-benzodiazole-6-carboxylic acid |
| 175 | 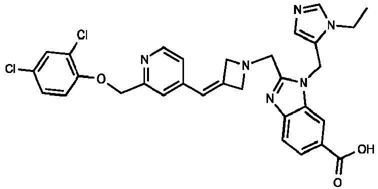 | 2-{[3-({2-[(2,4-dichlorophenoxy)methyl]pyridin-4-yl}methylidene)azetidin-1-yl]methyl}-1-[(1-ethyl-1H-imidazol-5-yl)methyl]-1H-1,3-benzodiazole-6-carboxylic acid |
| 176 | 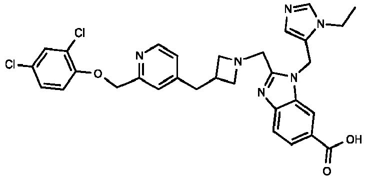 | 2-{[3-({2-[(2,4-dichlorophenoxy)methyl]pyridin-4-yl}methyl)azetidin-1-yl]methyl}-1-[(1-ethyl-1H-imidazol-5-yl)methyl]-1H-1,3-benzodiazole-6-carboxylic acid |
| 177 | 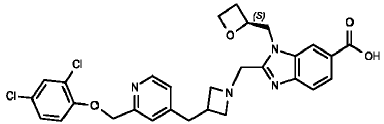 | 2-{[3-({2-[(2,4-dichlorophenoxy)methyl]pyridin-4-yl}methyl)azetidin-1-yl]methyl}-1-{[(2S)-oxetan-2-yl]methyl}-1H-1,3-benzodiazole-6-carboxylic acid |
| 180 | 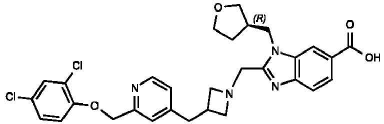 | 2-{[3-({2-[(2,4-dichlorophenoxy)methyl]pyridin-4-yl}methyl)azetidin-1-yl]methyl}-1-{[(3R)-oxolan-3-yl]methyl}-1H-1,3-benzodiazole-6-carboxylic acid |
| 181 | 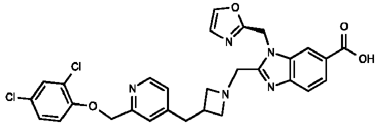 | 2-{[3-({2-[(2,4-dichlorophenoxy)methyl]pyridin-4-yl}methyl)azetidin-1-yl]methyl}-1-[(1,3-oxazol-2-yl)methyl]-1H-1,3-benzodiazole-6-carboxylic acid |
| 184 | 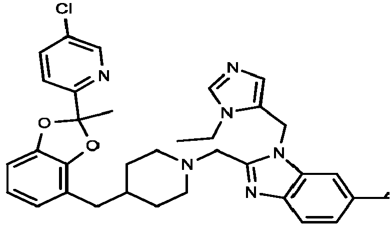 | 2-[(4-{[2-(5-chloropyridin-2-yl)-2-methyl-2H-1,3-benzodioxol-4-yl]methyl}piperidin-1-yl)methy-1-[(1-ethyl-1H-imidazol-5-yl)methyl]-1H-1,3-benzodiazole-6-carboxylic acid |
| 185 | 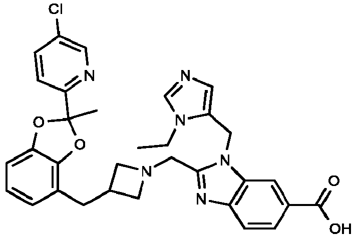 | 2-[(3-{[2-(5-chloropyridin-2-yl)-2-methyl-2H-1,3-benzodioxol-4-yl]methyl}azetidin-1-yl)methyl]-1-[(1-ethyl-1H-imidazol-5-yl)methyl]-1H-1,3-benzodiazole-6-carboxylic acid |
| 190 | 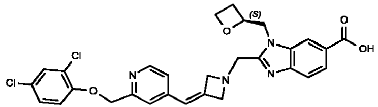 | 2-{[3-({2-[(2,4-dichlorophenoxy)methyl]pyridin-4-yl}methylidene)azetidin-1-yl]methyl}-1-{[(2S)-oxetan-2-yl]methyl}-1H-1,3-benzodiazole-6-carboxylic acid |
| 191 | 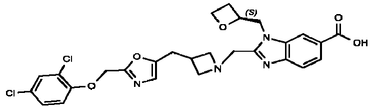 | 2-{[3-({2-[(2,4-dichlorophenoxy)methyl]pyridin-4-yl}methylidene)azetidin-1-yl]methyl}-1-{[(2S)-oxetan-2-yl]methyl}-1H-1,3-benzodiazole-6-carboxylic acid |
| 192 | 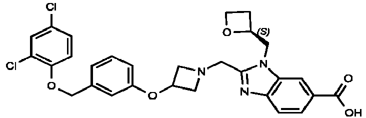 | 2-{[3-({2-[(2,4-dichlorophenoxy)methyl]-1,3-oxazol-5-yl}methyl)azetidin-1-yl]methyl}-1-{[(2S)-oxetan-2-yl]methyl}-1H-1,3 -benzodiazole-6-carboxylic acid |
| 193 | 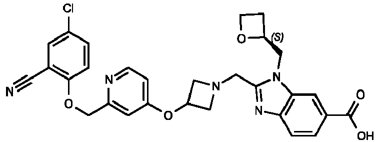 | 2-[(3-{3-[(2,4-dichlorophenoxy)methyl]phenoxy}azetidin-1-yl)methyl]-1-{[(2S)-oxetan-2-yl]methyl}-1H-1,3-benzodiazole-6-carboxylic acid |
| 194 | 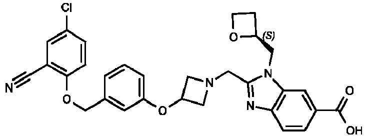 | 2-{[3-({2-[(4-chloro-2-cyanophenoxy)methyl]pyridin-4-yl}oxy)azetidin-1-yl]methyl}-1-{[(2S)-oxetan-2-yl]methyl}-1H-1,3-benzodiazole-6-carboxylic acid |
| 195 | 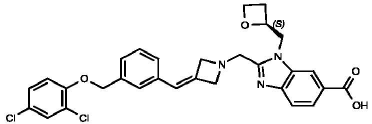 | 2-[(3-{3-[(4-chloro-2-cyanophenoxy)methyl]phenoxy}azetidin-1-yl)methyl]-1-{[(2S)-oxetan-2-yl]methyl}-1H-1,3-benzodiazole-6-carboxylic acid |
| 196 | 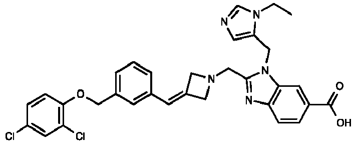 | 2-{[3-({3-[(2,4-dichlorophenoxy)methyl]phenyllmethylide ne)azetidin-1-yl]methyl}-1-{[(2S)-oxetan-2-yl]methyl}-1H-1,3-benzodiazole-6-carboxylic acid |
| 197 | 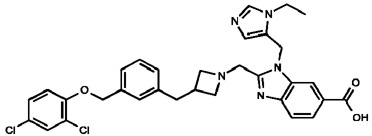 | 2-{[3-({3-[(2,4-dichlorophenoxy)methyl]phenyl}methylide ne)azetidin-1-yl]methyl}-1-[(1-ethyl-1H-imidazol-5-yl)methyl]-1H-1,3-benzodiazole-6-carboxylic acid |
| 201 | 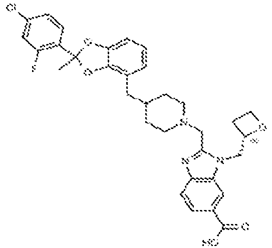 | 2-[(4-{[2-(4-chloro-2-fluorophenyl)-2-methyl-2H-1,3-benzodioxol-4-yl]methyl}piperidin-1-yl)methyl]-1-{[(2S)-oxetan-2-yl] methyl}-1H-1,3-benzodiazole-6-carboxylic acid |
| 209 | 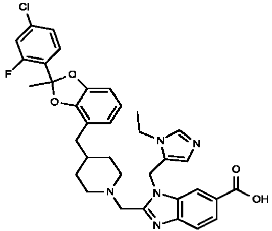 | 2-[(4-{[2-(4-chloro-2-fluorophenyl)-2-methyl-2H-1,3-benzodioxol-4-yl]methyl}piperidin-1-yl)methyl]-1-[(1-ethyl-1H-imidazol-5-yl)methyl]-1H-1,3-benzodiazole-6-carboxylic acid |
| 210 | 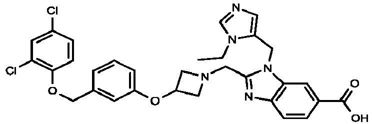 | 2-[(3-{3-[(2,4-dichlorophenoxy)methyl]phenoxy}azetidin-1-yl)methyl]-1-[(1-ethyl-1H-imidazol-5-yl)methyl]-1H-1,3-benzodiazole-6-carboxylic acid |
| 211 | 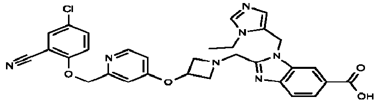 | 2-{[3-({2-[(4-chloro-2-cyanophenoxy)methyl]pyridin-4-yl}oxy)azetidin-1-yl]methyl}-1-[(1-ethyl-1H-imidazol-5-yl)methyl]-1H-1,3-benzodiazole-6-carboxylic acid |
| 212 | 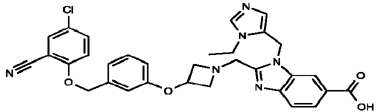 | 2-[(3-{3-[(4-chloro-2-cyanophenoxy)methyl]phenoxy}azetidin-1-yl)methyl]-1-[(1-ethyl-1H-imidazol-5-yl)methy1]-1H-1,3-benzodiazole-6-carboxylic acid |
| 213 | 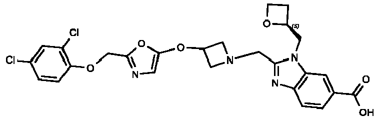 | 2-{[3-({2-[(2,4-dichlorophenoxy)methyl]-1,3-oxazol-5-yl}oxy)azetidin-1-yl]methyl}-1-{[(2S)-oxetan-2-yl]methyl}-1H-1,3-benzodiazole-6-carboxylic acid |
| 218 | 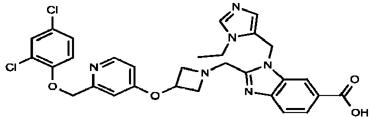 | 2-{[3-({2-[(2,4-dichlorophenoxy)methyl]pyridin-4-yl}oxy)azetidin-1-yl]methyl}-1-[(1-ethyl-1H-imidazol-5-yl)methyl]-1H-1,3-benzodiazole-6-carboxylic acid |
| 219 | 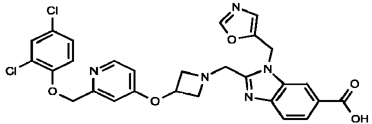 | 2-{[3-({2-[(2,4-dichlorophenoxy)methyl]pyridin-4-yl}oxy)azetidin-1-yl]methyl}-1-[(1,3-oxazol-5-yl)methyl]-1H-1,3-benzodiazole-6-carboxylic acid |
| 222 | 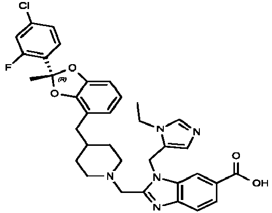 | 2-[(4-{[(2R)-2-(4-chloro-2-fluorophenyl)-2-methyl-2H-1,3-benzodioxol-4-yl] methyl}piperidin-1-yl)methyl]-1-[(1-ethyl-1H-imidazol-5-yl)methyl]-1H-1,3-benzodiazole-6-carboxylic acid |
| 223 | 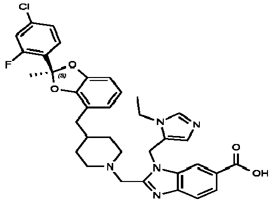 | 2-[(4-{[(2S)-2-(4-chloro-2-fluorophenyl)-2-methyl-2H-1,3-benzodioxol-4-yl]methyl}piperidin-1-yl)methyl]-1-[(1-ethyl-1H-imidazol-5-yl)methyl]-1H-1,3-benzodiazole-6-carboxylic acid |
| 225 | 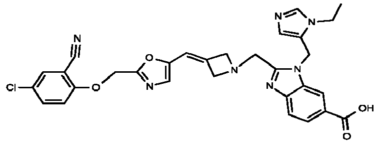 | 2-{[3-({2-[(4-chloro-2-cyanophenoxy)methyl]-1,3-oxazol-5-yl}methylidene)azetidin-1-yl]methyl}-1-[(1-ethyl-1H-imidazol-5-yl)methyl]-1H-1,3-benzodiazole-6-carboxylic acid |
| 226 | 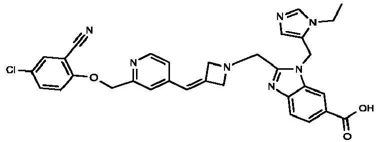 | 2-{[3-({2-[(4-chloro-2-cyanophenoxy)methyl]pyridin-4-yl}methylidene)azetidin-1-yl]methyl}-1-[(1-ethyl-1H-imidazol-5-yl)methyl]-1H-1,3-benzodiazole-6-carboxylic acid |
| 228 | 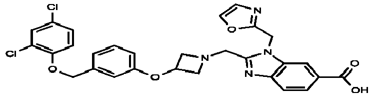 | 2-[(3-{3-[(2,4-dichlorophenoxy)methyl]phenoxy}azetidin-1-yl)methyl]-1-[(1,3-oxazol-2-yl)methyl]-1H-1,3-benzodiazole-6-carboxylic acid |
| 229 | 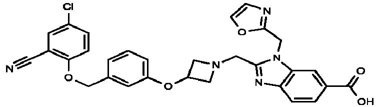 | 2-[(3-{3-[(4-chloro-2-cyanophenoxy)methyl]phenoxy}azetidin-1-yl)methyl]-1-[(1,3-oxazol-2-yl)methyl]-1H-1,3-benzodiazole-6-carboxylic acid |
| 233 | 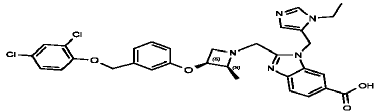 | 2-{[(2S,3S)-3-{3-[(2,4-dichlorophenoxy)methyl]phenoxy}-2-methylazetidin-1-yl]methyl}-1-[(1-ethyl-1H-imidazol-5-yl)methyl]-1H-1,3-benzodiazole-6-carboxylic acid |
| 234 | 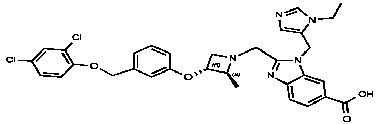 | 2-{[(2S,3R)-3-{3-[(2,4-dichlorophenoxy)methyl]phenoxy}-2-methylazetidin-1-yl]methyl}-1-[(1-ethyl-1H-imidazol-5-yl)methyl]-1H-1,3-benzodiazole-6-carboxylic acid |
| 235 | 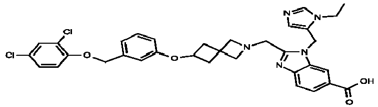 | 2-[(6-{3-[(2,4-dichlorophenoxy)methyl]phenoxy}-2-azaspiro[3.3]heptan-2-yl)methyl]-1-[(1-ethyl-1H-imidazol-5-yl)methyl]-1H-1,3-benzodiazole-6-carboxylic acid |
| 236 | 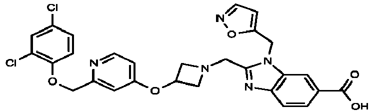 | 2-{[3-({2-[(2,4-dichlorophenoxy)methyl]pyridin-4-yl}oxy)azetidin-1-yl]methyl}-1-[(1,2-oxazol-5-yl)methyl]-1H-1,3-benzodiazole-6-carboxylic acid |
| 237 | 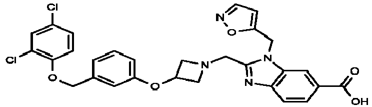 | 2-[(3-{3-[(2,4-dichlorophenoxy)methyl]phenoxy}azetidin-1-yl)methyl]-1-[(1,2-oxazol-5-yl)methyl]-1H-1,3-benzodiazole-6-carboxylic acid |
| 238 | 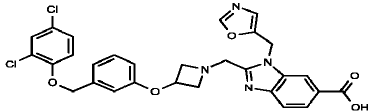 | 2-[(3-{3-[(2,4-dichlorophenoxy)methyl]phenoxy}azetidin-1-yl)methyl]-1-[(1,3-oxazol-5-yl)methyl]-1H-1,3-benzodiazole-6-carboxylic acid |
| 244 | 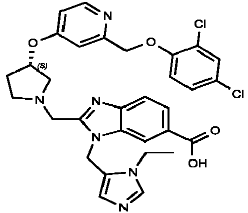 | 2-{[(3S)-3-({2-[(2,4-dichlorophenoxy)methyl]pyridin-4-yl}oxy)pyrrolidin-1-yl]methyl}-1-[(1-ethyl-1H-imidazol-5-yl)methyl]-1H-1,3-benzodiazole-6-carboxylic acid |
| 245 | 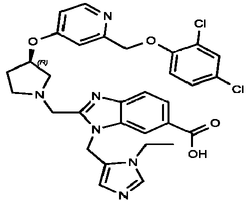 | 2-{[(3R)-3-({2-[(2,4-dichlorophenoxy)methyl]pyridin-4-yl}oxy)pyrrolidin-1-yl]methyl}-1-[(1-ethyl-1H-imidazol-5-yl)methyl]-1H-1,3-benzodiazole-6-carboxylic acid |
| 273 | 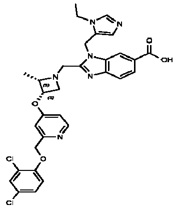 | 2-{[(2S,3S)-3-({2-[(2,4-dichlorophenoxy)methyl]pyridin-4-yl}oxy)-2-methylazetidin-1-yl]methyl}-1-[(1-ethyl-1H-imidazol-5-yl)methyl]-1H-1,3-benzodiazole-6-carboxylic acid |
| 281 | 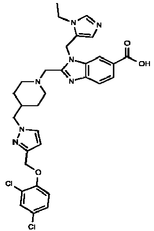 | 2-{[4-({3-[(2,4-dichlorophenoxy)methyl]-1H-pyrazol-1-yl}methyl)piperidin-1-yl]methyl}-1-[(1-ethyl-1H-imidazol-5-yl)methyl]-1H-1,3-benzodiazole-6-carboxylic acid |
| 282 | 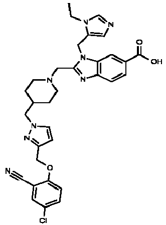 | 2-{[4-({3-[(4-chloro-2-cyanophenoay)methyl]-1H-pyrazol-1-yl}methyl)piperidin-1-yl]methyl}-1-[(1-ethyl-1H-imidazol-5-yl)methyl]-1H-1,3-benzodiazole-6-carboxylic acid |
| 289 | 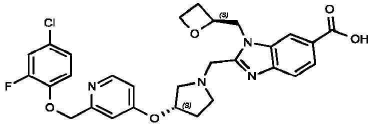 | 2-{[(3S)-3-({2-[(4-chloro-2-fluorophenoxy)methyl]pyridin-4-yl}oxy)pyrrolidin-1-yl]methyl}-1-{[(2S)-oxetan-2-yl]methyl}-1H-1,3-benzodiazole-6-carboxylic acid |
| 293 | 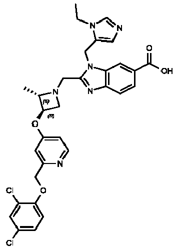 | 2-{[(2S,3R)-3-({2-[(2,4-dichlorophenoxy)methyl]pyridin-4-yl}oxy)-2-methylazetidin-1-yl]methyl}-1-[(1-ethyl-1H-imidazol-5-yl)methyl]-1H-1,3-benzodiazole-6-carboxylic acid |
| 298 | 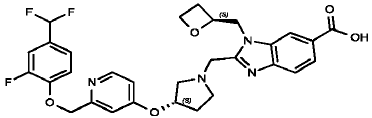 | 2-{[(3S)-3-[(2-{[4-(difluoromethyl)-2-fluorophenoxy]methyl}pyridin-4-yl)oxy]pyrrolidin-1-yl]methyl}-1-{[(2S)-oxetan-2-yl]methyl}-1H-1,3-benzodiazole-6-carboxylic acid |
| 350 | 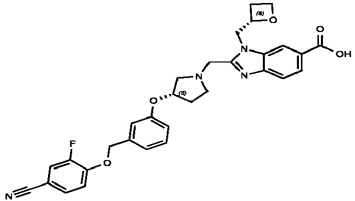 | 2-{[(3S)-3-{3-[(4-cyano-2-fluorophenoxy)methyl]phenoxy}pyrrolidin-1-yl]methyl}-1-{[(2S)-oxetan-2-yl]methyl}-1H-1,3-benzodiazole-6-carboxylic acid |
| 351 | 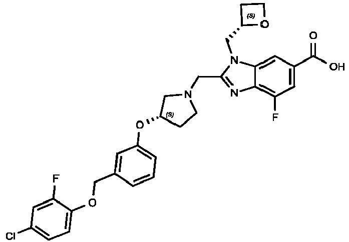 | 2-{[(3S)-3-{3-[(4-chloro-2-fluorophenoxy)methyl]phenoxy}pyrrolidin-1-yl]methyl}-4-fluoro-1-{[(2S)-oxetan-2-yl]methyl}-1H-1,3-benzodiazole-6-carboxylic acid |
| 360 | 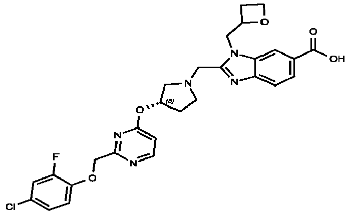 | 2-{[(3S)-3-({2-[(4-chloro-2-fluorophenoxy)methyl]pyrimidin-4-yl}oxy)pyrrolidin-1-yl]methyl}-1-[(oxetan-2-yl)methyl]-1H-1,3-benzodiazole-6-carboxylic acid |
| 361 | 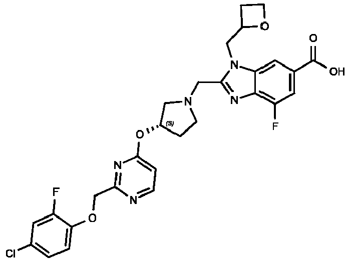 | 2-{[(3S)-3-({2-[(4-chloro-2-fluorophenoxy)methyl]pyrimidin-4-yl}oxy)pyrrolidin-1-yl]methyl}-4-fluoro-1-[(oxetan-2-yl)methyl]-1H-1,3-benzodiazole-6-carboxylic acid |
| 383 | 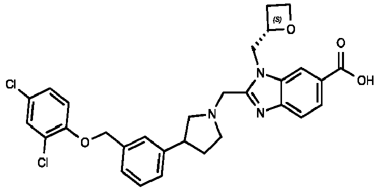 | 2-[(3-{3-[(2,4-dichlorophenoxy)methyl]phenyl}pyrrolidin-1-yl)methyl]-1-{[(2S)-oxetan-2-yl]methyl}-1H-1,3-benzodiazole-6-carboxylic acid |
| 384 | 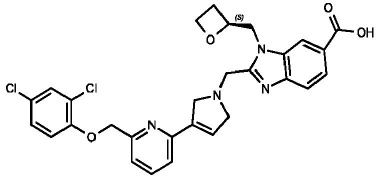 | 2-[(3-{6-[(2,4-dichlorophenoxy)methyl]pyridin-2-yl}-2,5-dihydro-1H-pyrrol-1-yl)methyl]-1-{[(2S)-oxetan-2-yl]methyl}-1H-1,3-benzodiazole-6-carboxylic acid |
| 385 | 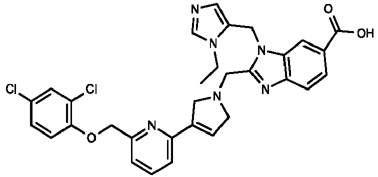 | 2-[(3-{6-[(2,4-dichlorophenoxy)methyl]pyridin-2-yl}-2,5-dihydro-1H-pyrrol-1-yl)methyl]-1-[(1-ethyl-1H-imidazol-5-yl)methyl]-1H-1,3-benzodiazole-6-carboxylic acid |
| 386 | 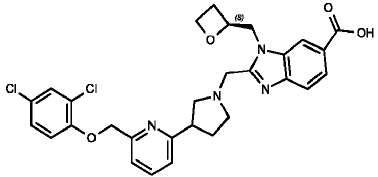 | 2-[(3-{6-[(2,4-dichlorophenoxy)methyl]pyridin-2-yl}pyrrolidin-1-yl)methyl]-1-{[(2S)-oxetan-2-yl] methyl}-1H-1,3-benzodiazole-6-carboxylic acid |
| 387 | 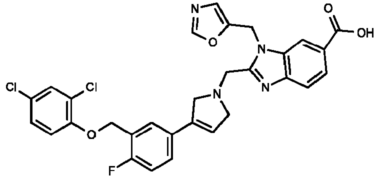 | 2-[(3-{3-[(2,4-dichlorophenoxy)methyl] -4-fluorophenyl}-2,5-dihydro-1H-pyrrol-1-yl)methyl]-1-[(1,3-oxazol-5-yl)methyl]-1H-1,3-benzodiazole-6-carboxylic acid |
| 388 | 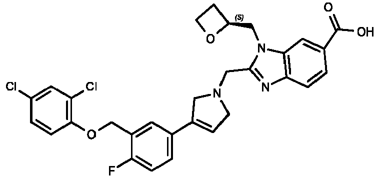 | 2-[(3-{3-[(2,4-dichlorophenoxy)methyl]-4-fluorophenyl}-2,5-dihydro-1H-pyrrol-1-yl)methyl]-1-{[(2S)-oxetan-2-yl]methyl}-1H-1,3-benzodiazole-6-carboxylic acid |
| 389 | 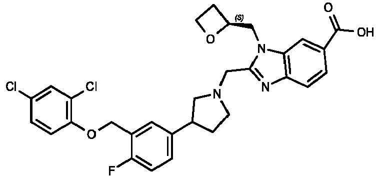 | 2-[(3-{3-[(2,4-dichlorophenoxy)methyl]-4-fluorophenyl}pyrrolidin-1-yl)methyl]-1-{[(2S)-oxetan-2-yl]methyl}-1H-1,3-benzodiazole-6-carboxylic acid |
| 390 | 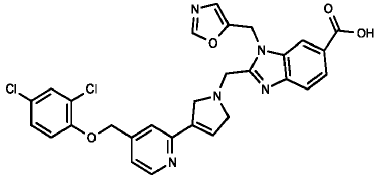 | 2-[(3-{4-[(2,4-dichlorophenoxy)methyl]pyridin-2-yl}-2,5-dihydro-1H-pyrrol-1-yl)methyl]-1-[(1,3-oxazol-5-yl)methyl]-1H-1,3-benzodiazole-6-carboxylic acid |
| 391 | 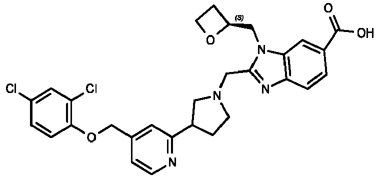 | 2-[(3-{4-[(2,4-dichlorophenoxy)methyl]pyridin-2-yl}pyrrolidin-1-yl)methyl]-1-{[(2S)-oxetan-2-yl]methyl}-1H-1,3-benzodiazole-6-carboxylic acid |
| 392 | 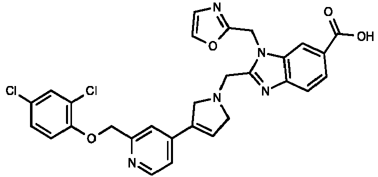 | 2-[(3-{2-[(2,4-dichlorophenoxy)methyl]pyridin-4-yl}-2,5-dihydro-1H-pyrrol-1-yl)methyl]-1-[(1,3-oxazol-2-yl)methyl]-1H-1,3-benzodiazole-6-carboxylic acid |
| 393 | 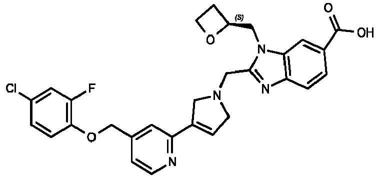 | 2-[(3-{4-[(4-chloro-2-fluorophenoxy)methyl]pyridin-2-yl}-2,5-dihydro-1H-pyrrol-1-yl)methyl]-1-{[(2S)-oxetan-2-yl] methyl}-1H-1,3-benzodiazole-6-carboxylic acid |
| 394 | 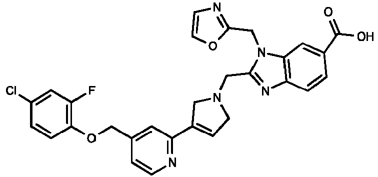 | 2-[(3-{4-[(4-chloro-2-fluorophenoxy)methyl]pyridin-2-yl}-2,5-dihydro-1H-pyrrol-1-yl)methyl]-1-[(1,3-oxazol-2-yl)methyl]-1H-1,3-benzodiazole-6-carboxylic acid |
| 395 | 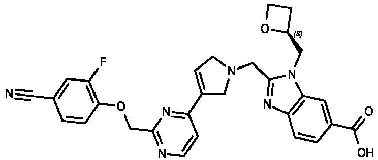 | 2-[(3-{2-[(4-cyano-2-fluorophenoxy)methyl]pyrimidin-4-yl}-2,5-dihydro-1H-pyrrol-1-yl)methyl]-1-{[(2S)-oxetan-2-yl] methyl}-1H-1,3-benzodiazole-6-carboxylic acid |
| 396 | 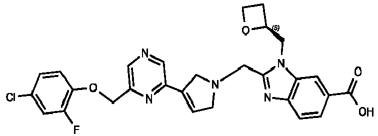 | 2-[(3-{6-[(4-chloro-2-fluorophenoxy)methyl]pyrazin-2-yl}-2,5-dihydro-1H-pyrrol-1-yl)methyl]-1-{[(2S)-oxetan-2-yl] methyl}-1H-1,3-benzodiazole-6-carboxylic acid |
| 397 | 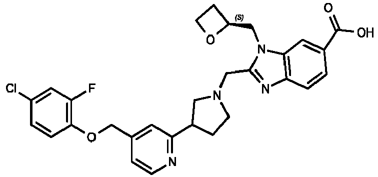 | 2-[(3-{4-[(4-chloro-2-fluorophenoxy)methyl]pyridin-2-yl}pyrrolidin-1-yl)methyl]-1-{[(2S)-oxetan-2-yl] methyl}-1H-1,3-benzodiazole-6-carboxylic acid |
| 398 | 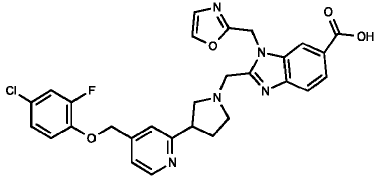 | 2-[(3-{4-[(4-chloro-2-fluorophenoxy)methyl]pyridin-2-yl}pyrrolidin-1-yl)methyl]-1-[(1,3-oxazol-2-yl)methyl]-1H-1,3-benzodiazole-6-carboxylic acid |
| 399 | 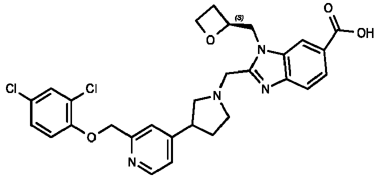 | 2-[(3-{2-[(2,4-dichlorophenoxy)methyl]pyridin-4-yl}pyrrolidin-1-yl)methyl]-1-{ [(2 S)-oxetan-2-yl] methyl}-1H-1,3-benzodiazole-6-carboxylic acid |
| 400 | 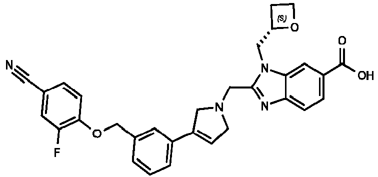 | 2-[(3-{3-[(4-cyano-2-fluorophenoxy)methyl]phenyl}-2,5-dihydro-1H-pyrrol-1-yl)methyl]-1-{[(2S)-oxetan-2-yl] methyl}-1H-1,3-benzodiazole-6-carboxylic acid |
| 401 | 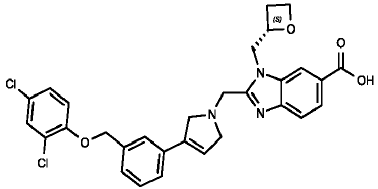 | 2-[(3-{3-[(2,4-dichlorophenoxy)methyl]phenyl}-2,5-dihydro-1H-pyrrol-1-yl)methyl]-1-{[(2S)-oxetan-2-yl]methyl}-1H-1,3-benzodiazole-6-carboxylic acid |
| 402 | 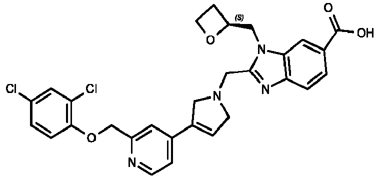 | 2-[(3-{2-[(2,4-dichlorophenoxy)methyl]pyridin-4-yl}-2,5-dihydro-1H-pyrrol-1-yl)methyl]-1-{[(2S)-oxetan-2-yl]methyl}-1H-1,3-benzodiazole-6-carboxylic acid |
| 403 | 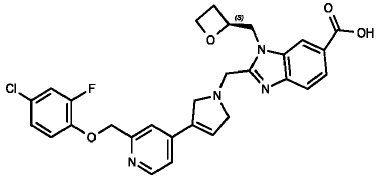 | 2-[(3-{2-[(4-chloro-2-fluorophenoxy)methyl]pyridin-4-yl}-2,5-dihydro-1H-pyrrol-1-yl)methyl]-1-{[(2S)-oxetan-2-yl]methyl}-1H-1,3-benzodiazole-6-carboxylic acid |
| 404 | 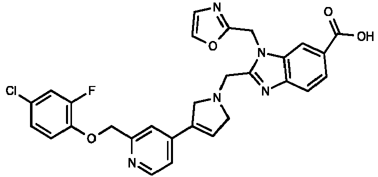 | 2-[(3-{2-[(4-chloro-2-fluorophenoxy)methyl]pyridin-4-yl}-2,5-dihydro-1H-pyrrol-1-yl)methyl]-1-[(1,3-oxazol-2-yl)methyl]-1H-1,3-benzodiazole-6-carboxylic acid |
| 405 | 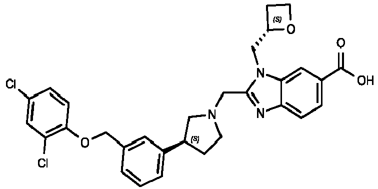 | 2-{[(3S)-3-{3-[(2,4-dichlorophenoxy)methyl]phenyl}pyrrolidin-1-yl] methyl}-1-{[(2S)-oxetan-2-yl]methyl}-1H-1,3-benzodiazole-6-carboxylic acid |
| 406 | 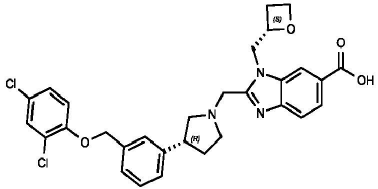 | 2-{[(3R)-3-{3-[(2,4-dichlorophenoxy)methyl]phenyl}pyrrolidin-1-yl]methyl}-1-{[(2S)-oxetan-2-yl]methyl}-1H-1,3-benzodiazole-6-carboxylic acid |
| 407 | 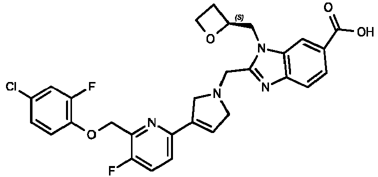 | 2-[(3-{6-[(4-chloro-2-fluorophenoxy)methyl]-5-fluoropyridin-2-yl}-2,5-dihydro-1H-pyrrol-1-yl)methyl]-1-{[(2S)-oxetan-2-yl]methyl}-1H-1,3-benzodiazole-6-carboxylic acid |
| 408 | 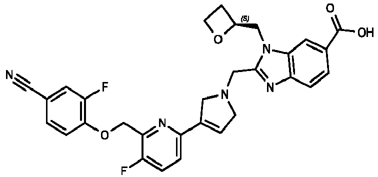 | 2-[(3-{6-[(4-cyano-2-fluorophenoxy)methyl]-5-fluoropyridin-2-yl}-2,5-dihydro-1H-pyrrol-1-yl)methyl]-1-{[(2S)-oxetan-2-yl]methyl}-1H-1,3-benzodiazole-6-carboxylic acid |
| 409 | 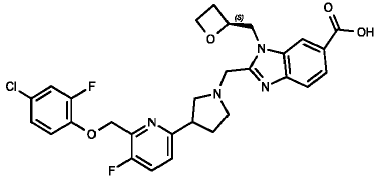 | 2-[(3-{6-[(4-chloro-2-fluorophenoxy)methyl]-5-fluoropyridin-2-yl}pyrrolidin-1-yl)methyl]-1-{[(2S)-oxetan-2-yl]methyl}-1H-1,3-benzodiazole-6-carboxylic acid |
| 410 | 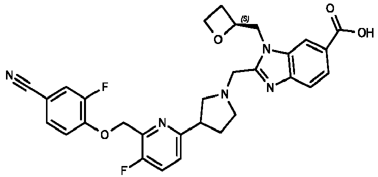 | 2-[(3-{6-[(4-cyano-2-fluorophenoxy)methyl]-5-fluoropyridin-2-yl}pyrrolidin-1-yl)methyl]-1-{[(2S)-oxetan-2-yl]methyl}-1H-1,3-benzodiazole-6-carboxylic acid |
| 411 | 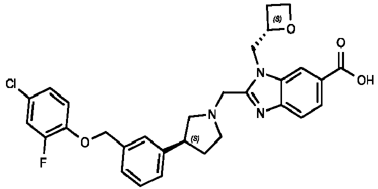 | 2-{[(3S)-3-{3-[(4-chloro-2-fluorophenoxy)methyl]phenyl}pyrrolidin-1-yl]methyl}-1-{[(2S)-oxetan-2-yl]methyl}-1H-1,3-benzodiazole-6-carboxylic acid |
| 412 | 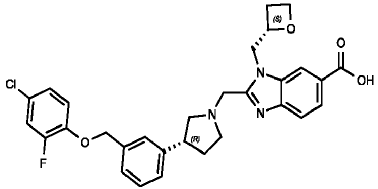 | 2-{[(3R)-3-{3-[(4-chloro-2-fluorophenoxy)methyl]phenyl}pyrrolidin-1-yl]methyl}-1-{[(2S)-oxetan-2-yl]methyl}-1H-1,3-benzodiazole-6-carboxylic acid |
| 413 | 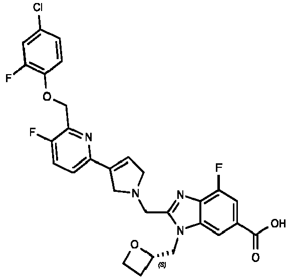 | 2-[(3-{6-[(4-chloro-2-fluorophenoxy)methyl]-5-fluoropyridin-2-yl}-2,5-dihydro-1H-pyrrol-1-yl)methyl]-4-fluoro-1-{[(2S)-oxetan-2-yl] methyl}-1H-1,3-benzodiazole-6-carboxylic acid |
| 414 | 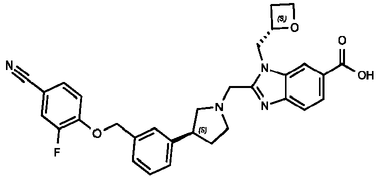 | 2-{[(3S)-3-{3-[(4-cyano-2-fluorophenoxy)methyl]phenyl}pyrrolidin-1-yl]methyl}-1-{[(2S)-oxetan-2-yl]methyl}-1H-1,3-benzodiazole-6-carboxylic acid |
| 415 | 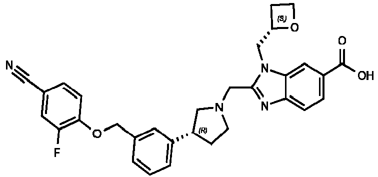 | 2-{[(3R)-3-{3-[(4-cyano-2-fluorophenoxy)methyl]phenyl}pyrrolidin-1-yl]methyl}-1-{[(2S)-oxetan-2-yl]methyl}-1H-1,3-benzodiazole-6-carboxylic acid |
| 416 | 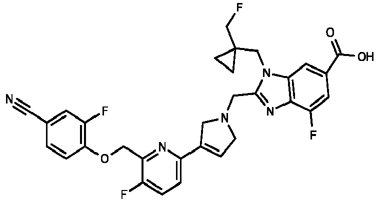 | 2-[(3-{6-[(4-cyano-2-fluorophenoxy)methyl]-5-fluoropyridin-2-yl}-2,5-dihydro-1H-pyrrol-1-yl)methyl]-4-fluoro-1-{[1-(fluoromethyl)cyclopropyl]methyl}-1H-1,3-benzodiazole-6-carboxylic acid |
| 417 | 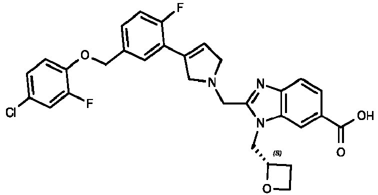 | 2-[(3-{5-[(4-chloro-2-fluorophenoxy)methyl]-2-fluorophenyl}-2,5-dihydro-1H-pyrrol-1-yl)methyl]-1-{[(2S)-oxetan-2-yl]methyl}-1H-1,3-benzodiazole-6-carboxylic acid |
| 418 | 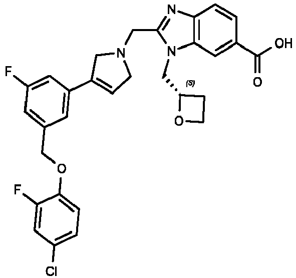 | 2-[(3-{3-[(4-chloro-2-fluorophenoxy)methyl]-5-fluorophenyl}-2,5-dihydro-1H-pyrrol-1-yl)methyl]-1-{[(2S)-oxetan-2-yl]methyl}-1H-1,3-benzodiazole-6-carboxylic acid |
| 419 | 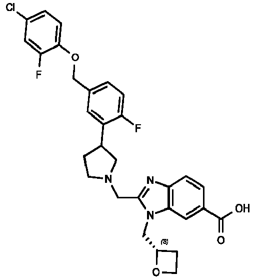 | 2-[(3-{5-[(4-chloro-2-fluorophenoxy)methyl]-2-fluorophenyl}pyrrolidin-1-yl)methyl]-1-{[(2S)-oxetan-2-yl]methyl}-1H-1,3-benzodiazole-6-carboxylic acid |
| 420 | 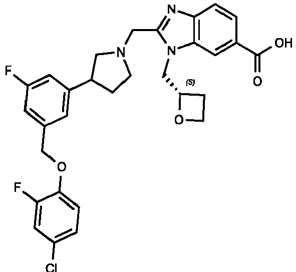 | 2-[(3-{3-[(4-chloro-2-fluorophenoxy)methyl]-5-fluorophenyl}pyrrolidin-1-yl)methyl]-1-{[(2S)-oxetan-2-yl]methyl}-1H-1,3-benzodiazole-6-carboxylic acid |
| 421 | 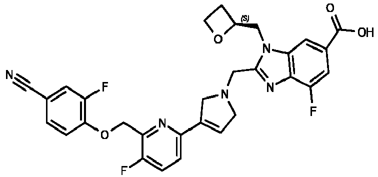 | 2-[(3-{6-[(4-cyano-2-fluorophenoxy)methyl]-5-fluoropyridin-2-yl}-2,5-dihydro-1H-pyrrol-1-yl)methyl]-4-fluoro-1-{[(2S)-oxetan-2-yl]methyl}-1H-1,3-benzodiazole-6-carboxylic acid |
| 422 | 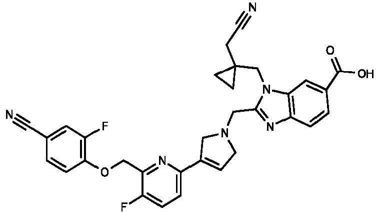 | 2-[(3-{6-[(4-cyano-2-fluorophenoxy)methyl] -5-fluoropyridin-2-yl}-2,5-dihydro-1H-pyrrol-1-yl)methyl]-1-{[1-(cyanomethyl)cyclopropyl]methyl}-1H-1,3-benzodiazole-6-carboxylic acid |
| 423 | 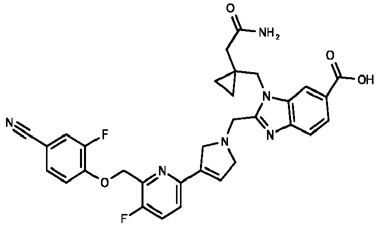 | 1-{[1-(carbamoylmethyl)cyclopropyl]methyl}-2-[(3-{6-[(4-cyano-2-fluorophenoxy)methyl]-5-fluoropyridin-2-yl}-2,5-dihydro-1H-pyrrol-1-yl)methyl]-1H-1,3-benzodiazole-6-carboxylic acid |
| 424 | 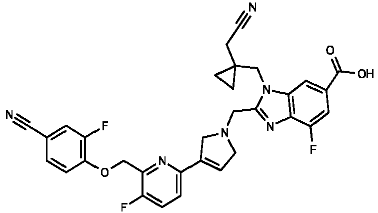 | 2-[(3-{6-[(4-cyano-2-fluorophenoxy)methyl] -5-fluoropyridin-2-yl}-2,5-dihydro-1H-pyrrol-1-yl)methyl]-1-{ [1-(cyanomethyl)cyclopropyl] methyl}-4-fluoro-1H-1,3-benzodiazole-6-carboxylic acid |
| 425 | 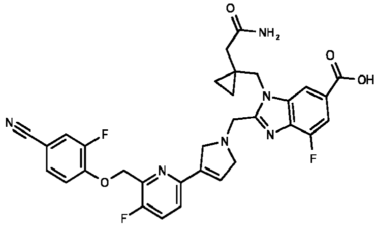 | 1-{[1-(carbamoylmethyl)cyclopropyl]methyl}-2-[(3-{6-[(4-cyano-2-fluorophenoxy)methyl]-5-fluoropyridin-2-yl}-2,5-dihydro-1H-pyrrol-1-yl)methyl]-4-fluoro-1H-1,3-benzodiazole-6-carboxylic acid |
| 426 | 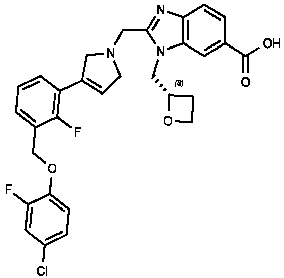 | 2-[(3-{3-[(4-chloro-2-fluorophenoxy)methyl]-2-fluorophenyl}-2,5-dihydro-1H-pyrrol-1-yl)methyl]-1-{[(2S)-oxetan-2-yl]methyl}-1H-1,3-benzodiazole-6-carboxylic acid |
| 427 | 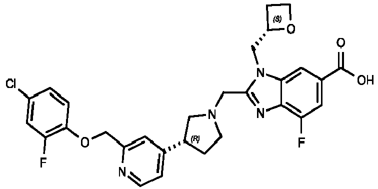 | 2-1[(3R)-3-12-[(4-chloro-2-fluorophenoxy)methyl]pyridin-4-yl}pyrrolidin-1-yl]methyl}-4-fluoro-1-{[(2S)-oxetan-2-yl]methyl}-1H-1,3-benzodiazole-6-carboxylic acid |
| 428 | 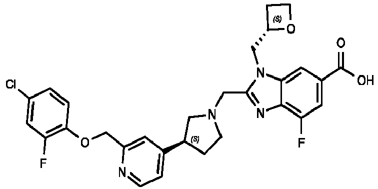 | 2-{[(3S)-3-{2-[(4-chloro-2-fluorophenoxy)methyl]pyridin-4-yl}pyrrolidin-1-yl]methyl}-4-fluoro-1-{[(2S)-oxetan-2-yl]methyl}-1H-1,3-benzodiazole-6-carboxylic acid |
| 429 | 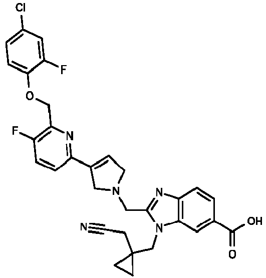 | 2-[(3-{6-[(4-chloro-2-fluorophenoxy)methyl]-5-fluoropyridin-2-yl}-2,5-dihydro-1H-pyrrol-1-yl)methyl]-1-{[1-(cyanomethyl)cyclopropyl]methyl}-1H-1,3-benzodiazole-6-carboxylic acid |
| 430 | 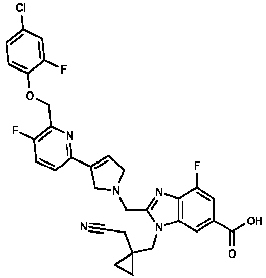 | 2-[(3-{6-[(4-chloro-2-fluorophenoxy)methyl]-5-fluoropyridin-2-yl}-2,5-dihydro-1H-pyrrol-1-yl)methyl]-1-{[1-(cyanomethyl)cyclopropyl]methyl}-4-fluoro-1H-1,3-benzodiazole-6-carboxylic acid |
| 431 | 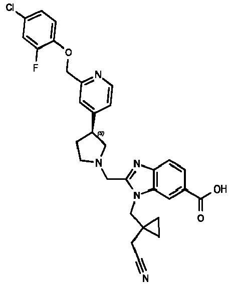 | 2-{[(3S)-3-{2-[(4-chloro-2-fluorophenoxy)methyl]pyridin-4-yl}pyrrolidin-1-yl]methyl}-1-{[1-(cyanomethyl)cyclopropyl]methyl}-1H-1,3-benzodiazole-6-carboxylic acid |
| 432 | 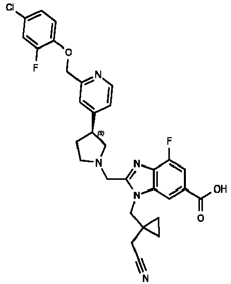 | 2-{[(3S)-3-{2-[(4-chloro-2-fluorophenoxy)methyl]pyridin-4-yl}pyrrolidin-1-yl]methyl}-1-{[1-(cyanomethyl)cyclopropyl]methyl}-4-fluoro-1H-1,3-benzodiazole-6-carboxylic acid |
| 433 | 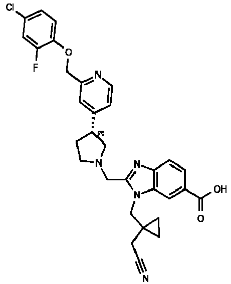 | 2-1[(3R)-3-12-[(4-chloro-2-fluorophenoxy)methyl]pyridin-4-yl}pyrrolidin-1-yl]methyl}-1-{[1-(cyanomethyl)cyclopropyl]methyl}-1H-1,3-benzodiazole-6-carboxylic acid |
| 434 | 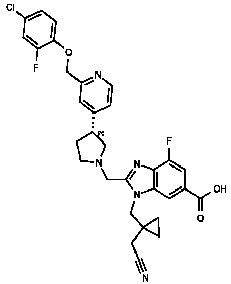 | 2-{[(3R)-3-{2-[(4-chloro-2-fluorophenoxy)methyl]pyridin-4-yl}pyrrolidin-1-yl]methyl}-1-{[1-(cyanomethyl)cyclopropyl]methyl}-4-fluoro-1H-1,3-benzodiazole-6-carboxylic acid |
| 435 | 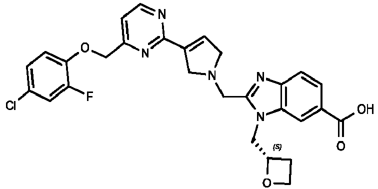 | 2-[(3-{4-[(4-chloro-2-fluorophenoxy)methyl]pyrimidin-2-yl}-2,5-dihydro-1H-pyrrol-1-yl)methyl]-1-{[(2S)-oxetan-2-yl]methyl}-1H-1,3-benzodiazole-6-carboxylic acid |
| 436 | 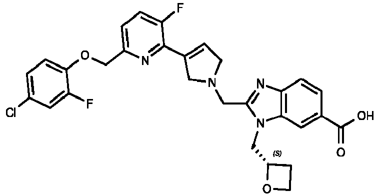 | 2-[(3-{6-[(4-chloro-2-fluorophenoxy)methyl]-3-fluoropyridin-2-yl}-2,5-dihydro-1H-pyrrol-1-yl)methyl]-1-{[(2S)-oxetan-2-yl]methyl}-1H-1,3-benzodiazole-6-carboxylic acid |
| 437 | 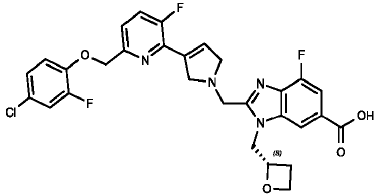 | 2-[(3-{6-[(4-chloro-2-fluorophenoxy)methyl]-3-fluoropyridin-2-yl}-2,5-dihydro-1H-pyrrol-1-yl)methyl]-4-fluoro-1-{[(2S)-oxetan-2-yl]methyl}-1H-1,3-benzodiazole-6-carboxylic acid |
| 438 | 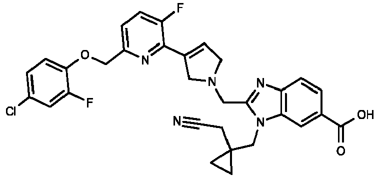 | 2-[(3-{6-[(4-chloro-2-fluorophenoxy)methyl]-3-fluoropyridin-2-yl}-2,5-dihydro-1H-pyrrol-1-yl)methyl]-1-{[1-(cyanomethyl)cyclopropyl]methyl}-1H-1,3-benzodiazole-6-carboxylic acid |
| 439 | 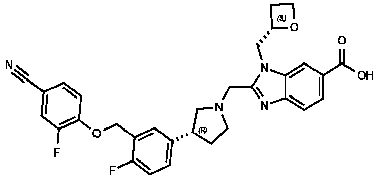 | 2-{[(3R)-3-{3-[(4-cyano-2-fluorophenoxy)methyl]-4-fluorophenyl}pyrrolidin-1-yl]methyl}-1-{[(2S)-oxetan-2-yl]methyl}-1H-1,3-benzodiazole-6-carboxylic acid |
| 440 | 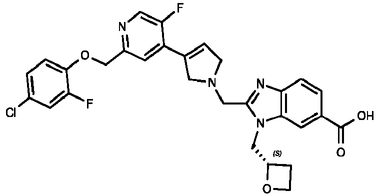 | 2-[(3-{2-[(4-chloro-2-fluorophenoxy)methyl]-5-fluoropyridin-4-yl}-2,5-dihydro-1H-pyrrol-1-yl)methyl]-1-{[(2S)-oxetan-2-yl]methyl}-1H-1,3-benzodiazole-6-carboxylic acid |
| 441 | 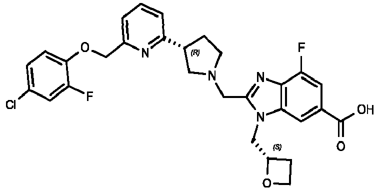 | 2-{[(3R)-3-{6-[(4-chloro-2-fluorophenoxy)methyl]pyridin-2-yl}pyrrolidin-1-yl]methyl}-4-fluoro-1-{[(2S)-oxetan-2-yl]methyl}-1H-1,3-benzodiazole-6-carboxylic acid |
| 442 | 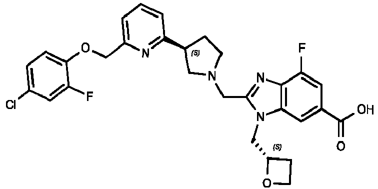 | 2-{[(3S)-3-{6-[(4-chloro-2-fluorophenoxy)methyl]pyridin-2-yl}pyrrolidin-1-yl]methyl}-4-fluoro-1-{[(2S)-oxetan-2-yl]methyl}-1H-1,3-benzodiazole-6-carboxylic acid |
| 443 | 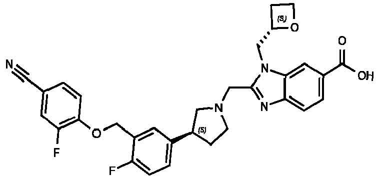 | 2-{[(3S)-3-{3-[(4-cyano-2-fluorophenoxy)methyl]-4-fluorophenyl}pyrrolidin-1-yl]methyl}-1-{[(2S)-oxetan-2-yl]methyl}-1H-1,3-benzodiazole-6-carboxylic acid |
| 444 | 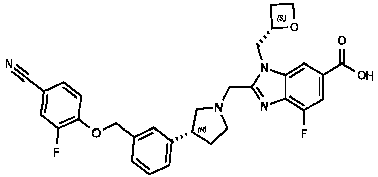 | 2-{[(3R)-3-{3-[(4-cyano-2-fluorophenoxy)methyl]phenyl}pyrrolidin-1-yl]methyl}-4-fluoro-1-{[(2S)-oxetan-2-yl]methyl}-1H-1,3-benzodiazole-6-carboxylic acid |
| 445 | 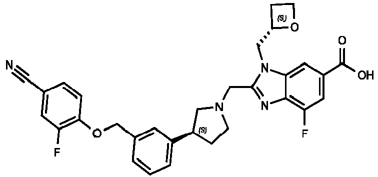 | 2-{[(3S)-3-{3-[(4-cyano-2-fluorophenoxy)methyl]phenyl}pyrrolidin-1-yl]methyl}-4-fluoro-1-{[(2S)-oxetan-2-yl]methyl}-1H-1,3-benzodiazole-6-carboxylic acid |
| 446 | 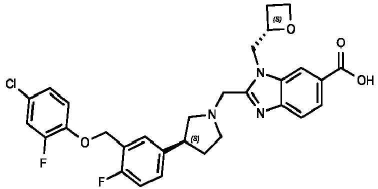 | 2-{[(3S)-3-{3-[(4-chloro-2-fluorophenoxy)methyl]-4-fluorophenyl}pyrrolidin-1-yl]methyl}-1-{[(2S)-oxetan-2-yl]methyl}-1H-1,3-benzodiazole-6-carboxylic acid |
| 447 | 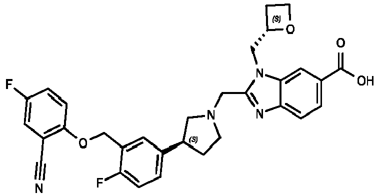 | 2-{[(3S)-3-{3-[(2-cyano-4-fluorophenoxy)methyl]-4-fluorophenyl}pyrrolidin-1-yl]methyl}-1-{[(2S)-oxetan-2-yl]methyl}-1H-1,3-benzodiazole-6-carboxylic acid |
| 448 | 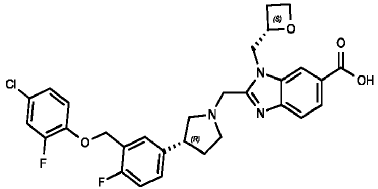 | 2-{[(3R)-3-{3-[(4-chloro-2-fluorophenoxy)methyl]-4-fluorophenyl}pyrrolidin-1-yl]methyl}-1-{[(2S)-oxetan-2-yl]methyl}-1H-1,3-benzodiazole-6-carboxylic acid |
| 449 | 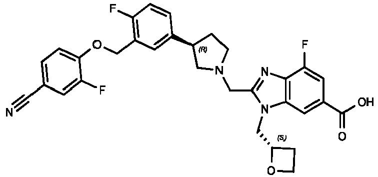 | 2-{[(3R)-3-{3-[(4-cyano-2-fluorophenoxy)methyl]-4-fluorophenyl}pyrrolidin-1-yl]methyl}-4-fluoro-1-{[(2S)-oxetan-2-yl]methyl}-1H-1,3-benzodiazole-6-carboxylic acid |
| 450 | 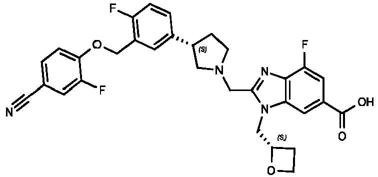 | 2-{[(3S)-3-{3-[(4-cyano-2-fluorophenoxy)methyl]-4-fluorophenyl}pyrrolidin-1-yl]methyl}-4-fluoro-1-{[(2S)-oxetan-2-yl]methyl}-1H-1,3-benzodiazole-6-carboxylic acid |
| 451 | 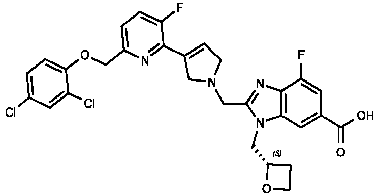 | 2-[(3-16-[(2,4-dichlorophenoxy)methyl]-3-fluoropyridin-2-yl}-2,5-dihydro-1H-pyrrol-1-yl)methyl]-4-fluoro-1-{[(2S)-oxetan-2-yl]methyl}-1H-1,3-benzodiazole-6-carboxylic acid |
| 452 | 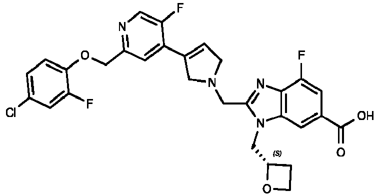 | 2-[(3-16-[(2,4-dichlorophenoxy)methyl]-3-fluoropyridin-2-yl}-2,5-dihydro-1H-pyrrol-1-yl)methyl]-4-fluoro-1-{[(2S)-oxetan-2-yl]methyl}-1H-1,3-benzodiazole-6-carboxylic acid |
| 453 | 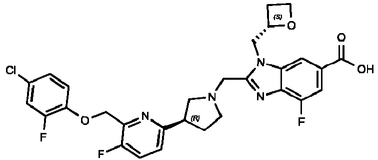 | 2-{[(3R)-3-{6-[(4-chloro-2-fluorophenoxy)methyl]-5-fluoropyridin-2-yl}pyrrolidin-1-yl]methyl}-4-fluoro-1-{[(2S)-oxetan-2-yl]methyl}-1H-1,3-benzodiazole-6-carboxylic acid |
| 454 | 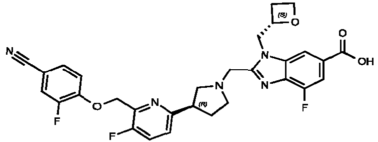 | 2-{[(3R)-3-{6-[(4-cyano-2-fluorophenoxy)methyl]-5-fluoropyridin-2-yl}pyrrolidin-1-yl]methyl}-4-fluoro-1-{[(2S)-oxetan-2-yl]methyl}-1H-1,3-benzodiazole-6-carboxylic acid |
| 455 | 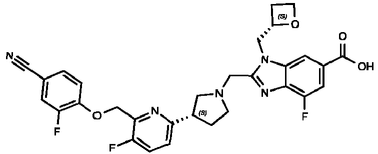 | 2-{[(3S)-3-{6-[(4-cyano-2-fluorophenoxy)methyl]-5-fluoropyridin-2-yl}pyrrolidin-1-yl]methyl}-4-fluoro-1-{[(2S)-oxetan-2-yl]methyl}-1H-1,3-benzodiazole-6-carboxylic acid |
| 456 | 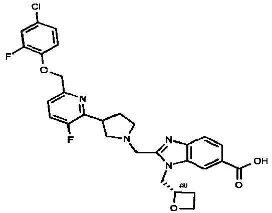 | 2-[(3-{6-[(4-chloro-2-fluorophenoxy)methyl]-3-fluoropyridin-2-yl}pyrrolidin-1-yl)methyl]-1-{[(2S)-oxetan-2-yl]methyl}-1H-1,3-benzodiazole-6-carboxylic acid |
| 457 | 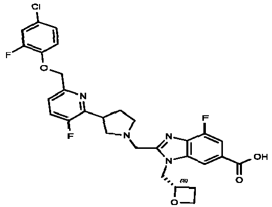 | 2-[(3-{6-[(4-chloro-2-fluorophenoxy)methyl]-3-fluoropyridin-2-yl}pyrrolidin-1-yl)methyl]-4-fluoro-1-{[(2S)-oxetan-2-yl]methyl}-1H-1,3-benzodiazole-6-carboxylic acid |
| 458 | 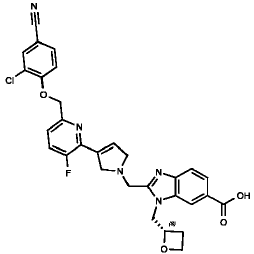 | 2-[(3-{6-[(2-chloro-4-cyanophenoxy)methyl]-3-fluoropyridin-2-yl}-2,5-dihydro-1H-pyrrol-1-yl)methyl]-1-{[(2S)-oxetan-2-yl]methyl}-1H-1,3-benzodiazole-6-carboxylic acid |
| 459 | 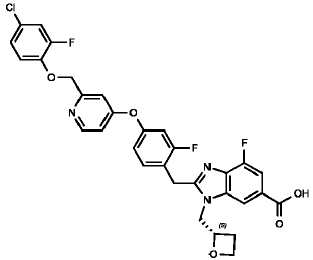 | 2-{[4-({2-[(4-chloro-2-fluorophenoxy)methyl]pyridin-4-yl}oxy)-2-fluorophenyl]methyl}-4-fluoro-1-{[(2S)-oxetan-2-yl]methyl}-1H-1,3-benzodiazole-6-carboxylic acid |
| 460 | 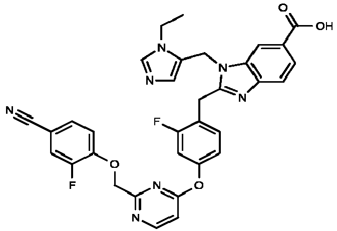 | 2-{[4-({2-[(4-cyano-2-fluorophenoxy)methyl]pyrimidin-4-yl}oxy)-2-fluorophenyl]methyl}-1-[(1-ethyl-1H-imidazol-5-yl)methyl]-1H-1,3-benzodiazole-6-carboxylic acid |
| 461 | 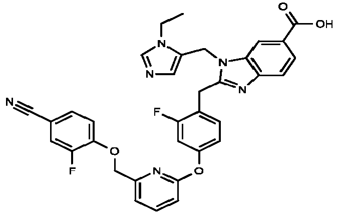 | 2-{[4-({6-[(4-cyano-2-fluorophenoxy)methyl]pyridin-2-yl}oxy)-2-fluorophenyl]methyl}-1-[(1-ethyl-1H-imidazol-5-yl)methyl]-1H-1,3-benzodiazole-6-carboxylic acid |
| 462 | 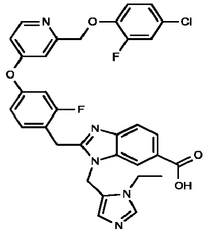 | 2-{[4-({2-[(4-chloro-2-fluorophenoxy)methyl]pyridin-4-yl}oxy)-2-fluorophenyl]methyl}-1-[(1-ethyl-1H-imidazol-5-yl)methyl]-1H-1,3-benzodiazole-6-carboxylic acid |
| 463 | 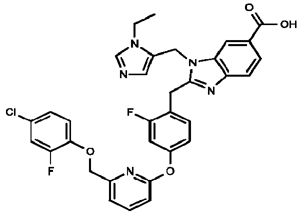 | 2-{[4-({6-[(4-chloro-2-fluorophenoxy)methyl]pyridin-2-yl}oxy)-2-fluorophenyl]methyl)-1-[(1-ethyl-1H-imidazol-5-yl)methyl]- 1H-1,3-benzodiazole-6-carboxylic acid |
| 464 | 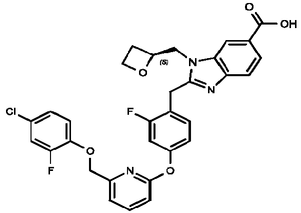 | 2-{[4-({6-[(4-chloro-2-fluorophenoxy)methyl]pyridin-2-yl}oxy)-2-fluorophenyl]methyl)-1-{[(2S)-oxetan-2-yl]methyl}-1H-1,3-benzodiazole-6-carboxylic acid |
| 465 | 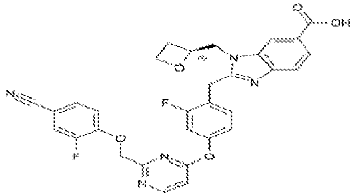 | 2-{[4-({2-[(4-cyano-2-fluorophenoxy)methyl]pyrimidin-4-yl}oxy)-2-fluorophenyl]methyl}-1-{[(2S)-oxetan-2-yl]methyl}-1H-1,3-benzodiazole-6-carboxylic acid |
| 466 | 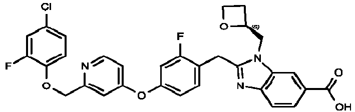 | 2-{[4-({2-[(4-chloro-2-fluorophenoxy)methyl]pyridin-4-yl}oxy)-2-fluorophenyl]methyl)-1-{[(2S)-oxetan-2-yl]methyl}-1H-1,3-benzodiazole-6-carboxylic acid |
| 467 | 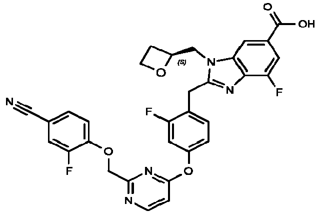 | 2-{[4-({6-[(4-cyano-2-fluorophenoxy)methyl]pyridin-2-yl}oxy)-2-fluorophenyl]methyl}-4-fluoro-1-{[(2S)-oxetan-2-yl]methyl}-1H-1,3-benzodiazole-6-carboxylic acid |
| 468 | 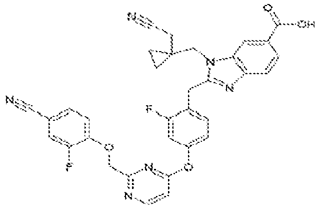 | 2-{[4-({2-[(4-cyano-2-fluorophenoxy)methyl]pyrimidin-4-yl}oxy)-2-fluorophenyl]methyl}-1-{[1-(cyanomethyl)cyclopropyl]methyl}-1H-1,3-benzodiazole-6-carboxylic acid |
| 469 | 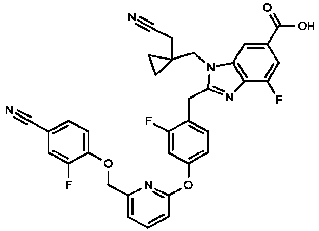 | 2-{[4-({6-[(4-cyano-2-fluorophenoxy)methyl]pyridin-2-yl}oxy)-2-fluorophenyl]methyl}-1-{[1-(cyanomethyl)cyclopropyl]methyl}-4-fluoro-1H-1,3-benzodiazole-6-carboxylic acid |
| 470 | 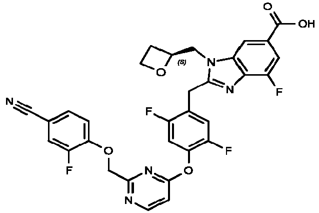 | 2-{[4-({2-[(4-cyano-2-fluorophenoxy)methyl]pyrimidin-4-yl}oxy)-2,5-difluorophenyl]methyl}-4-fluoro-1-{[(2S)-oxetan-2-yl]methyl}-1H-1,3-benzodiazole-6-carboxylic acid |
| 471 | 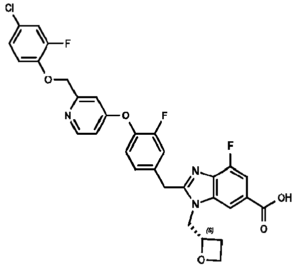 | 2-{[4-({2-[(4-chloro-2-fluorophenoxy)methyl]pyridin-4-yl} oxy)-3-fluorophenyl]methyl)-4-fluoro-1-{[(2S)-oxetan-2-yl]methyl}-1H-1,3-benzodiazole-6-carboxylic acid |
| 472 | 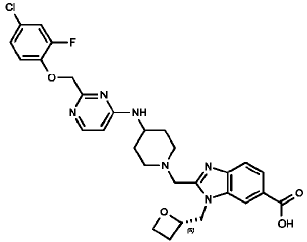 | 2-{[4-({2-[(4-chloro-2-fluorophenoxy)methyl]pyrimidin-4-yl}amino)piperidin-1-yl]methyl}-1-{[(2S)-oxetan-2-yl]methyl}-1H-1,3-benzodiazole-6-carboxylic acid |
| 473 | 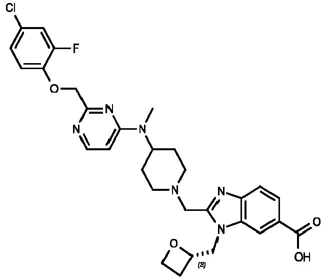 | 2-{[4-({2-[(4-chloro-2-fluorophenoxy)methyl]pyrimidin-4-yl}(methyl)amino)piperidin-1-yl]methyl}-1-{[(2S)-oxetan-2-yl]methyl}-1H-1,3-benzodiazole-6-carboxylic acid |
| 474 | 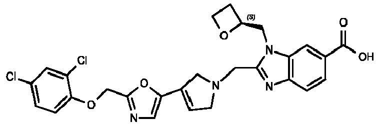 | 2-[(3-{2-[(2,4-dichlorophenoxy)methyl]-1,3-oxazol-5-yl}-2,5-dihydro-1H-pyrrol-1-yl)methyl]-1-{[(2S)-oxetan-2-yl]methyl}-1H-1,3-benzodiazole-6-carboxylic acid |
| 516 | 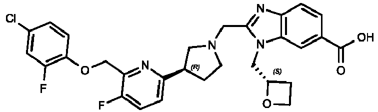 | 2-{[(3R)-3-{6-[(4-chloro-2-fluorophenoxy)methyl]-5-fluoropyridin-2-yl}pyrrolidin-1-yl]methyl}-1-{[(2S)-oxetan-2-yl]methyl}-1H-1,3-benzodiazole-6-carboxylic acid |
| 517 | 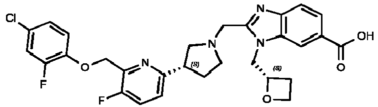 | 2-{[(3S)-3-{6-[(4-chloro-2-fluorophenoxy)methyl]-5-fluoropyridin-2-yl}pyrrolidin-1-yl]methyl}-1-{[(2S)-oxetan-2-yl]methyl}-1H-1,3-benzodiazole-6-carboxylic acid |
| 518 | 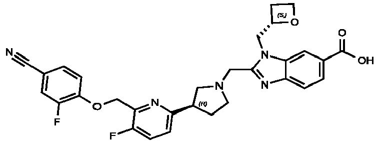 | 2-{[(3R)-3-{6-[(4-cyano-2-fluorophenoxy)methyl] -5-fluoropyridin-2-yl}pyrrolidin-1-yl]methyl}-1-{[(2S)-oxetan-2-yl]methyl}-1H-1,3-benzodiazole-6-carboxylic acid |
| 519 | 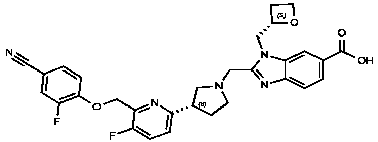 | 2-{[(3S)-3-{6-[(4-cyano-2-fluorophenoxy)methyl]-5-fluoropyridin-2-yl}pyrrolidin-1-yl]methyl}-1-{[(2S)-oxetan-2-yl] methyl}-1H-1,3-benzodiazole-6-carboxylic acid |
| 520 | 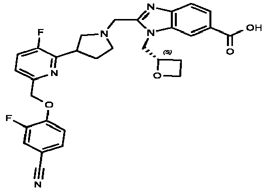 | 2-[(3-{6-[(4-cyano-2-fluorophenoxy)methyl]-3-fluoropyridin-2-yl}pyrrolidin-1-yl)methyl]-1-{ [(2 S)-oxetan-2-yl] methyl}-1H-1,3-benzodiazole-6-carboxylic acid |
| 521 | 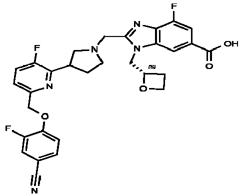 | 2-[(3-{6-[(4-cyano-2-fluorophenoxy)methyl]-3-fluoropyridin-2-yl}pyrrolidin-1-yl)methyl]-4-fluoro-1-{[(2S)-oxetan-2-yl]methyl}-1H-1,3-benzodiazole-6-carboxylic acid |
| 522 | 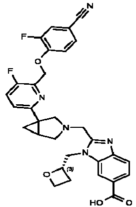 | 2-[(1-{6-[(4-cyano-2-fluorophenoxy)methyl]-5-fluoropyridin-2-yl}-3-azabicyclo[3.1.0]hexan-3-yl)methyl]-1-{[(2S)-oxetan-2-yl]methyl}-1H-1,3-benzodiazole-6-carboxylic acid |
| 523 | 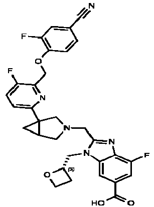 | 2-[(1-{6-[(4-cyano-2-fluorophenoxy)methyl]-5-fluoropyridin-2-yl}-3-azabicyclo[3.1.0]hexan-3-yl)methyl]-4-fluoro-1-{[(2S)-oxetan-2-yl]methyl}-1H-1,3-benzodiazole-6-carboxylic acid |
| 524 | 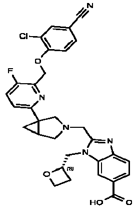 | 2-[(1-{6-[(2-chloro-4-cyanophenoxy)methyl]-5-fluoropyridin-2-yl}-3 -azabicyclo[3.1.0] hexan-3-yl)methyl]-1-{[(2S)-oxetan-2-yl]methyl}-1H-1,3-benzodiazole-6-carboxylic acid |
| 525 | 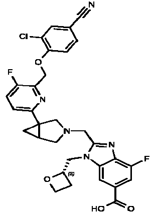 | 2-[(1-16-[(2-chloro-4-cyanophenoxy)methyl]-5-fluoropyridin-2-yl}-3-azabicyclo[3.1.0]hexan-3-yl)methyl]-4-fluoro-1-{[(2S)-oxetan-2-yl]methyl}-1H-1,3-benzodiazole-6-carboxylic acid |
| 526 | 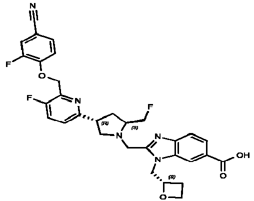 | 2-{[(2S,4R)-4-{6-[(4-cyano-2-fluorophenoxy)methyl]-5-fluoropyridin-2-yl}-2-(fluoromethyl)pyrrolidin-1-yl]methyl}-1-{[(2S)-oxetan-2-yl]methyl}-1H-1,3-benzodiazole-6-carboxylic acid |
| 527 | 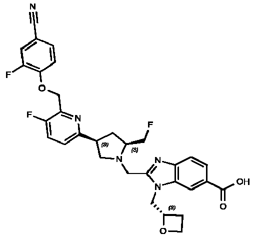 | 2-{[(2S,4S)-4-{6-[(4-cyano-2-fluorophenoxy)methyl]-5-fluoropyridin-2-yl}-2-(fluoromethyl)pyrrolidin-1-yl]methyl}-1-{[(2S)-oxetan-2-yl]methyl}-1H-1,3-benzodiazole-6-carboxylic acid |
| 528 | 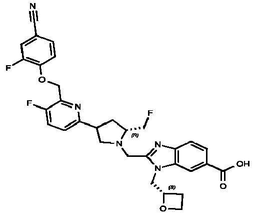 | 2-{[(2R)-4-{6-[(4-cyano-2-fluorophenoxy)methyl]-5-fluoropyridin-2-yl}-2-(fluoromethyl)pyrrolidin-1-yl]methyl}-1-{[(2S)-oxetan-2-yl]methyl}-1H-1,3-benzodiazole-6-carboxylic acid |
| 531 | 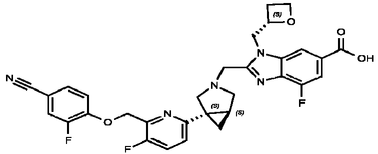 | 2-{[(1S,5S)-1-{6-[(4-cyano-2-fluorophenoxy)methyl]-5-fluoropyridin-2-yl}-3-azabicyclo[3.1.0]hexan-3-yl]methyl}-4-fluoro-1-{[(2S)-oxetan-2-yl] methyl}-1H-1,3-benzodiazole-6-carboxylic acid |
| 532 | 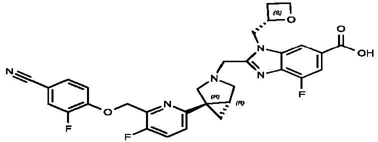 | 2-{[(1R,5R)-1-{6-[(4-cyano-2-fluorophenoxy)methyl]-5-fluoropyridin-2-yl}-3-azabicyclo[3.1.0]hexan-3-yl]methyl}-4-fluoro-1-{[(2S)-oxetan-2-yl]methyl}-1H-1,3-benzodiazole-6-carboxylic acid |
| 536 | 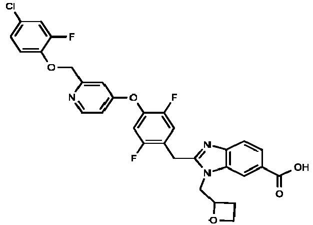 | 2-{[4-({2-[(4-chloro-2-fluorophenoxy)methyl]pyridin-4-yl}oxy)-2,5-difluorophenyl]methyl}-1-[(oxetan-2-yl)methyl]-1H-1,3-benzodiazole-6-carboxylic acid |
| 537 | 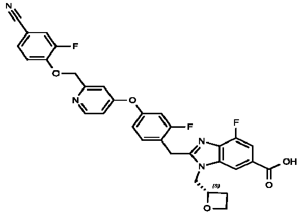 | 2-{[4-({2-[(4-cyano-2-fluorophenoxy)methyl]pyridin-4-yl}oxy)-2-fluorophenyl]methyl}-4-fluoro-1-{[(2S)-oxetan-2-yl]methyl}-1H-1,3-benzodiazole-6-carboxylic acid |
| 538 | 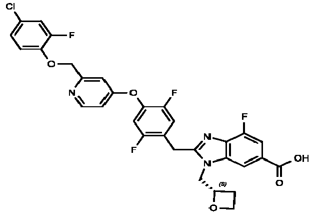 | 2-{[4-({2-[(4-chloro-2-fluorophenoxy) methyl] pyridin-4-yl}oxy)-2,5-difluorophenyl]methyl}-4-fluoro-1-{[(2S)-oxetan-2-yl]methyl}-1H-1,3-benzodiazole-6-carboxylic acid |
| 539 | 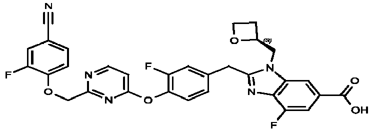 | 2-{[4-({2-[(4-cyano-2-fluorophenoxy)methyl]pyrimidin-4-yl}oxy)-3-fluorophenyl]methyl}-4-fluoro-1-{[(2S)-oxetan-2-yl]methyl}-1H-1,3-benzodiazole-6-carboxylic acid |
| 540 | 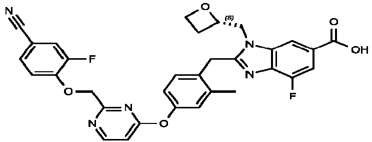 | 2-{[4-({2-[(4-cyano-2-fluorophenoxy)methyl]pyrimidin-4-yl}oxy)-2-methylphenyl]methyl}-4-fluoro-1-{[(2S)-oxetan-2-yl]methyl}-1H-1,3-benzodiazole-6-carboxylic acid |
| 541 | 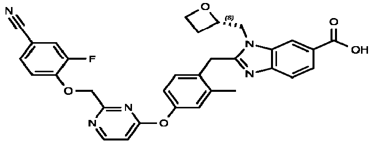 | 2-{[4-({2-[(4-cyano-2-fluorophenoxy)methyl]pyrimidin-4-yl}oxy)-2-methylphenyl]methyl}-1-{[(2S)-oxetan-2-yl]methyl}-1H-1,3-benzodiazole-6-carboxylic acid |
| 542 | 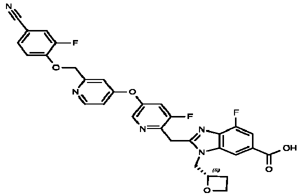 | 2-{[5-({2-[(4-cyano-2-fluorophenoxy)methyl]pyridin-4-yl]oxy)-3-fluoropyridin-2-yl]methyl}-4-fluoro-1-{[(2S)-oxetan-2-yl]methyl}-1H-1,3-benzodiazole-6-carboxylic acid |
| 543 | 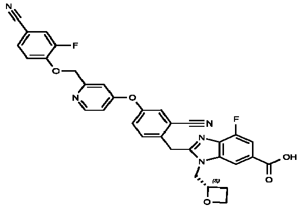 | 2-{[2-cyano-4-({2-[(4-cyano-2-fluorophenoxy)methyl]pyridin-4-yl}oxy)phenyl]methyl}-4-fluoro-1-{[(2S)-oxetan-2-yl]methyl}-1H-1,3-benzodiazole-6-carboxylic acid |
| 544 | 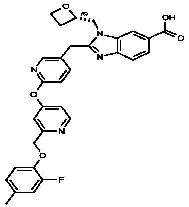 | 2-{[6-({2-[(2-fluoro-4-methylphenoxy)methyl]pyridin-4-yl}oxy)pyridin-3-yl]methy}-1-{[(2S)-oxetan-2-yl]methyl}-1H-1,3-benzodiazole-6-carboxylic acid |
| 545 | 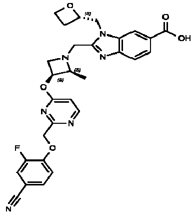 | 2-{[(2S,3S)-3-({2-[(4-cyano-2-fluorophenoxy)methyl]pyrimidin-4-yl}oxy)-2-methylazetidin-1-yl]methyl}-1-{[(2S)-oxetan-2-yl]methyl}-1H-1,3-benzodiazole-6-carboxylic acid |
| 546 | 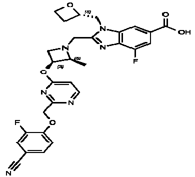 | 2-{[(2S,3S)-3-({2-[(4-cyano-2-fluorophenoxy)methyl]pyrimidin-4-yl}oxy)-2-methylazetidin-1-yl]methyl}-4-fluoro-1-{[(2S)-oxetan-2-yl]methyl}-1H-1,3-benzodiazole-6-carboxylic acid |
| 547 | 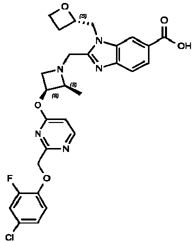 | 2-{[(2S,3S)-3-({2-[(4-chloro-2-fluorophenoxy)methyl]pyrimidin-4-yl}oxy)-2-methylazetidin-1-yl]methyl}-1-{[(2S)-oxetan-2-yl]methyl}-1H-1,3-benzodiazole-6-carboxylic acid |
| 548 | 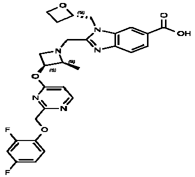 | 2-{[(2S,3S)-3-({2-[(2,4-difluorophenoxy)methyl]pyrimidin-4-yl}oxy)-2-methylazetidin-1-yl]methyl}-1-{[(2S)-oxetan-2-yl]methyl}-1H-1,3-benzodiazole-6-carboxylic acid |
| 549 | 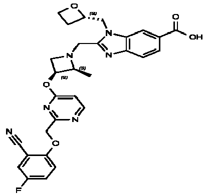 | 2-{[(2S,3S)-3-({2-[(2-cyano-4-fluorophenoxy)methyl]pyrimidin-4-yl}oxy)-2-methylazetidin-1-yl]methyl}-1-{[(2S)-oxetan-2-yl]methyl}-1H-1,3-benzodiazole-6-carboxylic acid |
| 550 | 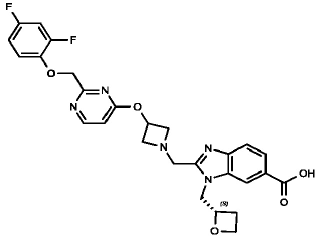 | 2-{[3-({2-[(2,4-difluorophenoxy)methyl]pyrimidin-4-yl}oxy)azetidin-1-yl]methyl}-1-{[(2S)-oxetan-2-yl]methyl}-1H-1,3-benzodiazole-6-carboxylic acid |

### Optically Active Compounds

In certain embodiments, compounds provided herein may have several chiral centers and may exist in and be isolated in optically active and racemic forms. In certain embodiments, some compounds may exhibit polymorphism. A person of skill in the art will appreciate that compounds provided herein can exist in any racemic, optically-active, diastereomeric, polymorphic, or stereoisomeric form, and/or mixtures thereof. A person of skill in the art will also appreciate that such compounds described herein that possess the useful properties also described herein is within the scope of this disclosure. A person of skill in the art will further appreciate how to prepare optically active forms of the compounds described herein, for example, by resolution of racemic forms via recrystallization techniques, by synthesis from optically-active starting materials, by chiral synthesis, or by chromatographic separation using a chiral stationary phase. In addition, most amino acids are chiral (i.e., designated as L- or D-, wherein the L- enantiomer is the naturally occurring configuration) and can exist as separate enantiomers.

Examples of methods to obtain optically active materials are known in the art, and include at least the following:
i) physical separation of crystals - a technique whereby macroscopic crystals of the individual enantiomers are manually separated. This technique can be used if crystals of the separate enantiomers exist (i.e., the material is a conglomerate, and the crystals are visually distinct);
ii) simultaneous crystallization - a technique whereby the individual enantiomers are separately crystallized from a solution of the racemate, only if the latter is a conglomerate in the solid state;
iii) enzymatic resolutions - a technique wherein partial, or complete separation of a racemate is accomplished by virtue of different rates of reaction of the enantiomers in the presence of an enzyme;
iv) enzymatic asymmetric synthesis - a synthetic technique wherein at least one step of the synthesis uses an enzymatic reaction to obtain an enantiomerically pure, or enriched synthetic precursor of the desired enantiomer;
v) chemical asymmetric synthesis - a synthetic technique wherein the desired enantiomer is synthesized from an achiral precursor using chiral catalysts, or chiral auxiliaries to produce asymmetry (i.e., chirality) in the product;
vi) diastereomer separations - a technique wherein a racemic compound is treated with an enantiomerically pure reagent (a chiral auxiliary) that converts the individual enantiomers to diastereomers. The resulting diastereomers are then separated by chromatography, or crystallization by virtue of their now more distinct diastereomeric differences, and then the chiral auxiliary is removed to obtain each enantiomer;
vii) first- and second-order asymmetric transformations - a technique wherein diastereomers of the racemate equilibrate in solution to yield a preponderance of a diastereomer of the desired enantiomer, or where kinetic, or thermodynamic crystallization of the diastereomer of the desired enantiomer perturbs the equilibrium such that eventually in principle all the material is converted to the crystalline diastereomer of the desired enantiomer. The desired enantiomer is then derived from the diastereomer;
viii) kinetic resolutions - this technique refers to the achievement of partial or complete resolution of a racemate (or of a further resolution of a partially resolved compound) by virtue of unequal reaction rates of the enantiomers with a chiral, or non-racemic reagent, or catalyst under kinetic conditions;
ix) enantiospecific synthesis from non-racemic precursors - a synthetic technique wherein the desired enantiomer is obtained from chiral starting materials, and where the stereochemical integrity is not or is only minimally compromised over the course of the synthesis;
x) chiral liquid chromatography - a technique wherein the enantiomers of a racemate are separated in a liquid mobile phase by virtue of their different interactions with a stationary phase. The stationary phase can be made of chiral material, or the mobile phase can contain an additional chiral material to provoke the different interactions;
xi) chiral gas chromatography - a technique wherein the racemate is volatilized and enantiomers are separated by virtue of their different interactions in the gaseous mobile phase with a column containing a fixed non-racemic adsorbent phase;
xii) extraction with chiral solvents - a technique wherein the enantiomers are separated by virtue of kinetic or thermodynamic dissolution of one enantiomer into a particular chiral solvent;
xiii) transport across chiral membranes - a technique wherein a racemate is placed in contact with a thin membrane barrier. The barrier typically separates two miscible fluids, one containing the racemate, and a driving force such as a concentration, or pressure differential causes preferential transport across the membrane barrier. Separation occurs as a result of the non-racemic nature of the membrane which allows only one enantiomer of the racemate to pass through.

In some embodiments, provided herein are compositions of compounds of any of Formulas **(I)-(LVI),** that are substantially free of a designated stereoisomer of that compound. In certain embodiments, in the methods and compounds of this disclosure, the compounds are substantially free of other stereoisomers. In some embodiments, the composition includes a compound that is at least 85%, 90%, 95%, 98%, or 99% to 100% by weight of the compound, the remainder comprising other chemical species, or enantiomers. In some embodiments, provided herein are compositions of compounds of any of Formulas **(I)-(LVI),** that are substantially free of a designated enantiomer of that compound. In certain embodiments, in the methods and compounds of this disclosure, the compounds are substantially free of other enantiomers. In some embodiments, the composition includes a compound that is at least 85%, 90%, 95%, 98%, or 99% to 100% by weight of the compound, the remainder comprising other chemical species or enantiomers.

### Isotopically Enriched Compounds

Also provided herein are isotopically enriched compounds including, but not limited to, isotopically enriched compounds of any of Formulas **(I)-(LVI).**

Isotopic enrichment (for example, deuteration) of pharmaceuticals to improve pharmacokinetics ("PK"), pharmacodynamics ("PD"), and/or toxicity profiles, has been previously demonstrated within some classes of drugs. See, for example, Lijinsky et al., Food Cosmet. Toxicol., 20: 393 (1982); Lijinsky et al., J. Nat. Cancer Inst., 69: 1127 (1982); Mangold et al., Mutation Res. 308: 33 (1994); Gordon et al., Drug Metab. Dispos., 15: 589 (1987); Zello et al., Metabolism, 43: 487 (1994); Gately et al., J. Nucl. Med., 27: 388 (1986); Wade D, Chem. Biol. Interact. 117: 191 (1999).

Isotopic enrichment of a drug can be used, for example, to (1) reduce or eliminate unwanted metabolites; (2) increase the half-life of the parent drug; (3) decrease the number of doses needed to achieve a desired effect; (4) decrease the amount of a dose necessary to achieve a desired effect; (5) increase the formation of active metabolites if any are formed; and/or (6) decrease the production of deleterious metabolites in specific tissues. Isotopic enrichment of a drug can also be used to create a more effective and/or safer drug for combination therapy, whether the combination therapy is intentional or not.

Replacement of an atom for one of its isotopes often will result in a change in the reaction rate of a chemical reaction. This phenomenon is known as the Kinetic Isotope Effect ("KIE"). For example, if a C-H bond is broken during a rate-determining step in a chemical reaction (i.e., the step with the highest transition state energy), substitution of a (heavier) isotope for that reactive hydrogen will cause a decrease in the reaction rate. The Deuterium Kinetic Isotope Effect ("DKIE") is the most common form of KIE. (*See, e.g.,* Foster et al., Adv. Drug Res., vol. 14, pp. 1-36 (1985); Kushner et al., Can. J. Physiol. Pharmacol., vol. 77, pp. 79-88 (1999)).

The magnitude of the DKIE can be expressed as the ratio between the rates of a given reaction in which a C-H bond is broken, and the same reaction where deuterium is substituted for hydrogen and the C-D bond is broken. The DKIE can range from about one (no isotope effect) to very large numbers, such as 50, or more, meaning that the reaction can be fifty, or more, times slower when deuterium has been substituted for hydrogen.

Substitution of tritium ("T") for hydrogen results in yet a stronger bond than deuterium and gives numerically larger isotope effects. Similarly, substitution of isotopes for other elements including, but not limited to, ¹³C, or ¹⁴C for carbon; ³³S, ³⁴S, or ³⁶S for sulfur; ¹⁵N for nitrogen; and ¹⁷O, or ¹⁸O for oxygen may lead to a similar kinetic isotope effect.

The animal body expresses a variety of enzymes for the purpose of eliminating foreign substances, such as therapeutic agents, from its circulation system. Examples of such enzymes include the cytochrome P450 enzymes ("CYPs"), esterases, proteases, reductases, dehydrogenases, and monoamine oxidases to react with and convert these foreign substances to more polar intermediates, or metabolites for renal excretion. Some of the most common metabolic reactions of pharmaceutical compounds involve the oxidation of a carbon-hydrogen (C-H) bond to either a carbon-oxygen (C-O), or carbon-carbon (C=C) pi-bond. The resultant metabolites may be stable, or unstable under physiological conditions, and can have substantially different PK/PD, and acute, and long-term toxicity profiles relative to the parent compounds. For many drugs, such oxidations are rapid. Therefore, these drugs often require the administration of multiple or high daily doses.

Therefore, isotopic enrichment at certain positions of a compound provided herein will produce a detectable KIE that will affect the pharmacologic, PK, PD, and/or toxicological profiles of a compound provided herein in comparison with a similar compound having a natural isotopic composition.

### Compositions and Uses

### Pharmaceutical Compositions and Methods of Administration

The compounds provided herein can be formulated into pharmaceutical compositions using methods available in the art and those disclosed herein. Any of the compounds provided herein can be provided in the appropriate pharmaceutical composition and be administered by a suitable route of administration.

The methods provided herein encompass administering pharmaceutical compositions comprising at least one compound provided herein and one or more compatible and pharmaceutically acceptable carriers. In this context, the term "pharmaceutically acceptable" means approved by a regulatory agency of the Federal, or state government, or listed in the U.S. Pharmacopeia, or other generally recognized pharmacopeia for use in animals, and in certain embodiments in humans. The term "carrier" includes a diluent, adjuvant (e.g., Freund's adjuvant (complete and incomplete)), excipient, or vehicle with which the therapeutic is administered. Such pharmaceutical carriers can be sterile liquids, such as water and oils including petroleum, animal, vegetable, or oils of synthetic origin, such as peanut oil, soybean oil, mineral oil, sesame oil, and the like. Water can be used as a carrier when the pharmaceutical composition is administered intravenously. Saline solutions, and aqueous dextrose, and glycerol solutions can also be employed as liquid carriers, particularly for injectable solutions. Examples of suitable pharmaceutical carriers are described in Martin, E.W., *Remington's Pharmaceutical Sciences.*

In clinical practice the pharmaceutical compositions, or compounds provided herein may be administered by any route known in the art. Exemplary routes of administration include, but are not limited to, inhalation, intraarterial, intradermal, intramuscular, intraperitoneal, intravenous, nasal, parenteral, pulmonary, and subcutaneous routes. In some embodiments, a pharmaceutical composition or compound provided herein is administered parenterally.

The compositions for parenteral administration can be emulsions or sterile solutions. Parenteral compositions may include, for example, propylene glycol, polyethylene glycol, vegetable oils, and injectable organic esters (e.g., ethyl oleate). These compositions can also contain wetting, isotonizing, emulsifying, dispersing, and stabilizing agents. Sterilization can be carried out in several ways, for example, using a bacteriological filter, via radiation, or via heating. Parenteral compositions can also be prepared in the form of sterile solid compositions which can be dissolved at the time of use in sterile water, or any other injectable sterile medium.

In some embodiments, a composition provided herein is a pharmaceutical composition, or a single unit dosage form. Pharmaceutical compositions, and single unit dosage forms provided herein comprise a prophylactically, or therapeutically effective amount of one, or more prophylactic, or therapeutic compounds.

The pharmaceutical composition may comprise one, or more pharmaceutical excipients. Any suitable pharmaceutical excipient may be used, wherein a person of ordinary skill in the art is capable of selecting suitable pharmaceutical excipients. Non-limiting examples of suitable excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol, and the like. Whether a particular excipient is suitable for incorporation into a pharmaceutical composition, or dosage form depends on a variety of factors well known in the art including, but not limited to, the way in which the dosage form will be administered to a subject and the specific compound in the dosage form. The composition, or single unit dosage form, if desired, can also contain minor amounts of wetting, or emulsifying agents, or pH buffering agents. Accordingly, the pharmaceutical excipients provided below are intended to be illustrative, and not limiting. Additional pharmaceutical excipients include, for example, those described in the Handbook of Pharmaceutical Excipients, Rowe et al. (Eds.) 6th Ed. (2009), incorporated by reference herein in its entirety.

In some embodiments, the pharmaceutical composition comprises an anti-foaming agent. Any suitable anti-foaming agent may be used. In some aspects, the anti-foaming agent is selected from an alcohol, an ether, an oil, a wax, a silicone, a surfactant, and combinations thereof. In some aspects, the anti-foaming agent is selected from a mineral oil, a vegetable oil, ethylene bis stearamide, a paraffin wax, an ester wax, a fatty alcohol wax, a long-chain fatty alcohol, a fatty acid soap, a fatty acid ester, a silicon glycol, a fluorosilicone, a polyethylene glycol-polypropylene glycol copolymer, polydimethylsiloxane-silicon dioxide, ether, octyl alcohol, capryl alcohol, sorbitan trioleate, ethyl alcohol, 2-ethyl-hexanol, dimethicone, oleyl alcohol, simethicone, and combinations thereof.

In some embodiments, the pharmaceutical composition comprises a co-solvent. Illustrative examples of co-solvents include, ethanol, poly(ethylene) glycol, butylene glycol, dimethylacetamide, glycerin, and propylene glycol.

In some embodiments, the pharmaceutical composition comprises a buffer. Illustrative examples of buffers include, acetate, borate, carbonate, lactate, malate, phosphate, citrate, hydroxide, diethanolamine, monoethanolamine, glycine, methionine, guar gum, and monosodium glutamate.

In some embodiments, the pharmaceutical composition comprises a carrier, or filler. Illustrative examples of carriers, or fillers include, lactose, maltodextrin, mannitol, sorbitol, chitosan, stearic acid, xanthan gum, and guar gum.

In some embodiments, the pharmaceutical composition comprises a surfactant. Illustrative examples of surfactants, include d-alpha tocopherol, benzalkonium chloride, benzethonium chloride, cetrimide, cetylpyridinium chloride, docusate sodium, glyceryl behenate, glyceryl monooleate, lauric acid, macrogol 15 hydroxystearate, myristyl alcohol, phospholipids, polyoxyethylene alkyl ethers, polyoxyethylene sorbitan fatty acid esters, polyoxyethylene stearates, polyoxylglycerides, sodium lauryl sulfate, sorbitan esters, and vitamin E polyethylene(glycol) succinate.

In some embodiments, the pharmaceutical composition comprises an anti-caking agent. Illustrative examples of anti-caking agents include, calcium phosphate (tribasic), hydroxymethyl cellulose, hydroxypropyl cellulose, and magnesium oxide.

Other excipients that may be used with the pharmaceutical compositions include, for example, albumin, antioxidants, antibacterial agents, antifungal agents, bioabsorbable polymers, chelating agents, controlled release agents, diluents, dispersing agents, dissolution enhancers, emulsifying agents, gelling agents, ointment bases, penetration enhancers, preservatives, solubilizing agents, solvents, stabilizing agents, and sugars. Specific examples of each of these agents are described, for example, in the Handbook of Pharmaceutical Excipients, Rowe et al. (Eds.) 6th Ed. (2009), The Pharmaceutical Press, incorporated by reference herein in its entirety.

In some embodiments, the pharmaceutical composition comprises a solvent. In some aspects, the solvent is saline solution, such as a sterile isotonic saline solution, or dextrose solution. In some aspects, the solvent is water for injection.

In some embodiments, the pharmaceutical compositions are in a particulate form, such as a microparticle or a nanoparticle. Microparticles, and nanoparticles may be formed from any suitable material, such as a polymer, or a lipid. In some aspects, the microparticles, or nanoparticles are micelles, liposomes, or polymersomes.

Further provided herein are anhydrous pharmaceutical compositions, and dosage forms comprising a compound, since, in some embodiments, water can facilitate the degradation of some compounds.

Anhydrous pharmaceutical compositions, and dosage forms provided herein can be prepared using anhydrous, or low moisture containing ingredients, and low moisture, or low humidity conditions. Pharmaceutical compositions, and dosage forms that comprise lactose, and at least one active ingredient that comprises a primary, or secondary amine can be anhydrous if substantial contact with moisture, and/or humidity during manufacturing, packaging, and/or storage is expected.

An anhydrous pharmaceutical composition can be prepared and stored such that its anhydrous nature is maintained. Accordingly, anhydrous compositions can be packaged using materials known to prevent exposure to water such that they can be included in suitable formulary kits. Examples of suitable packaging include, but are not limited to, hermetically sealed foils, plastics, unit dose containers (e.g., vials), blister packs, and strip packs.

Lactose-free compositions provided herein can comprise excipients that are well known in the art and are listed, for example, in the U.S. Pharmocopeia (USP) SP (XXI)/NF (XVI). In general, lactose-free compositions comprise an active ingredient, a binder/filler, and a lubricant in pharmaceutically compatible, and pharmaceutically acceptable amounts. Exemplary lactose-free dosage forms comprise an active ingredient, microcrystalline cellulose, pre gelatinized starch, and magnesium stearate.

Also provided are pharmaceutical compositions, and dosage forms that comprise one, or more excipients that reduce the rate by which a compound will decompose. Such excipients, which are referred to herein as "stabilizers," include, but are not limited to, antioxidants such as ascorbic acid, pH buffers, or salt buffers.

### Parenteral Dosage Forms

In certain embodiments, provided are parenteral dosage forms. Parenteral dosage forms can be administered to subjects by various routes including, but not limited to, subcutaneous, intravenous (including bolus injection), intramuscular, and intraarterial. Because their administration typically bypasses subjects' natural defenses against contaminants, parenteral dosage forms are typically sterile, or capable of being sterilized prior to administration to a subject. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms are well known to those skilled in the art. Examples include, but are not limited to Water for Injection USP; aqueous vehicles such as, but not limited to, Sodium Chloride Injection, Ringer's Injection, Dextrose Injection, Dextrose, and Sodium Chloride Injection, and Lactated Ringer's Injection; water miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Excipients that increase the solubility of one, or more of the antibodies disclosed herein can also be incorporated into the parenteral dosage forms.

### Dosage and Unit Dosage Forms

In human therapeutics, the doctor will determine the posology which he considers most appropriate according to a preventive, or curative treatment, and according to the age, weight, condition, and other factors specific to the subject to be treated.

In certain embodiments, a composition provided herein is a pharmaceutical composition, or a single unit dosage form. Pharmaceutical compositions, and single unit dosage forms provided herein comprise a prophylactically, or therapeutically effective amount of one, or more prophylactic, or therapeutic antibodies, or antigen binding fragments thereof.

The amount of the compound, or composition which will be effective in the prevention, or treatment of a disorder, or one, or more symptoms thereof will vary with the nature, and severity of the disease, or condition, and the route by which the compound is administered. The frequency and dosage will also vary according to factors specific for each subject depending on the specific therapy (e.g., therapeutic or prophylactic agents) administered, the severity of the disorder, disease, or condition, the route of administration, as well as age, body, weight, response, and the past medical history of the subject. Effective doses may be extrapolated from dose-response curves derived from *in vitro,* or animal model test systems.

In certain embodiments, exemplary doses of a composition include milligram, or microgram amounts of the compound per kilogram of subject, or sample weight (e.g., about 10 micrograms per kilogram to about 50 milligrams per kilogram, about 100 micrograms per kilogram to about 25 milligrams per kilogram, or about 100 microgram per kilogram to about 10 milligrams per kilogram). In certain embodiments, the dosage of the compound provided herein, based on weight of the compound, administered to prevent, treat, manage, or ameliorate a disorder, or one, or more symptoms thereof in a subject is 0.1 mg/kg, 1 mg/kg, 2 mg/kg, 3 mg/kg, 4 mg/kg, 5 mg/kg, 6 mg/kg, 10 mg/kg, or 15 mg/kg or more of a subject's body weight. In another embodiment, the dosage of the composition, or a composition provided herein administered to prevent, treat, manage, or ameliorate a disorder, or one, or more symptoms thereof in a subject is 0.1 mg to 200 mg, 0.1 mg to 100 mg, 0.1 mg to 50 mg, 0.1 mg to 25 mg, 0.1 mg to 20 mg, 0.1 mg to 15 mg, 0.1 mg to 10 mg, 0.1 mg to 7.5 mg, 0.1 mg to 5 mg, 0.1 to 2.5 mg, 0.25 mg to 20 mg, 0.25 to 15 mg, 0.25 to 12 mg, 0.25 to 10 mg, 0.25 mg to 7.5 mg, 0.25 mg to 5 mg, 0.25 mg to 2.5 mg, 0.5 mg to 20 mg, 0.5 to 15 mg, 0.5 to 12 mg, 0.5 to 10 mg, 0.5 mg to 7.5 mg, 0.5 mg to 5 mg, 0.5 mg to 2.5 mg, 1 mg to 20 mg, 1 mg to 15 mg, 1 mg to 12 mg, 1 mg to 10 mg, 1 mg to 7.5 mg, 1 mg to 5 mg, or 1 mg to 2.5 mg.

The dose can be administered according to a suitable schedule, for example, once, two times, three times, or four times weekly. It may be necessary to use dosages of the compound outside the ranges disclosed herein in some cases, as will be apparent to those of ordinary skill in the art. Furthermore, it is noted that the clinician, or treating physician will know how, and when to interrupt, adjust, or terminate therapy in conjunction with subject response.

Different therapeutically effective amounts may be applicable for different diseases, and conditions, as will be readily known by those of ordinary skill in the art. Similarly, amounts sufficient to prevent, manage, treat, or ameliorate such disorders, but insufficient to cause, or sufficient to reduce, adverse effects associated with the antibodies, or antigen binding fragments thereof provided herein are also encompassed by the described dosage amounts, and dose frequency schedules herein. Further, when a subject is administered multiple dosages of a composition provided herein, not all of the dosages need be the same. For example, the dosage administered to the subject may be increased to improve the prophylactic, or therapeutic effect of the composition, or it may be decreased to reduce one, or more side effects that a particular subject is experiencing.

In certain embodiments, treatment, or prevention can be initiated with one, or more loading doses of a compound, or composition provided herein followed by one, or more maintenance doses.

In certain embodiments, a dose of a compound, or composition provided herein can be administered to achieve a steady-state concentration of the compound in blood, or serum of the subject. The steady-state concentration can be determined by measurement according to techniques available to those of skill or can be based on the physical characteristics of the subject such as height, weight, and age.

In certain embodiments, administration of the same composition may be repeated and the administrations may be separated by at least one day, two days, three days, five days, ten days, fifteen days, thirty days, forty-five days, two months, seventy-five days, three months, or six months. In other embodiments, administration of the same prophylactic, or therapeutic agent may be repeated, and the administration may be separated by at least one day, two days, three days, five days, ten days, fifteen days, thirty days, forty-five days, two months, seventy-five days, three months, or six months.

### Therapeutic Applications

For therapeutic applications, the compounds are administered to a mammal, in certain embodiments, a human, in a pharmaceutically acceptable dosage suitable for admisntration form such as those known in the art, and those discussed herein, intravenously as a bolus or by continuous infusion over a period of time, by intramuscular, intraperitoneal, intra-cerebrospinal, subcutaneous, intra-articular, intrasynovial, intrathecal, or intratumoral routes. The compounds also are suitably administered by peritumoral, intralesional, or perilesional routes, to exert local as well as systemic therapeutic effects. In certain embodiments, the compounds are administered to a mammal, in certain embodiments, a human, in a pharmaceutically acceptable dosage suitable for oral admisntration form such as those known in the art, and those discussed herein. For example, the compounds of this disclosure may be administered orally to a human as a liquid, or solid form. Solid dosage forms include, capsules, tablets, pills, powders, and granules. In such solid dosage forms, the chemical entity is mixed with one or more pharmaceutically acceptable excipients, such as sodium citrate or dicalcium phosphate and/or: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like.

The compounds provided herein may be useful for the treatment of any disease, or condition described herein (e.g., a metabolic disease or condition). In certain embodiments, the disease, or condition is any disease, or condition that benefits from modulation of GLP-1 receptor activity. In certain embodiments, the disease, or condition is any disease, or condition that benefits from agonizing GLP-1 receptor activity. In certain embodiments, the methods reduce blood glucose levels. In certain embodiments, the methods promote insulin synthesis, stimulate insulin secretion, increase the mass of β-cells, modulate gastric acid secretion, modulate gastric emptying, and/or decrease glucagon production. In certain embodiments, the disease, or condition is type 2 diabetes.

In certain embodiments, the disease, or condition is obesity, or one, or more diseases, or conditions associated with obesity. Non-limiting examples of obesity, and obesity related conditions include, symptomatic obesity, simple obesity, childhood obesity, morbid obesity, and abdominal obesity (central obesity characterized by abdominal adiposity). Non-limiting examples of symptomatic obesity include, endocrine obesity (e.g., Cushing syndrome, hypothyroidism, insulinoma, obese type II diabetes, pseudohypoparathyroidism, hypogonadism), hypothalamic obesity, hereditary obesity (e.g., Prader-Willi syndrome, Laurence-Moon-Biedl syndrome), and drug-induced obesity (e.g., steroid, phenothiazine, insulin, sulfonylurea agent, or β-blocker-induced obesity).

Examples of such diseases, and conditions associated with obesity include, without limitation, glucose tolerance disorders, diabetes (e.g., type 2 diabetes, obese diabetes), lipid metabolism abnormality, hyperlipidemia, hypertension, cardiac failure, hyperuricemia, gout, fatty liver (including non-alcoholic steatohepatitis (NASH)), coronary heart disease (e.g., myocardial infarction, angina pectoris), cerebral infarction (e.g., brain thrombosis, transient cerebral ischemic attack), bone, or articular disease (e.g., knee osteoarthritis, hip osteoarthritis, spondylitis deformans, lumbago), sleep apnea syndrome, obesity hypoventilation syndrome (Pickwickian syndrome), menstrual disorder (e.g., abnormal menstrual cycle, abnormality of menstrual flow and cycle, amenorrhea, abnormal catamenial symptom), visceral obesity syndrome, and metabolic syndrome. In certain embodiments, the compounds described herein can be used to treat subjects exhibiting symptoms of both obesity, and insulin deficiency.

In some embodiments, the disease, or condition is diabetes. Non-limiting examples of diabetes include, type 1 diabetes, type 2 diabetes (e.g., diet-treated type 2-diabetes, sulfonylurea-treated type 2-diabetes, a far-advanced stage type 2-diabetes, long-term insulin-treated type 2-diabetes), diabetes mellitus (e.g., non-insulin-dependent diabetes mellitus, insulin-dependent diabetes mellitus), gestational diabetes, obese diabetes, autoimmune diabetes, and borderline type diabetes.

In some embodiments, the disease, or condition is associated with diabetes (e.g., a complication of diabetes). Non-limiting examples of disorders associated with diabetes include, obesity, obesity-related disorders, metabolic syndrome, neuropathy, nephropathy (e.g., diabetic nephropathy), retinopathy, diabetic cardiomyopathy, cataract, macroangiopathy, osteopenia, hyperosmolar diabetic coma, infectious disease (e.g., respiratory infection, urinary tract infection, gastrointestinal infection, dermal soft tissue infections, inferior limb infection), diabetic gangrene, xerostomia, hypacusis, cerebrovascular disorder, diabetic cachexia, delayed wound healing, diabetic dyslipidemia peripheral blood circulation disorder, cardiovascular risk factors, (e.g., coronary artery disease, peripheral artery disease, cerebrovascular disease, hypertension, and risk factors related to unmanaged cholesterol, and/or lipid levels, and/or inflammation), NASH, bone fracture, and cognitive dysfunction

Other non-limiting examples of diseases, or conditions related to diabetes include, pre-diabetes, hyperlipidemia (e.g., hypertriglyceridemia, hypercholesterolemia, high LDL-cholesterolemia, low HDL-cholesterolemia, postprandial hyperlipemia), metabolic syndrome (e.g., metabolic disorder where activation of GLP-1R is beneficial, metabolic syndrome X), hypertension, impaired glucose tolerance (IGT), insulin resistance, and sarcopenia.

In some embodiments, the disease, or condition is diabetes, and obesity (diabesity). In certain embodiments, the compounds described herein are useful in improving the therapeutic effectiveness of metformin.

In some embodiments, the disease or condition is a disorder of a metabolically important tissue.

In some embodiments, the disease, or condition is a fatty liver disease. Fatty liver diseases include, but are not limited to, non-alcoholic fatty acid liver disease (NAFLD), steatohepatitis, non-alcoholic steatohepatitis (NASH), fatty liver disease resulting from hepatitis, fatty liver disease resulting from obesity, fatty liver disease resulting from diabetes, fatty liver disease resulting from insulin resistance, fatty liver disease resulting from hypertriglyceridemia, Abetalipoproteinemia, glycogen storage diseases, Weber-Christian disease, Wolmans disease, acute fatty liver of pregnancy, and lipodystrophy.

Non-alcoholic fatty liver disease (NAFLD) represents a spectrum of disease occurring in the absence of alcohol abuse, and is typically characterized by the presence of steatosis (fat in the liver). NAFLDis believed to be linked to a variety of conditions, e.g., metabolic syndrome (including obesity, diabetes, and hypertriglyceridemia), and insulin resistance. It can cause liver disease in adults and children, and may ultimately lead to cirrhosis (Skelly et al., J Hepatol 2001; 35: 195-9; Chitturi et al., Hepatology 2002; 35(2):373-9). The severity of NAFLD ranges from the relatively benign isolated predominantly macrovesicular steatosis (i.e., nonalcoholic fatty liver or NAFL) to non-alcoholic steatohepatitis (NASH) (Angulo et al., J Gastroenterol Hepatol 2002; 17 SuppkS 186-90). In certain embodiments, the subject is a pediactric subject (e.g., 6-16 years old; or 6-12 years old; or 6-10 years old). In certain embodiments, the subject is an adult subject.

Other non-limiting examples of diseases, or conditions in metabolically important tissues include, joint disorders (e.g., osteoarthritis, secondary osteoarthritis), steatosis (e.g. in the liver); gall stones; gallbladder disorders; gastroesophageal reflux; sleep apnea; hepatitis; fatty liver; bone disorder characterized by altered bone metabolism, such as osteoporosis, including post-menopausal osteoporosis, poor bone strength, osteopenia, Paget's disease, osteolytic metastasis in cancer patients, osteodistrophy in liver disease, and the altered bone metabolism caused by renal failure, or haemodialysis, bone fracture, bone surgery, aging, pregnancy, protection against bone fractures, and malnutritionpolycystic ovary syndrome; renal disease (e.g., chronic renal failure, glomerulonephritis, glomerulosclerosis, nephrotic syndrome, hypertensive nephrosclerosis, end-stage renal disease); muscular dystrophy, angina pectoris, acute, or chronic diarrhea, testicular dysfunction, respiratory dysfunction, frailty, sexual dysfunction (e.g., erectile dysfunction), and geriatric syndrome. In certain embodiments, the chemical entities described herein can be used for treating surgical trauma by improving recovery after surgery, and/or by preventing the catabolic reaction caused by surgical trauma.

In some embodiments, the disease, or condition is a cardiovascular disease. Non-limiting examples of cardiovascular disease include, congestive heart failure, atherosclerosis, arteriosclerosis, coronary heart disease, or peripheral artery disease, stroke, coronary artery disease, congestive heart failure, coronary heart disease, hypertension, cardiac failure, cerebrovascular disorder (e.g., cerebral infarction), vascular dysfunction, myocardial infarction, elevated blood pressure (e.g., 130/85 mm Hg or higher), and prothrombotic state (exemplified by high fibrinogen, or plasminogen activator inhibitor in the blood).

In some embodiments, the disease, or condition is a neurological disorder (e.g., neurodegenerative disorder), or a psychiatric disorder. Non-limiting examples of neurological disorders include, brain insulin resistance, mild cognitive impairment (MCI), Alzheimer's disease (AD), Parkinson's disease (PD), anxiety, dementia (e.g., senile dementia), traumatic brain injury, Huntington's chores, tardive dyskinesia, hyperkinesia, mania, Morbus Parkinson, steel-Richard syndrome, Down's syndrome, myasthenia gravis, nerve trauma, brain trauma, vascular amyloidosis, cerebral hemorrhage I with amyloidosis, brain inflammation, Friedrich's ataxia, acute confusion disorder, amyotrophic lateral sclerosis (ALS), glaucoma, and apoptosis-mediated degenerative diseases of the central nervous system (e.g., Creutzfeld-Jakob Disease, bovine spongiform encephalopathy (mad cow disease), chronic wasting syndrome). See, e.g., US20060275288A1.

Non-limiting examples of psychiatric disorders include, drug dependence/addiction (narcotics, amphetamines, and attention deficit/hyperactivity disorder (ADHD). The chemical entities described herein can be useful in improving behavioral response to addictive drugs, decreasing drug dependence, prevention drug abuse relapse, and relieving anxiety caused by the absence of a given addictive substance. See, e.g., US20120021979A1.

In certain embodiments, the chemical entities described herein are useful in improving learning, and memory by enhancing neuronal plasticity, and facilitation of cellular differentiation, and also in preserving dopamine neurons, and motor function in Morbus Parkinson.

In some embodiments, the disease, or condition is impaired fasting glucose (IFG), impaired fasting glycemia (IFG), hyperglycemia, insulin resistance (impaired glucose homeostasis), hyperinsulinemia, elevated blood levels of fatty acids, or glycerol, a hypoglycemic condition, insulin resistant syndrome, paresthesia caused by hyperinsulinemia, hyperlipidaemia, hypercholesteremia, impaired wound healing, leptin resistance, glucose intolerance, increased fasting glucose, dyslipidemia (e.g., hyperlipidemia, atherogenic dyslipidemia characterized by high triglycerides and low HDL cholesterol), glucagonoma, hyperuricacidemia, hypoglycemia (e.g., nighttime hypoglycemia), and concomitant comatose endpoint associated with insulin.

In certain embodiments, the compounds described herein can reduce, or slow down the progression of borderline type, impaired fasting glucose, or impaired fasting glycemia into diabetes.

In some embodiments, the disease, or condition is an autoimmune disorder. Non-limiting examples of autoimmune disorders include, multiple sclerosis, experimental autoimmune encephalomyelitis, autoimmune disorder is associated with immune rejection, graft versus host disease, uveitis, optic neuropathies, optic neuritis, transverse myelitis, inflammatory bowel disease, rheumatoid arthritis, ankylosing spondylitis, systemic lupus erythematosus, myasthenia gravis, and Graves disease. See, e.g., US20120148586A1.

In some embodiments, the disease, or condition is a stomach, or intestine related disorder. Non-limiting examples of these disorders include, ulcers of any etiology (e.g. peptic ulcers, Zollinger-Ellison syndrome, drug-induced ulcers, ulcers related to infections, or other pathogens), digestion disorders, malabsorption, short bowel syndrome, cul-de-sac syndrome, inflammatory bowel diseases (Crohn's disease, and ulcerative colitis), celiac sprue, hypogammaglobulinemic sprue, chemotherapy, and/or radiation therapy-induced mucositis, and diarrhea, gastrointestinal inflammation, short bowel syndrome, colitis ulcerosa, gastric mucosal injury (e.g., gastric mucosal injury caused by aspirin), small intestinal mucosal injury, and cachexia (e.g., cancerous cachexia, tuberculous cachexia, cachexia associated with blood disease, cachexia associated with endocrine disease, cachexia associated with infectious disease, cachexia caused by acquired immunodeficiency syndrome).

In some embodiments, the compounds described herein can be used to reduce body weight (e.g., excess body weight), prevent body weight gain, induce weight loss, decrease body fat, or reduce food intake in a subject (e.g., a subject in need thereof). In certain embodiments, the weight increase in a subject may be attributed to excessive ingestion of food, or unbalanced diets, or may be weight increase derived from a concomitant drug (e.g., insulin sensitizers having a PPARy agonist-like action, such as troglitazone, rosiglitazone, englitazone, ciglitazone, pioglitazone, and the like). Alternatively, the weight increase may be weight increase before reaching obesity, or may be weight increase in an obese subject. The weight increase may also be medication-induced weight gain, or weight gain subsequent to cessation of smoking.

In some embodiments, the condition, disease, or disorder is an eating disorder, such as hyperphagia, binge eating, bulimia, or compulsive eating.

In some embodiments, the disease, or condition is an inflammatory disorder. Non-limiting examples of inflammatory disorders include, chronic rheumatoid arthritis, spondylitis deformans, arthritis deformans, lumbago, gout, post-operational or post-traumatic inflammation, bloating, neuralgia, laryngopharyngitis, cystitis, pneumonia, pancreatitis, enteritis, inflammatory bowel disease (including inflammatory large bowel disease), inflammation in metabolically important tissues including liver, fat, pancreas, kidney, and gut, and a proinflammatory state (e.g., elevated levels of proinflammatory cytokines or, markers of inflammation-like C-reactive protein in the blood).

In some embodiments, the disease, or condition is cancer. Suitable examples of cancer include, breast cancer (e.g., invasive ductal breast cancer, noninvasive ductal breast cancer, inflammatory breast cancer), prostate cancer (e.g., hormone-dependent prostate cancer, hormone-independent prostate cancer), pancreatic cancer (e.g., ductal pancreatic cancer), gastric cancer (e.g., papillary adenocarcinoma, mucous adenocarcinoma, adenosquamous carcinoma), lung cancer (e.g., non-small cell lung cancer, small-cell lung cancer, malignant mesothelioma), colon cancer (e.g., gastrointestinal stromal tumor), rectal cancer (e.g., gastrointestinal stromal tumor), colorectal cancer (e.g., familial colorectal cancer, hereditary non-polyposis colorectal cancer, gastrointestinal stromal tumor), small intestinal cancer (e.g., non-Hodgkin's lymphoma, gastrointestinal stromal tumor), esophageal cancer, duodenal cancer, tongue cancer, pharyngeal cancer (e.g., nasopharyngeal cancer, oropharynx cancer, hypopharyngeal cancer), salivary gland cancer, brain tumor (e.g., pineal astrocytoma, pilocytic astrocytoma, diffuse astrocytoma, anaplastic astrocytoma), neurilemmoma, liver cancer (e.g., primary liver cancer, extrahepatic bile duct cancer), renal cancer (e.g., renal cell cancer, transitional cell cancer of the renal pelvis and ureter), bile duct cancer, endometrial cancer, uterine cervical cancer, ovarian cancer (e.g., epithelial ovarian cancer, extragonadal germ cell tumor, ovarian germ cell tumor, ovarian tumor of low malignant potential), bladder cancer, urethral cancer, skin cancer (e.g., intraocular (ocular) melanoma, Merkel cell carcinoma), hemangioma, malignant lymphoma, malignant melanoma, thyroid cancer (e.g., medullary thyroid cancer), parathyroid cancer, nasal cavity cancer, sinus cancer, bone tumor (e.g., osteosarcoma, Ewing tumor, uterine sarcoma, soft tissue sarcoma), angiofibroma, sarcoma of the retina, penis cancer, testicular tumor, pediatric solid tumor (e.g., Wilms' tumor, childhood kidney tumor), Kaposi's sarcoma, Kaposi's sarcoma caused by AIDS, tumor of maxillary sinus, fibrous histiocytoma, leiomyosarcoma, rhabdomyosarcoma, and leukemia (e.g., acute myeloid leukemia, acute lymphoblastic leukemia).

In certain embodiments, provided herein are methods for the treatment that include, the administration of an effective amount of compounds provided herein, or a pharmaceutically acceptable salt thereof. In certain embodiments, the methods encompass the step of administering to the subject in need thereof an amount of a compound described herein effective for the treatment of disease, or condition in combination with a second agent effective for the treatment, or prevention of the disease, or condition. In certain embodiments, the compound is in the form of a pharmaceutical composition, or dosage form, as described elsewhere herein.

In certain embodiments, the subject is a treatment naive subject. In further embodiments, the subject has previously received therapy. For instance, in certain embodiments, the subject has not responded to a single agent treatment regimen.

In certain embodiments, the subject is a subject that discontinued some other therapy because of one or more adverse events associated with the other therapy. In certain embodiments, the subject has received some other therapy and discontinued that therapy prior to administration of a method provided herein. In further embodiments, the subject has received therapy and continues to receive that therapy along with administration of a compound provided herein. The compounds described herein can be co-administered with other therapy for treatment of the disease or condition according to the judgment of one of skill in the art. In certain embodiments, the methods or compositions provided herein can be co-administered with a reduced dose of the other therapy for the treatment of the disease or condition.

### Diagnostic Applications

In some embodiments, the compounds provided herein are used in diagnostic applications. These applications may be useful, for example, in making a diagnosis, and/or prognosis for a disease, or condition, such as a metabolic disease, or condition.

In some diagnostic and prognostic applications or embodiments, the compound may be labeled with a detectable moiety. Suitable detectable moieties include, but are not limited to, radioisotopes, fluorescent labels, and enzyme-substrate labels. In another embodiment, the compound need not be labeled, and the presence of the compound can be detected using a labeled antibody, or antigen binding fragment thereof which specifically binds to the compound.

### Kits

In some embodiments, a compound provided herein is provided in the form of a kit (i.e., a packaged combination of reagents in predetermined amounts with instructions for performing a procedure). In some embodiments, the procedure is a diagnostic assay. In certain embodiments, the procedure is a therapeutic procedure.

In some embodiments, the kit further comprises a solvent for the reconstitution of the compound. In some embodiments, the compound is provided in the form of a pharmaceutical composition.

In some embodiments, the kits can include a compound, or composition provided herein, an optional second agent, or composition, and instructions providing information to a health care provider regarding usage for treating the disorder. Instructions may be provided in printed form, or in the form of an electronic medium such as a floppy disc, CD, or DVD, or in the form of a website address where such instructions may be obtained. A unit dose of a compound, or a composition provided herein, or a second agent, or composition, can include a dosage such that when administered to a subject, a therapeutically, or prophylactically effective plasma level of the compound, or composition can be maintained in the subject for at least one day. In some embodiments, a compound, or composition can be included as a sterile aqueous pharmaceutical composition, or dry powder (e.g., lyophilized) composition.

In some embodiments, suitable packaging is provided. As used herein, "packaging" includes a solid matrix, or material customarily used in a system, and capable of holding within fixed limits a compound provided herein, and/or a second agent suitable for administration to a subject. Such materials include, glass, and plastic (e.g., polyethylene, polypropylene, and polycarbonate) bottles, vials, paper, plastic, plastic-foil laminated envelopes, and the like. If e-beam sterilization techniques are employed, the packaging should have sufficiently low density to permit sterilization of the contents.

### Preparation and Synthetic Procedures

In certain embodiments, compounds described herein are according to compounds of Formula **IIj-IIn** shown in **Scheme G1.**

### Scheme G1

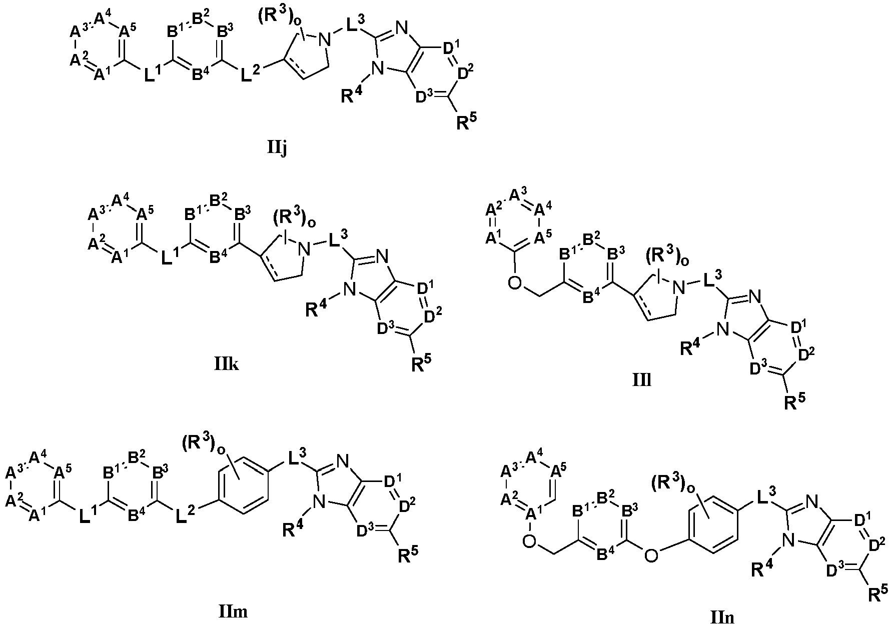

In some embodiments, the compounds described herein are prepared as outlined in **Schemes 1-3.** The synthesis of the compounds in this application is not limited to these general reaction schemes illustrated here. For detailed synthesis of each individual compound, please check the Examples section.

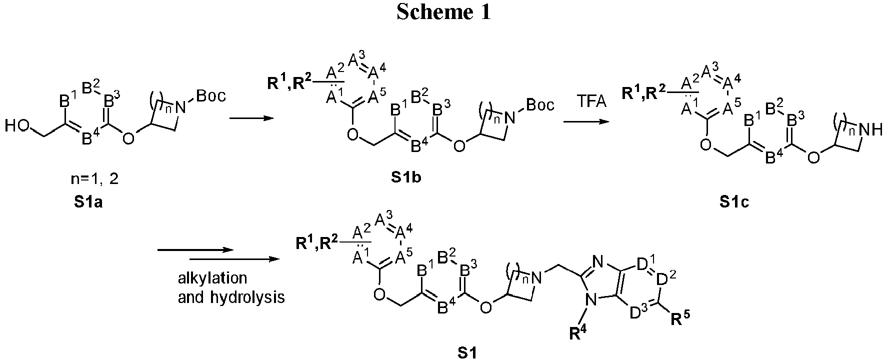

**Scheme 1.** Compound with a Formula **S1** can be made as shown in **Scheme 1. S1b** can be prepared from **S1a** through Mitsunobu reaction or alkylation of a phenol with mesylate of **S1a.** TFA catalyzed cleavage of the Boc-protecting group in **S1b** will furnished azetidine **S1c.** Alkylation of **S1c,** followed by hydrolysis will give **S1.**

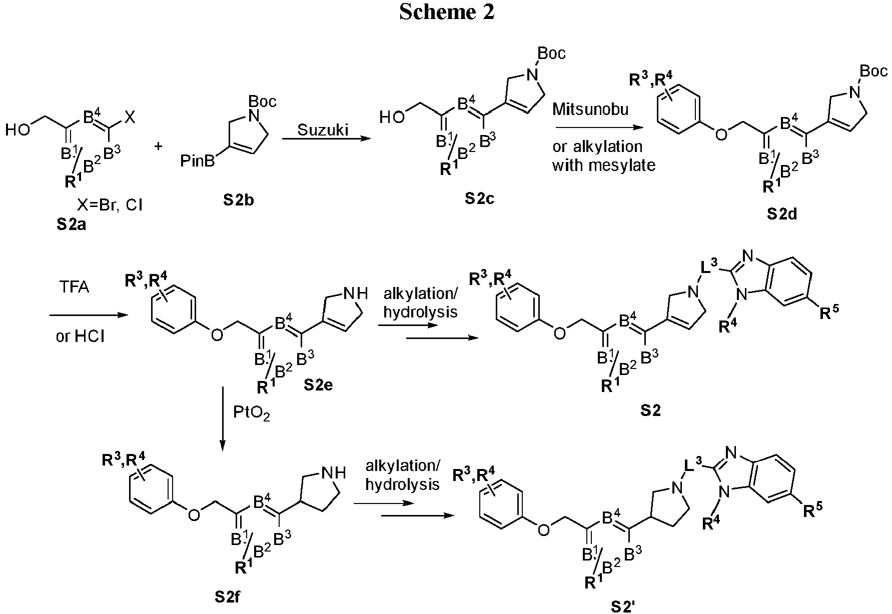

**Scheme 2.** Compounds with Formula **S2** and **S2'** can be made as shown in **Scheme 2.** Compound **S2c** can be prepared from halide **S2a** and pinacolborane **S2b** through Suzuki reaction. Mitsunobu reaction with **S2c** or alkylation of a phenol with mesylate of **S2c** will provide **S2d.** TFA catalyzed cleavage of the Boc-protecting group in **S2d** will furnished pyrroline **S2e.** Alkylation of **S2e,** followed by hydrolysis will give **S2.** Similarly compound **S2'** can be prepared via alkylation, followed by hydrolysis of pyrrolidine **S2f,** which can be obtained from selective hydrogenation of the endo double bond in **S2e.**

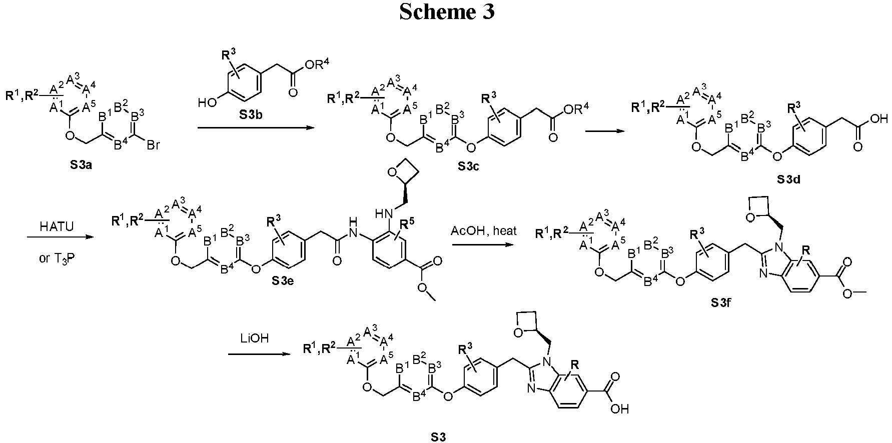

**Scheme 3.** Compound with a Formula **S3** can be made as shown in **Scheme 3.** SNAr reaction or metal-catalyzed coupling reaction of **S3a** and **S3b** will provide **S3c.** Hydrolysis of the R⁴ group will give acid **S3d,** which will undergo amidation reaction to give ester **S3e.** Acetic acid-mediated imidazole formation give **S3f,** which upon hydrolysis will provide **S3.**

### EXAMPLES

### Preparation of the Compounds

The compounds used in the reactions described herein are made according to organic synthesis techniques known to those skilled in this art, starting from commercially available chemicals, and/or from compounds described in the chemical literature. "Commercially available chemicals" are obtained from standard commercial sources such as Acros Organics (Pittsburgh, PA), Advanced ChemBlocks, Inc (Burlingame, CA), Aldrich Chemical (Milwaukee, WI, including Sigma Chemical and Fluka), AK Scientific (Union City, CA), AstaTech, Inc. (Bristol, PA), Aurum Pharmatech LLC (Franklin Park, NJ), Combi-Blocks, Inc. (San Diego, CA), Enamine (Monmouth Jct., NJ), Fisher Scientific Co. (Pittsburgh, PA), Frontier Scientific (Logan, UT), TCI America (Portland, OR), and VWR (Radnor, PA). Specific and analogous reactants are optionally identified through the indices of known chemicals prepared by the Chemical Abstract Service of the American Chemical Society, which are available in most public and university libraries, as well as through on-line databases.

Suitable reference books that detail the synthesis of reactants useful in the preparation of compounds described herein, or provide references to articles that describe their preparation, include for example, "Synthetic Organic Chemistry", John Wiley & Sons, Inc., New York; S. R. Sandler et al., "Organic Functional Group Preparations," 2nd Ed., Academic Press, New York, 1983; "T. L. Gilchrist, "Heterocyclic Chemistry", 2nd Ed., John Wiley & Sons, New York, 1992; and J. March, "Advanced Organic Chemistry: Reactions, Mechanisms and Structure", 4th Ed., Wiley-Interscience, New York, 1992; R. C. Larock "Comprehensive Organic Transformations: A Guide to Functional Group Preparations" 2nd Edition (1999) Wiley-VCH, ISBN: 0-471-19031-4; "Organic Reactions" (1942-2000) John Wiley & Sons, in over 55 volumes; and "Chemistry of Functional Groups" John Wiley & Sons, in 73 volumes. Some compounds require the application of protecting groups. The need for such protection is within the skill in the art. For a general description of protecting groups and their use, see T.W. Greene, P.G.M. Nuts, and Protective Groups in Organic Synthesis, John Wiley & Sons, New York, 1999.

### Analytical Methods and Instrumentation

Proton nuclear magnetic resonance (NMR) spectra were obtained on either Bruker, or Varian spectrometers at 400, or 600 MHz. NMR spectra are reported relative to residual solvent signals as follows: chemical shift δ (ppm), multiplicity, coupling constant *J* (Hz), and integration. Tetramethylsilane (TMS) was used as an internal standard in some of the cases. Mass spectral data were measured using one of the two following systems: System A: Waters Acquity i-class ultra-performance liquid chromatography (UPLC) system with Acquity Photo Diode Array Detector, Acquity Evaporative Light Scattering Detector (ELSD) and Waters ZQ Mass Spectrometer. Data was acquired using Waters MassLynx 4.1 software and purity characterized by UV wavelength 220 nm, evaporative light scattering detection (ELSD) and electrospray positive ion (ESI) (column: Acquity UPLC BEH C18 1.7 µ 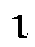 2.1 × 50mm). System B: Agilent LC/MS consisting of a 1200 series LC and 6140 Quadrupole MS detector (column: Agilent USGYL01131, HPH-C18 2.7 µM, 2.1 × 50 mm). Solvents used: acetonitrile/water, containing 0.1% formic acid; flow rate 0.7 mL/min. Preparatory HPLC purifications were conducted with a flow rate of 15 mL/min and detection by UV wavelength 214 nm and 254 nm (Column: Jupiter^{©} 10 µM Proteo 90 Å, 250 × 21.2 mm A, solvent: acetonitrile/water, containing modifier such as 0.1% trifluoroacetic acid, formic acid or acetic acid). Compound purity was checked on an analytical HPLC (Waters Acquity UPLC H-Class instrument), with a flow rate of 0.5 mL/min (Acquity BEH C18, 50 × 2.1 mm column).

Abbreviations used in the examples include:

| **Abbreviation** | **Name** |
|---|---|
| CH₃CN | acetonitrile |
| aq. | aqueous |
| atm | atmospheres |
| BINAP | (1,1'-binaphthalene-2,2'-diyl)bis(diphenylphosphine) |
| Boc | *t*-butoxycarbonyl |
| CCl₄ | carbon tetrachloride |
| CDCl₃ | deuterated chloroform |
| CO | carbon monoxide gas |
| CO₂ | carbon dioxide |
| Cs₂CO₃ | cesium carbonate |
| CuBr | copper(I) bromide |
| Cu(OAc)₂ | copper(II) acetate |
| DCM | dichloromethane |
| DEAD | diethyl azodicarboxylate |
| DIPEA | diisopropylethylamine |
| DMF | *N,N*-dimethylformamide |
| DMSO | dimethylsulfoxide |
| ESI | electrospray ionization |
| Et₃N | triethylamine |
| EtOAc | ethyl acetate |
| EtOH | ethanol |
| h | hours |
| H₂O | water |
| HATU | 1-[bis(dimethylamino)methylene]-1H-1,2,3-triazolo[4,5-b]pyridinium 3-oxid hexafluorophosphate |
| HCl | hydrochloric acid |
| HOAc | acetic acid |
| K₂CO₃ | potassium carbonate |
| KI | potassium iodide |
| KOH | potassium hydroxide |
| KOtBu | potassium *tert*-butoxide |
| LiAlH₄ | lithium aluminum hydride |
| LiOH | lithium hydroxide |
| MeOH | methanol |
| min | minutes |
| N₂ | nitrogen |
| Na₂CO₃ | sodium carbonate |
| Na₂SO₄ | sodium sulfate |
| NaH | sodium hydride |
| NaHCO₃ | sodium bicarbonate |
| NaN₃ | sodium azide |
| NaOH | sodium hydroxide |
| NBS | *N*-bromosuccinimide |
| NH₄Cl | ammonium chloride |
| NH₄HCO₃ | ammonium bicarbonate |
| NMR | nuclear magnetic resonance |
| Pd₂(dba)₃ | tris(dibenzylideneacetone)dipalladium(0) |
| Pd(dppf)Cl₂ | [1,1'-bis(diphenylphosphino)ferrocene]dichloropalladiun(II) |
| Pd/C | palladium on carbon |
| Pd(OAc)₂ | palladium(II) acetate |
| Prep-TLC | preparatory thin layer chromatography |
| RuPhos-Pd-G2 | chloro(2-dicyclohexylphosphino-2',6'-diisopropoxy-1,1'-biphenyl)[2-(2'-amino-1,1'-biphenyl)]palladium(II) |
| sat. | saturated |
| t-BuOH | *tert*-butanol |
| T₃P | propylphosphonic anhydride |
| TBD | 1,5,7-triazabicyclo[4.4.0]dec-5-ene |
| TFA | trifluoracetic acid |
| THF | tetrahydrofuran |

Unless otherwise noted, reagents, and solvents were used as received from commercial suppliers. Anhydrous solvents and oven-dried glassware were used for synthetic transformations sensitive to moisture, and/or oxygen. Reaction times, and yields were not optimized. Example numbers and compound numbers are the same.

### Preparation of common intermediate:

### Intermediate Ih. Methyl (S)-2-(chloromethyl)-1-(oxetan-2-ylmethyl)-1H-benzo[d]imidazole-6-carboxylate (Ih)

### Step A. (S)-2-((Benzyloxy)methyl)oxetane (Ia)

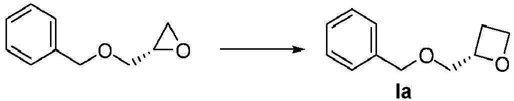

To a solution of KOtBu (2.97 g, 30.4 mmol) in tBuOH (50 mL) was added trimethylsulfoxonium (6.68 g, 30.4 mmol). The reaction mixture was stirred at 60 °C for 30min, then (2S)-2-[(benzyloxy)methyl]oxirane (5 g, 30.4 mmol) was added. The mixture was heated at 80 °C for 2 h. After completion, the mixture was cooled to 25 °C and filtered through celite. The filtered cake was washed with petroleum ether (10 mL). The filtrate was added water (20 mL), and extracted with petroleum ether (2x10 mL). The combined organic layer was washed with saturated NH₄Cl (10 mL), dried over magnesium sulfate, filtered and concentrated *in vacuo* to obtain the crude product. The crude material was purified by silica gel column chromatography (Petroleum ether: EtOAc=1:1) to afford the title compound (**Ia**) (2.5 g, 47%) as a yellow oil. m/z (ESI, +ve ion) = 201.1 [M+Na] ⁺.

### Step B. (S)-Oxetan-2-ylmethanol (Ib)

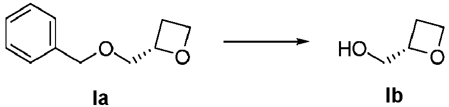

To a solution of **Ia** (7 g, 39.3 mmol) in THF (90 mL) was added Pd(OH)₂ (700 mg, 3.93 mmol). The reaction mixture was stirred at 45 °C under 0.4 Mpa H₂ for 48 h. After completion, the crude mixture was filtered through celite and the resulting crude mixture (**Ib**) in THF was carried to next step without any further purification. m/z (ESI, +ve ion) = 111.1 [M+Na] ⁺.

### Step C. (S)-Oxetan-2-ylmethyl methanesulfonate (Ic)

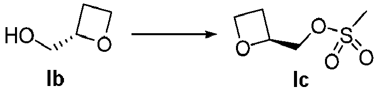

To a solution of **Ib** (6 g, 68 mmol) in THF (100 mL) was added methanesulfonic anhydride (17.1 g, 102 mmol) and TEA (13.6 g, 136.5 mmol) at 0 °C. The reaction mixture was stirred at 20 °C for 2 h. The reaction was quenched with water (30 mL), extracted with EtOAc (3x30 mL). The combined organic layers were dried over anhydrous Na₂SO₄, filtered, and concentrated under reduced pressure. The residue was purified by silica gel column chromatography (Petroleum: EtOAc = 1:1) to afford the title product **Ic** (6 g, 53.1%) as a yellow oil. m/z (ESI, +ve ion) = 167.1 [M+H]⁺.

### Step D. (S)-2-(Azidomethyl)oxetane (Id)

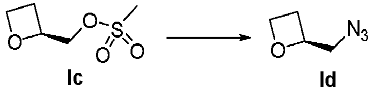

To a solution of **Ic** (6 g, 36.1 mmol) in DMF (100 mL) was added NaN₃ (7.02 g, 108 mmol). The reaction mixture was stirred at 80 °C for 16 h. The reaction was added water (20 mL), extracted with EtOAc (3* 20 mL), washed with H₂O (10 mL). The organic layer (**Id**) was concentrated and carried to next step without any further purification. m/z (ESI, +ve ion) = 114.1 [M+H]⁺.

### Step E. (5)-Oxetan-2-ylmethanamine (Ie)

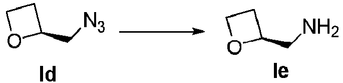

To a solution of **Id** (900 mg, 7.96 mmol) in THF (30 mL) was added Pd/C (10 mg). The reaction mixture was stirred at 20 °C for 2 h under hydrogen. After completion, the crude reaction mixture was filtered through celite. The filtrate (**Ie**) was used to next step without any purification. m/z (ESI, +ve ion) = 88.2 [M+H]⁺.

### Step F. Methyl (S)-4-nitro-3-((oxetan-2-ylmethyl)amino)benzoate (If)

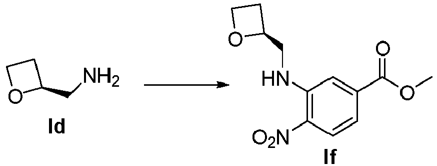

To a solution of **Ie** (700 mg, 10.5 mmol) in THF (3 mL) was added methyl 3-fluoro-4-nitrobenzoate (2.09 g, 10.5 mmol), and TEA (3.18 g, 31.5 mmol). The reaction mixture was stirred at 25 °C for 16 h, then added water (15 mL). The solution was extracted with EtOAc (3x 10mL). The combined organic layers were dried over anhydrous Na₂SO₄, and filtered. The filtrate was concentrated under reduced pressure. The residue was purified by silica gel column chromatography (Petroleum:EtOAc = 1:1) to afford the title product (**If**) (1.2 g, 43.9%) as a yellow solid. m/z (ESI, +ve ion) = 267.1 [M+H]⁺.

### Step G. Methyl (S)-4-amino-3-((oxetan-2-ylmethyl)amino)benzoate (Ig)

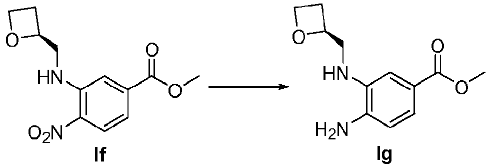

To a solution of **If** (800 mg, 7.9 mmol) in THF (8 mL) was added Pd/C (100 mg). The reaction mixture was stirred at 20 °C for 3 h under 50 psi H₂. After completion, the crude mixture was filtered through celite, and the filtrate was concentrated to give the title compound (Ig) (0.6 g, 43.9%) as a yellow solid. m/z (ESI, +ve ion) = 237.2 [M+H]⁺.

### Step H. Methyl (S)-2-(chloromethyl)-1-(oxetan-2-ylmethyl)-1H-benzo[d]imidazole-6-carboxylate (Ih)

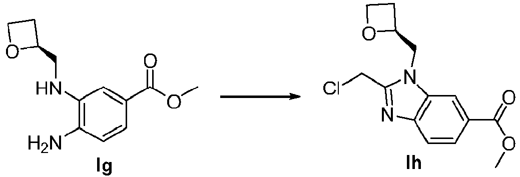

To a solution of **Ig** (700 mg, 2.96 mmol) in MeCN (10 mL) was added 2-chloro-1,1,1-trimethoxyethane (456 mg, 2.96 mmol) and 4-methyl-benzenesulfonic acid (25 mg, 0.14 mmol). The reaction mixture was stirred at 60 °C for 2 h. The residue was purified by silica gel column chromatography (Petroleum:EtOAc = 1:1) to afford the title product (**Ih**) (0.6 g, 68.9%) as a yellow solid. m/z (ESI, +ve ion) = 295.1 [M+H]⁺.

### Intermediate IIf. Methyl 2-(chloromethyl)-1-((1-ethyl-1H-imidazol-5-yl)methyl)-1H benzo[d]imidazole-6-carboxylate (IIf)

### Step A. Ethyl 1-ethyl-1H-imidazole-5-carboxylate (IIa)

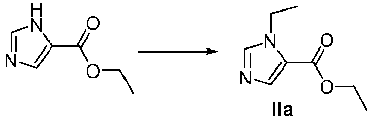

To a solution of ethyl *1H*-imidazole-5-carboxylate (2.8 g, 20.0 mmol) in DMF (10 mL) was added NaH (60% in mineral oil, 1.2 g, 30.0 mmol) at 0 °C. After stirring at the same temperature for 0.5 h, iodoethane was added (3.12 g, 20.0 mmol), and the resulting mixture was stirred at 0 °C for another 0.5 h. After completion, the mixture was quenched with H₂O (30 mL), and extracted with EtOAc (30 mL × 3). The organic layer was washed with H₂O (30 mL), brine (30 mL), dried over anhydrous Na₂SO₄, filtered, and concentrated *in vacuo* to give a crude residue. The residue was purified by silica gel column chromatography (petroleum ether:EtOAc = 5:1) to afford the title product (**IIa**) (850 mg, 25%) as a white solid. *m*/*z* (ESI, +ve ion) = 169.1 [M+H]⁺.

### Step B. 1-Ethyl-1H-imidazole-5-carboxamide (IIb)

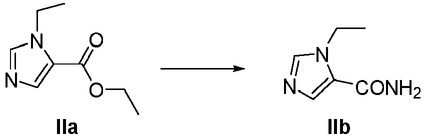

A solution of **IIa** (3.6 g, 21.4 mmol) in 7 N NH₃ solution in MeOH (50 mL) was stirred at 80 °C for 36 h in a sealed reactor. After completion, the mixture was concentrated *in vacuo* to give a crude residue. The crude residue was purified by silica gel column chromatography (DCM:MeOH = 10:1) to afford the title product (**IIb**) (1.9 g, 64%) as a white solid. *m*/*z* (ESI, +ve ion) = 140.1 [M+H]⁺.

### Step C. (1-Ethyl-1H-imidazol-5-yl)methanamine (IIc)

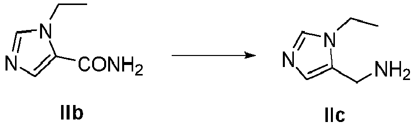

To a solution of **IIb** (1.9 g, 13.6 mmol) in THF (20 mL) was added LiAlH₄ (1.1 g, 27.2 mmol) at 0 °C. The mixture was stirred at 60 °C for 16 h under N₂. After completion, the mixture was quenched with Na₂SO₄·10H₂O, and filtered. The filtrate was extracted by DCM (100 mL). The organic layer was concentrated *in vacuo* to afford the crude title product (**IIc**) (1.4 g) as a colorless oil, which was used in the next step without further purification. *m*/*z* (ESI, +ve ion) = 126.2 [M+H]⁺.

### Step D. Methyl 3-(((1-ethyl-1H-imidazol-5-yl)methyl)amino)-4-nitrobenzoate (IId)

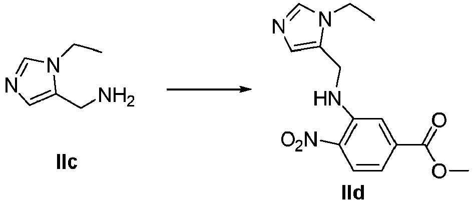

A mixture of **IIc** (1.4 g, 11.1 mmol), methyl 3-fluoro-4-nitrobenzoate (2.4 g, 12.2 mmol), and TEA (3.3 g, 33.3 mmol) in THF (20 mL) was stirred at 25 °C for 16 h. After completion, the mixture was diluted with H₂O (50 mL), and extracted with DCM (50 mL × 3). The organic layer was dried over anhydrous Na₂SO₄, filtered, and concentrated *in vacuo* to give a crude residue. The residue was purified by silica gel column chromatography (DCM:MeOH = 10:1) to afford the title product (**IId**) (1.6 g, 47%) as a white solid. *m*/*z* (ESI, +ve ion) = 305.2 [M+H]⁺.

### Step E. Methyl 4-amino-3-(((1-ethyl-1H-imidazol-5-yl)methyl)amino)benzoate (IIe)

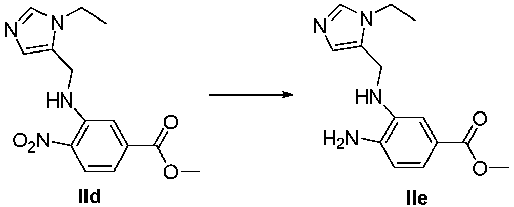

A mixture of **IId** (1.6 g, 5.26 mmol), and 10% Pd/C (200 mg) in MeOH (10 mL) was stirred at 25 °C for 2 h under H₂. After completion, the mixture was filtered through a pad of celite, which was rinsed with additional MeOH (100 mL). The organic layers were combined, and concentrated *in vacuo* to give a crude residue. The residue was purified by silica gel column chromatography (DCM:MeOH = 10:1) to afford the title product (**IIe**) (1.3 g, 90%) as a brown solid. *m*/*z* (ESI, +ve ion) = 275.2 [M+H]⁺.

### Step F. Methyl 2-(chloromethyl)-1-((1-ethyl-1H-imidazol-5-yl)methyl)-1H benzo[d]imidazole-6-carboxylate (Ilf)

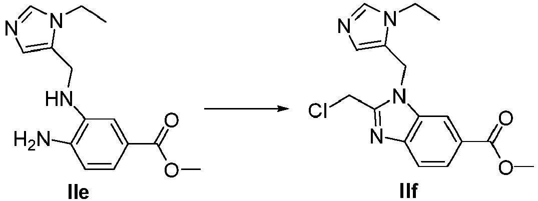

To a solution of **57e** (100 mg, 0.36 mmol) in CH₃CN (3 mL) was added 2-chloro-1,1,1-trimethoxyethane (67.7 mg, 0.44 mmol), and p-toluenesulfonic acid monohydrate (3.4 mg, 0.018 mmol) at room temperature. The mixture was heated to 60 °C, and stirred overnight. The crude reaction mixture was purified by reverse phase HPLC (CH₃CN and water with 0.1% HOAc as a modifier) to give the title product (**IIf**) (46.6 mg, 39.0 %) as a white solid. *m*/*z* (ESI, +ve ion) = 333.1 [M+H]⁺.

### Intermediate IIIf. tert-Butyl 2-(2,5-difluoro-4-hydroxyphenyl)acetate (IIIf)

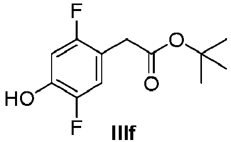

### Step A. 1-Allyl-2,5-difluoro-4-methoxybenzene (IIIa)

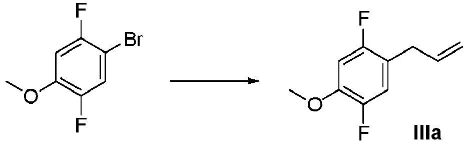

A solution of 1-bromo-2,5-difluoro-4-methoxybenzene (5g, 22.4 mmol), allyltributyltin (8.9 g, 26.8 mmol), Pd(PPh₃)₄ (2.59 g, 2.2 mmol) in DMF (100 mL) was stirred at 110 °C for 18 h under N₂. The reaction was poured into water (500 mL) and extracted with EtOAc (150 mL × 2). The combined organic layer was concentrated and purified by silica gel column chromatography (EtOAc:petroleum ether= 1:15) to afford the title product (**IIIa**) (2.63 g, 57.6%) as a colorless oil. ¹H NMR (400 MHz, CDCl₃) δ ppm 6.89 (dd, *J* = 11.56, 7.04 Hz, 1H), 6.67 (dd, *J* = 10.10, 7.14 Hz, 1H), 5.83 - 5.95 (m, 1H), 5.03 - 5.12 (m, 2H), 3.85 (s, 3H), 3.31 (dd, *J* = 6.50, 1.05 Hz, 2H).

### Step B. 2-(2,5-Difluoro-4-methoxyphenyl)acetic acid (IIIb)

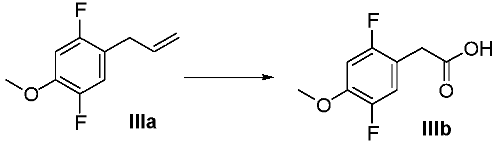

To a mixture of **IIIa** (2.5 g, 13.6 mmol), NaIO₄ (11.64 g, 54.4 mmol) in a mixed solution of CCl₄/CH₃CN/H₂O (40 mL/40 mL/70 mL) was added ruthenium(III) chloride (560 mg, 2.7 mmol) at 0 °C. The reaction was stirred at 0 °C for 2 h, poured into water (50 mL) and extracted with DCM (50 mL × 2). The combined organic layer was dried over Na₂SO₄, filtered and concentrated *in vacuo* to provide an impure crude title product (**IIIb**) (2.2 g) as a dark green solid, which was used in the next step without further purification. *m*/*z* (ESI, +ve ion) = 203.1 [M+H]⁺.

### Step C. 2-(2,5-Difluoro-4-hydroxyphenyl)acetic acid (IIIc)

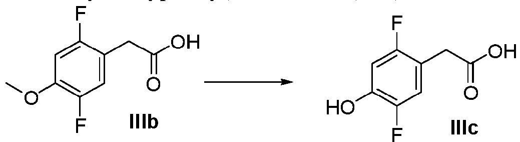

A stirred solution of crude **IIIb** (650 mg, 3.2 mmol) in DCM (6 mL) was added BBr₃ (1.5 mL) at 0 °C. The reaction was stirred at 25 °C for 2 h, poured into ice-water, and extracted with EtOAc (50 mL × 3). The combined organic layer was concentrated and purified by reverse phase HPLC (32% CH₃OH in H₂O) to afford the title product (**IIIc**) (441 mg, 65%) as a gray solid. *m*/*z* (ESI, +ve ion) = 189.1 [M+H]⁺.

### Step D. 2-(4-Acetoxy-2,5-difluorophenyl)acetic acid (IIId)

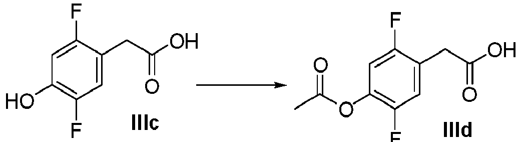

To a mixture of **IIIc** (50 mg, 0.26 mmol) and AczO (54 mg, 0.53 mmol) in DCM (3.0 mL) was added one drop of conc. H₂SO₄. The resulting clear solution was stirred at 25 °C for 1.5 h, poured into water (5 mL) and extracted with EtOAc (15 mL × 2). The combined organic layer was concentrated *in vacuo* and purified by reverse phase HPLC (37% CH₃OH in H₂O) to afford the title product (**IIId**) (37 mg, 54%) as a white solid. *m*/*z* (ESI, +ve ion) = 253.0 [M+Na]⁺.

### Step E. tert-Butyl 2-(4-acetoxy-2,5-difluorophenyl)acetate (IIIe)

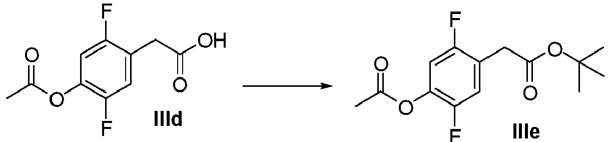

A solution of **IIId** (100 mg, 0.43 mmol), Boc₂O (190 mg, 0.87 mmol), DMAP (11 mg, 0.09 mmol) in t-BuOH (5 mL) was stirred at 50 °C for 1 h. The reaction was concentrated and purified by silica gel column chromatography (EtOAc/petroleum ether = 1:10) to afford the title product (**IIIe**) (69 mg, 50%) as a colorless oil. *m*/*z* (ESI, +ve ion) = 309.0 [M+Na]⁺.

### Step F. tert-Butyl 2-(2,5-difluoro-4-hydroxyphenyl)acetate (IIIf)

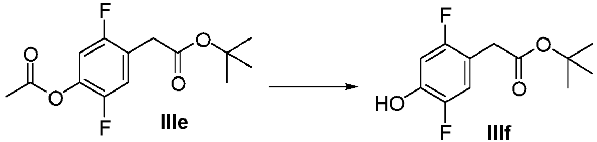

A mixture of IIIe (380 mg, 1.33 mmol) and K₂CO₃ (275 mg, 2 mmol, 1.5 eq) in CH₃OH (5 mL) was stirred at 25 °C for 0.5 h. The reaction was poured into water (25 mL) and extracted with EtOAc (15 mL × 3). The combined organic layer was concentrated and purified by reversed phase HPLC (37% CH₃CN in H₂O, with 0.5% TFA as a modifier) to afford the title product (IIIf) (320 mg, 88%) as a white solid. *m*/*z* (ESI, +ve ion) = 267.0 [M+Na]⁺. ¹H NMR (400 MHz, DMSO-*d6*) δ ppm 10.27 (s, 1H), 7.12 (dd, *J* = 11.50, 7.19 Hz, 1H), 6.74 (dd, *J* = 10.80, 7.44 Hz, 1H), 3.47 (s, 2H), 1.39 (s, 9H).

### Example 119. 1-(((S)-Oxetan-2-yl)methyl)-2-((3-(3-phenoxyphenyl)pyrrolidin-1-yl)methyl)-1H-benzo[d]imidazole-6-carboxylic acid (119)

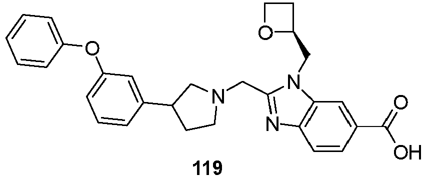

### Step A. Methyl 1-(((S)-oxetan-2-yl)methyl)-2-((3-(3-phenoxyphenyl)pyrrolidin-1-yl)methyl)-1H-benzo[d]imidazole-6-carboxylate (119a)

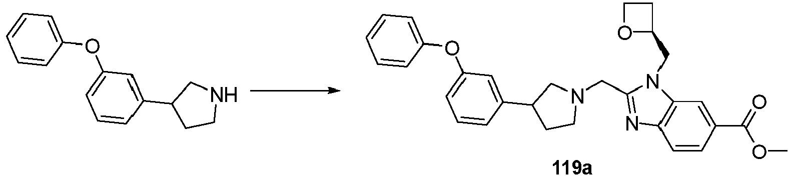

A solution of 3-(3-phenoxyphenyl)pyrrolidine (20 mg, 0.084 mmol), **Ih** (19.7 mg, 0.084 mmol) and DIPEA (0.029 mL, 0.17 mmol) in DCM (0.84 mL) was heated to 40 °C overnight. The crude was directly purified by silica gel column chromatography (gradient elution, 0-100% EtOAc in hexanes) to provide the title product (**119a**) (32 mg, 77%). *m*/*z* (ESI, +ve ion) = 498.1 [M+H]⁺.

### Step B. 1-(((S)-Oxetan-2-yl)methyl)-2-((3-(3-phenoxyphenyl)pyrrolidin-1-yl)methyl)-1H-benzo[d]imidazole-6-carboxylic acid (119)

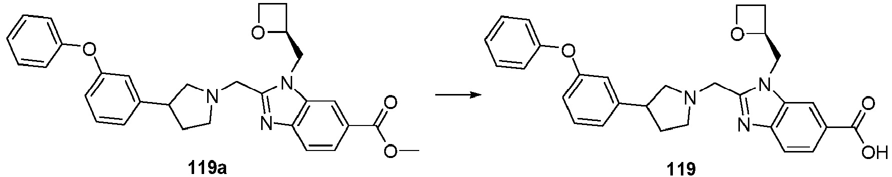

To a solution of **119a** (30 mg, 0.06 mmol) in THF/H₂O (0.6 mL, 2:1) was added LiOH (2.5 mg, 0.06 mmol) and stirred for 2 h. The reaction was neutralized with 1 N HCl and extracted with EtOAc. The organic layer was dried, filtered, concentrated and purified by silica gel column chromatography (gradient elution, 0-10% MeOH in DCM) to provide the title product (**119**) (17 mg, 58%) as a white solid. *m*/*z* (ESI, +ve ion) = 484.3 [M+H]⁺.

### Example 127. (S)-2-((4-((3-((2,4-dichlorophenoxy)methyl)-1H-pyrazol-1-yl)methyl)piperidin-1-yl)methyl)-1-(oxetan-2-ylmethyl)-1H-benzo[d]imidazole-6-carboxylic acid (127)

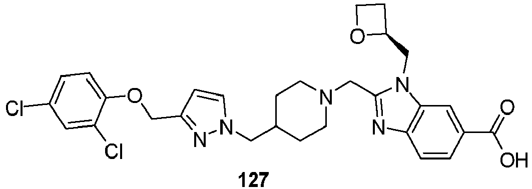

### Step A. tert-Butyl 4-((3-(methoxycarbonyl)-1H-pyrazol-1-yl)methyl)piperidine-1-carboxylate (127a)

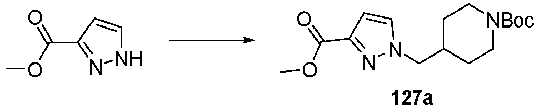

A solution of methyl 1*H*-pyrazole-3-carboxylate (1.26 g, 10.0 mmol), tert-butyl 4-(bromomethyl)piperidine-1-carboxylate (2.7 7g, 10.0 mmol) and Cs₂CO₃ (9.78 g, 30.0 mmol) in CH₃CN (50 mL) was stirred at 60 °C for 5 h. After completion, the reaction was diluted with H₂O (30 mL) and extracted with EtOAc (30 mL × 3). The organic layer was washed with H₂O (30 mL), brine (30 mL), dried over anhydrous Na₂SO₄, filtered and concentrated to give a crude residue, which was purified by reverse phase HPLC (CH₃CN in H₂O, with 0.1% formic acid as a modifier) to afford the title product (**127a**) (1.60 g, 49%) as a white solid. *m*/*z* (ESI, +ve ion) = 346.1 [M+Na]⁺.

### Step B. tert-Butyl 4-((3-(hydroxymethyl)-1H-pyrazol-1-yl)methyl)piperidine-1-carboxylate (127b)

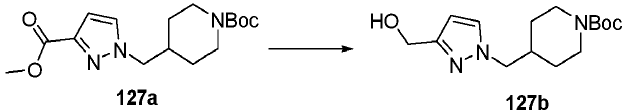

To a solution of **127a** (1.60 g, 4.9 mmol) in 20 mL of anhydrous THF was added DIBAL-H solution (1 N in hexanes, 14.7 mL, 14.7 mmol) at 0 °C. The reaction was stirred at 15 °C for 2 h under N₂. After completion, the reaction was quenched with Na₂SO₄.10H₂O. The mixture was filtered and the filtrate cake was rinsed with DCM (100 mL). The organic layer was concentrated to afford the title product (**127b**) (970 mg, 67%) as a colorless oil. *m*/*z* (ESI, +ve ion) = 296.2 [M+H]⁺.

### Step C. tert-Butyl 4-((3-((2,4-dichlorophenoxy)methyl)-1H-pyrazol-1-yl)methyl)piperidine-1-carboxylate (127c)

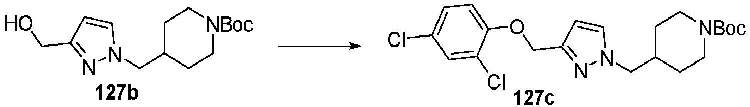

To a solution of **127b** (310 mg, 1.1 mmol), 2,4-dichlorophenol (187 mg, 1.2 mmol) and PPh₃ (303 mg, 1.2 mmol) in THF (10 mL) was added DEAD (201 mg, 1.2 mmol) at 0 °C. The reaction was stirred at 15 °C for 5 h under N₂. After completion, the reaction was diluted with H₂O (20 mL) and extracted with EtOAc (30 mL × 3). The organic layer was washed with H₂O (30 mL), brine (30 mL), dried over anhydrous Na₂SO₄, filtered and concentrated *in vacuo* to give a crude residue, which was purified by silica gel column chromatography (petroleum:EtOAc = 10:1) to afford the title product (**127c**) (81 mg, 17%) as a colorless oil. *m*/*z* (ESI, +ve ion) = 462.0 [M+Na]⁺.

### Step D. 4-((3-((2,4-Dichlorophenoxy)methyl)-1H-pyrazol-1-yl)methyl)piperidine (127d)

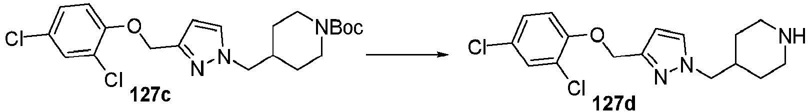

A solution of **127c** (81 mg, 0.18 mmol) in DCM (10 mL) was added 2,6-lutidine (432 mg, 3.7 mmol) and TMSOTf (400 mg, 1.8 mmol) at 0 °C. After stirring at 0 °C for 1 h, the reaction was quenched with NH₄Cl (10 mL) and extracted with EtOAc (20 mL × 3). The combined organic layer was washed with H₂O (20 mL), brine (20 mL), dried over anhydrous Na₂SO₄, filtered and concentrated *in vacuo* to afford the crude title product (**127d**) as a yellow oil, which was used in the next step without further purification. *m*/*z* (ESI, +ve ion) = 340.1 [M+H]⁺.

### Step E. Methyl (S)-2-((4-((3-((2,4-Dichlorophenoxy)methyl)-1H-pyrazol-1-yl)methyl)piperidin-1-yl)methyl)-1-(oxetan-2-ylmethyl)-1H-benzo[d]imidazole-6-carboxylate (127e)

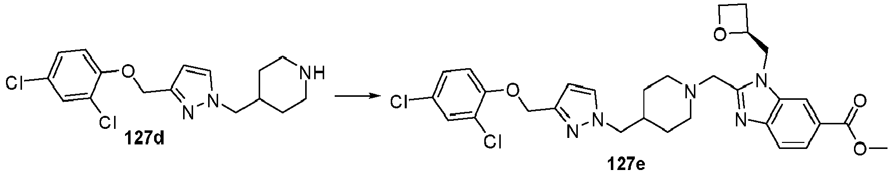

A mixture of **127d** (crude from Step D, 0.18 mmol) and K₂CO₃ (75 mg, 0.54 mmol) in DMF (5 mL) was added **Ih** (53 mg, 0.18 mmol) at 10 °C. After stirring for 16 h at the same temperature, the reaction was diluted with H₂O (5 mL) and extracted with DCM (20 mL × 3). The organic layer was washed with brine (10 mL), dried over anhydrous Na₂SO₄, filtered and concentrated *in vacuo* to give a crude residue, which was purified by silica gel column chromatography (DCM/MeOH =50:1) to afford the title product (**127e**) (61 mg, 57%) as a white solid. *m*/*z* (ESI, +ve ion) = 598.1 [M+H]⁺.

### Step F. (S)-2-((4-((3-((2,4-Dichlorophenoxy)methyl)-1H-pyrazol-1-yl)methyl)piperidin-1-yl)methyl)-1-(oxetan-2-ylmethyl)-1H-benzo[d]imidazole-6-carboxylic acid (127)

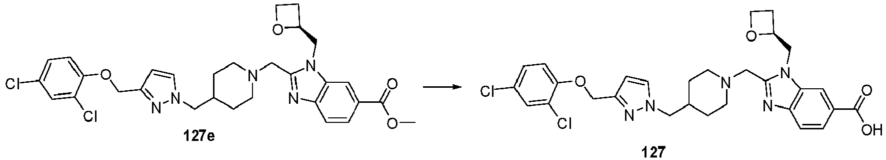

A mixture of **127e** (61 mg, 0.1 mmol) in THF (3 ml) and H₂O (1 mL) was added lithium hydroxide (24 mg, 1.0 mmol) and stirred at 30 °C for 5 h. After completion, 1 N HCl was added to the reaction to adjust to pH=7. The solvents were removed under reduced pressure and the residue was purified by reverse phase HPLC (CH₃CN in H₂O with 0.1% TFA as a modifier) to afford the title product (**127**) (23.2 mg, 39%) as a white solid. *m*/*z* (ESI, +ve ion) = 584.2 [M+H]⁺. ¹H NMR (400 MHz, CD₃OD) δ ppm 8.28 - 8.41 (m, 1H), 7.97 - 8.11 (m, 1H), 7.79 (d, *J* = 8.56 Hz, 1H), 7.63 (d, *J* = 2.20 Hz, 1H), 7.38 (dd, *J=* 2.32, 1.10 Hz, 1H), 7.13 - 7.28 (m, 2H), 6.42 (d, *J* = 1.96 Hz, 1H), 5.16 - 5.26 (m, 1 H), 5.14 (s, 2 H), 4.79 (d, *J* = 3.42 Hz, 2H), 4.70 - 4.76 (m, 1H), 4.58 - 4.70 (m, 2H), 4.39 (dt, *J* = 9.11, 5.96 Hz, 1H), 4.14 (d, *J* = 6.85 Hz, 2H), 3.78 (br d, *J* = 10.03 Hz, 2H), 3.14 - 3.29 (m, 2H), 2.68 - 2.87 (m, 1H), 2.41 - 2.56 (m, 1H), 2.15 - 2.32 (m, 1H), 1.74 - 1.93 (m, 2H), 1.49 - 1.68 (m, 2H).

### Example 134. 1-((1-Ethyl-1H-imidazol-5-yl)methyl)-2-((3-phenoxypyrrolidin-1-yl)methyl)-1H-benzo[d]imidazole-6-carboxylic acid (134)

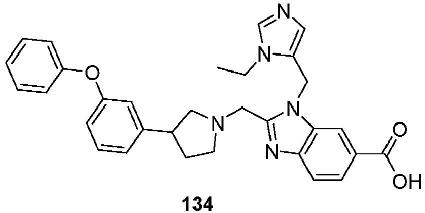

### Step A. Methyl 1-((1-ethyl-1H-imidazol-5-yl)methyl)-2-((3-(3-phenoxyphenyl)pyrrolidin-1-yl)methyl)-1H-benzo[d]imidazole-6-carboxylate (134a)

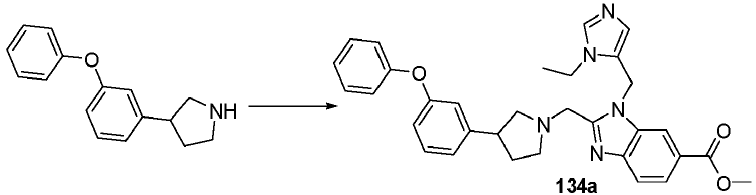

A solution of 3-(3-phenoxyphenyl)pyrrolidine (4 mg, 0.017 mmol), **IIf** (4.6 mg, 0.014 mmol) and Et₃N (14 mg, 0.14 mmol) in DMF (1 mL) was stirred at room temperature for 20 h. After completion, the reaction was purified by reverse phase HPLC (gradient elution, 0-90% CH₃CN in water, with 0.1% TFA as a modifier) to provide the title compound (**134a**) (8.2 mg) as a white solid with some impurity, which was used in the next step without further purification. *m*/*z* (ESI, +ve ion) = 536.4 [M+H]⁺.

### Step B. 1-((1-Ethyl-1H-imidazol-5-yl)methyl)-2-((3-(3-phenoxyphenyl)pyrrolidin-1-yl)methyl)-1H-benzo[d]imidazole-6-carboxylic acid (134)

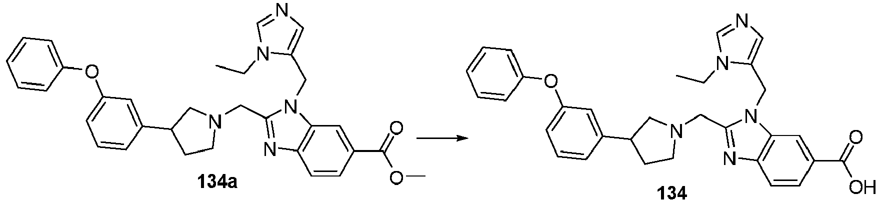

A solution of **134a** (8.2 mg, crude) in THF (0.5 mL) was treated with 2 N NaOH (0.5 mL) and MeOH (4 drops). The resulting mixture was stirred at room temperature for 20 h. After completion, the reaction was purified by reverse phase HPLC (gradient elution, 0-60% CH₃CN in water, with 0.1% TFA as a modifier) to provide the title product (**134**) (3.7 mg, 41% for 2 steps, racemic) as a white solid. ¹H NMR (600 MHz, CD₃OD ) δ ppm 8.99 (s, 1H), 8.24 (br d, *J* = 0.73 Hz, 1H), 8.04 - 8.07 (m, 1H), 7.83 (d, *J* = 8.44 Hz, 1H), 7.33 - 7.39 (m, 3H), 7.08 - 7.15 (m, 3H), 7.03 (br t, *J* = 2.02 Hz, 1H), 6.96 - 7.00 (m, 2H), 6.88 - 6.92 (m, 1H), 5.81 (s, 2H), 4.31 (q, *J* = 7.34 Hz, 2H), 3.92 - 4.01 (m, 2H), 3.65 - 3.75 (m, 3H), 2.53 - 2.60 (m, 2H), 2.21 - 2.29 (m, 2H), 1.53 (t, *J* = 7.34 Hz, 3H) *m*/*z* (ESI, +ve ion) = 522.3 [M+H]⁺.

### Example 147. 2-((3-((2-((2,4-Dichlorophenoxy)methyl)oxazol-5-yl)methyl)azetidin-1-yl)methyl)-1-((1-ethyl-1H-imidazol-5-yl)methyl)-1H-benzo[d]imidazole-6-carboxylic acid (147)

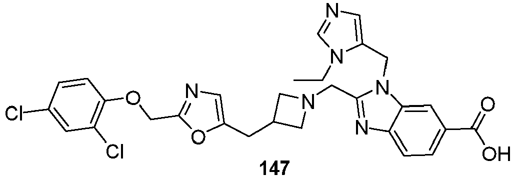

### Step A. Methyl 5-((1-(tert-butoxycarbonyl)azetidin-3-ylidene)methyl)oxazole-2-carboxylate (147a)

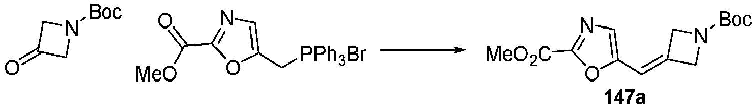

To a solution of methyl 5-((bromotriphenyl-λ5-phosphaneyl)methyl)oxazole-2-carboxylate (211 mg, 0.44 mmol, prepared by heating triphenylphosphine with methyl 5-(bromomethyl)-1,3-oxazole-2-carboxylate in toluene at 90 °C for 24 h, and decanting the solvent and drying the solid residue on high vacuum pump) in anhydrous DMF (2.2 mL) was added NaH (19 mg, 0.47 mmol, 60% in mineral oil) at 0 °C. After stirring for 10 min, a solution of tert-butyl 3-oxoazetidine-1-carboxylate (50 mg, 0.29 mmol) in DMF (1.5 mL) was added dropsise and the reaction was allowed to warm to room temperature. After 16 h, the reaction was quenched with sat. NH₄Cl, and extracted with EtOAc (5 mL × 2). The organic layer was washed with H₂O (5 mL), brine (5 mL), dried with Na₂SO₄, filtered, and concentrated to give a crude residue, which was purified by silica gel column chromatography (gradient elution, 0-90% EtOAc in hexanes) to afford the title product (**147a**) (48 mg, 56%). *m*/*z* (ESI, +ve ion) = 239.3 [M-*t*Bu+H]⁺.

### Step B. Methyl 5-((1-(tert-butoxycarbonyl)azetidin-3-yl)methyl)oxazole-2-carboxylate (147b)

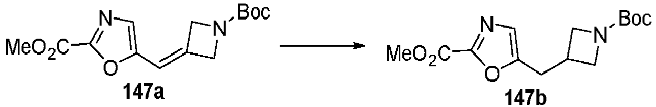

10% Pd/C (8 mg, 0.008 mmol) was added to **147a** (45 mg, 0.15 mmol) in EtOH (1.5 mL) at room temperature. The mixture was degassed with hydrogen for 10 min, then stirred under a H₂ balloon for 4 h. The reaction was filtered through a pad of celite, rinsed with EtOAc and concentrated to provide the title product (**147b**) (42 mg), which was used in the next step without further purification. *m*/*z* (ESI, +ve ion) = 241.1 [M - tBu + H]⁺.

### Step C. tert-Butyl 3-((2-(hydroxymethyl)oxazol-5-yl)methyl)azetidine-1-carboxylate (147c)

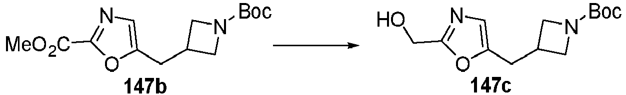

To a solution of crude **147b** (42 mg) in anhydrous EtOH (1.4 mL) at 0 °C was added NaBH₄ (16 mg, 0.43 mmol) and CaCh (47 mg, 0.43 mmol). The reaction was then stirred at room temperature for 4 h, quenched with dropwise addition of 1 N HCl (1 mL), and extracted with EtOAc (5 mL × 3). The organic layer was washed with H₂O (5 mL), brine (5 mL), dried over Na₂SO₄, filtered, concentrated and the crude residue was purified by silica gel column chromatography (gradient elution, 0-15% MeOH in DCM) to provide the title product (**147c**) (32 mg, 84% for 2 steps). *m*/*z* (ESI, +ve ion) = 291.1 [M+Na]⁺.

### Step D. tert-Butyl 3-((2-((2,4-Dichlorophenoxy)methyl)oxazol-5-yl)methyl)azetidine-1-carboxylate (147d)

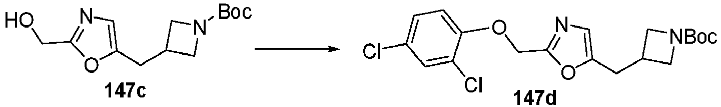

DIAD (41 mg, 0.20 mmol), PPh₃ (53 mg, 0.20 mmol) and 2,4-dichlorophenol (22 mg, 0.14 mmol) were added to **147c** (30 mg, 0.11 mmol) in anhydrous THF (1.1 mL) at 0 °C. The reaction was warmed to room temperature. After 3 h, H₂O (3 mL) was added and the reaction was extracted with EtOAc (3 mL × 3). The organic layer was washed with brine (3 mL), dried over Na₂SO₄, filtered, and concentrated to give a crude residue, which was purified by silica gel column chromatogrpahy (gradient elution, 0-90% EtOAc in hexanes) to provide the title product (**147d**) (12 mg, 21%). *m*/*z* (ESI, +ve ion) = 436.0 [M+Na]⁺.

### Step E. 5-(Azetidin-3-ylmethyl)-2-((2,4-dichlorophenoxy)methyl)oxazole (147e)

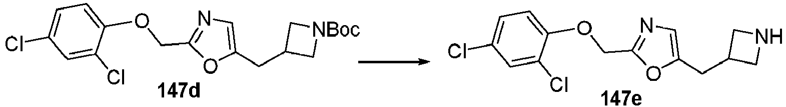

A solution of **147d** (11 mg, 0.027 mmol) in DCM (0.6 mL) was added TFA (0.051 mL). After stirring at room temperature for 5 h, the reaction was concentrated, redissolved in EtOAc and washed with saturated NaHCO₃. The aqueous layer was extracted with EtOAc (x 2) and the combined organic layer was dried, filtered and concentrated to provide the crude title product (**147e**) (8.3 mg), which was used in the next step without further purification.

### Step E. 2-((3-((2-((2,4-Dichlorophenoxy)methyl)oxazol-5-yl)methyl)azetidin-1-yl)methyl)-1-((1-ethyl-1H-imidazol-5-yl)methyl)-1H-benzo[d]imidazole-6-carboxylic acid (147)

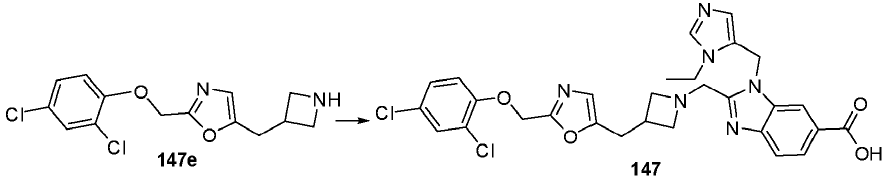

The title product (**147**) was synthesized in similar procedures from the crude **147e** as described in Example **119**, Steps A and B. *m*/*z* (ESI, +ve ion) = 595.1 [M+H]⁺. ¹H NMR (600 MHz, MeOD) δ ppm 9.07 (d, *J* = 1.10 Hz, 1H), 8.24 (d, *J* = 0.73 Hz, 1H), 8.05 (dd, *J* = 8.44, 1.47 Hz, 1H), 7.82 (d, *J* = 8.44 Hz, 1H), 7.40 (d, *J* = 2.20 Hz, 1H), 7.28 (dd, *J* = 8.80, 2.57 Hz, 1H), 7.20 (d, *J* = 8.80 Hz, 1H), 7.13 (d, *J*=1.47 Hz, 1H), 6.98 (s, 1H), 5.78 (s, 2H), 5.22 (s, 2H), 4.91 (s, 2H), 4.47 - 4.57 (m, 2H), 4.21 - 4.36 (m, 4H), 3.33 - 3.37 (m, 1H), 3.17 - 3.24 (m, 2H), 1.53 (t, *J* = 7.34 Hz, 3H).

Examples **167** and **191** were synthesized in similar procedures as described in Example **147.**

Example **225** was synthesized from **147a** in similar procedures as described in Example **127,** Step B (toluene as solvent), followed by Example **147,** Steps D and E.

### Example 173. 2-((4-((2-(5-Chloropyridin-2-yl)-2-methylbenzo[d][1,3]dioxol-4-yl)methyl)piperidin-1-yl)methyl)-1-(((S)-oxetan-2-yl)methyl)-1H-benzo[d]imidazole-6-carboxylic acid (173)

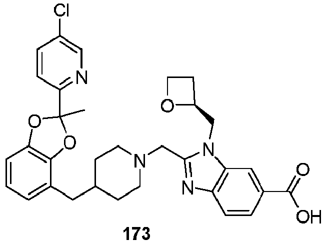

### Step A. 2-(4-Bromo-2-methylbenzo[d][1,3]dioxol-2-yl)-5-chloropyridine (173a)

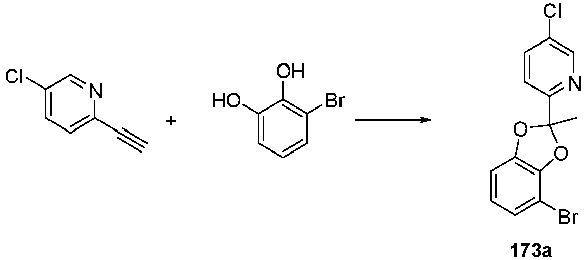

A mixture of 5-chloro-2ethynylpyridine (0.18 g, 1.31 mmol), 3-bromobenzene-1,2-diol (0.25 g, 1.31 mmol) and triruthenium dodecacarbonyl (0.017 g, 0.026 mmol) in toluene (2.6 mL) was degassed by Argon for one minute. The resulting mixture was stirred at 100 °C overnight. After completion, the reaction mixture was diluted with EtOAc (5 mL) and filtered through a pad of celite. The filtrate was concentrated and purified by silica gel column chromatography (gradient elution, 0-10% EtOAc in hexanes) to afford the title product (**173a**) (0.25 g, 59%) as a pale-yellow oil. *m*/*z* (ESI, +ve ion) = 326.1 [M+H]⁺.

### Step B. tert-Butyl 4-((2-(5-chloropyridin-2-yl)-2-methylbenzo[d][1,3]dioxol-4-yl)methyl)piperidine-1-carboxylate (173b)

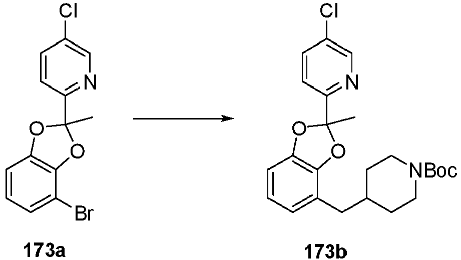

Neat 1-N-Boc-4-methylenepiperidine (0.17 mL, 0.85 mmol) was degassed with Argon for one minute, followed by addition of 9-BBN solution in THF (1.7 mL, 0.85 mmol). The reaction was stirred at 70 °C for one hour. Then it was cooled down to room temperature and transferred to a solution of **173a** (0.25 g, 0.76 mmol), Pd(dppf)Cl₂ (0.017 g, 0.023 mmol) and K₂CO₃ (0.14 g, 1.01 mmol) in DMF (1.76 mL) and water (0.18 mL). The resulting mixture was further degassed with Argon for one minute and then stirred at 60 °C overnight. After completion, the reaction mixture was poured into water (5 mL) and adjusted to pH = 11 with 1 N NaOH. It was then diluted with EtOAc (10 mL). The organic layer was washed with water (5 mL), brine (5 mL), dried over Na₂SO₄, filtered, and concentrated to give a crude residue, which was purified by silica gel column chromatography (gradient elution, 0-30% EtOAc in hexanes) to provide the title product (**173b**) (0.19 g, 56%) as a colorless oil. *m*/*z* (ESI, +ve ion) = 445.4 [M+H]⁺.

### Step C. 5-Chloro-2-(2-methyl-4-(piperidin-4-ylmethyl)benzo[d][1,3]dioxol-2-yl)pyridine (173c)

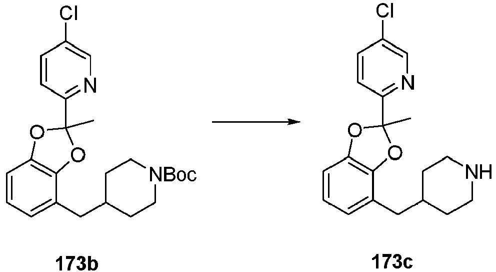

A solution of **173b** (192 mg, 0.43 mmol) in DCM (2 mL) and TFA (1 mL) was stirred at room temperature for 10 min. The reaction was concentrated to remove the volatiles and diluted with EtOAc (10 mL). The organic layer was washed with sat. NaHCO₃ (5 mL), brine (3 mL), dried over Na₂SO₄, filtered and concentrated to afford the crude title product (**173c**) (159 mg) as a yellow oil, which was used in the next step without further purification. *m*/*z* (ESI, +ve ion) = 345.3 [M+H]⁺.

### Step D. Methyl 2-((4-((2-(5-chloropyridin-2-yl)-2-methylbenzo[d][1,3]dioxol-4-yl)methyl)piperidin-1-yl)methyl)-1-(((S)-oxetan-2-yl)methyl)-1H-benzo[d]imidazole-6-carboxylate (173d)

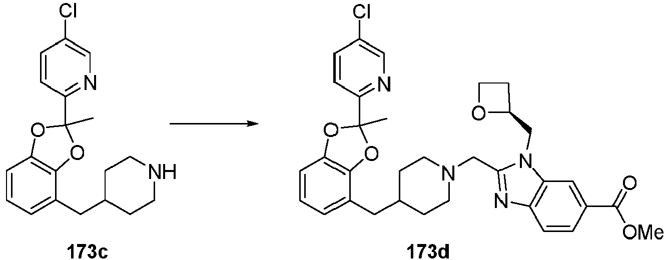

A solution of **173c** (9.8 mg, 0.028 mmol), **Ih** (8.8 mg, 0.030 mmol) and DIPEA (0.099 mL, 0.57 mmol) in DMF (0.5 mL) was stirred at room temperature overnight. The reaction mixture was diluted with EtOAc (5 mL). The organic layer was washed with water (3 mL × 2), brine (3 mL), dried over Na₂SO₄, filtered and concentrated to give a crude residue, which was purified by silica gel column chromatography (gradient elution, 80-100% EtOAc in hexanes and then 0-20% MeOH in DCM) to afford the title product (**173d**) (6 mg, 35%) as a colorless oil. *m*/*z* (ESI, +ve ion) = 603.3 [M+H]⁺.

### Step E. 2-((4-((2-(5-Chloropyridin-2-yl)-2-methylbenzo[d][1,3]dioxol-4-yl)methyl)piperidin-1-yl)methyl)-1-(((S)-oxetan-2-yl)methyl)-1H-benzo[d]imidazole-6-carboxylic acid (173)

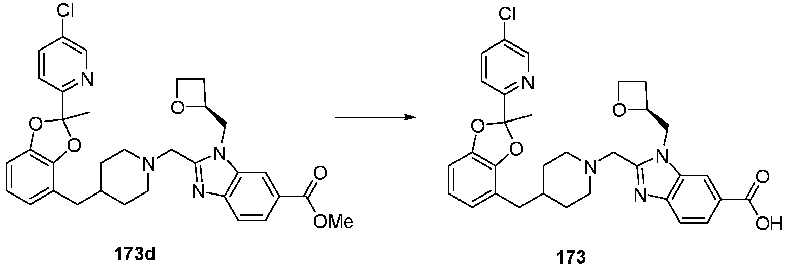

A solution of **173d** (6 mg, 0.010 mmol) and LiOH (2.1 mg, 0.05 mmol) in THF:MeOH:H₂O = 2:1:1 (0.4 mL) was stirred at 30 °C overnight. After completion, reaction mixture was diluted with water to 1 mL and purified by reverse phase HPLC (gradient elution, 30-70% CH₃CN in H₂O, with 0.1% TFA as a modifier) to afford the title product (**173**) (6.9 mg, 99%) as a white solid. *m*/*z* (ESI, +ve ion) = 589.3 [M+H]⁺. ¹H NMR (600 MHz, CD₃OD) δ ppm 8.60 (br t, *J* = 2.4 Hz, 1H), 8.33 (br d, *J* = 1.10 Hz, 1H), 8.04 (dd, *J* = 8.4, 1.5 Hz, 1H), 7.88 (dd, *J* = 8.44 Hz, 1H), 7.81 (d, *J* = 8.4 Hz, 1H), 7.65 - 7.68 (m, 1H), 6.81 (t, *J* = 1.0 Hz, 1H), 6.76 (d, *J* = 1.0 Hz, 1H), 6.69 (br d, *J* = 7.7 Hz, 1H), 5.17 - 5.23 (m, 1H), 4.55 - 4.80 (m, 5H), 4.34 - 4.41 (m, 1H), 3.67 - 3.78 (m, 2H), 3.10 - 3.25 (m, 2H), 2.75 - 2.84 (m, 1H), 2.65 (br d, *J* = 6.6 Hz, 2H), 2.48 (ddd, *J* = 16.9, 11.4, 7.3 Hz, 1H), 2.02 (s, 3H), 1.80 - 2.01 (m, 3H), 1.46 - 1.64 (m, 2H).

### Examples 172, 184, and 185 were synthesized in similar procedures as described in Example 173.

### Example 177. (S)-2-((3-((2-((2,4-Dichlorophenoxy)methyl)pyridin-4-yl)methyl)azetidin-1-yl)methyl)-1-(oxetan-2-ylmethyl)-1H-benzo[d]imidazole-6-carboxylic acid (177)

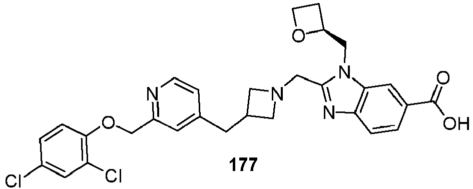

### Step A. 4-Bromo-2-((2,4-dichlorophenoxy)methyl)pyridine (177a)

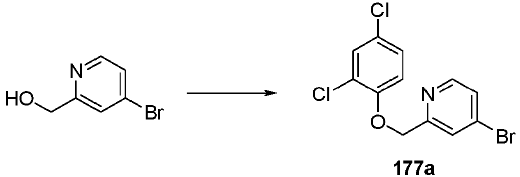

DIAD (5.00 g, 24.7 mmol), PPh₃ (6.49 g, 24.7 mmol) and 2,4-dichlorophenol (2.69 g, 16.5 mmol) were added to (4-bromopyridin-2-yl)methanol (3.10 g, 16.5 mmol) in anhydrous THF (33 mL) at 0 °C. After 1 h, H₂O (30 mL) was added and the reaction was extracted with EtOAc (20 mL × 3). The organic layer was washed with brine (15 mL), dried with Na₂SO₄, filtered, and concentrated to give a crude residue, which was purified by silica gel column chromatography (gradient elution, 0-50% EtOAc in hexanes), and further purified through recrystallization with EtOAc and hexanes to provide the title product (**177a**) (4.65 g, 85%). *m*/*z* (ESI, +ve ion) = 334.0 [M + H]⁺.

### Step B. tert-Butyl 3-((2-((2,4-dichlorophenoxy)methyl)pyridin-4-yl)methylene)azetidine-1-carboxylate (177b)

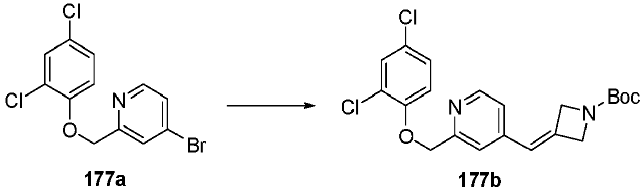

To a solution of **177a** (1.2 g, 3.60 mmol) in anhydrous CH₃CN (36 mL) was added Pd(OAc)₂ (162 mg, 0.72 mmol), tri-o-tolylphosphane (439 mg, 1.44 mmol), *tert-*butyl *3-*methyleneazetidine-1-carboxylate (732 mg, 4.32 mmol) and *i*Pr₂NEt (1.88 mL, 1.40 g, 10.80 mmol) at room temperature. The mixture was flushed with argon for 15 min, and was heated to 110 °C. After 16 h, the reaction was cooled down to room temperature, added sat. NaHCO₃ (30 mL), and extracted with EtOAc (20 mL × 3). The organic layer was washed with brine, dried with Na₂SO₄, filtered, and concentrated to give a crude residue, which was purified by silica gel column chromatography (gradient elution, 0-40% EtOAc in hexanes) to afford the title product (**177b**) (450 mg, 30%) as a yellow solid. *m*/*z* (ESI, +ve ion) = 421.4 [M + H]⁺.

### Step C. tert-Butyl 3-((2-((2,4-dichlorophenoxy)methyl)pyridin-4-yl)methyl)azetidine-1-carboxylate (177c)

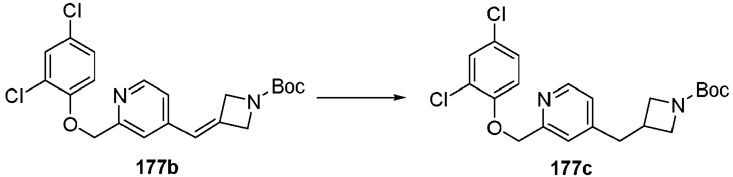

PtO₂ (1.4 mg, 0.006 mmol) was added to **177b** (50 mg, 0.12mmol) in EtOAc (0.6 mL) at room temperature. The mixture was degassed with hydrogen for 10 min, then stirred under a H₂ balloon for 16 h. The reaction was filtered through a pad of celite, rinsed with EtOAc and concentrated to afford the crude title product (**177c**) (55 mg), which was used in the next step without further purification. *m*/*z* (ESI, +ve ion) = 423.3 [M + H]⁺.

### Step D. (S)-2-((3-((2-((2,4-Dichlorophenoxy)methyl)pyridin-4-yl)methyl)azetidin-1-yl)methyl)-1-(oxetan-2-ylmethyl)-1H-benzo[d]imidazole-6-carboxylic acid (177)

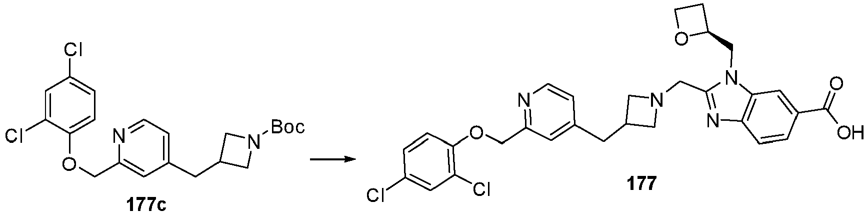

The title product (**177**) was synthesized in similar procedures from **177c** as described in Example **173,** Steps C to E. ¹H NMR (600 MHz, MeOD) δ ppm 8.53 (d, *J* = 5.14 Hz, 1H), 8.30 (d, *J* = 1.10 Hz, 1H), 8.00 - 8.04 (m, 1H), 7.75 (d, *J* = 8.07 Hz, 1H), 7.58 (s, 1H), 7.42 (d, *J* = 2.57 Hz, 1H), 7.34 (dd, *J=* 5.14, 1.47 Hz, 1H), 7.27 (dd, *J=* 8.80, 2.57 Hz, 1H), 7.13 (d, *J* = 8.80 Hz, 1H), 5.27 (s, 2H), 5.17 (qd, *J* = 7.09, 2.57 Hz, 1H), 4.91 - 5.01 (m, 2H), 4.61 - 4.68 (m, 2H), 4.52 - 4.59 (m, 1H), 4.41 - 4.50 (m, 2H), 4.37 (dt, *J* = 9.26, 6.01 Hz, 1H), 4.17 - 4.27 (m, 2H), 3.37 (dt, *J* = 16.41, 8.12 Hz, 1H), 3.14 - 3.23 (m, 2H), 2.79 (dtd, *J*=11.51, 8.09, 8.09, 6.05 Hz, 1H), 2.38 - 2.51 (m, 1H). *m*/*z* (ESI, +ve ion) = 567.3 [M+H]⁺

Example compounds **175, 176, 180, 181,** and **190** were synthesized in similar procedures as described in Example **177.**

Example **226** was synthesized in similar procedures as described in Example **177,** without the hydrogenation step.

### Example 192. (S)-2-((3-(3-((2,4-Dichlorophenoxy)methyl)phenoxy)azetidin-1-yl)methyl)-1-(oxetan-2-ylmethyl)-1H-benzo[d]imidazole-6-carboxylic acid (192)

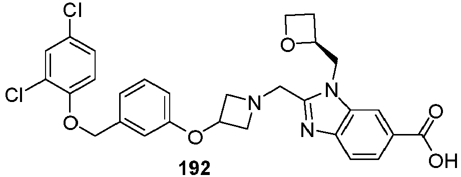

### Step A. tert-Butyl 3-(tosyloxy)azetidine-1-carboxylate (192a)

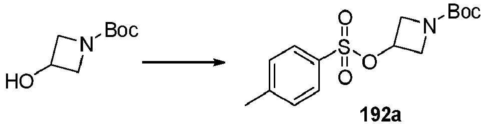

A solution of *tert-*butyl 3-hydroxyazetidine-1-carboxylate (1.99 g, 11.5 mmol) in DMF (5 mL) was stirred at 0 °C for 10 min. 4-Methylbenzenesulfonyl chloride (2.41 g, 12.6 mmol) and Et₃N (11.2 g, 34.5 mmol) were added at 0 °C. The resulting mixture was raised to room temperature and stirred for 16 h. After completion, the reaction solution was concentrated to remove the solvents. H₂O (10 mL) was added and the reaction was extracted with EtOAc (20 mL × 3). The organic layer was dried, filtered and concentrated to afford the title product (**192a**) (2.5 g, 63 %) as a yellow oil, which was used in the next step without further purification. *m*/*z* (ESI, +ve ion) = 330.2 [M+H]⁺.

### Step B. tert-Butyl 3-(3-(methoxycarbonyl)phenoxy)azetidine-1-carboxylate (192b)

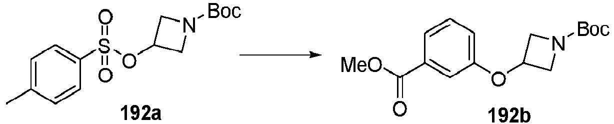

A solution of **192a** (2.5 g, 7.6 mmol), methyl 3-hydroxybenzoate (1.16 g, 7.6 mmol) and Cs₂CO₃ (7.43 g, 22.8 mmol) in DMF (5 mL) was stirred at 80 °C for 3 h. After completion, the reaction solution was diluted with H₂O (10 mL) and extracted with EtOAc (10 mL × 3). The organic layer was dried, filtered and concentrated to give a crude residue, which was purified by silica gel column chromatography (petroleum ether: EtOAc = 10:1) to afford the title product (**192b**) (1.20 g, 49.9%) as a yellow oil. *m*/*z* (ESI, +ve ion) = 330.1 [M+Na]⁺.

### Step C. tert-Butyl 3-(3-(hydroxymethyl)phenoxy)azetidine-1-carboxylate (192c)

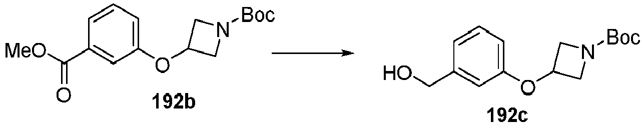

To a solution of **192b** (600 mg, 1.9 mmol) in THF (5 mL) was added LiAlH₄ (36.92 mg, 0.97 mmol). The mixture was stirred at 20 °C for 1 h. After completion, the reaction was quenched with H₂O (2 mL) and extracted with EtOAc (5 mL × 3). The organic layer was dried, filtered and concentrated to give a crude residue, which was purified by silica gel column chromatography (petroleum ether:EtOAc = 5: 1) to afford the title product (**192c**) (460 mg, 84.3%) as a white solid. *m*/*z* (ESI, +ve ion) = 302.2 [M+Na]⁺.

### Step D. tert-Butyl 3-(3-((2,4-dichlorophenoxy)methyl)phenoxy)azetidine-1-carboxylate (192d)

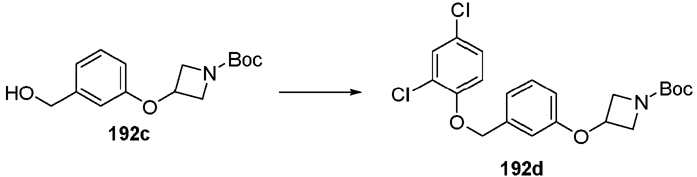

A solution of 2,4-dichlorophenol (122 mg, 0.75 mmol), **192c** (210 mg, 0.75 mmol), PPh₃ (294 mg, 1.12 mmol) and DEAD (195 mg, 1.12 mmol) in anhydrous THF (5 mL) was stirred at 20 °C for 16 h under N₂. After completion, the reaction was diluted with H₂O (2 mL) and extracted with EtOAc (5 mL × 3). The organic layer was dried, filtered and concentrated to give a crude residue, which was purified by silica gel column chromatography (petroleum ether:EtOAc = 5: 1) to afford the title product (**192d**) (148 mg, 45.5 %) as a white solid. *m*/*z* (ESI, +ve ion) = 446.0 [M+Na]⁺.

### Step E. (S)-2-((3-(3-((2,4-Dichlorophenoxy)methyl)phenoxy)azetidin-1-yl)methyl)-1-(oxetan-2-ylmethyl)-1H-benzo[d]imidazole-6-carboxylic acid (192)

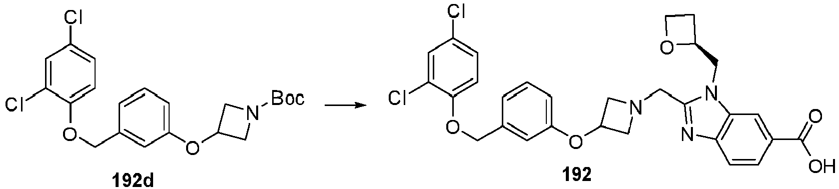

The title product (**192**) was synthesized in similar procedures from **192d** as described in Example **177,** Step D. *m*/*z* (ESI, +ve ion) = 568.3 [M+H]⁺.

Example **194** was synthesized in similar procedures as described in Example **192.**

Examples **210** and **212** was synthesized in similar procedures as described in Example **192.**

### Example 193. (S)-2-((3-((2-((4-Chloro-2-cyanophenoxy)methyl)pyridin-4-yl)oxy)azetidin-1-yl)methyl)-1-(oxetan-2-ylmethyl)-1H-benzo[d]imidazole-6-carboxylic acid

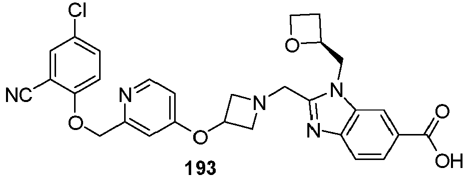

### Step A. tert-Butyl 3-((methylsulfonyl)oxy)azetidine-1-carboxylate (193a)

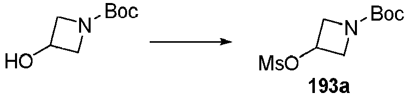

A mixture of *tert*-butyl (3-hydroxyazetidin-1-yl) formate (2.0 g, 11.5 mmol), DIPEA (3.8 mL, 23 mmol) in DCM (10 mL) was added a solution of methanesulfonic anhydride (3.0 g, 17.2 mmol) in DCM (5 mL) at 0 °C. The mixture was stirred at 0 °C for 1.5 h and then concentrated *in vacuo* to give a crude residue, which was purified by silica gel column chromatography (petroleum ether: EtOAc =10:1) to afford the title product (**193a**) (2.9 g, 89%) as a colorless oil. ¹H NMR (400 MHz, CDCl₃) δ ppm 5.20 (s, 1H), 4.27 (ddd, *J* = 10.3, 6.7, 1.1 Hz, 2H), 4.10 (ddd, *J=* 10.3, 4.2, 1.2 Hz, 2H), 3.06 (s, 3H), 1.44 (d, *J* = 2.4 Hz, 9H).

### Step B. 4-((1-(tert-Butoxycarbonyl)azetidin-3-yl)oxy)picolinic acid (193b)

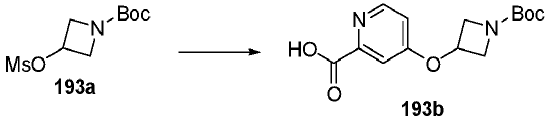

A mixture of **193a** (1.38 g, 5.48 mmol), methyl 4-hydroxypyridine-2-carboxylate (0.7 g, 4.57 mmol), Cs₂CO₃ (2.23g, 6.86 mmol), potassium iodide (151 mg, 0.91 mmol) in DMF (10 mL) was heated at 80 °C for 12 h. The mixture was cooled down to 18 °C and poured into water (10 mL), adjusted to pH = 7 with concentrated hydrochloric acid. The resulting mixture was purified by reverse phase HPLC (41% CH₃CN in H₂O, with 0.5%TFA as a modifier) to afford an impure title product (**193b**) (480 mg) as a white solid. *m*/*z* (ESI, +ve ion) = 295.1 [M+H]⁺.

### Step C. tert-Butyl 3-((2-(hydroxymethyl)pyridin-4-yl)oxy)azetidine-1-carboxylate (193c)

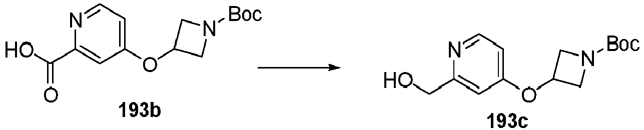

A mixture of **193b** (430 mg, 1.45 mmol) in THF (10 mL) was added BH₃-Me₂S (4.7 mL, 9.5 mmol, 2 N in THF) at 0 °C. The reaction was heated at 40 °C for 2 h and quenched with CH₃OH (15 mL). The resulting mixture was further stirred at 70 °C for 30 min. LCMS showed part of the product still complexed with BH₃. The mixture was concentrated *in vacuo* to give the crude title product (**193c**) which was used in the next step without further purification. *m*/*z* (ESI, +ve ion) = 281.3 [M+H]⁺.

### Step D. tert-Butyl 3-((2-((4-chloro-2-cyanophenoxy)methyl)pyridin-4-yl)oxy)azetidine-1-carboxylate (193d)

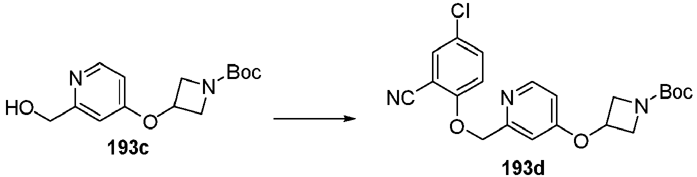

To a mixture of crude **193c** (430 mg, 1.63 mmol), 5-chloro-2-hydroxybenzonitrile (251 mg, 1.63 mmol), PPh₃ (557 mg, 2.12 mmol) in THF (15 mL) was added DEAD (370 mg, 1.12 mmol) at 0 °C under N₂. The reaction was raised to room temperature and stirred for 18 h, poured into water (20 mL), and extracted with EtOAc (15 mL × 3). The organic layer was washed with H₂O (10 mL), brine (10 mL), dried and concentrated *in vacuo* to give a crude residue, which was purified by reverse phase HPLC (69% CH₃CN in H₂O, with 0.05% TFA as a modifier) to afford the title product (**193d**) (35 mg) as a white solid. MS (ESI, pos. ion) m/z: 416.2 (M+1).

### Step E. (S)-2-((3-((2-((4-Chloro-2-cyanophenoxy)methyl)pyridin-4-yl)oxy)azetidin-1-yl)methyl)-1-(oxetan-2-ylmethyl)-1H-benzo[d]imidazole-6-carboxylic acid (193)

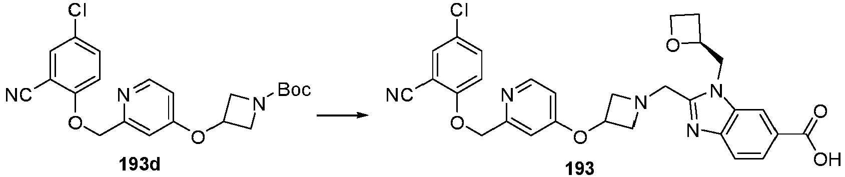

The title product (**193**) was synthesized in similar procedures from **193d** as described in Example **177,** Step D. *m*/*z* (ESI, +ve ion) = 560.3 [M+H]⁺.

Examples **211** and **218** were synthesized in similar procedures as described in Example **193.**

### Example 197. 2-((3-(3-((2,4-Dichlorophenoxy)methyl)benzyl)azetidin-1-yl)methyl)-1-((1-ethyl-1H-imidazol-5-yl)methyl)-1H-benzo[d]imidazole-6-carboxylic acid (197)

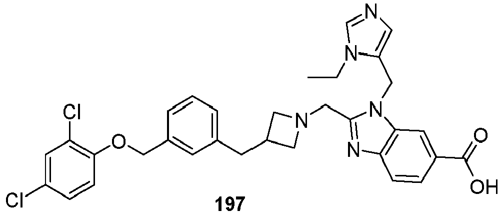

### Step A. 1-((3-Bromobenzyl)oxy)-2,4-dichlorobenzene (197a)

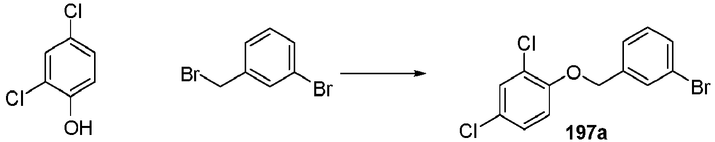

A mixture of 2,4-dichlorophenol (410 mg, 2.5 mmol), 1-bromo-3-(bromomethyl)benzene (629 mg, 2.5 mmol) and K₂CO₃ (695 mg, 5.0 mmol) in DMF (8 mL) was stirred at room temperature for 0.5 h. After completion, the reaction was quenched with water (25 mL), extracted with ethyl acetate (15 mL × 3). The combined organic phase was washed with brine (40 mL), dried with anhydrous Na₂SO₄, filtered, and concentrated to give a crude product which was purified by silica gel column chromatography (EtOAc in hexanes) to afford the title product (**197a**) (692 mg, 41%). *m*/*z* (ESI, +ve ion) = 334.2 [M+H]⁺.

### Step B. tert-Butyl 3-(3-((2,4-dichlorophenoxy)methyl)benzylidene)azetidine-1-carboxylate. (197b)

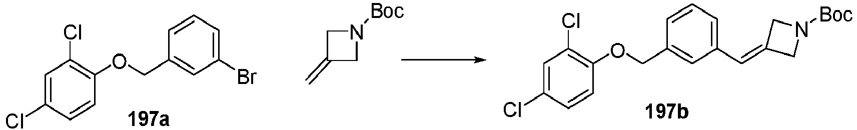

A solution of Pd₂(dba)₃ (169 mg, 0.2 mmol) and BINAP (260 mg, 0.4 mmol) in CH₃CN (3 mL) was heated at 85 °C for 5 min before transferred to a mixture of **197a** (692 mg, 2.1 mmol), *tert*-butyl 3-methylideneazetidine-1-carboxylate (388 mg, 2.3 mmol) and Cs₂CO₃ (1.36 g, 4.2 mmol) in CH₃CN (18 mL). The mixture was degassed with argon and stirred at 85 °C for 16 h under argon. After completion, the solvent was removed *in vacuo* and the residue was purified by silica gel column chromatography (EtOAc in hexanes) to afford the title product (**197b**) (265 mg, 30%) as a dark brown oil. *m*/*z* (ESI, +ve ion) = 442.2 [M+Na]⁺.

### Step C. tert-Butyl 3-(3-((2,4-dichlorophenoxy)methyl)benzyl)azetidine-1-carboxylate (197c)

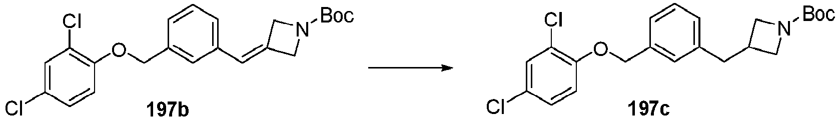

PtO₂ (7.6 mg, 0.3 mmol) was suspended in a solution of **197b** (20 mg, 0.05 mmol) in ethyl acetate (2 mL). The mixture was degassed with hydrogen and stirred at room temperature under a hydrogen balloon for 1 h. After completion, the reaction was filtered and the filtrate was concentrated and purified by silica gel column chromatography (EtOAc in hexanes) to afford the title product (**197c**) (11 mg, 56%) as a colorless oil. *m*/*z* (ESI, +ve ion) = 444.2 [M+Na]⁺.

### Step D. 3-(3-((2,4-Dichlorophenoxy)methyl)benzyl)azetidine (197d)

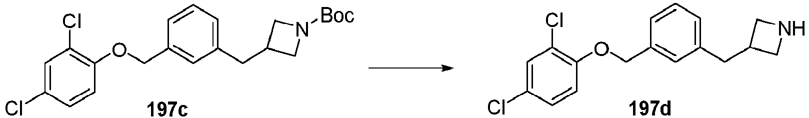

A solution of **197c** (11 mg, 0.03 mmol) in DCM (2 mL) was treated with TFA (1 mL) at room temperature for 10 min. After completion, the solvent was removed *in vacuo,* and the resulting crude was purified by reverse phase HPLC (gradient elution, 0-90% CH₃CN in water, with 0.1% TFA as a modifier) to provide the title product (**197d**) (9.1 mg, 80%) as a white solid. *m*/*z* (ESI, +ve ion) = 322.2 [M+H]⁺.

### Step E. Methyl 2-((3-(3-((2,4-dichlorophenoxy)methyl)benzyl)azetidin-1-yl)methyl)-1-((1-ethyl-1H-imidazol-5-yl)methyl)-imidazol-5-yl)methyl)-1H-benzo[d]imidazole-6-carboxylate (197e)

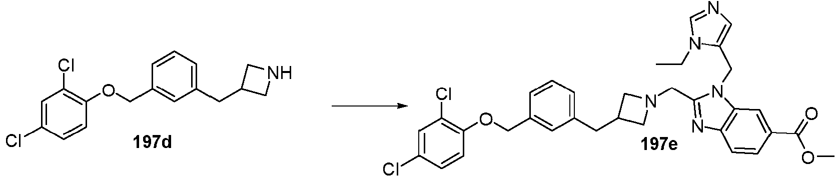

A solution of 197d (9.1 mg, 0.02 mmol), 57f (7.6 mg, 0.023 mmol) and triethylamine (42 mg, 0.42 mmol) in DMF (1 mL) was stirred at room temperature for 22 h. After completion, the reaction was purified by reverse phase HPLC (gradient elution, 20-100% CH₃CN in water, with 0.1% TFA as a modifier) to provide the title product (197e) (8.2 mg, 54%). m/z (ESI, +ve ion) = 618.3 [M+H]⁺.

### Step F. 2-((3-(3-((2,4-Dichlorophenoxy)methyl)benzyl)azetidin-1-yl)methyl)-1-((1-ethyl-1H-imidazol-5-yl)methyl)-1H-benzo[d]imidazole-6-carboxylic acid (197)

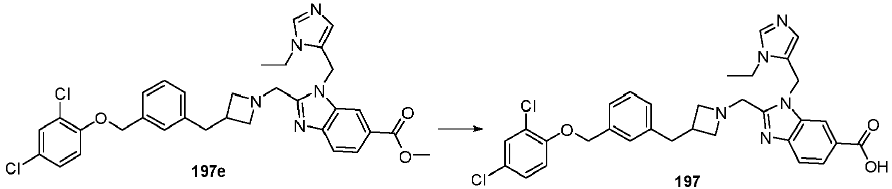

A solution of **197e** (8.2 mg, 0.011 mmol) in CH₃CN (0.5 mL) was treated with 2 N NaOH (0.5 mL). The resulting mixture was stirred at room temperature for 26 h. After completion, the reaction was purified by reverse phase HPLC (gradient elution, 0-75% CH₃CN in water, with 0.1% formic acid as modifier) to provide the title product (**197**) (5.3 mg, 66%) as a white solid. ¹H NMR (600 MHz, CD₃OD) δ ppm 8.16 (br d, *J* = 0.73 Hz, 1H), 7.98 (dd, *J* = 8.44, 1.47 Hz, 1H), 7.79-7.83 (m, 1H), 7.71 (d, *J* = 8.44 Hz, 1H), 7.34 (d, *J* = 2.57 Hz, 1H), 7.25 - 7.33 (m, 3H), 7.20 (dd, *J* = 8.80, 2.57 Hz, 1H), 7.12 - 7.15 (m, 1H), 7.07 (d, *J=* 8.80 Hz, 1H), 6.69 - 6.72 (m, 1H), 5.68 (s, 2H), 5.15 (s, 2H), 4.12 (s, 2H), 4.05 (q, *J* = 7.34 Hz, 2H), 3.65 (br t, *J* = 7.34 Hz, 2H), 3.32 - 3.38 (m, 2H), 2.89 - 2.93 (m, 2H), 2.83 - 2.89 (m, 1H), 1.24 (t, *J* = 7.34 Hz, 3H). *m*/*z* (ESI, +ve ion) = 604.3 [M+H]⁺.

Example compounds **195** and **196** were synthesized in similar procedures as described in Example **197.**

### Example 201. 2-((4-((2-(4-Chloro-2-fluorophenyl)-2-methylbenzo[d][1,3]dioxol-4-yl)methyl)piperidin-1-yl)methyl)-1-(((S)-oxetan-2-yl)methyl)-1H-benzo[d]imidazole-6-carboxylic acid (201)

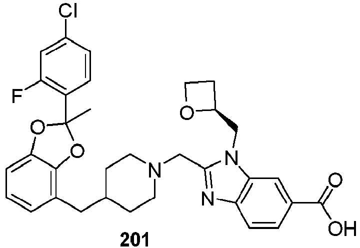

### Step A. 4-Chloro-1-(1,1-dimethoxyethyl)-2-fluorobenzene (201a)

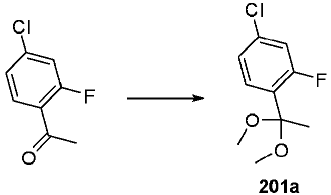

To a solution of 1-(4-chloro-2-fluorophenyl)ethanone (25 g, 0.145 mol) and trimethoxymethane (46 g, 0.435 mol) in MeOH (150 mL) was added TsOH (249 mg, 1.45 mmol). The mixture was stirred at 45 °C for 2 h. Then it was diluted with EtOAc (500 mL), washed with brine (500 mL × 3). The organic layer was dried with Na₂SO₄, filtered and concentrated to afford the crude title product (**201a**) (30 g) as a colorless oil.

### Step B. 4-Bromo-2-(4-chloro-2-fluorophenyl)-2-methylbenzo[d] [1,3]dioxole (201b)

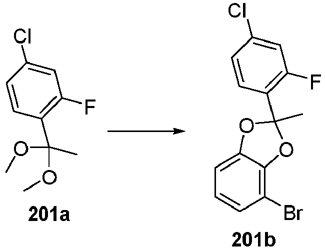

To a solution of **201a** (28 g, 0.128 mol) and 3-bromobenzene-1,2-diol (24.2 g, 0.128 mol) in toluene (200 mL) was added TsOH (44 mg, 0.256 mmol). The mixture was stirred at 80 °C for 2 h. Then it was evaporated to dryness and the crude residue was purified by silica gel column chromatography (petroleum ether) to afford the title product (**201b**, racemic mixture) (12.2 g, 27.7%) as a brown solid. ¹H NMR (400 MHz, CDCl₃) δ ppm 7.54 (t, *J* = 8.4 Hz, 1H), 7.11 - 7.17 (m, 2H), 6.95 (dd, *J* = 8.0, 1.4 Hz, 1H), 6.67 - 6.77 (m, 2H), 2.11 (d*, J* = 1.0 Hz, 3H).

### Step C. tert-Butyl 4-((2-(4-chloro-2-fluorophenyl)-2-methylbenzo[d][1,3]dioxol-4-yl)methyl)piperidine-1-carboxylate (201c)

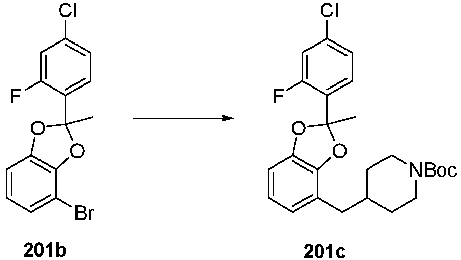

The title product (**201c**) was synthesized in similar procedures from **201b** as described in Example **173,** Step B. *m*/*z* (ESI, +ve ion) = 484.1 [M+Na]⁺.

### Step D. 4-((2-(4-Chloro-2-fluorophenyl)-2-methylbenzo[d][1,3]dioxol-4-yl)methyl)piperidine (201d)

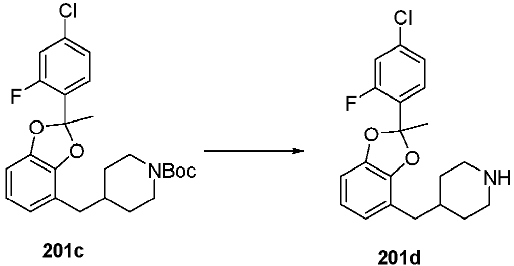

To a solution **of 201c** (350 mg, 0.76 mmol) and 2,6-lutidine (821 mg,7.60 mmol) in DCM (10 ml) was added TMSOTf (843 mg, 3.8 mmol). The reaction was stirred at 0 °C for 2 h and washed with brine (10 mL × 3). The DCM layer was dried, filtered, and concentrated to provide the crude title product (**201d**) (275 mg) as a colorless oil. *m*/*z* (ESI, +ve ion) = 362.1 [M+H]⁺.

### Step E. Methyl 2-((4-((2-(4-chloro-2-fluorophenyl)-2-methylbenzo[d|[1,3]dioxol-4-yl)methyl)piperidin-1-yl)methyl)-1-(((S)-oxetan-2-yl)methyl)-1H-benzo[d]imidazole-6-carboxylate (201e)

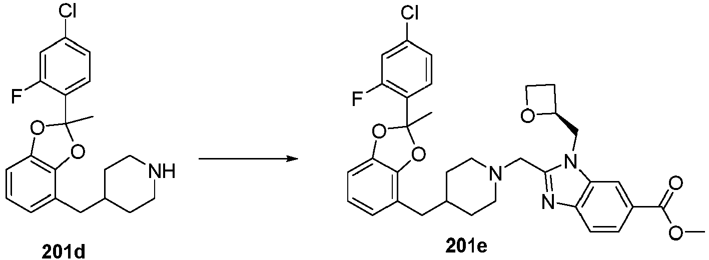

To a solution of **201d** (55 mg, 0.15 mmol) and **Ih** (45 mg, 0.15 mmol) in DMF (3 mL) was added K₂CO₃ (41 mg, 0.30 mmol). After stirring at 20 °C for 16 h, the mixture was diluted with EtOAc (20 mL) and washed with brine (20 mL × 3). The organic layer was dried over Na₂SO₄, filtered, concentrated and the crude residue was purified by silica gel column chromatography (5% MeOH in DCM) to afford the title product (**201e**) (85 mg, 91.4%) as a white solid. *m*/*z* (ESI, +ve ion) = 620.2 [M+H]⁺.

### Step F. 2-((4-((2-(4-Chloro-2-fluorophenyl)-2-methylbenzo[d][1,3]dioxol-4-yl)methyl)piperidin-1-yl)methyl)-1-(((S)-oxetan-2-yl)methyl)-1H-benzo[d]imidazole-6-carboxylic acid (201)

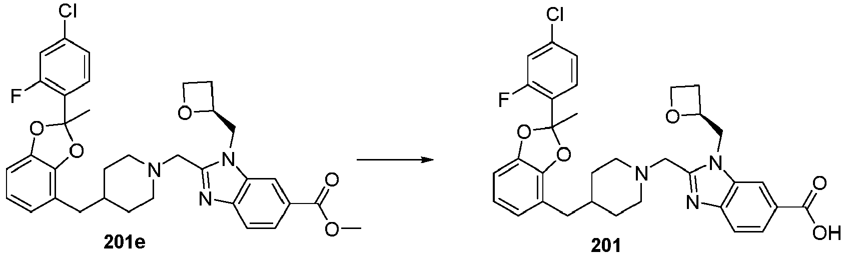

To a solution of **201e** (85 mg, 0.14 mmol) in THF/H₂O (1:1, 3 mL) was added LiOH (6.6 mg, 0.27 mmol). After stirring at 20 °C for 2 h, the reaction was adjusted to pH = 6 with TFA, and purified by reversed phase HPLC (40% CH₃CN/H₂O, with 0.05% TFA as a modifier) to provide the title product (**201**) (97 mg, 98.2%) as a white solid. *m*/*z* (ESI, +ve ion) =606.2 [M+H]⁺. ¹H NMR (400 MHz, CD₃OD) δ ppm 8.33 (s, 1H), 8.03 (dd, *J* = 8.5, 1.5 Hz, 1H), 7.79 (d, *J* = 8.7 Hz, 1H), 7.58 (t, *J* = 8.1 Hz, 1H), 7.25 (dt, *J* = 10.9, 2.4 Hz, 1H), 7.18 - 7.22 (m, 1H), 6.72 - 6.82 (m, 2H), 6.68 (dd, *J* = 7.4, 1.3 Hz, 1H), 5.15 - 5.24 (m, 1H), 4.58 - 4.82 (m, 5H), 4.35 - 4.43 (m, 1H), 3.56 - 3.68 (m, 2H), 3.00 - 3.15 (m, 2H), 2.73 - 2.84 (m, 1H), 2.57 - 2.70 (m, 2H), 2.43 - 2.54 (m, 1H), 2.03 (s, 3H), 1.76 - 2.02 (m, 3H), 1.45 - 1.60 (m, 2H).

Example **209** was synthesized in similar procedures as described in Example **201.**

Example **222** and **223** (stereochemistry arbitrarily assigned) were synthesized as Example **209,** with an added step of SFC chiral separation (Daicel CHIRALCEL AD column, mobile phase: CO₂/IPA with 0.2% NH₃ (7 M solution in MeOH) = 60/40) at the second to last step before final hydrolysis. Example **222** was derived from the early-eluting peak from chiral separation.

### Example 213. (S)-2-((3-((2-((2,4-Dichlorophenoxy)methyl)oxazol-5-yl)oxy)azetidin-1-yl)methyl)-1-(oxetan-2-ylmethyl)-1H-benzo[d]imidazole-6-carboxylic acid (213)

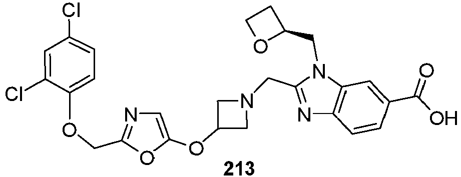

### Step A. tert-Butyl 3-((2-((2,4-dichlorophenoxy)methyl)oxazol-5-yl)oxy)azetidine-1-carboxylate (213a)

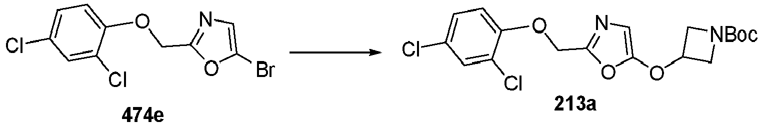

NaH (8.66 mg, 0.22 mmol, 60% dispersion in mineral oil) was added to a solution of tert-butyl 3-hydroxyazetidine-1-carboxylate (25 mg, 0.144 mmol) in anhydrous DMF (0.2 M) at 0 °C under Ar. The mixture was stirred at room temperature for 10 min, then heated to 50 °C for 25 min, followed by addition of **474e** (51.3 mg, 0.16 mmol) at the same temperature. After 30 min, the reaction was quenched with H₂O and extracted with EtOAc (x 2). The combined organic layer was washed with H₂O, brine, dried over Na₂SO₄ and concentrated. The crude residue was purified by silica gel column chromatography (gradient elution, 0-60% EtOAc in hexanes) to provide an impure title product (**213a**) (26 mg). *m*/*z* (ESI, +ve ion) = 415.2 [M+H]⁺.

### Step B. (S)-2-((3-((2-((2,4-Dichlorophenoxy)methyl)oxazol-5-yl)oxy)azetidin-1-yl)methyl)-1-(oxetan-2-ylmethyl)-1H-benzo[d]imidazole-6-carboxylic acid (213)

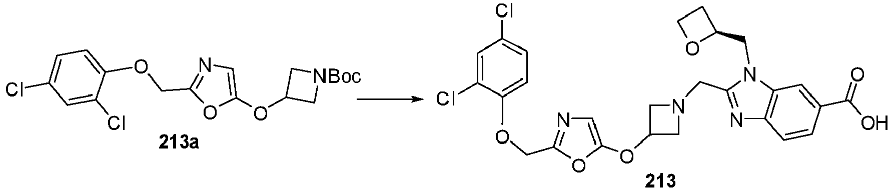

The title product (**213**) was synthesized from **213a** in similar procedures as described in Example **177,** Step D. *m*/*z* (ESI, +ve ion) = 559.2 [M+H]⁺.

### Example 219. 2-((3-((2-((2,4-Dichlorophenoxy)methyl)pyridin-4-yl)oxy)azetidin-1-yl)methyl)-1-(oxazol-5-ylmethyl)-1H-benzo[d]imidazole-6-carboxylic acid (219)

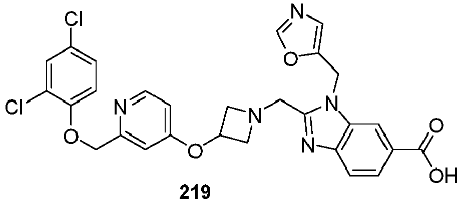

### Step A. tert-Butyl 2-((3-((2-((2,4-dichlorophenoxy)methyl)pyridin-4-yl)oxy)azetidin-1-yl)methyl)-1-(oxazol-5-ylmethyl)-1H-benzo[d]imidazole-6-carboxylate (219a)

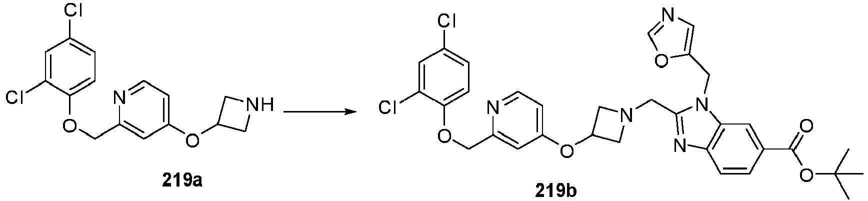

A solution of **219a** (prepared in similar procedures as **193d,** 6.4 mg, 0.020 mmol), tert-butyl 2-(chloromethyl)-1-(oxazol-2-ylmethyl)-1*H*-benzo[*d*]imidazole-6-carboxylate (6.8 mg, 0.020 mmol) and DIPEA (68.6 µL, 0.39 mmol) in DMF (0.2 mL) was stirred at room temperature for 2 h. After completion, the reaction mixture was diluted with EtOAc (5 mL). The organic layer was washed with water (3 mL × 2), brine (3 mL), dried over Na₂SO₄, filtered and concentrated. The resulting crude residue was purified by silica gel column chromatography (gradient elution, 80-100% EtOAc in hexanes then 0-20% MeOH in DCM) to afford the title product **(219b)** (9.6 mg, 77%) as a pale-yellow oil. *m*/*z* (ESI, +ve ion) = 636.3 [M+H]⁺.

### Step B. 2-((3-((2-((2,4-Dichlorophenoxy)methyl)pyridin-4-yl)oxy)azetidin-1-yl)methyl)-1-(oxazol-5-ylmethyl)-1H-benzo[d]imidazole-6-carboxylic acid (219)

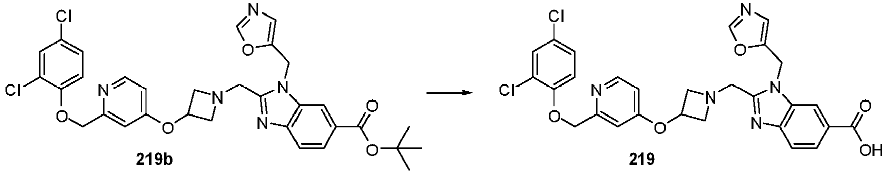

A solution of **219b** (10.0 mg, 0.016 mmol) in DCM (1.0 mL) and TFA (1.0 mL) was stirred at room temperature overnight. After completion, the reaction solvent was removed under reduced pressure. The resulting residue was diluted with water to 1 mL and purified by reverse phase HPLC (gradient elution, 35-65% CH₃CN in H₂O, with 0.1% TFA as a modifier) to afford the title product **(219)** (10.9 mg, 100%) as a white solid. ¹H NMR (600 MHz, CD₃OD) δ ppm 8.58 (d, *J =* 8.2 Hz, 1H), 8.40 (d, *J =* 1.1 Hz, 1H), 8.16 (s, 1H), 8.01 - 8.03 (m, 1H), 7.74 - 7.76 (m, 1H), 7.49 (d, *J* = 2.2 Hz, 1H), 7.38 (d, *J* = 2.6 Hz, 1H), 7.35 (s, 1H), 7.31 (dd, *J =* 8.8, 2.6 Hz, 1H), 7.16 - 7.19 (m, 2H), 5.72 (s, 2H), 5.46 (tt, *J =* 6.2, 4.4 Hz, 1H), 5.33 (s, 2H), 4.98 (s, 2H), 4.78 - 4.88 (m, 2H), 4.48 (br dd, *J =* 12.1, 3.7 Hz, 2H). *m*/*z* (ESI, +ve ion) = 580.2 [M+H]⁺.

Examples **228** (from **192d), 236, 237** (from **192d),** and **238** (from **192d)** were synthesized in similar procedures as described in Example **219.** Example **229** was synthesized in similar procedures as described in Example **228.**

### Example 233. 2-(((2S,3S)-3-(3-((2,4-Dichlorophenoxy)methyl)phenoxy)-2-methylazetidin-1-yl)methyl)-1-((1-ethyl-1H-imidazol-5-yl)methyl)-1H-benzo[d]imidazole-6-carboxylic acid

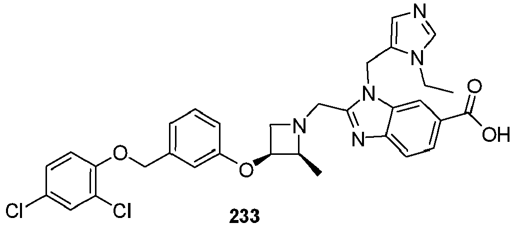

### Step A. tert-Butyl (2S,3S)-3-(3-((2,4-dichlorophenoxy)methyl)phenoxy)-2-methylazetidine-1-carboxylate (233)

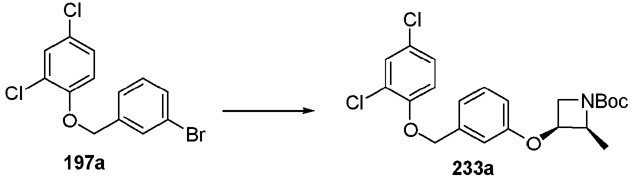

To a solution of **197a** (80 mg, 0.24 mmol) and *tert-*butyl *(2S,3S)*-3-hydroxy-2-methylazetidine-1-carboxylate (45 mg, 0.24 mmol) in anhydrous toluene (2.4 mL) was added Pd₂(dba)₃ (9.6 mg, 0.012 mmol), *t*-BuXphos (5.1 mg, 0.012 mmol), and Cs₂CO₃ (157 mg, 0.48 mmol) at room temperature. The mixture was flushed with argon for 15 min and heated to 90 °C. After stirring for 3 h, the reaction was cooled down to room temperature and 1 N HCl (2 mL) was added. The reaction was extracted with EtOAc (3 mL × 3), dried over Na₂SO₄, filtered, concentrated and the crude residue was purified by silica gel column chromatography (gradient elution, 0-20% EtOAc in hexanes) to afford the title product **(233a)** (41 mg, 43%) as a colorless oil. *m*/*z* (ESI, +ve ion) = 462.3 [M + Na]⁺.

### Step B. 2-(((2S,3S)-3-(3-((2,4-Dichlorophenoxy)methyl)phenoxy)-2-methylazetidin-1-yl)methyl)-1-((1-ethyl-1H-imidazol-5-yl)methyl)-1H-benzo[d]imidazole-6-carboxylic acid (233)

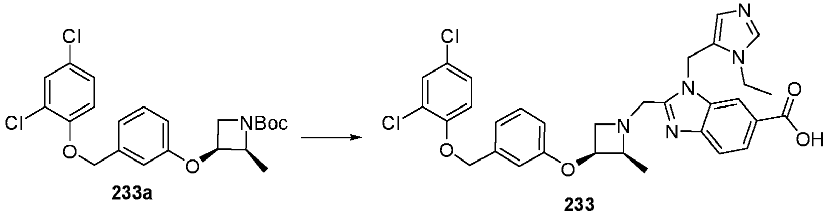

The title product **(233)** was synthesized in similar procedures as described in Example 177, Step D. *m*/*z* (ESI, +ve ion) = 620.3 [M+H]⁺. ¹H NMR (600 MHz, CD₃OD) δ ppm 9.05 (s, 1H), 8.23 (s, 1H), 8.05 (d, *J =* 8.80 Hz, 1H), 7.82 (d, *J =* 8.80 Hz, 1H), 7.41 (d, *J* = 2.57 Hz, 1H), 7.37 (t, *J =* 8.07 Hz, 1H), 7.24 (dd, *J* = 8.80, 2.20 Hz, 1H), 7.14 (br d, *J* = 7.70 Hz, 1H), 7.09 - 7.12 (m, 2H), 7.05 (s, 1H), 6.90 (br d, *J =* 8.44 Hz, 1H), 5.82 (br d, *J =* 2.57 Hz, 2H), 5.24 - 5.29 (m, 1H), 5.18 (s, 2H), 4.58 (br d, *J* = 2.93 Hz, 1H), 4.39 - 4.45 (m, 1H), 4.33 (q, *J*=7.34 Hz, 2H), 1.60 (br d, *J =* 6.97 Hz, 3H), 1.54 (t, *J =* 7.34 Hz, 3H).

Examples **234** and **235** were synthesized in similar procedures as described in Example **233.**

### Example 244. (S)-2-((3-((2-((2,4-Dichlorophenoxy)methyl)pyridin-4-yl)oxy)pyrrolidin-1-yl)methyl)-1-((1-ethyl-1H-imidazol-5-yl)methyl)-1H-benzo[d]imidazole-6-carboxylic acid (244)

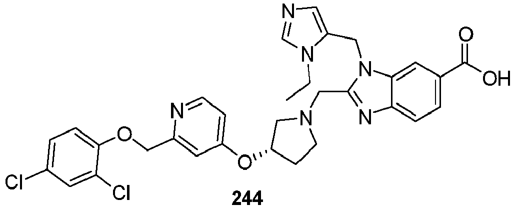

### Step A. Methyl (S)-4-((1-(tert-butoxycarbonyl)pyrrolidin-3-yl)oxy)picolinate (244a)

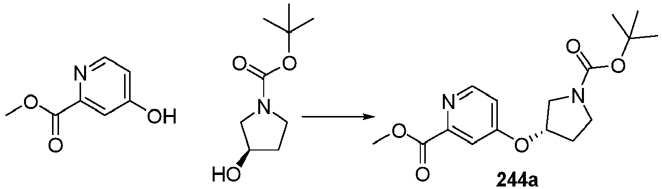

To a stirred suspension of tert-butyl (R)-3-hydroxypyrrolidine-1-carboxylate (128 mg, 0.69 mmol), methyl 4-hydroxypyridine-2-carboxylate (0.1 g, 0.65 mmol) and PPh₃ (214 mg, 0.82 mmol) in THF was added DIAD (165 mg, 0.82 mmol) dropwise at room temperature. After 15 min, reaction was heated at 55 °C overnight. After cooling down, the reaction was filtered over a pad of celite and rinsed with EtOAc. The filtrate was concentrated and purified by silica gel column chromatography (gradient elution, 10-60% acetone in hexanes) to afford an impure product with triphenylphosphine oxide **(244a)** as a white solid. *m*/*z* (ESI, +ve ion) = 323.3 [M+H]⁺.

### Step B. tert-Butyl (S)-3-((2-(hydroxymethyl)pyridin-4-yl)oxy)pyrrolidine-1-carboxylate (244b)

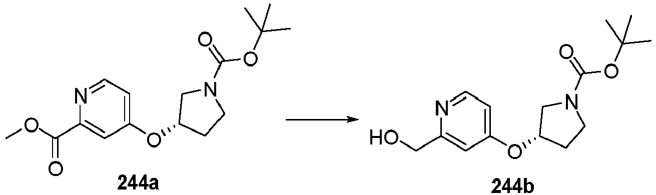

To a stirred solution of **244a** (211 mg, impure, 0.653 mmol) in EtOH (3.3 mL) at 0 °C was added eaCh (217 mg, 1.96 mmol), followed by the addition of NaBH₄ (70.2 mg, 1.96 mmol). After stirring for 1 h at room temperature, the reaction was cooled down in an icebath, quenched with 1 N HCl, diluted with sat. NH₄Cl, and extracted with EtOAc (x 3). The combined organic layer was washed with brine, dried over Na₂SO₄, filtered and concentrated under reduced pressure. The crude residue was purified by silica gel column chromatography (gradient elution, 0 to 15% MeOH in DCM) to provide the title product **(244b)** (87 mg, 45% over 2 steps). *m*/*z* (ESI, +ve ion) = 295.4 [M+H]⁺.

### Step C. (S)-2-((3-((2-((2,4-Dichlorophenoxy)methyl)pyridin-4-yl)oxy)pyrrolidin-1-yl)methyl)-1-((1-ethyl-1H-imidazol-5-yl)methyl)-1H-benzo[d]imidazole-6-carboxylic acid (244)

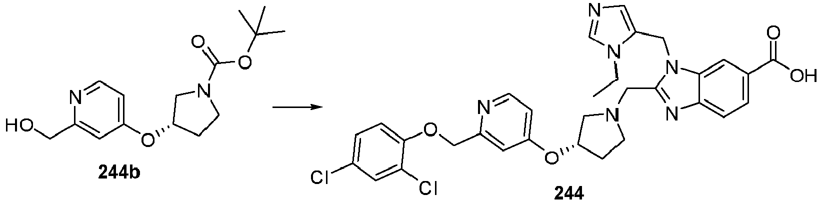

The title product **(244)** was synthesized in similar procedures as described in Example **193,** Steps D and E. *m*/*z* (ESI, +ve ion) = 621.3 [M+H]⁺.

Examples **245, 289,** and **298** were synthesized in similar procedures as described in Example **244.**

Example **273** was prepared in similar procedures from **177a** as described in Example **213,** Step A, Example **147,** Step E, followed by Example **201,** Steps E and F.

Example **293** was synthesized in similar procedures as described in Example **273.**

### Example 281. 2-((4-((3-((2,4-Dichlorophenoxy)methyl)-1H-pyrazol-1-yl)methyl)piperidin-1-yl)methyl)-1-((1-ethyl-1H-imidazol-5-yl)methyl)-1H-benzo[d]imidazole-6-carboxylic acid (281)

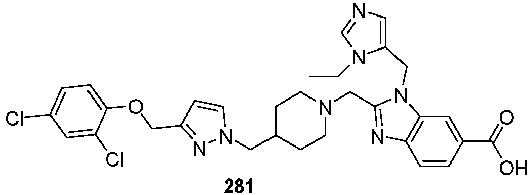

### Step A. tert-Butyl 4-((3-(((methylsulfonyl)oxy)methyl)-1H-pyrazol-1-yl)methyl)piperidine-1-carboxylate (281a)

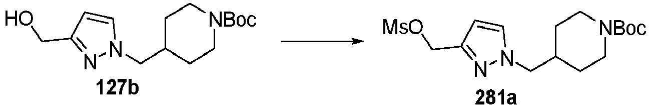

A solution of **127b** (105 mg, 0.36 mmol), MsCl (61 mg, 0.53 mmol) and DIPEA (184 mg, 1.42 mmol) in anhydrous DCM (2 mL) was stirred at 20 °C for 1 h. After completion, the organic layer was dried and concentrated to afford the crude title product **(281a)** (105 mg, 83%) as a yellow oil. *m*/*z* (ESI, +ve ion) = 310.1 [M-OMs+OMe]⁺.

### Step B. tert-Butyl 4-((3-((2,4-dichlorophenoxy)methyl)-1H-pyrazol-1-yl)methyl)piperidine-1-carboxylate (281b)

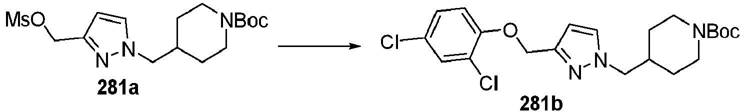

To a solution of 2,4-dichlorophenol (46 mg, 0.28 mmol) and **281a** (105 mg, 0.28 mmol) in DMF (2 mL) was added K₂CO₃ (117 mg, 0.84 mmol) and KI (47 mg, 0.28 mmol). The reaction mixture was stirred at 80 °C for 3 h under N₂. After completion, the mixture was quenched with H₂O (5 mL) and extracted with EtOAc (5 mL × 3). The combined organic layer was washed with H₂O (5 mL), brine (5 mL), dried over anhydrous Na₂SO₄, filtered and concentrated *in vacuo* to give a crude residue, which was purified by prep-TLC (DCM:MeOH = 10:1) to afford the title product **(281b)** (65 mg, 51%) as a yellow oil. *m*/*z* (ESI, +ve ion) = 462.1 [M+Na]⁺.

### Step C. 4-((3-((2,4-Dichlorophenoxy)methyl)-1H-pyrazol-1-yl)methyl)piperidine (281c)

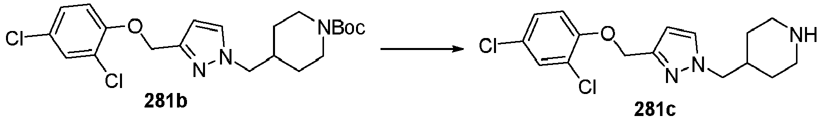

A solution of **281b** (65 mg, 0.15 mmol) in DCM/TFA (2 mL, 4:1) was stirred at 20 °C for 1 h. After completion, saturated aqueous sodium bicarbonate solution was added to the mixture to adjust to pH = 7 in an ice bath. Then the reaction was diluted with H₂O (10 mL) and extracted with EtOAc (10 mL × 3). The combined organic layer was dried, filtered, and concentrated to afford the crude title product **(281c)** (51 mg, 96%) as a yellow oil. *m*/*z* (ESI, +ve ion) = 340.0 [M+H]⁺.

### Step D. Methyl 2-((4-((3-((2,4-dichlorophenoxy)methyl)-1H-pyrazol-1-yl)methyl)piperidin-1-yl)methyl)-1-((1-ethyl-1H-imidazol-5-yl)methyl)-1H-benzo|i/|imidazole-6-carboxylate (281d)

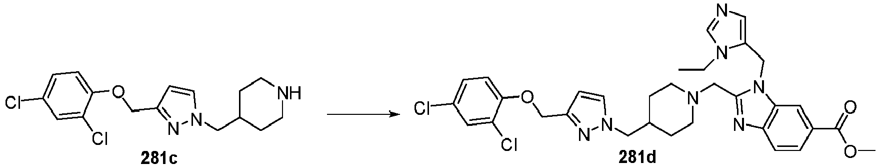

To a solution of **281c** (43 mg, 0.13 mmol) and methyl 1-((1-ethyl-1*H*-imidazol-5-yl)methyl)-2-(((methylsulfonyl)oxy)methyl)-1*H*-benzo[*d*]imidazole-6-carboxylate (51 mg, 0.13 mmol) in DMF (2 mL) was added K₂CO₃ (53 mg, 0.38 mmol) and KI (21 mg, 0.13 mmol) at 20 °C. The reaction was stirred at 20 °C for 3 h under N₂. After completion, the reaction was quenched with H₂O (5 mL) and extracted with EtOAc (5 mL × 3). The combined organic layer was washed with H₂O (5 mL), brine (5 mL), dried over anhydrous Na₂SO₄, filtered and concentrated *in vacuo* to give a crude residue, which was purified by silica gel column chromatography (DCM:MeOH = 10:1) to afford the title product **(281d)** (42 mg, 49.6%) as a yellow oil. *m*/*z* (ESI, +ve ion) = 636.2 [M+H]⁺.

### Step E. 2-((4-((3-((2,4-Dichlorophenoxy)methyl)-1H-pyrazol-1-yl)methyl)piperidin-1-yl)methyl)-1-((1-ethyl-1H-imidazol-5-yl)methyl)-1H-benzo[d]imidazole-6-carboxylic acid (281)

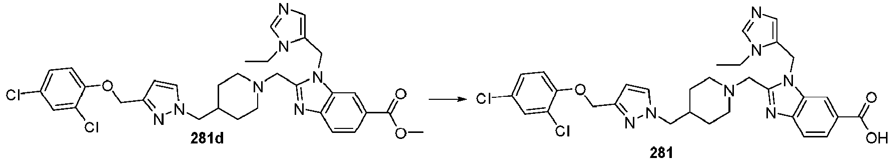

A solution of **281d** (42 mg, 0.066 mmol) and LiOH (8 mg, 0.33 mmol) in THF/H₂O (2 mL, 1:1) was stirred at 20 °C for 12 h. After completion, 1 N HCl was added to the mixture to adjust to pH = 5 in an ice bath and the reaction mixture was extracted with EtOAc (10 mL × 3). The combined organic layer was dried, filtered, concentrated and purified by reversed phase HPLC (30% CH₃CN in H₂O, with 0.05% TFA as a modifier) to afford the title product **(281)** (17.5 mg, 42%) as a white solid. *m*/*z* (ESI, +ve ion) = 622.1 [M+H]⁺.

Example **282** was synthesized in similar procedures as described in Example **281.**

Example **350** was synthesized from *tert*-butyl (3R)-3-hydroxypyrrolidine-1-carboxylate and **Ih** in similar procedures as described in Example **192,** Steps A (MszO was used) and B, then Example **127,** Step B and Example **281,** Steps A to E.

Example **351** was synthesized in similar procedures as described in Example **350.**

### Example 360. 2-(((S)-3-((2-((4-Chloro-2-fluorophenoxy)methyl)pyrimidin-4-yl)oxy)pyrrolidin-1-yl)methyl)-1-(((S)-oxetan-2-yl)methyl)-1H-benzo[d]imidazole-6-carboxylic acid (360)

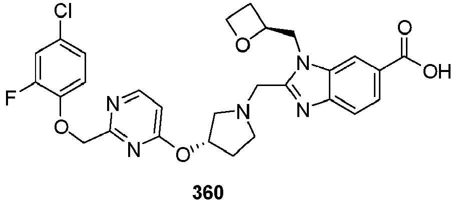

### Step A. 4-Chloro-2-((4-chloro-2-fluorophenoxy)methyl)pyrimidine (360a)

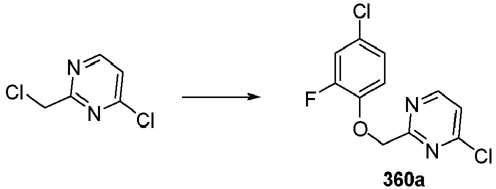

To a solution of 4-chloro-2-(chloromethyl)pyrimidine (230 mg, 1.41 mmol) and K₂CO₃ (388 mg, 2.81 mmol) in DMF (5 mL) was added 4-chloro-2-fluorophenol (206 mg, 1.41 mmol). The reaction was stirred at 25 °C for 16 h, diluted with EtOAc (30 mL) and washed with brine (30 mL × 2). The organic layer was dried, filtered, evaporated to dryness, and purified by silica gel column chromatography (petroleum ether:EtOAc = 2:1) to afford the title product **(360a)** (230 mg, 47.8%). *m*/*z* (ESI, +ve ion) = 269.1 [M+H]⁺.

### Step B. tert-Butyl (S)-3-((2-((4-chloro-2-fluorophenoxy)methyl)pyrimidin-4-yl)oxy)pyrrolidine-1-carboxylate (360b)

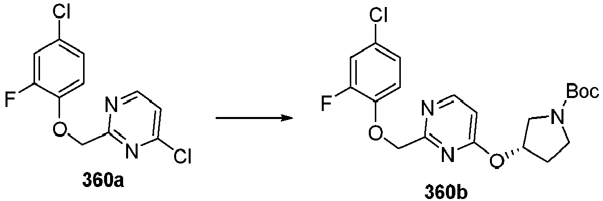

To a solution of tert-butyl (*S*)-3-hydroxypyrrolidine-1-carboxylate (16 mg, 0.88 mmol) in THF (10 mL) was added NaH (35 mg, 0.88 mmol) at 0 °C and stirred at the same temperature for 0.5 h. **360a** (200 mg, 0.73 mmol) was added to the reaction and the resulting mixture was stirred at 25 °C for 16 h. The reaction was diluted with EtOAc (30 mL) and washed with brine (30 mL × 2). The organic layer was dried, filtered, evaporated to dryness and the crude residue was purified by silica gel column chromatography (35% EtOAc in petroleum ether) to afford the title product **(360b)** (210 mg, 67.7%) as a colorless oil. *m*/*z* (ESI, +ve ion) = 424.1 [M+H]⁺.

### Step C. (S)-2-((4-Chloro-2-fluorophenoxy)methyl)-4-(pyrrolidin-3-yloxy)pyrimidine (360c)

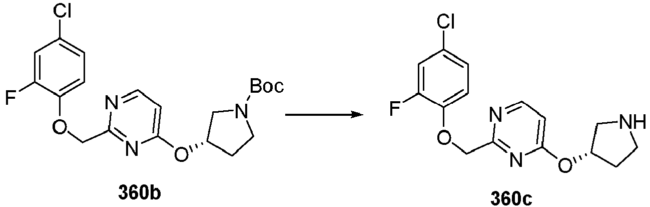

A solution of **360b** (200 mg, 0.47 mmol) in 4 N HCl in dioxane (5 mL) was stirred at 25 °C for 2 h. Then the mixture was concentrated *in vacuo* to afford the crude title product (**360c**) (170 mg) as a white solid, which was used in the next step without further purification. *m*/*z* (ESI, +ve ion) = 324.1 [M+H]⁺.

### Step D. Methyl 2-(((A)-3-((2-((4-chloro-2-fluorophenoxy)methyl)pyrimidin-4-yl)oxy)pyrrolidin-1-yl)methyl)-1-(((S)-oxetan-2-yl)methyl)-1H-benzo[d]imidazole-6-carboxylate (360d)

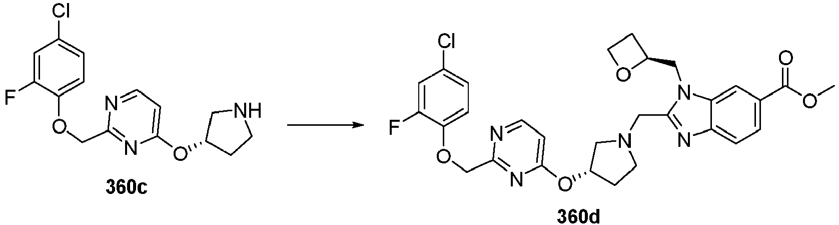

To a mixture of **360c** (35 mg, 0.11 mmol) and K₂CO₃ (30 mg, 0.22 mmol) in DMF (1 ml) was added **Ih** (32 mg, 0.11 mmol). The reaction was stirred at 25 °C for 16 h and filtered. The filtrate was purified by reversed phase HPLC (45% CH₃CN/H₂O, 0.05% TFA as a modifier) to afford the title product **(360d)** (35 mg) with some impurity as a yellow solid. *m*/*z* (ESI, +ve ion) = 582.1 [M+H]⁺.

### Step E. 2-(((5)-3-((2-((4-Chloro-2-fluorophenoxy)methyl)pyrimidin-4-yl)oxy)pyrrolidin-1-yl)methyl)-1-(((S)-oxetan-2-yl)methyl)-1H-benzo[d]imidazole-6-carboxylic acid (360)

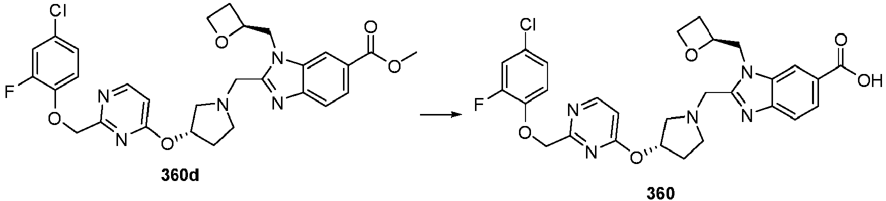

To a solution of **360d** (35 mg, 0.06 mmol) in THF/H₂O (1 mL, 1:1) was added LiOH (3 mg, 0.125 mmol). The reaction was stirred at 25 °C for 2 h. After completion, the reaction was adjusted to pH = 7 with 1 N HCl. The resulting residue was purified by reverse phase HPLC (40% CH₃CN in H₂O) to afford the title product **(360)** (20 mg, 55.8%) as a white solid. *m*/*z* (ESI, +ve ion) = 568.1 [M+H]⁺. ¹H NMR (400 MHz, CD₃OD) δ ppm 8.41 (d, *J* = 5.92 Hz, 1H), 8.29 (s, 1H), 7.95 (dd, *J* = 8.52, 1.4 Hz, 1H), 7.63 (*d, J* = 8.52 Hz, 1H), 7.13 (dd, *J* = 10.36, 1.72 Hz, 1H), 6.94 - 7.06 (m, 2H), 6.77 (d, *J* = 5.92 Hz, 1H), 5.42 (d, *J* = 6.12 Hz, 1H), 5.16 - 5.28 (m, 3H), 4.80 (dd, *J* = 9.84, 5.52 Hz, 1H), 4.56 - 4.71 (m, 2H), 4.43 (dt, *J* = 9.16, 5.92 Hz, 1H), 4.11 (d, *J* = 13.72 Hz, 1H), 3.99 (d, *J* = 13.72 Hz, 1H), 2.83 - 2.96 (m, 2H), 2.76 (dt, *J* = 14.92, 8.4 Hz, 2H), 2.43 - 2.62 (m, 2H), 2.28 (td, *J* = 13.8, 7.60 Hz, 1H), 1.92 (dd, *J* = 8.90, 6.24 Hz, 1H).

Example **361** was synthesized in similar procedures as described in Example 360.

### Example 383. 2-((3-(3-((2,4-Dichlorophenoxy)methyl)phenyl)pyrrolidin-1-yl)methyl)-1-(((S)-oxetan-2-yl)methyl)-1H-benzo[d]imidazole-6-carboxylic acid (383)

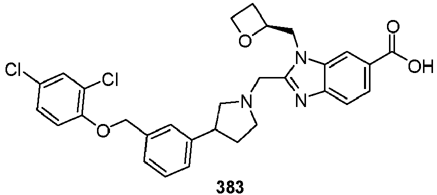

### Step A. tert-Butyl 3-(3-(hydroxymethyl)phenyl)-2,5-dihydro-1H-pyrrole-1-carboxylate (383a)

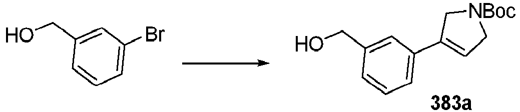

A mixture of of (3-bromophenyl)methanol (450 mg, 2.4 mmol), tert-butyl 3-(4,4,5,5-tetramethyl-1,3,2-dioxaborolan-2-yl)-2,5-dihydro-1*H*-pyrrole-1-carboxylate (0.5 g, 1.6 mmol), Pd(dppf)Cl₂ (0.24 g, 0.32 mmol) and K₂CO₃ (0.67 g, 4.8 mmol) in dioxane/H₂O (10/2.5 mL) was stirred at 95 °C for 2 h. After completion, water (30 mL) was added. The reaction was extracted with EtOAc (10 mL × 3) and the combined organic layer was washed with water (10 mL), brine (10 mL), dried with anhydrous Na₂SO₄, filtered, concentrated and purified by silica gel column chromatography (petroleum ether:EtOAc = 1:1) to afford the title product (383a) (0.42g, 79%, white oil). *m*/*z* (ESI, +ve ion) = 220.1 [M-^{t}Bu+H]⁺.

### Step B. tert-Butyl 3-(3-(((methylsulfonyl)oxy)methyl)phenyl)-2,5-dihydro-1H-pyrrole-1-carboxylate (383b)

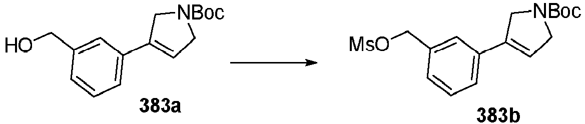

To a solution of **383a** (0.5 g, 1.8 mmol) in THF (10 mL) was added Et₃N (0.367 g, 3.6 mmol) and methanesulfonic anhydride (0.47 g, 2.7 mmol) at 0 °C. The reaction mixture was stirred at 20 °C for 1 h, quenched with water (10 mL), and extracted with EtOAc (10 mL × 3). The combined organic layer was dried over anhydrous Na₂SO₄, filtered, concentrated *in vacuo* to afford the crude title product (383b) (0.62 g) as a white solid. *m*/*z* (ESI, +ve ion) = 376.1 [M+Na]⁺.

### Step C. tert-Butyl 3-(3-((2,4-dichlorophenoxy)methyl)phenyl)-2,5-dihydro-1H-pyrrole-1-carboxylate (383c)

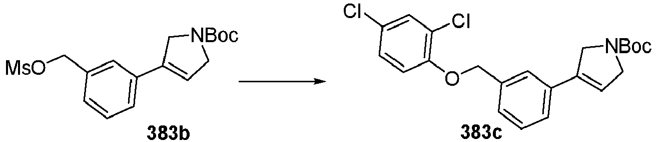

To a solution of **383b** (0.41 g, 1.16 mmol), 2,4-dichlorophenol (0.19 g, 1.16 mmol) in DMF (10 mL) was added Cs₂CO₃ (0.76 g, 2.23 mmol) at 20 °C. After stirring at 55 °C for 1 h, the reaction mixture was quenched with water (10 mL) and extracted with EtOAc (10 mL). The organic layer was washed with H₂O (10 mL), brine (10 mL), dried over anhydrous Na₂SO₄, filtered, concentrated and purified by reverse phase HPLC (70% CH₃CN in H₂O, with 0.05% formic acid as a modifier) to afford the title product **(383c)** (0.42 g, 84%) as a white solid. *m*/*z* (ESI, +ve ion) = 364.0 [M-^{t}Bu+H]⁺.

### Step D. tert-Butyl 3-(3-((2,4-dichlorophenoxy)methyl)phenyl)pyrrolidine-1-carboxylate (383d)

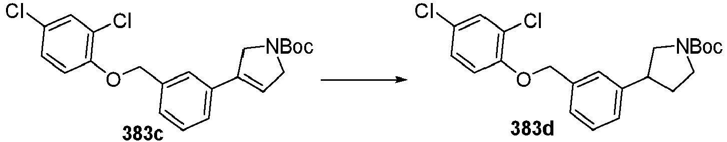

To a solution of **383c** (0.2 g, 0.48 mmol) in THF (5 mL) was added PtO₂ (5.4 mg, 0.024 mmol) at 20 °C. The reaction mixture was stirred at 20 °C for 10 h under 50 psi of H₂. After completion, the reaction was filtered and the filtrate was concentrated under reduced pressure. The crude residue was purified by reverse phase HPLC (70% CH₃CN in H₂O, with 0.05% formic acid as a modifier) to afford the title product **(383d,** racemic) (120 mg, 60%) as a white oil. *m*/*z* (ESI, +ve ion) = 444.0 [M+Na]⁺.

### Step E. 3-(3-((2,4-Dichlorophenoxy)methyl)phenyl)pyrrolidine (383e)

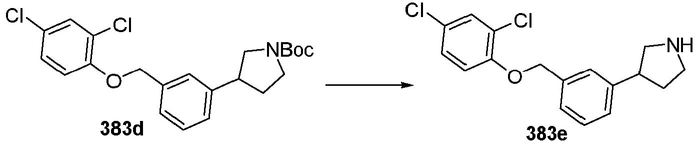

To a solution of **383d** (200 mg, 0.4 mmol) in DCM (2 mL) was added TFA (0.4 mL) at 0 °C. The mixture was stirred at 20 °C for 1 h under N₂. After completion, the mixture was concentrated *in vacuo* to afford the crude title product **(383e)** (102 mg) as a white solid, which was used in the next step without any further purification. *m*/*z* (ESI, +ve ion) = 322.0 [M+H]⁺.

### Step F. Methyl 2-((3-(3-((2,4-dichlorophenoxy)methyl)phenyl)pyrrolidin-1-yl)methyl)-1-(((S)-oxetan-2-yl)methyl)-1H-benzo[d]imidazole-6-carboxylate (383f)

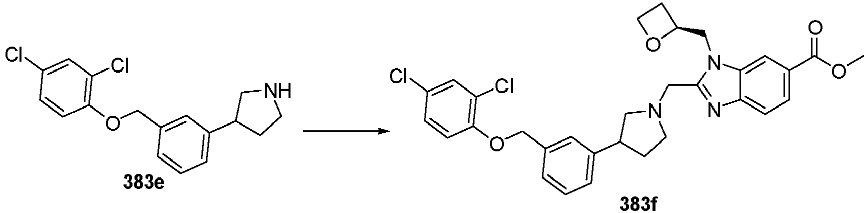

To a solution of **Ih** (76 mg, 0.26 mmol) in CH₃CN (2 mL) was added **383e** (84 mg, 0.26 mmol) and K₂CO₃ (216 mg,1.56 mmol). The reaction mixture was stirred at 20 °C for 5 h. After completion, the mixture was quenched with water (10 mL), extracted with EtOAc (10 mL × 3), washed with water, and brine. The combined organic layer was dried with anhydrous sodium sulfate, filtered, concentrated, and purified by silica gel column chromatography (gradient elution, 0-50% EtOAc in petroleum ether) to afford the title product **(383f)** (50 mg, 34.4%) as a white solid. *m*/*z* (ESI, +ve ion) = 580.2 [M+H]⁺.

### Step G. 2-((3-(3-((2,4-Dichlorophenoxy)methyl)phenyl)pyrrolidin-1-yl)methyl)-1-(((S)-oxetan-2-yl)methyl)-1H-benzo[d]imidazole-6-carboxylic acid (383)

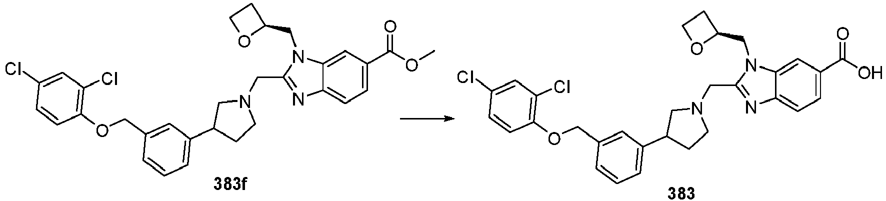

To a solution of **383f** (40 mg, 0.07 mmol) in a mixed solvent of MeOH/THF/H₂O (1:1:1,3 mL) was added LiOH (8.25 mg, 0.7 mmol). The reaction mixture was stirred at 20 °C for 5 h. After completion, the reaction was adjusted to pH = 6 by 10% citric acid, and extracted with EtOAc (20 mL × 3). The combined organic phase was washed with water (20 mL), brine (20 mL), dried with anhydrous sodium sulfate, filtered, concentrated and purified by reversed phase HPLC (60% CH₃CN in H₂O, with 0.05% NH₄HCO₃ as a modifier) to afford the title product **(383)** (15 mg, 38%, diastereomer mixture) as a white solid. *m*/*z* (ESI, +ve ion) = 566.1 [M+H]⁺. ¹H NMR (400 MHz, CD₃OD) δ ppm 8.29 (d, *J* = 0.88 Hz, 1H), 7.97 (dd, *J* = 8.33, 0.88 Hz, 1H), 7.67 (d, *J* = 8.33 Hz, 1H), 7.38 - 7.45 (m, 2H), 7.18 - 7.34 (m, 4H), 7.09 (dd, *J* = 8.99, 2.85 Hz, 1H), 5.18 - 5.27 (m, 1H), 5.15 (s, 2H), 4.83 (d, *J* = 7.0 Hz, 1H), 4.65 - 4.72 (m, 1H), 4.61 (td, *J* = 7.78, 5.92, 1H), 4.38 - 4.44 (m, 1H), 4.26 - 4.35 (m, 1H), 4.18 (dd, *J =* 13.59, 6.58 Hz, 1H), 3.43 - 3.56 (m, 1H), 3.14 - 3.25 (m, 1H), 2.91 - 3.09 (m, 2H), 2.70 - 2.88 (m, 2H), 2.33 - 2.54 (m, 2H), 1.91 - 2.06 (m, 1H).

Examples **400** and **401** were synthesized in similar procedures as described in Example **383,** except without Step D (the hydrogenation step).

### Example 384. (S)-2-((3-(6-((2,4-Dichlorophenoxy)methyl)pyridin-2-yl)-2,5-dihydro-1H-pyrrol-1-yl)methyl)-1-(oxetan-2-ylmethyl)-1H-benzo[d]imidazole-6-carboxylic acid (384)

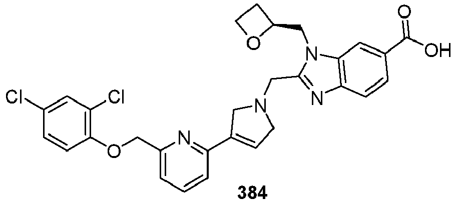

### Step A. tert-Butyl 3-(6-(hydroxymethyl)pyridin-2-yl)-2,5-dihydro-1H-pyrrole-1-carboxylate (384a)

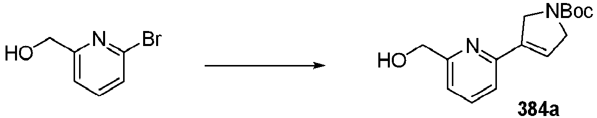

A solution of (6-bromopyridin-2-yl)methanol (191.0 mg, 1.02 mmol), *tert*-butyl 3-(4,4,5,5-tetramethyl-1,3,2-dioxaborolan-2-yl)-2,5-dihydro-1*H*-pyrrole-1-carboxylate (200 mg, 0.68 mmol), Pd(dppf)Cl₂ (99.2 mg, 0.14 mmol) and K₂CO₃ (187.0 mg, 1.36 mmol) in 1,4-dioxane (5.0 mL) and water (1.25 mL) was purged with argon for 5 min. The reaction was heated at 90 °C for 1 h. Upon completion, the reaction mixture was diluted with water and EtOAc. The aqueous layer was extracted with EtOAc (x 3). The combined organic layer was washed with brine, dried over Na₂SO₄, filtered, concentrated and the resulting crude residue was purified by silica gel column chromatography (gradient elution, 30-80% EtOAc in hexanes) to afford the title product **(384a)** (152.0 mg, 81%) as a yellow oil. *m*/*z* (ESI, +ve ion) = 277.4 [M+H]⁺.

### Step B. tert-Butyl 3-(6-((2,4-dichlorophenoxy)methyl)pyridin-2-yl)-2,5-dihydro-1H-pyrrole-1-carboxylate (384b)

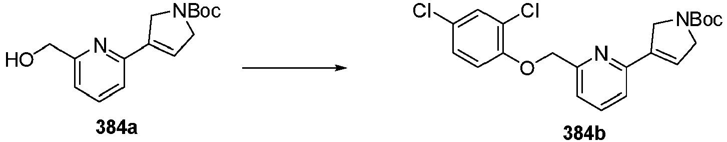

To a solution of **384a** (50.0 mg, 0.18 mmol), 2,4-dichlorophenol (31.0 mg, 0.19 mmol), PPh₃ (71.2 mg, 0.27 mmol) in THF (0.9 mL) was added DIAD (53.4 µL, 0.27 mmol). After stirring at room temperature for 1 h, the reaction solvent was removed under reduced pressure. The resulting residue was purified by silica gel column chromatography (gradient elution, 10-50% EtOAc in hexanes) to afford the title product (384b) (61.0 mg, 80%) as a white solid. *m*/*z* (ESI, +ve ion) = 421.3 [M+H]⁺.

### Step C. 2-((2,4-Dichlorophenoxy)methyl)-6-(2,5-dihydro-tH-pyrrol-3-yl)pyridine 2,2,2-trifluoroacetate (384c)

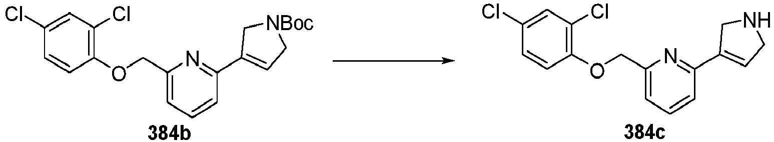

A solution of **384b** (19.0 mg, 0.045 mmol) in DCM (1.0 mL) and TFA (1.0 mL) was stirred at room temperature for 5 min. After completion, the reaction solvent was removed under reduced pressure to afford the crude title product **(384c)** (19.6 mg) as a yellow oil, which was used in the next step reaction without further purification. *m*/*z* (ESI, +ve ion) = 321.3 [M+H]⁺.

### Step D. Methyl (S)-2-((3-(6-((2,4-dichlorophenoxy)methyl)pyridin-2-yl)-2,5-dihydro-1H-pyrrol-1-yl)methyl)-1-(oxetan-2-ylmethyl)-1H-benzo[d]imidazole-6-carboxylate (384d)

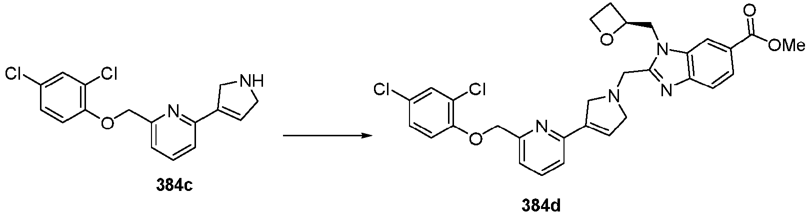

A solution of **384c** (8.8 mg, 0.020 mmol), **Ih** (7.1 mg, 0.024 mmol) and DIPEA (70.4 µL, 0.40 mmol) in DMF (0.2 mL) was stirred at room temperature overnight. After completion, the reaction mixture was diluted with EtOAc (5 mL). The organic layer was washed with water (3 mL × 2), brine (3 mL), dried over Na₂SO₄, filtered and concentrated and the resulting crude residue was purified by silica gel column chromatography (gradient elution, 80-100% EtOAc in hexanes then 0-20% MeOH in DCM) to afford the title product **(384d)** (6.7 mg, 57%) as a white solid. *m*/*z* (ESI, +ve ion) = 579.3 [M+H]⁺.

### Step E. (S)-2-((3-(6-((2,4-Dichlorophenoxy)methyl)pyridin-2-yl)-2,5-dihydro-1H-pyrrol-1-yl)methyl)-1-(oxetan-2-ylmethyl)-1H-benzo[d]imidazole-6-carboxylic acid (384)

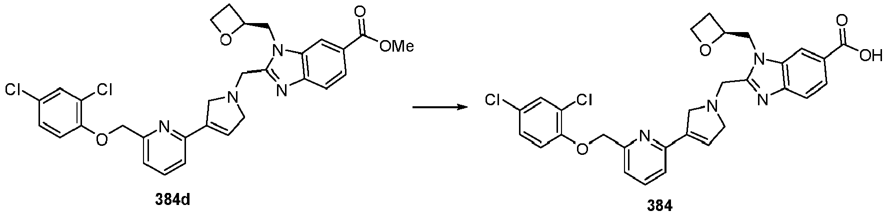

A solution of **384d** (6.7 mg, 0.012 mmol) and LiOH (1.4 mg, 0.058 mmol) in THF:MeOH:H₂O = 2:1:1 (0.4 mL) was stirred at 30 °C overnight. After completion, the reaction mixture was diluted with water to 1 mL and purified by reverse phase HPLC (gradient elution, 35-65% CH₃CN in H₂O, with 0.05% NH₄OH as a modifier) to afford the title product

**(384)** (6.0 mg, 89%) as a white solid. ¹H NMR (600 MHz, DMSO-*d₆*) δ ppm 12.7 (s, 1H), 8.27 (s, 1H), 7.80 - 7.85 (m, 2H), 7.68 (br d, *J* = 8.4 Hz, 1H), 7.59 (br d, *J =* 7.3, 1H), 7.57 (br d, *J* = 2.6 Hz, 1H), 7.39 (br d, *J* = 7.7 Hz, 1H), 7.32 (br dd, *J* = 8.8, 2.6 Hz, 1H), 7.25 (d, *J* = 8.8 Hz, 1H), 6.68 (s, 1H), 5.24 (s, 2H), 5.08 (ddd, *J* = 14.4, 7.3, 2.9 Hz, 1H), 4.81 (br dd, *J* = 15.2, 7.2 Hz, 1H), 4.66 (br dd, *J* = 15.4, 2.9 Hz, 1H), 4.48 (br dd, *J* = 13.4, 7.9 Hz, 1H), 4.36 (dt, *J* = 9.1, 5.9 Hz, 1H), 4.12 - 4.35 (m, 2H), 3.70 - 4.00 (m, 4H), 2.65 - 2.74 (m, 1H), 2.36 - 2.44 (m, 1H). *m*/*z* (ESI, +ve ion) = 565.2 [M+H]⁺.

Examples **385, 388, 393, 402, 403, 417, 418, 426, 436, 437,** and **438** were synthesized in similar procedures as described in Example **384.**

### Example 386. 2-((3-(6-((2,4-Dichlorophenoxy)methyl)pyridin-2-yl)pyrrolidin-1-yl)methyl)-1-(((S)-oxetan-2-yl)methyl)-1H-benzo[d]imidazole-6-carboxylic acid (386)

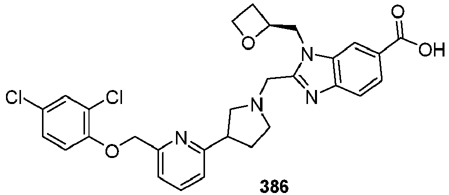

### Step A. tert-Butyl 3-(6-((2,4-dichlorophenoxy)methyl)pyridin-2-yl)pyrrolidine-1-carboxylate (386a)

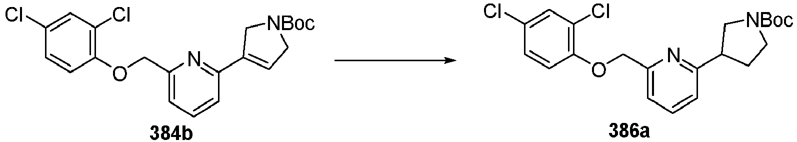

To a solution of **384b** (45 mg, 0.11 mmol) in THF (2 mL) was added PtO₂ (1.22 mg, 0.005 mmol) at 20 °C. The reaction mixture was purged with H₂ for 5 min and stirred overnight under a balloon of H₂. The reaction was filtered and concentrated to provide a crude title product **(386a,** racemic) (7.9 mg, 18%), which was carried to the next step without further purification. *m*/*z* (ESI, +ve ion) = 423.3 [M+H]⁺.

### Step B. 2-((3-(6-((2,4-Dichlorophenoxy)methyl)pyridin-2-yl)pyrrolidin-1-yl)methyl)-1-(((S)-oxetan-2-yl)methyl)-1H-benzo[d]imidazole-6-carboxylic acid (386)

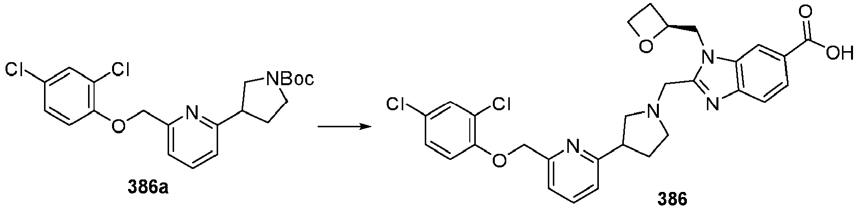

The title product **(386,** diastereomer mixture) was synthesized in similar procedures as described in Example **384,** Steps C to E. *m*/*z* (ESI, +ve ion) = 567.3 [M+H]⁺.

Example **389, 391, 399, 419,** and **420** were synthesized in similar procedures as described in Example **386.**

### Example 390. 2-((3-(4-((2,4-Dichlorophenoxy)methyl)pyridin-2-yl)-2,5-dihydro-1H-pyrrol-1-yl)methyl)-1-(oxazol-5-ylmethyl)-1H-benzo[d]imidazole-6-carboxylic acid (390)

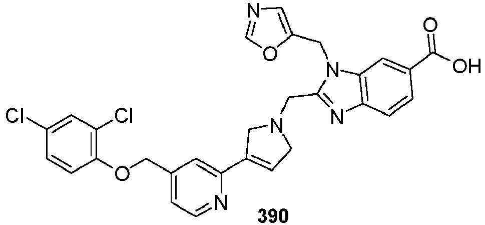

### Step A. 2-((3-(4-((2,4-Dichlorophenoxy)methyl)pyridin-2-yl)-2,5-dihydro-1H-pyrrol-1-yl)methyl)-1-(oxazol-5-ylmethyl)-1H-benzo[d]imidazole-6-carboxylic acid (390)

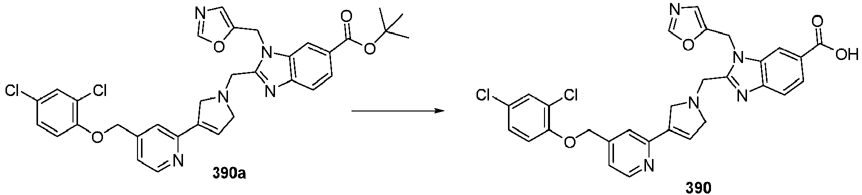

A solution of **390a** (prepared in similar procedures as **384d,** 27.6 mg, 0.044 mmol) in DCM (1.0 mL) and TFA (1.0 mL) was stirred at room temperature overnight. After completion, the reaction solvent was removed under reduced pressure and the resulting residue was diluted with water to 1 mL and purified by reverse phase HPLC (gradient elution, 20-60% CH₃CN in H₂O, with 0.1% TFA as a modifier) to afford the title product **(390)** (15.0 mg, 50%) as a white solid. *m*/*z* (ESI, +ve ion) = 576.3 [M+H]⁺. ¹H NMR (600 MHz, CD₃OD) δ ppm 8.57 (d, *J* = 5.1 Hz, 1H), 8.41 (d, *J* = 1.1 Hz, 1H), 8.15 (s, 1H), 8.04 (dd, *J* = 8.6, 1.7 Hz, 1H), 7.87 (s, 1H), 7.80 (d, *J* = 1.2 Hz, 1H), 7.49 (br d, *J* = 5.1 Hz, 1H), 7.46 (d, *J* = 2.2 Hz, 1H), 7.34 (s, 1H), 7.28 (dd, *J* = 8.8, 2.6 Hz, 1H), 7.13 (d, *J* = 9.2 Hz, 1H), 6.72 - 6.74 (m, 1H), 5.75 (s, 2H), 5.28 (s, 2H), 5.12 (s, 2H), 4.77 - 4.89 (m, 2H), 4.64 (br s, 2H).

Examples **387, 392, 394,** and **404** were synthesized in similar procedures as described in Example **390.**

Example **398** was synthesized in similar procedures as described for Example **397,** except the last step followed Example **390,** Step A.

### Example 395. (S)-2-((3-(2-((4-Cyano-2-fluorophenoxy)methyl)pyrimidin-4-yl)-2,5-dihydro-1H-pyrrol-1-yl)methyl)-1-(oxetan-2-ylmethyl)-1H-benzo[d]imidazole-6-carboxylic acid (395)

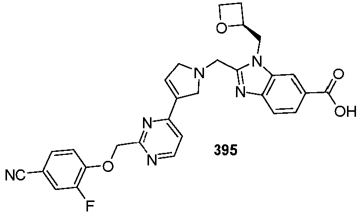

### Step A. 4-((4-Chloropyrimidin-2-yl)methoxy)-3-fluorobenzonitrile (395a)

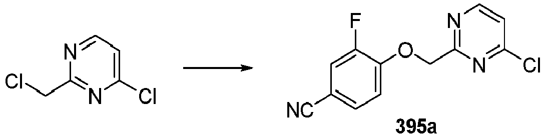

A mixture of 3-fluoro-4-hydroxybenzonitrile (81.8 mg, 0.6 mmol) and 4-chloro-2-(chloromethyl)pyrimidine (81 mg, 0.5 mmol) in DMF (4 mL) was treated with Cs₂CO₃ (324 mg, 1 mmol) at room temperature. The reaction mixture was stirred for 2 h, quenched with water (50 mL) and extracted with EtOAc (20 mL × 3). The combined organic layer was washed with brine, dried with Na₂SO₄, filtered and concentrated *in vacuo.* The crude residue was purified by silica gel column chromatography (EtOAc in hexanes) to provide the title product **(395a)** (93 mg, 71%) as a white solid. *m*/*z* (ESI, +ve ion) = 264.1 [M+H]⁺.

### Step B. tert-Butyl 3-(2-((4-cyano-2-fluorophenoxy)methyl)pyrimidin-4-yl)-2,5-dihydro-1H-pyrrole-1-carboxylate (395b)

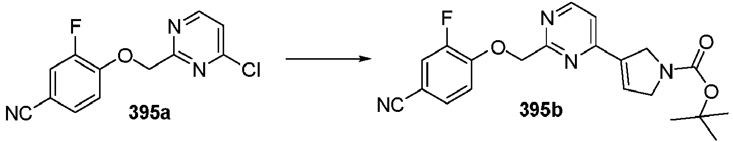

A suspension of **395a** (26 mg, 0.1 mmol), tert-butyl 3-(4,4,5,5-tetramethyl-1,3,2-dioxaborolan-2-yl)-2,5-dihydro-1*H*-pyrrole-1-carboxylate (29 mg, 0.1 mmol) and K₃PO4 (42 mg, 0.2 mmol) in 1,4-dioxane/water (8 mL/4 mL) was degassed with argon and charged with an argon balloon. Pd(dppf)Cl₂ (7.2 mg, 0.01 mmol) was added into the mixture, followed by the same degas and argon protection procedure. The mixture was heated up to 100 °C overnight. Upon completion, the reaction was poured into EtOAc (20 mL) and the aqueous phase was extracted with EtOAc (20 mL × 2). The combined organic layer was dried over Na₂SO₄, filtered, concentrated and purified by silica gel column chromatography (EtOAc in hexanes) to provide the title product **(395b)** (10 mg, 23%). *m*/*z* (ESI, +ve ion) = 397.4 [M+H]⁺.

### Step C. 4-((4-(2,5-Dihydro-1H-pyrrol-3-yl)pyrimidin-2-yl)methoxy)-3-fluorobenzonitrile (395c)

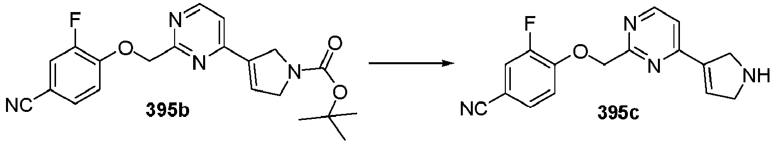

**395b** (10 mg, 0.025 mmol) was dissolved in DCM (2 mL) and treated with TFA (1 mL) at room temperature. After 10 min, the resulting solution was concentrated *in vacuo,* redissolved in DMSO (1 mL) and purified by reverse phase HPLC (gradient elution, 0-90% CH₃CN in water, with 0.1% TFA as a modifier) to provide the title product **(395c)** (7 mg, 68%) as a white solid. *m*/*z* (ESI, +ve ion) = 297.3 [M+H]⁺.

### Step D. Methyl (S)-2-((3-(2-((4-cyano-2-fluorophenoxy)methyl)pyrimidin-4-yl)-2,5-dihydro-1H-pyrrol-1-yl)methyl)-1-(oxetan-2-ylmethyl)-1H-benzo[d]imidazole-6-carboxylate (395d)

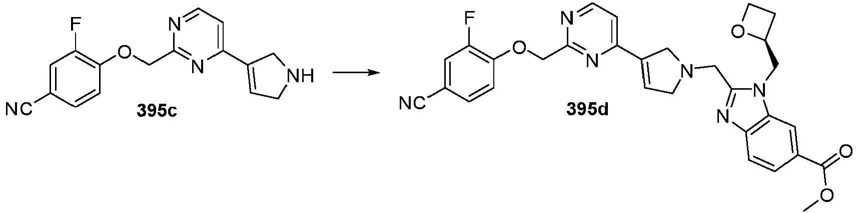

To a solution of **395c** (7 mg, 0.024 mmol) and **Ih** (7 mg, 0.024 mmol) in DMF (1 mL) was added Et₃N (47.8 mg, 0.47 mmol). The reaction mixture was stirred at room temperature overnight then purified by reverse phase HPLC (gradient elution, 0-60% CH₃CN in water, with 0.1% TFA as a modifier) to provide the title product **(395d)** (11 mg, 72%) as a white solid. *m*/*z* (ESI, +ve ion) = 555.3 [M+H]⁺.

### Step E. (S)-2-((3-(2-((4-Cyano-2-fluorophenoxy)methyl)pyrimidin-4-yl)-2,5-dihydro-1H-pyrrol-1-yl)methyl)-1-(oxetan-2-ylmethyl)-1H-benzo[d]imidazole-6-carboxylic acid (395)

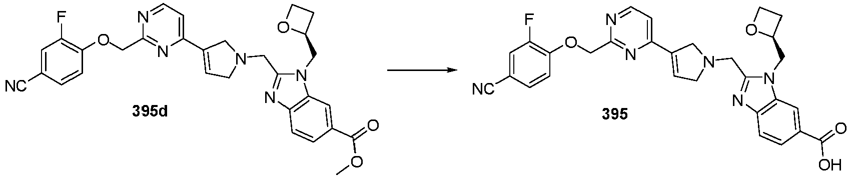

**395d** (10 mg, 0.018 mmol) was dissolved in CH₃CN (1 mL) and treated with 2 N NaOH (0.5 mL). The mixture was stirred vigorously at room temperature for 24 h and then purified by reverse phase HPLC (gradient elution, 0-50% CH₃CN in water, with 0.05% NH₄OH as a modifier) to provide the title product **(395)** (6.3 mg, 63%) as a white solid. *m*/*z* (ESI, +ve ion) = 541.3 [M+H]⁺. ¹H NMR (600 MHz, CD₃OD) δ ppm 8.67 (d, *J* = 5.50 Hz, 1H), 8.25 (d, *J* = 0.73 Hz, 1H), 7.98 (dd, *J* = 8.62, 1.65 Hz, 1H), 7.66 (d, *J* = 8.07 Hz, 1H), 7.50 (d, *J* = 5.50 Hz, 1H), 7.40 - 7.46 (m, 2H), 7.20 - 7.24 (m, 1H), 6.91 - 6.94 (m, 1H), 5.42 (s, 2H), 5.25 (qd, *J* = 7.07, 2.89 Hz, 1H), 4.87 (m, 1H), 4.69 (br dd, *J* = 15.41, 2.93 Hz, 1H), 4.60 - 4.65 (m, 1H), 4.45 (dt, *J* = 9.17, 5.87 Hz, 1H), 4.35 (d, *J* = 13.57 Hz, 1H), 4.27 (d, *J* = 13.57 Hz, 1H), 3.89 - 3.94 (m, 2H), 3.82 - 3.87 (m, 2H), 2.78 (dtd, *J* = 11.23, 8.14, 8.14, 6.24 Hz, 1H), 2.51 (ddt, *J* = 11.28, 8.99, 7.11, 7.11 Hz, 1H).

### Example 396. (S)-2-((3-(6-((4-Chloro-2-fluorophenoxy)methyl)pyrazin-2-yl)-2,5-dihydro-1H-pyrrol-1-yl)methyl)-1-(oxetan-2-ylmethyl)-1H-benzo[d]imidazole-6-carboxylic acid (396)

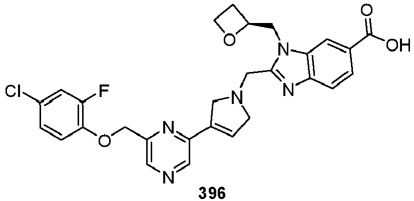

### Step A. Methyl 6-(1-(tert-butoxycarbonyl)-2,5-dihydro-1H-pyrrol-3-yl)pyrazine-2-carboxylate (396a)

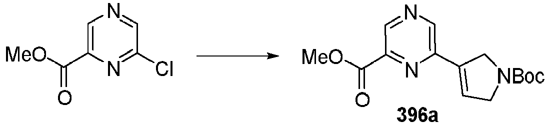

To a solution of methyl 6-chloropyrazine-2-carboxylate (200 mg, 1.16 mmol) and *tert*-butyl 3-(4,4,5,5-tetramethyl-1,3,2-dioxaborolan-2-yl)-2,5-dihydro-1*H*-pyrrole-1-carboxylate (342 mg, 1.16 mmol) in 1,4-dioxane/H₂O (9:1, 3.1 mL) was added Pd(dppf)Cl₂ (42 mg, 0.058 mmol) and Cs₂CO₃ (755 mg, 2.32 mmol) at room temperature. The mixture was flushed with argon for 15 min, then heated to 90 °C. After 2 h, the mixture was cooled down to room temperature and H₂O (2 mL) was added. The reaction was extracted with EtOAc (3 mL × 3). The combined organic layer was dried over Na₂SO₄, filtered, concentrated and purified by silica gel column chromatography (gradient elution, 0-40% EtOAc in hexanes) to afford the title product **(396a)** (263 mg, 74%) as a yellow solid. *m*/*z* (ESI, +ve ion) = 328.3 [M+Na]+.

### Step B. tert-Butyl 3-(6-(hydroxymethyl)pyrazin-2-yl)-2,5-dihydro-1H-pyrrole-1-carboxylate (396b)

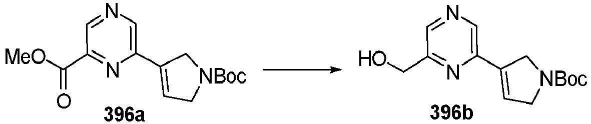

To a solution of **396a** (100 mg, 0.33 mmol) in anhydrous EtOH (3.3 mL) at 0 °C was added NaBH₄ (35 mg, 0.99 mmol) and eaCh (109 mg, 0.99 mmol). After stirring for 1.5 h at room temperature, 1 N HCl (1 mL) was added dropwise at 0 °C and the reaction was extracted with EtOAc (5 mL × 3). The combined organic layer was dried over Na₂SO₄, filtered, concentrated and purified by silica gel column chromatography (gradient elution, 0-15% MeOH in CH₂Cl₂) to provide the title product **(396b)** (35 mg, 39%). *m*/*z* (ESI, +ve ion) = 278.3 [M+H]⁺.

### Step C. tert-Butyl 3-(6-((4-chloro-2-fluorophenoxy)methyl)pyrazin-2-yl)-2,5-dihydro-1H-pyrrole-1-carboxylate (396c)

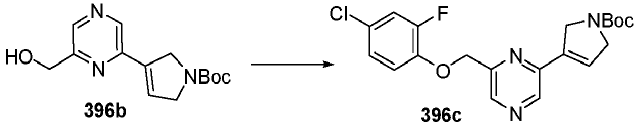

To a solution of **396b** (35 mg, 0.13 mmol) in anhydrous CH₂Cl₂ (0.7 mL) at 0 °C was added PPh₃ (49.7 mg, 0.19 mmol), 4-chloro-2-fluorophenol (18.5 mg, 0.13 mmol), followed by addition of a solution of DCAD (69.5 mg, 0.19 mmol) in anhydrous CH₂Cl₂ (0.7 mL) dropwise. The resulting mixture was stirred for 1 h at room temperature, filtered and concentrated. The crude residue was purified by silica gel column chromatography (gradient elution, 0-40% EtOAc in hexanes) to provide the title product **(396c)** (42 mg, 82%). *m*/*z* (ESI, +ve ion) = 428.3 [M+Na]⁺.

### Step D. (S)-2-((3-(6-((4-Chloro-2-fluorophenoxy)methyl)pyrazin-2-yl)-2,5-dihydro-1H-pyrrol-1-yl)methyl)-1-(oxetan-2-ylmethyl)-1H-benzo[d]imidazole-6-carboxylic acid (16)

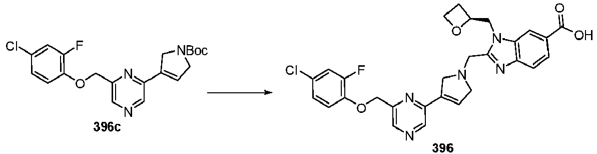

The title product **(396)** was synthesized in similar procedures from **396c** as described in Example **384,** Steps C to E. Formic acid was used as the modifier in the last step purification. m/z (ESI, +ve ion) = 550.2 [M+H]⁺. ¹H NMR (600 MHz, CD₃OD) ppm δ 8.78 (s, 1H), 8.59 (s, 1 H), 8.33 (d, *J* = 0.73 Hz, 1H), 7.99 (dd, *J* = 8.44, 1.47 Hz, 1H), 7.71 (d, *J* = 8.44 Hz, 1H), 7.17 - 7.21 (m, 2H), 7.07 - 7.10 (m, 1H), 6.83 (t, *J =* 2.02 Hz, 1H), 5.23 - 5.28 (m, 3H), 4.87 - 4.91 (m, 1H), 4.72 (dd, *J* = 15.41, 2.57 Hz, 1H), 4.61 - 4.66 (m, 1H), 4.46 (dt, *J* = 9.26, 6.01 Hz, 1H), 4.29 - 4.42 (m, 2H), 4.01 - 4.09 (m, 2H) 3.84 - 3.94 (m, 2H), 2.76 - 2.83 (m, 1H), 2.49 - 2.55 (m, 1H).

### Example 397. 2-((3-(4-((4-Chloro-2-fluorophenoxy)methyl)pyridin-2-yl)pyrrolidin-1-yl)methyl)-1-(((S)-oxetan-2-yl)methyl)-1H-benzo[d]imidazole-6-carboxylic acid (397)

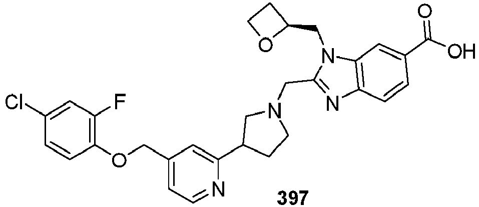

### Step A. tert-Butyl 3-(4-(hydroxymethyl)pyridin-2-yl)pyrrolidine-1-carboxylate (397b)

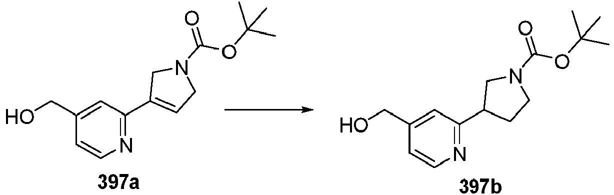

To a solution of **397a** (synthesized as **384a,** 56.5 mg, 0.20 mmol) in THF (5 mL) was added PtO₂ (2.3 mg, 0.01 mmol) at 20 °C. The reaction mixture was purged with H₂ for 5 min and stirred overnight under a balloon of H₂. The reaction was filtered and concentrated to provide a crude title product **(397b,** racemic) with some unreacted starting material **397a,** which was carried to the next step without further purification. *m*/*z* (ESI, +ve ion) = 279.4 [M+H]⁺.

### Step B. tert-Butyl 3-(4-((4-chloro-2-fluorophenoxy)methyl)pyridin-2-yl)pyrrolidine-1-carboxylate (397c)

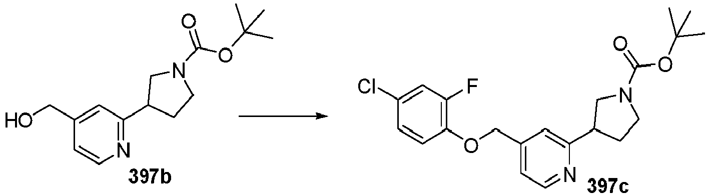

The title product **(397c)** was synthesized from the **397b** in similar procedure as described in Example **384,** Step B. *m*/*z* (ESI, +ve ion) = 407.3 [M+H]⁺.

### Step C. 2-((3-(4-((4-Chloro-2-fluorophenoxy)methyl)pyridin-2-yl)pyrrolidin-1-yl)methyl)-1-(((S)-oxetan-2-yl)methyl)-1H-benzo[d]imidazole-6-carboxylic acid (397)

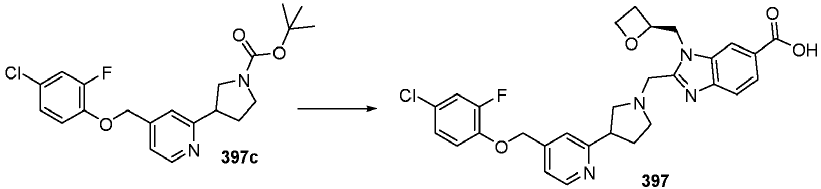

The title product **(397,** diastereomer mixture) was synthesized in similar procedures from **397c** as described in Example **384,** Steps C to E. *m*/*z* (ESI, +ve ion) = 551.3 [M+H]⁺. ¹H NMR (600 MHz, DMSO-*d₆*) δ ppm 8.50 (d, *J =* 5.1 Hz, 1H), 8.08 (s, 1H), 7.77 (dd, *J =* 8.3, 1.3 Hz, 1H), 7.42 - 7.49 (m, 2H), 7.35 (s, 1H), 7.18 - 7.26 (m, 3H), 5.22 (s, 2H), 5.05 (qd, *J =* 7.0, 3.1 Hz, 1H), 4.71 (ddd, *J =* 15.2, 7.0, 4.2 Hz, 1H), 4.57 (ddd, *J =* 15.2, 8.6, 3.3 Hz, 1H), 4.43 - 4.48 (m, 1H), 4.33 (dq, *J =* 9.1, 5.9 Hz, 1H), 4.07 (dd, *J =* 13.4, 5.7 Hz, 1H), 3.90 (dd, *J =* 13.2, 7.7 Hz, 1H), 3.48 - 3.56 (m, 1H), 2.98 - 3.08 (m, 1H), 2.60 - 2.85 (m, 4H), 2.34 - 2.44 (m, 1H), 2.17 - 2.26 (m, 1H), 2.03 (td, *J =* 13.3, 7.15 Hz, 1H). *m*/*z* (ESI, +ve ion) = 551.30 [M+H]⁺.

Example **456** and **457** were synthesized from (6-bromo-5-fluoropyridin-2-yl)methanol as described in Example **397.**

### Examples 405 and 406. 2-(((S)-3-(3-((2,4-Dichlorophenoxy)methyl)phenyl)pyrrolidin-1-yl)methyl)-1-(((S)-oxetan-2-yl)methyl)-1H-benzo[d]imidazole-6-carboxylic acid (405) and 2-(((R)-3-(3-((2,4-dichlorophenoxy)methyl)phenyl)pyrrolidin-1-yl)methyl)-1-(((S)-oxetan-2-yl)methyl)-1H-benzo[d]imidazole-6-carboxylic acid (406)

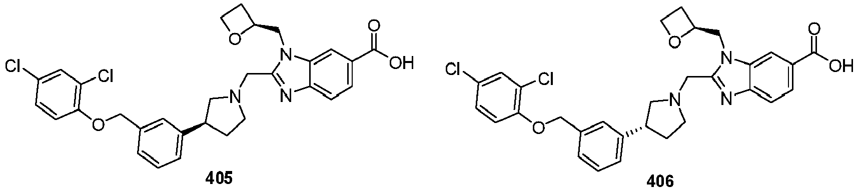

### Step A. tert-Butyl (S)-3-(3-((2,4-dichlorophenoxy)methyl)phenyl)pyrrolidine-1-carboxylate (383d-P1) and tert-butyl (R)-3-(3-((2,4-dichlorophenoxy)methyl)phenyl)pyrrolidine-1-carboxylate (383d-P2)

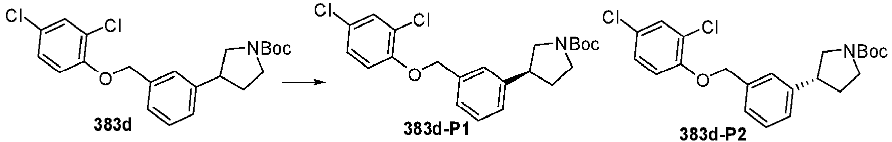

800 mg of **383d** was separated by SFC chiral separation (Acquity UPC 2 column; Daicel CHIRALPAK OX-3 3mm*150mm, 3µm) with a flow rate of 2.0 mL/min. Mobile phase: CO₂/MeOH with 0.1% DEA = 85:15. **383d-P1** (300 mg) and **383d-P2** (305 mg) were obtained respectively (stereochemistry is arbitrarily assigned). **383d-P1** is the early eluting peak.

### Step B. 2-(((S)-3-(3-((2,4-Dichlorophenoxy)methyl)phenyl)pyrrolidin-1-yl)methyl)-1-(((S)-oxetan-2-yl)methyl)-1H-benzo[d]imidazole-6-carboxylic acid (405) and 2-(((R)-3-(3-((2,4-dichlorophenoxy)methyl)phenyl)pyrrolidin-l-yl)methyl)-1-(((S)-oxetan-2-yl)methyl)-1H-benzo[d]imidazole-6-carboxylic acid (406)

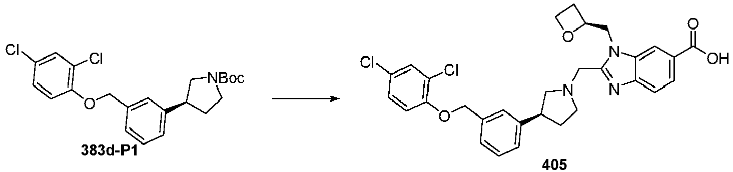

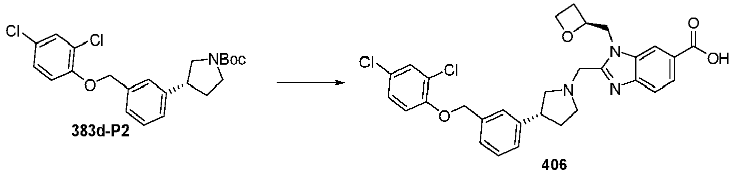

The title product **(405)** was synthesized from **383d-P1** in similar procedures as described in Example **383,** Steps E to G. *m*/*z* (ESI, +ve ion) = 566.1 [M+H]⁺. ¹H NMR (400 MHz, MeOD) δ ppm 8.27 (d, *J* = 0.9 Hz, 1H), 7.96 (dd, *J* = 8.5, 1.5 Hz, 1H), 7.66 (d, *J* = 8.5 Hz, 1H), 7.42 (br s, 1H), 7.39 (d, *J =* 2.5 Hz, 1H), 7.24 - 7.34 (m, 3H), 7.21 (dd, *J =* 8.8, 2.5 Hz, 1H), 7.09 (d, *J=* 8.9 Hz, 1H), 5.16 - 5.25 (m, 1H), 5.15 (d, *J=* 7.4 Hz, 2H), 4.81 - 4.83 (m, 1H), 4.69 (dd, *J* = 15.4, 2.7 Hz, 1H), 4.60 (td, *J* = 7.9, 6.0 Hz, 1H), 4.41 (dt, *J* = 9.1, 6.0 Hz, 1H), 4.28 (d, *J* = 13.8 Hz, 1H), 4.14 (d, *J* = 13.8 Hz, 1H), 3.41 - 3.53 (m, 1H), 3.14 (dd, *J* = 10.8, 6.4 Hz, 1H), 2.88 - 3.03 (m, 2H), 2.81 (dd, *J=* 9.5, 7.2 Hz, 1H), 2.69 - 2.77 (m, 1H), 2.32 - 2.53 (m, 2H), 1.96 (dt, *J =* 15.6, 7.4 Hz, 1H).

The title product **(406)** was synthesized from **383d-P2** in similar procedures as described in Example **383,** Steps E to G. *m*/*z* (ESI, +ve ion) = 566.2 [M+H]⁺. ¹H NMR (400 MHz, MeOD) δ 8.16 (s, 1H), 7.93 (dd, *J* = 8.4, 1.3 Hz, 1H), 7.57 (d, *J* = 8.4 Hz, 1H), 7.40 (d, *J =* 2.5 Hz, 2H), 7.19 - 7.32 (m, 4H), 7.09 (d, *J* = 8.9 Hz, 1H), 5.21 - 5.25 (m, 1H), 5.14 (s, 2H), 4.88 - 4.91 (m, 1H), 4.69 (dd, *J =* 15.3, 2.9 Hz, 1H), 4.56 - 4.63 (m, 1H), 4.43 (dd, *J* = 6.0, 3.1 Hz, 1H), 4.17 (d, *J* = 13.5 Hz, 1H), 4.04 (d, *J* = 13.5 Hz, 1H), 3.38 - 3.45 (m, 1H), 3.07 (t, *J =* 8.5 Hz, 1H), 2.83 - 2.92 (m, 1H), 2.84 - 2.70 (m, 2H), 2.65 (dd, *J* = 9.1, 7.0 Hz, 1H), 2.45 - 2.55 (m, 1H), 2.31 - 2.40 (m, 1H), 1.88 - 1.96 (m, 1H).

### Example 407. (S)-2-((3-(6-((4-Chloro-2-fluorophenoxy)methyl)-5-fluoropyridin-2-yl)-2,5-dihydro-1H-pyrrol-1-yl)methyl)-1-(oxetan-2-ylmethyl)-1H-benzo[d]imidazole-6-carboxylic acid (407)

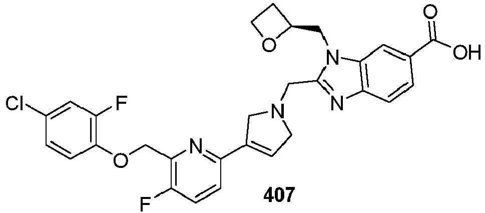

### Step A. tert-Butyl 3-(5-fluoro-6-(hydroxymethyl)pyridin-2-yl)-2,5-dihydro-1H-pyrrole-1-carboxylate (407a)

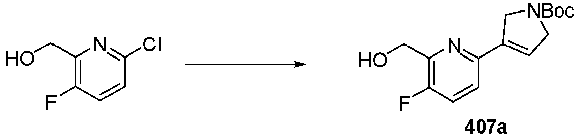

A solution of (6-chloro-3-fluoropyridin-2-yl)methanol (185.0 mg, 1.15 mmol), *tert-*butyl 3-(4,4,5,5-tetramethyl-1,3,2-dioxaborolan-2-yl)-2,5-dihydro-*1H*-pyrrole-1-carboxylate (260 mg, 0.88 mmol), Pd(dppf)Cl₂ (129.0 mg, 0.18 mmol) and K₂CO₃ (243.0 mg, 1.76 mmol) in 1,4-dioxane (5.0 mL) and water (1.25 mL) was purged with argon for 5 min. It was stirred at 90 °C for 2 h. Upon completion, the reaction mixture was diluted with water and EtOAc. The aqueous layer was extracted with EtOAc (x 3). The combined organic layer was washed with brine, dried over Na₂SO₄, filtered and concentrated and the resulting crude residue was purified by silica gel column chromatography (gradient elution, 20-70% EtOAc/hexanes) to afford the title product **(407a)** (183.0 mg, 71%) as a colorless oil. *m*/*z* (ESI, +ve ion) = 317.3 [M+Na]⁺.

### Step B. tert-Butyl 3-(6-((4-chloro-2-fluorophenoxy)methyl)-5-fluoropyridin-2-yl)-2,5-dihydro-1H-pyrrole-1-carboxylate (407b)

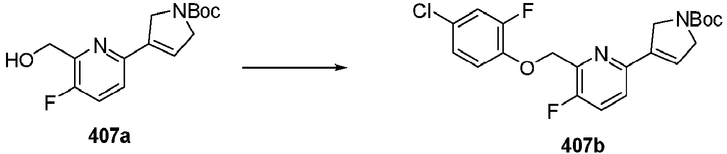

To a solution of **407a** (90.0 mg, 0.31 mmol), 4-chloro-2-fluorophenol (47.1 mg, 0.32 mmol), PPh₃ (120.0 mg, 0.46 mmol) in THF (1.5 mL) was added DIAD (90.3 µL, 0.46 mmol). After stirring at room temperature for 1 h, the reaction solvent was removed under reduced pressure. The resulting crude residue was purified by silica gel column chromatography (gradient elution, 0-40% EtOAc/hexanes) to afford the title product **(407b)** (115.0 mg, 89%) as a white solid. *m*/*z* (ESI, +ve ion) = 445.3 [M+Na]⁺.

### Step C. (S)-2-((3-(6-((4-Chloro-2-fluorophenoxy)methyl)-5-fluoropyridin-2-yl)-2,5-dihydro-1H-pyrrol-1-yl)methyl)-1-(oxetan-2-ylmethyl)-1H-benzo[d]imidazole-6-carboxylic acid (407)

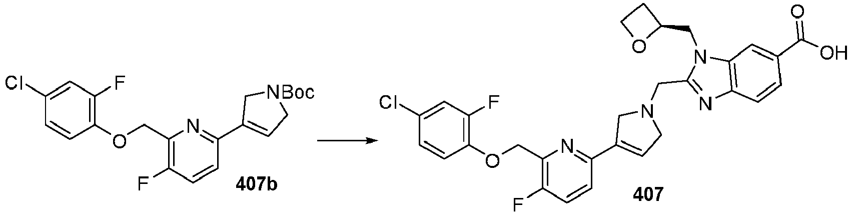

The title product **(407)** was synthesized in similar procedures as described in Example **384,** Steps C to E. *m*/*z* (ESI, +ve ion) = 567.3 [M+H]⁺. ¹H NMR (600 MHz, DMSO-*d*₆) δ ppm 8.00 (s, 1H), 7.72 - 7.79 (m, 3H), 7.34 - 7.42 (m, 3H), 7.17 - 7.20 (m, 1H), 6.60 - 6.63 (m, 1H), 5.27 (s, 2H), 5.07 (ddd, *J*=14.31, 6.97, 3.30 Hz, 1H), 4.70 (br dd, *J*=15.22, 7.15 Hz, 1H), 4.53 - 4.58 (m, 1H), 4.44 - 4.50 (m, 1H), 4.36 (dt, *J*=8.89, 6.01 Hz, 1H), 4.23 (d, *J*=13.57 Hz, 1H), 4.11 (br d, *J*=13.57 Hz, 1H), 3.83 - 3.92 (m, 2H), 3.68 - 3.80 (m, 2H), 2.64 - 2.71 (m, 1H), 2.40 - 2.46 (m, 1H).

Example **408, 413, 416, 421, 422, 424, 429, 430, 440, 451, 452,** and **458** were synthesized in similar procedures as described in Example **407.**

Example **423** was the side product isolated at the last step synthesis of Example **422.**

Example **425** was the side product isolated at the last step synthesis of Example **424.**

Example **409** was synthesized from **407b** in similar procedures as described in Example **386,** Step A, followed by Example **384,** Steps C to E.

Example **410** was synthesized in similar procedures as described in Example **409.**

### Examples 411 and 412. 2-(((S)-3-(3-((4-Chloro-2-fluorophenoxy)methyl)phenyl)pyrrolidin-1-yl)methyl)-1-(((S)-oxetan-2-yl)methyl)-1H-benzo[d]imidazole-6-carboxylic acid (411) and 2-(((R)-3-(3-((4-Chloro-2-fluorophenoxy)methyl)phenyl)pyrrolidin-1-yl)methyl)-1-(((S)-oxetan-2-yl)methyl)-1H-benzo[d]imidazole-6-carboxylic acid (412)

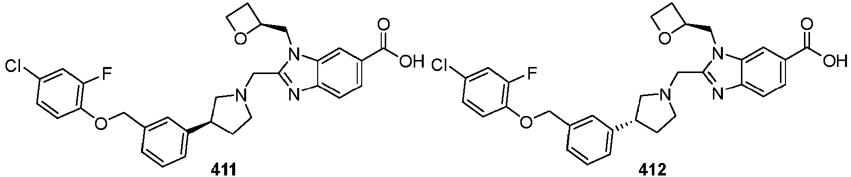

### Step A. Methyl 2-(((S)-3-(3-(hydroxymethyl)phenyl)pyrrolidin-1-yl)methyl)-1-(((S)-oxetan-2-yl)methyl)-1H-benzo[d]imidazole-6-carboxylate (411a-P1) and methyl 2-(((R)-3-(3-(hydroxymethyl)phenyl)pyrrolidin-1-yl)methyl)-1-(((S)-oxetan-2-yl)methyl)-1H-benzo[d]imidazole-6-carboxylate (411a-P2)

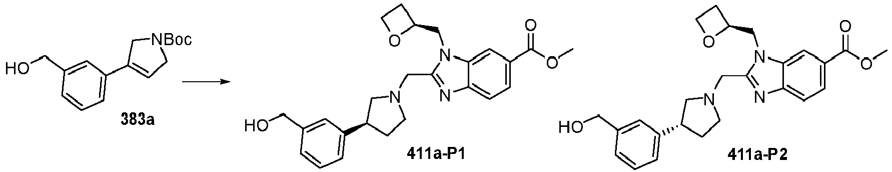

The title products **(411a-P1** and **411a-P2)** were synthesized in similar procedures from **383a** as described in Example **383,** Steps D to F, followed by chiral separation: Daicel CHIRALPAK AZ-3, mobile phase: CO₂/MeOH with 0.2% NH₃ (7M Solution in MeOH) = 60/40. **411a-P1** is the early eluting peak. Stereochemistry is arbitrarily assigned. *m*/*z* (ESI, +ve ion) = 436.2 [M+H]⁺.

### Step B. Methyl 2-(((S)-3-(3-(((methylsulfonyl)oxy)methyl)phenyl)pyrrolidin-1-yl)methyl)-1-(((S)-oxetan-2-yl)methyl)-1H-benzo[d]imidazole-6-carboxylate (411b)

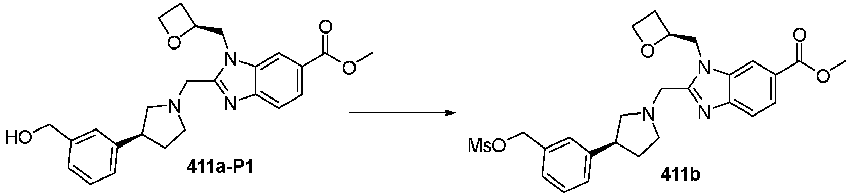

To a stirred solution of **411a-P1** (40 mg, 0.09 mmol), methanesulfonic anhydride (40 mg, 0.23 mmol) in DCM (2 mL) at 25 °C was added DIPEA (36 mg, 0.28 mmol). After stirring for 2 h, the reaction was diluted with H₂O (10 mL) and extracted with EtOAc (10 mL × 3). The combined organic layer was dried and concentrated to afford the crude title product (**411b**) (50 mg, 95%) as a white solid. *m*/*z* (ESI, +ve ion) = 514.2 [M+H]⁺.

### Step C. Methyl 2-(((S)-3-(3-((4-chloro-2-fluorophenoxy)methyl)phenyl)pyrrolidin-1-yl)methyl)-1-(((S)-oxetan-2-yl)methyl)-1H-benzo[d]imidazole-6-carboxylate (411c)

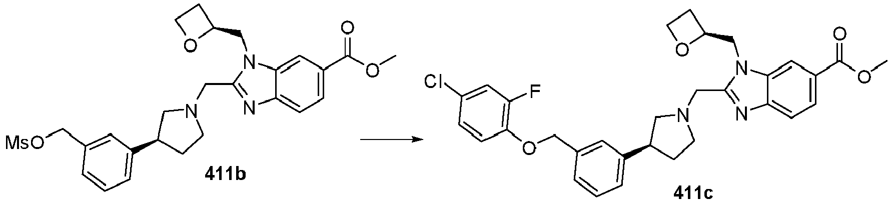

To a stirred solution of **411b** (50 mg, 0.10 mmol), 4-chloro-2-fluorophenol (14 mg, 0.10 mmol) in DMF (2 mL) at 80 °C was added K₂CO₃ (40 mg, 0.29 mmol). After heating for 6 h, the reaction was diluted with H₂O (10 mL) and extracted with EtOAc (10 mL × 3). The combined organic layer was dried, filtered, concentrated and purified by silica gel column chromatography (petroleum ether: EtOAc = 2:1) to afford the title product (411c) (40 mg, 67% yield) as a white solid. *m*/*z* (ESI, +ve ion) = 564.2 [M+H]⁺.

### Step D. 2-(((S)-3-(3-((4-Chloro-2-fluorophenoxy)methyl)phenyl)pyrrolidin-1-yl)methyl)-1-(((S)-oxetan-2-yl)methyl)-1H-benzo[d]imidazole-6-carboxylic acid (411)

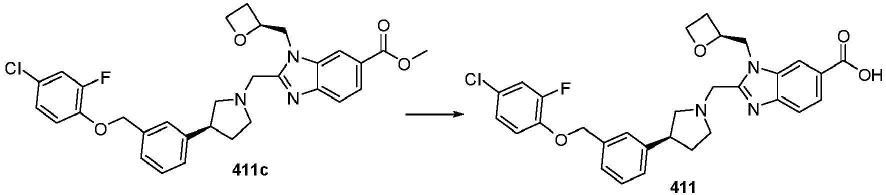

To a solution of **411c** (30 mg, 0.05 mmol) in MeOH (1.0 mL) stirred at 25 °C was added a solution of lithium hydroxide (13 mg, 0.53 mmol) in H₂O (0.5 mL). After stirring for 3 h, 1 N HCl was added to the mixture to adjust to pH = 5 in an ice bath. The reaction was then extracted with EtOAc (5 mL × 3). The cobmined organic layer was dried, filtered, concentrated and purified by silica gel column chromatography (DCM:MeOH = 10:1) to afford the title product **(411)** (24 mg, 78%) as a white solid. *m*/*z* (ESI, +ve ion) = 550.2 [M+H]⁺. ¹H NMR (400 MHz, CD₃OD) δ ppm 8.28 (d, *J* = 0.88 Hz, 1H), 7.96 (dd, *J* = 8.55, 1.53 Hz, 1H), 7.66 (d, *J* = 8.33 Hz, 1H), 7.39 (s, 1H), 7.23 - 7.35 (m, 3 H), 7.17 (dd, *J* = 10.96, 2.19 Hz, 1H), 7.03 - 7.14 (m, 2H), 5.17 - 5.26 (m, 1H), 5.12 (s, 2H), 4.81 - 4.86 (m, 1H), 4.70 (dd, *J* = 15.35, 2.63 Hz, 1H), 4.56 - 4.65 (m, 1H), 4.41 (dt, *J* = 8.99, 6.03 Hz, 1H), 4.10 - 4.31 (m, 2H), 3.41 - 3.52 (m, 1H), 3.14 (t, *J* = 8.77 Hz, 1H), 2.69 - 3.03 (m, 4H), 2.33 - 2.52 (m, 2H), 1.90 - 2.02 (m, 1H).

Example **412** was synthesized in similar procedures from **411a-P2** as described in Example **411.** *m*/*z* (ESI, +ve ion) = 550.2 [M+H]⁺. ¹H NMR (400 MHz, CD₃OD) δ ppm 8.28 (s, 1 H), 7.97 (dd, *J* = 8.56, 1.47 Hz, 1H), 7.66 (d, *J* = 8.31 Hz, 1H), 7.39 (s, 1H), 7.23 - 7.35 (m, 3H), 7.17 (dd, *J* = 10.88, 2.32 Hz, 1H), 7.02 - 7.14 (m, 2H), 5.22 (qd, *J* = 7.17, 2.69 Hz, 1H), 5.12 (s, 2H), 4.88 - 4.91 (m, 1H), 4.69 (dd, *J* = 15.41, 2.69 Hz, 1H), 4.61 (td, *J* = 7.83, 5.87 Hz, 1H), 4.43 (dt, *J* = 9.11, 5.96 Hz, 1H), 4.28 (d, *J* = 13.69 Hz, 1H), 4.11 - 4.19 (m, 1H), 3.47 (dt, *J* = 16.20, 7.92 Hz, 1H), 3.17 (t, *J* = 8.80 Hz, 1H), 2.85 - 3.05 (m, 2H), 2.70 -2.82 (m, 2H), 2.33 - 2.56 (m, 2H), 1.90 - 2.03 (m, 1H).

Examples **414** and **415; 439** and **443** were synthesized in similar procedures as described in Example **411** and **412.**

### Example 427. 2-(((R)-3-(2-((4-Chloro-2-fluorophenoxy)methyl)pyridin-4-yl)pyrrolidin-1-yl)methyl)-4-fluoro-1-(((S)-oxetan-2-yl)methyl)-1H-benzo[d]imidazole-6-carboxylic acid (427)

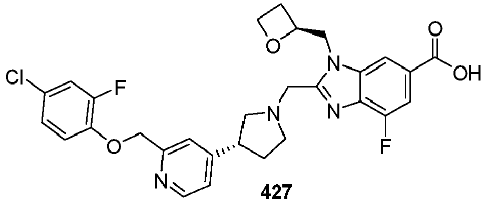

### Step A. tert-Butyl 3-(2-(hydroxymethyl)pyridin-4-yl)pyrrolidine-1-carboxylate (427b)

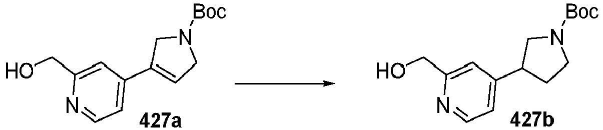

To a stirred solution of **427a** (synthesized as described for **383a,** 4.5 g, 16.2 mmol) in THF (50 mL) under H₂ at 25 °C was added PtO₂ (0.37 g, 1.6 mmol). After stirring for 5 h, the reaction was diluted with H₂O (50 mL) and extracted with EtOAc (20 mL × 3). The combined organic layer was dried, filtered, concentrated and purified by silica gel column chromatography (petroleum ether: EtOAc = 2:1) to afford the title product **(427b)** (2.5 g, 53%) as a colorless oil. *m*/*z* (ESI, +ve ion) = 279.2 [M+H]⁺.

### Step B. tert-Butyl (S)-3-(2-((4-chloro-2-fluorophenoxy)methyl)pyridin-4-yl)pyrrolidine-1-carboxylate (427c-P1) and tert-butyl (R)-3-(2-((4-chloro-2-fluorophenoxy)methyl)pyridin-4-yl)pyrrolidine-1-carboxylate (427c-P2)

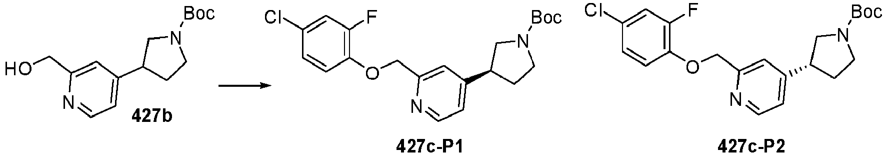

The title products (**427c-P1** and **427c-P2)** were synthesized as described in Example **383,** Steps B and C, followed by chiral separation: chiralpak-OJ column, mobile Phase: CO₂-MeOH with 0.1% DEA. **427c-P1** is the early eluting peak and **427c-P2** is the late eluting peak.

### Step C. (R)-2-((4-Chloro-2-fluorophenoxy)methyl)-4-(pyrrolidin-3-yl)pyridine (427d)

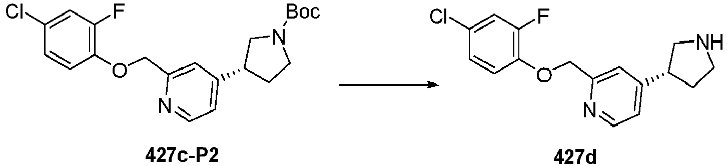

A solution of **427c-P2** (20 mg, 0.05 mmol) in HCl (4 M in 1,4 dioxane, 1 mL) was stirred for 2 h at 25 °C. After completion, 1 N NaOH was added to the reaction to adjust to pH = 8 in an ice bath. The reaction mixture was extracted with EtOAc (15 mL × 3). The combined organic layer was dried, filtered and concentrated to afford the crude title product **(427d)** (15 mg) as a white solid. *m*/*z* (ESI, +ve ion) = 307.1 [M+H]⁺.

### Step D. 2-(((R)-3-(2-((4-Chloro-2-fluorophenoxy)methyl)pyridin-4-yl)pyrrolidin-1-yl)methyl)-4-fluoro-1-(((S)-oxetan-2-yl)methyl)-1H-benzo[d]imidazole-6-carboxylic acid (427)

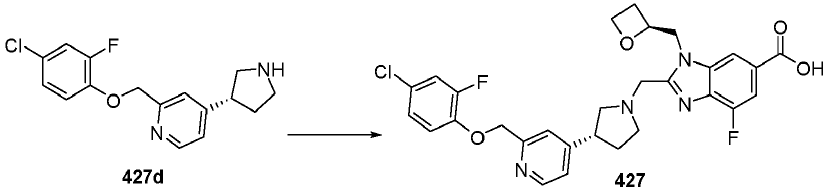

The title product **(427)** was prepared in similar procedures as described in Example **383,** Steps F and G. *m*/*z* (ESI, +ve ion) = 569.2 [M+H]⁺. ¹H NMR (400 MHz, CD₃OD) δ ppm 8.42 (d, *J* = 5.26 Hz, 1H), 8.11 (s, 1H), 7.62 (br d, *J* = 10.96 Hz, 1H), 7.56 (s, 1H), 7.32 (dd, *J* = 5.26, 1.32 Hz, 1H), 7.19 (dd, *J* = 10.96, 2.63 Hz, 1H), 7.04 - 7.15 (m, 2H), 5.14 - 5.25 (m, 3H), 4.84 (br d, *J* = 7.02 Hz, 1H), 4.67 (dd, *J =* 15.57, 2.41 Hz, 1H), 4.55 - 4.63 (m, 1H), 4.41 (dt, *J* = 9.21, 5.92 Hz, 1H), 4.06 - 4.29 (m, 2H), 3.45 - 3.56 (m, 1H), 3.10 (t, *J*=8.55 Hz, 1H), 3.02 (td, *J =* 8.77, 4.39 Hz, 1H), 2.68 - 2.88 (m, 3H), 2.36 - 2.52 (m, 2 H), 1.87 - 1.99 (m, 1 H).

Example **428** was synthesized from **427c-P1** in similar procedures as described in Example **427.** Examples **433** and **434** were synthesized in similar procedures as described in Example **427.**

Examples **431** and **432** were synthesized in similar procedures as described in Example **428.**

### Example 435. (S)-2-((3-(4-((4-Chloro-2-fluorophenoxy)methyl)pyrimidin-2-yl)-2,5-dihydro-1H-pyrrol-1-yl)methyl)-1-(oxetan-2-ylmethyl)-1H-benzo[d]imidazole-6-carboxylic acid (435)

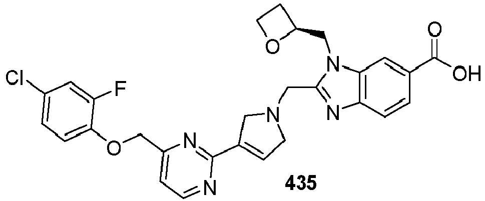

### Step A. (2-Chloropyrimidin-4-yl)methanol (435a)

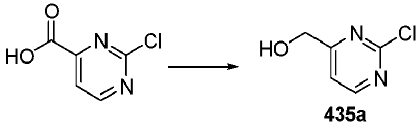

2-Chloropyrimidine-4-carboxylic acid (793 mg, 5 mmol) was dissolved in THF (16.7 mL) and cooled to 0 °C. Triethylamine (704 µL, 5.05 mmol) was slowly added and stirred for 5 min. Isobutylchloroformate (655 µL, 5.05 mmol) was added dropwise and the reaction mixture was warmed to 21 °C and stirred for 1 h. Then the reaction was filtered and rinsed with THF (5 mL). The filtrate was cooled to 0 °C and a solution of NaBH₄ (378 mg, 2 eq., 10 mmol) in water (3 mL) was added dropwise. After stirring at 21 °C for 2 h, the mixture was poured into H₂O (40 mL), and extracted with EtOAc (30 mL × 3). The combined organic layer was washed with brine (15 mL), dried with Na₂SO₄, concentrated, and purified by silica gel column chromatography (gradient elution, 15 to 100% EtOAc in DCM) to provide the tittle product **(435a)** (347 mg, 48%). *m*/*z* (ESI, +ve ion) = 145.2 [M+H]⁺.

### Step B. tert-Butyl 3-(4-(hydroxymethyl)pyrimidin-2-yl)-2,5-dihydro-1H-pyrrole-1-carboxylate (435b)

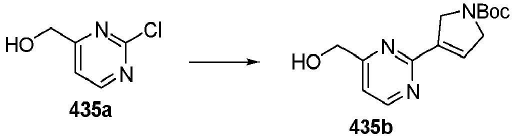

**435a** (347 mg, 2.4 mmol), *tert*-butyl 3-(4,4,5,5-tetramethyl-1,3,2-dioxaborolan-2-yl)-2,5-dihydro-1*H*-pyrrole-1-carboxylate (709 mg, 2.4 mmol), K₂CO₃ (2.4 mL, 4.8 mmol, 2 M in water), and 1,4-dioxane (9.6 mL) were thoroughly purged with N₂ for 5 min. Pd(dppf)Cl₂ (87.8 mg, 120 µmol) was added and the reaction mixture was heated to 90 °C for 3 h. After cooling down, the reaction was diluted with EtOAc (20 mL) and washed with NH₄Cl (5 mL), H₂O (5 mL) and brine (5 mL). The organic layer was dried with Na₂SO₄, concentrated, and purified by silica gel column chromatography (gradient elution, 15 to 100% EtOAc in DCM) to give the title product **(435b)** (461 mg, 69%). *m*/*z* (ESI, +ve ion) = 300.3 [M+Na]⁺.

### Step C. tert-Butyl 3-(4-((4-chloro-2-fluorophenoxy)methyl)pyrimidin-2-yl)-2,5-dihydro-1H-pyrrole-1-carboxylate (435c)

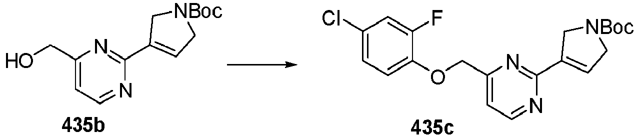

**435b** (139 mg, 0.5 mmol), 4-chloro-2-fluorophenol (55.8 µL, 525 µmol), PPh₃ (197 mg, 750 µmol), and DCM (1.97 mL) were cooled to 0 °C. *N*-({[(4-chlorophenyl)methoxy]carbonyl}imino)[(4-chlorophenyl)methoxy]formamide (DCAD) (275 mg, 750 µmol) was added slowly. After stirring at room temperature for 1 h, the reaction was filtered and directly purified by silica gel column chromatography (gradient elution, 0 to 50% EtOAc in DCM) to provide the title product **(435c)** (178 mg, 87%) as a colorless solid. *m*/*z* (ESI, +ve ion) = 428.3 [M+Na]⁺.

### Step D. (S)-2-((3-(4-((4-Chloro-2-fluorophenoxy)methyl)pyrimidin-2-yl)-2,5-dihydro-1H-pyrrol-1-yl)methyl)-1-(oxetan-2-ylmethyl)-1H-benzo[d]imidazole-6-carboxylic acid (435)

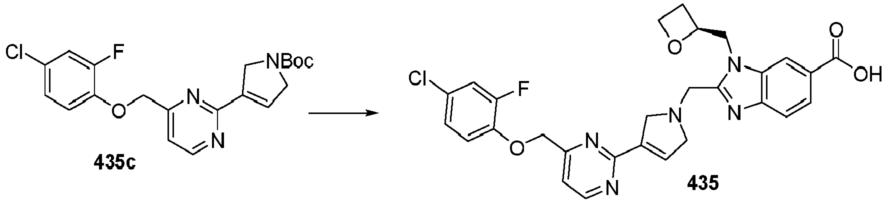

The title product **(435)** was synthesized from **435c** in similar procedures as described in Example **384,** Steps C to E. *m*/*z* (ESI, +ve ion) = 550.2 [M+H]⁺. ¹H NMR (400 MHz, CD₃OD) δ ppm 8.72 (d, *J* = 5.27 Hz, 1H), 8.17 (d, *J* = 0.75 Hz, 1H), 7.96 (dd, *J* = 8.28, 1.51 Hz, 1H), 7.61 (d, *J* = 8.53 Hz, 1H), 7.44 (d, *J* = 5.02 Hz, 1H), 7.20 - 7.24 (m, 1H), 7.08 - 7.15 (m, 2H), 6.97 (t, *J* = 1.88 Hz, 1H), 5.26 (qd, *J* = 7.07, 2.89 Hz, 1H), 5.21 (s, 2H), 4.72 - 4.80 (m, 1 H), 4.66 - 4.74 (m, 1H), 4.59 - 4.66 (m, 1H), 4.46 (dt, *J* = 9.03, 6.02 Hz, 1H), 4.35 - 4.41 (m, 1H), 4.25 - 4.31 (m, 1H), 3.96 - 4.06 (m, 2H), 3.80 - 3.91 (m, 2H), 2.74 - 2.82 (m, 1H), 2.48 - 2.57 (m, 1H).

Examples **441** and **442** were synthesized as described in Example **427,** Steps A to C, except chiral separation was performed after Boc was removed (SFC separation, Daicel CHIRALCEL AD, 250mm*30 mm I.D., 10µm; mobile phase: CO₂/MeOH with 0.2% NH₃ (7 M in MeOH) = 85/15, followed by Example **384,** Steps D and E.

Examples **444** and **445** were synthesized in similar procedures as described in Example **427,** Step A, followed by Example **383,** Steps B, C, E, F G and then chiral separation (SFC, Daicel CHIRALCEL AZ, 250mm*30 mm I.D., 10µm; mobile phase: CO₂/MeOH with 0.2% NH₃ (7 M in MeOH) = 60/40).

Example **446** was synthesized in similar procedures as described in Example **411,** Step A, followed by Example **383,** Steps G, B and C.

Example **447** was synthesized in similar procedures as described in Example **411,** Step A, followed by Example **383,** Steps B, C and G. Example **448** was synthesized in similar procedures as described in Example **447.**

### Example 449. 2-(((R)-3-(3-((4-Cyano-2-fluorophenoxy)methyl)-4-fluorophenyl)pyrrolidin-1-yl)methyl)-4-fluoro-1-(((S)-oxetan-2-yl)methyl)-1H-benzo[d]imidazole-6-carboxylic acid (449)

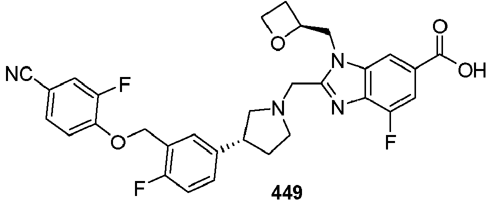

### Step A. tert-Butyl 3-(4-fluoro-3-(methoxycarbonyl)phenyl)-2,5-dihydro-1H-pyrrole-1-carboxylate (449a)

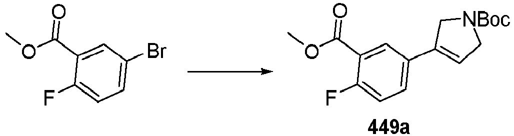

The title product **(449a)** was synthesized in similar procedures as described in Example 383, Step A. *m*/*z* (ESI, +ve ion) = 344.0 [M+Na]⁺.

### Step B. tert-Butyl 3-(4-fluoro-3-(methoxycarbonyl)phenyl)pyrrolidine-1-carboxylate (449b)

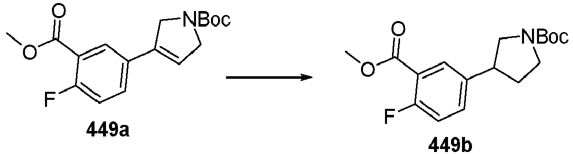

The title product **(449b)** was synthesized in similar procedures as described in Example **427,** Step A. *m*/*z* (ESI, +ve ion) = 346.1 [M+Na]⁺.

### Step C. tert-Butyl 3-(4-fluoro-3-(hydroxymethyl)phenyl)pyrrolidine-1-carboxylate (449c)

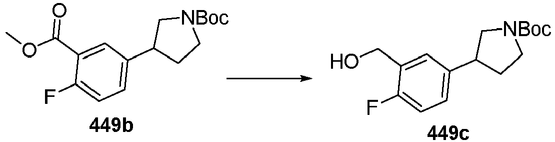

To a mixture of **449b** (1.6 g, 4.95 mmol) in THF was added LiAlH₄ (188 mg, 4.95 mmol) at 0 °C. The resulting mixture was stirred at 0 °C for 1.5 h. After completion, the reaction was quenched with Na₂SO₄.10H₂O, and filtered. The filter cake was rinsed with THF (100 mL). The combined organic layer was concentrated to afford the crude title product **(449c)** (1.5 g) as a yellow solid, which was used in the next step without further purification. *m*/*z* (ESI, +ve ion) = 318.1 [M+Na]⁺.

### Step D. (R)-3-Fluoro-4-((2-fluoro-5-(pyrrolidin-3-yl)benzyl)oxy)benzonitrile (449d-P1) and (5)-3-fluoro-4-((2-fluoro-5-(pyrrolidin-3-yl)benzyl)oxy)benzonitrile (449d-P2)

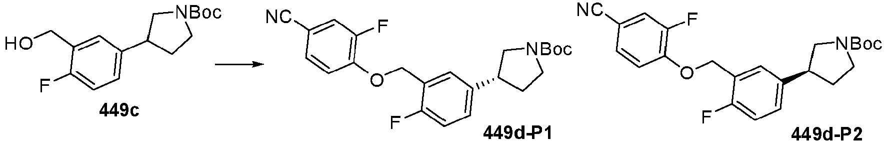

The title products **(449d-P1** and **449d-P2)** were synthesized from **449c** in similar procedures as described in Example **383,** Steps B and C, followed by chiral SFC separation: Daicel CHIRALCEL OZ, 250mm*30 mm I.D., 10µm; mobile phase: CO₂/MeOH with 0.2%NH₃ (7M Solution in MeOH) = 85/15. *m*/*z* (ESI, +ve ion) = 437.1 [M+Na]⁺. **449d-P1** is the early eluting peak. Stereochemistry is arbitrarily assigned.

### Step E. 2-(((R)-3-(3-((4-Cyano-2-fluorophenoxy)methyl)-4-fluorophenyl)pyrrolidin-1-yl)methyl)-4-fluoro-1-(((S)-oxetan-2-yl)methyl)-1H-benzo[d]imidazole-6-carboxylic acid (449)

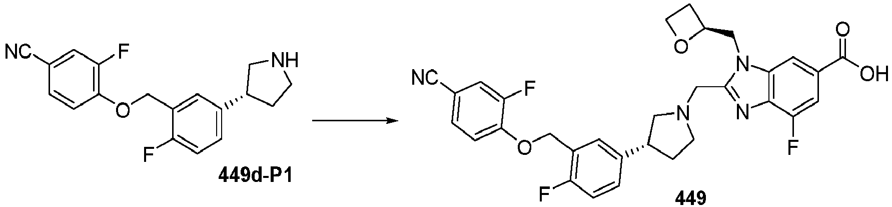

The title product **(449)** was synthesized in similar procedures from **449d-P1** as described in Example **384,** Steps C to E. ¹H NMR (400 MHz, DMSO-d6 ) δ ppm 7.78 - 7.83 (m, 1 H), 7.75 (s, 1 H), 7.61 - 7.69 (m, 1 H), 7.41 - 7.48 (m, 2 H), 7.32 - 7.38 (m, 1 H), 7.25 - 7.32 (m, 1 H), 7.12 (dd, *J* = 9.91, 8.66 Hz, 1 H), 5.20 (s, 2 H), 4.94 - 5.04 (m, 1 H), 4.63 (dd, *J* = 15.31, 7.28 Hz, 1 H), 4.48 - 4.56 (m, 1 H), 4.33 - 4.43 (m, 1 H), 4.24 (dt, *J* =8.91, 5.96 Hz, 1 H), 3.99 (d, *J* = 13.55 Hz, 1 H), 3.83 (d, *J* =13.30 Hz, 1 H), 3.13 - 3.19 (m, 1 H), 2.88 (t, *J* = 8.41 Hz, 1 H), 2.62 - 2.75 (m, 2 H), 2.28 - 2.35 (m, 2 H), 2.16 - 2.24 (m, 2 H), 1.63 - 1.75 (m, 1 H).

Example **450** was synthesized from **449d-P2** (the late eluting peak) in similar procedures as described in Example **449.**

Example **453** was synthesized in similar procedures from methyl 6-chloro-3-fluoropicolinate as described in Example **449,** Step A to C, followed by Example **383,** Steps B, C, E, and F, then chiral UPCC separation of the enantiomers (Daicel CHIRALCEL AZ, 250mm × 30 mm I.D., 10µm; CO₂/MeOH with 0.2% NH₃ (7M in MeOH) = 70/30), followed by Example **383,** Step G. **453** is formed from the early eluting peak.

Example **454** (from the early eluting peak) and **455** (from the late eluting peak) were synthesized in similar procedures as described in Example **453.** Chiral separation condition: UPCC (Daicel CHIRALPAK AZ_3, 3*150mm, 3µm; mobile phase: CO₂/MeOH with 0.1%DEA = 60/40).

### Example 459. (S)-2-(4-((2-((4-Chloro-2-fluorophenoxy)methyl)pyridin-4-yl)oxy)-2-fluorobenzyl)-4-fluoro-1-(oxetan-2-ylmethyl)-1H-benzo[d]imidazole-6-carboxylic acid (459)

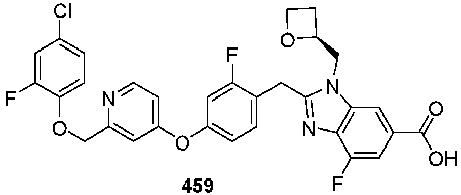

### Step A. (4-Bromopyridin-2-yl)methyl methanesulfonate (459a)

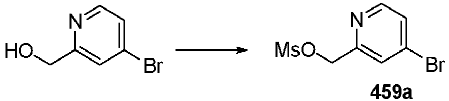

To a DCM (50 mL) solution of (4-bromopyridin-2-yl)methanol (3 g, 0.016 mol) was added DIPEA (6.2 g, 0.05 mol). After stirring for 3 min, methanesulfonic anhydride (4.18 mg, 0.02 mol) was added at 0 °C. After stirring at 20 °C for 1 h, the reaction was quenched with H₂O (80 mL) and extracted with EtOAc (50 mL × 3). The combined organic layer was dried over anhydrous Na₂SO₄, filtered and concentrated to afford the crude title product **(459a)** (3 g), which was used in the next step without further purification. *m*/*z* (ESI, +ve ion) = 267.9 [M+H]⁺.

### Step B. 4-Bromo-2-((4-chloro-2-fluorophenoxy)methyl)pyridine (459b)

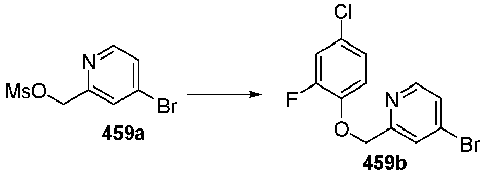

To a solution of **459a** (3 g, 0.01mol) in in DMF (20 mL) was added 4-chloro-2-fluorophenol (1.82 g, 0.01 mol) and K₂CO₃ (15.62 g, 0.113 mol). The resulting mixture was heated at 80 °C for 12 h. The reaction mixture was quenched with H₂O (8 mL) and extracted with EtOAc (50 mL × 3). The combined organic layer was dried over anhydrous Na₂SO₄, filtered and concentrated to afford the title product **(459b)** (3.0 g, 79%) as a colorless oil. *m*/*z* (ESI, +ve ion) = 317.9 [M+H]⁺.

### Step C. Methyl 2-(4-((2-((4-chloro-2-fluorophenoxy)methyl)pyridin-4-yl)oxy)-2-fluorophenyl)acetate (459c)

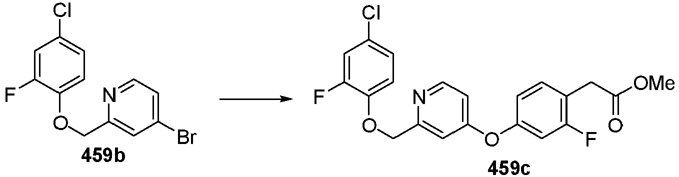

A vial was charged with **459b** (1.0g, 3.2 mmol), methyl 2-(2-fluoro-4-hydroxyphenyl)acetate (0.59 g, 3.2 mmol), CuI (0.06 g, 0.0003 mol), pyridine-2-carboxylic acid, (0.08 g, 0.6mmol) and K₃PO4 (1.36 g, 0.0064 mol) and flushed with argon. DMSO (20 mL) was added. The reaction was heated at 100 °C for 12 h, cooled down, quenched with H₂O (50 mL) and extracted with EtOAc (50 mL × 3). The combined organic layer was concentrated and purified by Prep-HPLC (MeCN in H₂O, with 0.05%TFA as a modifier) to afford the title product **(459c)** (300 mg, 22%) as a brown solid. *m*/*z* (ESI, +ve ion) = 420.3 [M+H]⁺.

### Step D. 2-(4-((2-((4-Chloro-2-fluorophenoxy)methyl)pyridin-4-yl)oxy)-2-fluorophenyl)acetic acid (459d)

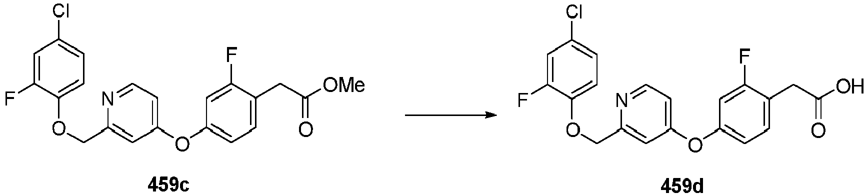

To a stirred solution of **459c** (300 mg, 0.71mmol) in MeOH/THF/H₂O (1:1:2, 20 mL) was added LiOH (171.1 mg, 7.146 mmol). After stirring at 20 °C for 3 h, the reaction was quenched by 1 N HCl to adjusted to pH = 6 and extracted with EtOAc (50 mL × 3). The combined organic layer was dried over anhydrous Na₂SO₄, filtered, concentrated, and purified by Prep-HPLC (CH₃CN in H₂O, with 0.5% TFA as a modifier) to afford the title product (**459d**) (200 mg, 68.2%) as a yellow oil. *m*/*z* (ESI, +ve ion) = 406.2 [M+H]⁺.

### Step E. Methyl (A)-4-(2-(4-((2-((4-chloro-2-fluorophenoxy)methyl)pyridin-4-yl)oxy)-2-fluorophenyl)acetamido)-3-fluoro-5-((oxetan-2-ylmethyl)amino)benzoate (459e)

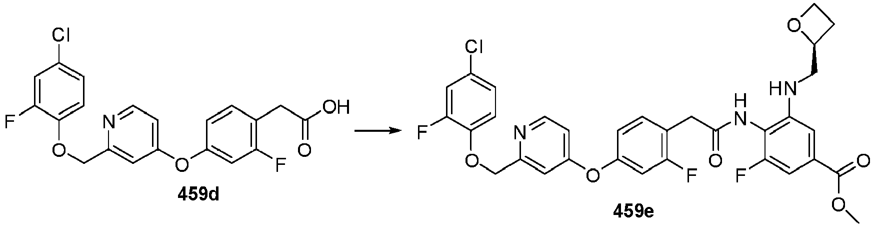

To a stirred solution of **459d** (100 mg, 0.24 mmol) in DCM( 5 mL) was added methyl 4-amino-3-fluoro-5-{[(2*S*)-oxetan-2-ylmethyl]amino}benzoate (62.65 mg, 0.24mmol), DIPEA (95.5 mg, 0.73 mmol). After stirring for 30 min, HATU (234.2 mg, 0.61 mmol) was added. The resulting mixture was stirred at 20 °C for 2 h, quenched with H₂O (8 mL), and extracted with EtOAc (5 mL × 3). The combined organic layer was dried over anhydrous Na₂SO₄, filtered, and concentrated to afford the crude title product **(459e)** (80 mg, 50%) as a white solid. *m*/*z* (ESI, +ve ion) = 642.2 [M+H]⁺.

### Step F. Methyl (S)-2-(4-((2-((4-chloro-2-fluorophenoxy)methyl)pyridin-4-yl)oxy)-2-fluorobenzyl)-4-fluoro-1-(oxetan-2-ylmethyl)-1H-benzo[d]imidazole-6-carboxylate (459f)

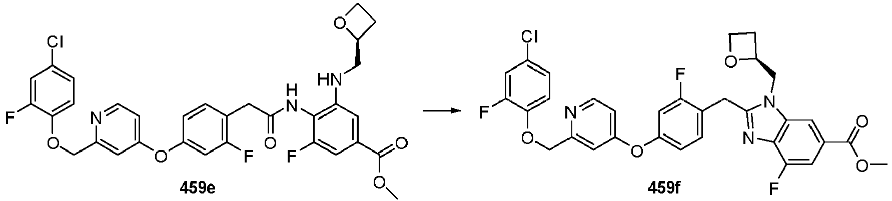

A solution of **459e** (50 mg, 0.077 mmol) in HOAc (10 ml) was heated at 100 °C for 5 h. The mixture was concentrated to afford the crude title product **(4591)** (40 mg, 78.2%) as a white solid, which was used in the next step without further purification. *m*/*z* (ESI, +ve ion) = 624.2 [M+H]⁺.

### Step G. (S)-2-(4-((2-((4-Chloro-2-fluorophenoxy)methyl)pyridin-4-yl)oxy)-2-fluorobenzyl)-4-fluoro-1-(oxetan-2-ylmethyl)-1H-benzo[d]imidazole-6-carboxylic acid (459)

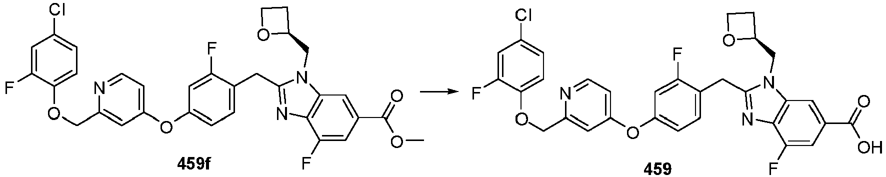

A solution of **459f** (50 mg, 0.08 mmol) in CH₃OH/H₂O (2 mL, 1:1) was added LiOH (19 mg, 0.8 mmol). After stirring at room temperature for 2 h, the reaction was quenched with 1 N HCl and adjusted to pH = 7. The mixture was extracted with DCM/CH₃OH (10:1) (5 mL × 2). The combined organic layer was concentrated and purified by prep-TLC (silica gel, DCM:CH₃OH = 10:1) to provide the title product **(459)** (15 mg, 30.3%) as a white solid. *m*/*z* (ESI, +ve ion) = 610.2 [M+H]⁺. ¹H NMR (400 MHz, CD3OD) δ 8.42 (d, *J* = 5.8 Hz, 1H), 8.08 (d, *J* = 0.9 Hz, 1H), 7.63 (d, *J* = 11.4 Hz, 1H), 7.36 (t, *J* = 8.5 Hz, 1H), 7.03 - 7.21 (m, 4H), 6.90 - 7.01 (m, 3H), 5.09 - 5.23 (m, 3H), 4.33 - 4.75 (m, 6H), 2.77 (dd, *J* = 18.5, 7.0 Hz, 1H), 2.41 - 2.52 (m, 1H).

Example **462 466** were synthesized in similar procedures as described in Example **459.**

### Example 460. 2-(4-((2-((4-Cyano-2-fluorophenoxy)methyl)pyrimidin-4-yl)oxy)-2-fluorobenzyl)-1-((1-ethyl-1H-imidazol-5-yl)methyl)-1H-benzo[d]imidazole-6-carboxylic acid (460)

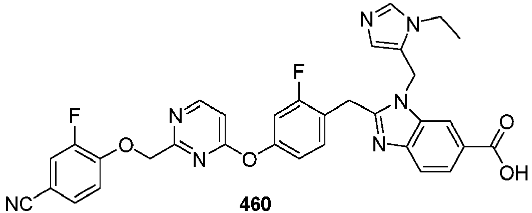

### Step A. 4-((4-Chloropyrimidin-2-yl)methoxy)-3-fluorobenzonitrile (460a)

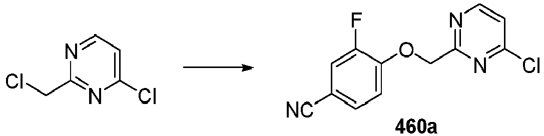

A mixture of 3-fluoro-4-hydroxybenzonitrile (81.8 mg, 0.6 mmol) and 4-chloro-2-(chloromethyl)pyrimidine (81 mg, 0.5 mmol) in DMF (4 mL) was treated with Cs₂CO₃ (324 mg, 1 mmol) at room temperature. The reaction mixture was stirred for 2 h before quenched with water (50 mL) and extracted with EtOAc (20 mL × 3). The combined organic layer was washed with brine, dried with Na₂SO₄, filtered and concentrated *in vacuo.* The crude residue was purified by silica gel column chromatography (EtOAc in hexanes) to provide the title product **(460a)** (93 mg, 71%) as a white solid. *m*/*z* (ESI, +ve ion) = 264.1 [M+H]⁺.

### Step B. Methyl 2-(4-((2-((4-cyano-2-fluorophenoxy)methyl)pyrimidin-4-yl)oxy)-2-fluorophenyl)acetate (460b)

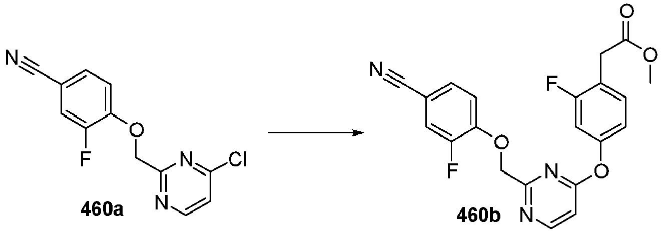

A vial was charged with **460a** (175 mg, 0.66 mmol), methyl 2-(2-fluoro-4-hydroxyphenyl)acetate (134 mg, 0.73 mmol), Cs₂CO₃ (433 mg, 1.33 mmol) and degassed and charged with an Ar balloon. Anhydrous toluene (27 mL) was added (vial A). The same degas and Ar protection procedure was applied to a separate vial containing a solution of Pd₂(dba)₃ (27 mg, 0.05 mmol) and BINAP (92 mg, 0.22 mmol) in dry toluene (3 mL), which was then heated up to 110 °C for 5 min and transferred to vial A. The resulting reaction mixture was heated at 110 °C overnight. After cooling down, the reaction was diluted with water and extracted with EtOAc. The organic layer was dried over Na₂SO₄, filtered, concentrated and purified by silica gel column chromatography to provide the title product **(460b)** (233 mg, 85%).

### Step C. 2-(4-((2-((4-Cyano-2-fluorophenoxy)methyl)pyrimidin-4-yl)oxy)-2-fluorophenyl)acetic acid (460c)

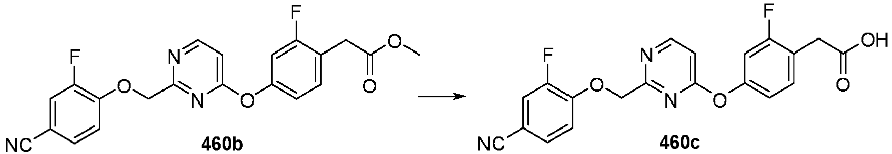

460b (66 mg, 0.16 mmol) was dissolved in CH₃CN (4 mL) and added 2 N NaOH (2 mL). The mixture was stirred at room temperature for 2 h, and directly purified by reverse phase HPLC (gradient elution, 20-100% CH₃CN in water, with 0.1% TFA as a modifier) to provide the title product **(460c)** (15 mg, 95%). *m*/*z* (ESI, +ve ion) = 398.2 [M+H]⁺.

### Step D. Methyl 4-(2-(4-((2-((4-cyano-2-fluorophenoxy)methyl)pyrimidin-4-yl)oxy)-2-fluorophenyl)acetamido)-3-(((1-ethyl-1H-imidazol-5-yl)methyl)amino)benzoate (460d)

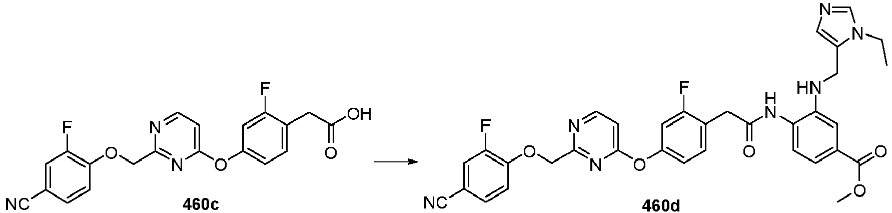

To a stirred solution **of460c** (15 mg, 0.038 mmol) and HATU (29 mg, 0.076 mmol) in DMF (1 mL) was added DIPEA (14.6 mg, 0.11 mmol). After 5 min, **IIe** (11 mg, 0.04 mmol) was added and the mixture was stirred at room temperature for 1 h. Upon completion, the mixture was directly purified by reverse phase HPLC (CH₃CN in water, with 0.1% TFA as a modifier) to provide the title product **(460d)** (8 mg, 32%) as a white solid. *m*/*z* (ESI, +ve ion) = 654.3 [M+H]⁺.

### Step E. Methyl 2-(4-((2-((4-cyano-2-fluorophenoxy)methyl)pyrimidin-4-yl)oxy)-2-fluorobenzyl)-1-((1-ethyl-1H-imidazol-5-yl)methyl)-1H-benzo[d]imidazole-6-carboxylate (460e)

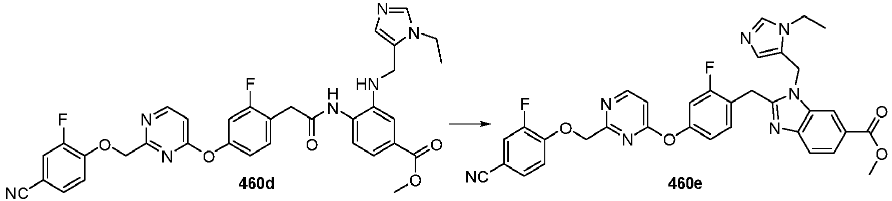

**460d** (8 mg, 0.012 mmol) was heated in HOAc (3 mL) at 65 °C overnight. Upon completion the reaction was concentrated and then co-evaporated with hexanes and water to provide the crude title product **(460e),** which was used in the next step without further purification. *m*/*z* (ESI, +ve ion) = 636.3 [M+H]⁺.

### Step F. 2-(4-((2-((4-Cyano-2-fluorophenoxy)methyl)pyrimidin-4-yl)oxy)-2-fluorobenzyl)-1-((1-ethyl-1H-imidazol-5-yl)methyl)-1H-benzo[d]imidazole-6-carboxylic acid (460)

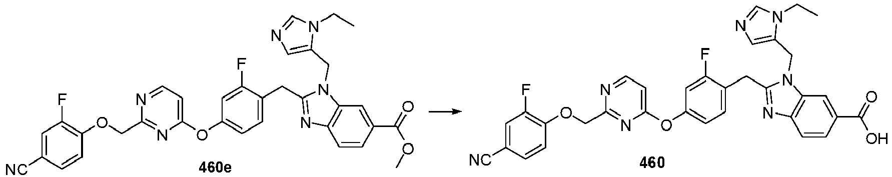

A solution of **460e** (crude) in CH₃CN (1 mL) was added 2 N NaOH (0.5 mL). The reaction was heated at 60 °C for 1 h, and directly purified by reverse phase HPLC (gradient elution, 0-75% CH₃CN in water, with 0.1% TFA as a modifier) to provide the title product **(460)** as a white solid (3.6 mg, 47% for 2 steps). *m*/*z* (ESI, +ve ion) = 622.3 [M+H]⁺. ¹H NMR (600 MHz, CD₃OD) δ ppm 9.00 (s, 1H), 8.65 (d, *J =* 5.87 Hz, 1H), 8.22 (d, *J =* 0.73 Hz, 1H), 8.04 (dd, *J =* 8.62, 1.65 Hz, 1H), 7.76 (d, *J =* 8.80 Hz, 1H), 7.33 - 7.39 (m, 2H), 7.30 (t, *J =* 8.44 Hz, 1H), 7.09 (t, *J =* 8.44 Hz, 1H), 7.03 (d, *J =* 5.87 Hz, 1H), 6.80 - 6.88 (m, 3H), 5.87 (d, *J =* 0.73 Hz, 2H), 5.35 (s, 2H), 4.46 (s, 2H), 4.33 (q, *J =* 7.34 Hz, 2H), 1.56 (t, *J =* 7.34 Hz, 3H).

Examples **461** and **463** were synthesized in similar procedures starting from 2-chloro-6-(chloromethyl)pyridine as described in Example **460.** Examples **469** were synthesized in similar procedures as described in Example **460**

Example **464, 465, 467, 468,** and **470** (from **IIIf**) were synthesized in similar procedures as described in Example **470,** using NH₄OH as a modifier for the last step purification.

### Example 471. (S)-2-(4-((2-((4-Chloro-2-fluorophenoxy)methyl)pyridin-4-yl)oxy)-3-fluorobenzyl)-4-fluoro-1-(oxetan-2-ylmethyl)-1H-benzo[d]imidazole-6-carboxylic acid (471)

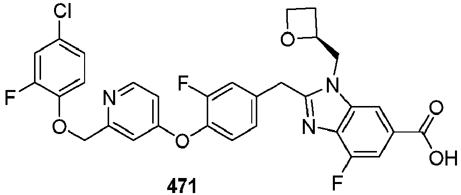

### Step A. Methyl 2-(3-fluoro-4-hydroxyphenyl)acetate (471a)

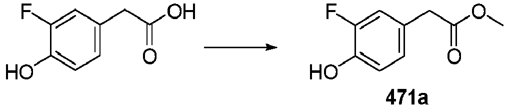

To a stirred solution of (3-fluoro-4-hydroxyphenyl)acetic acid (1.5 g, 8.8 mmol) and DMF (0.06 g, 0.8 mmol) in MeOH (20 mL) at 0 °C was added thionyl chloride (1.57 g, 13.2 mmol). After stirring at 0 °C for 2 h, the reaction was diluted with H₂O (25 mL) and extracted with EtOAc (30 mL × 3). The combined organic layer was dried and concentrated to afford the crude title product **(471a)** (1.6 g, 94%) as a colorless oil. *m*/*z* (ESI, +ve ion) = 185.1 [M+H]⁺.

### Step B. Methyl 2-(4-((2-((4-chloro-2-fluorophenoxy)methyl)pyridin-4-yl)oxy)-3-fluorophenyl)acetate (471b)

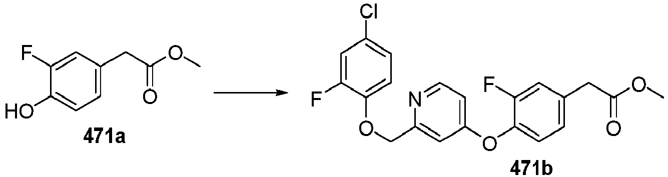

A solution of **471a** (200 mg, 1.09 mmol), **471b** (378 mg, 1.19 mmol), palladium diacetate (49 mg, 0.22 mmol), tBuXphos (92 mg, 0.22 mmol) and K₃PO4 (461 mg, 2.17 mmol) in toluene (10 mL) was heated under nitrogen at 100 °C for 6 h. After completion, the reaction was cooled down, diluted with H₂O (15 mL) and extracted with EtOAc (20 mL × 3). The combined organic layer was dried, filtered, concentrated and purified by silica gel column chromatography (EtOAc:petroleum ether = 1:3) to afford the title product **(471b)** (260 mg, 54%) as a white solid. *m*/*z* (ESI, +ve ion) = 420.1 [M+H]⁺.

### Step C. 2-(4-((2-((4-Chloro-2-fluorophenoxy)methyl)pyridin-4-yl)oxy)-3-fluorophenyl)acetic acid (471c)

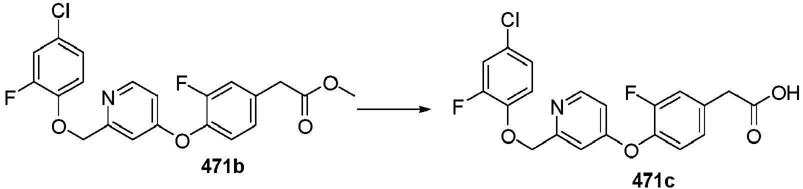

To a stirred solution of **471b** (370 mg, 0.88 mmol) in THF (15 mL) at 25 °C was added a solution of lithium hydroxide (211 mg, 8.81 mmol) in H₂O (5 mL). The reaction mixture was stirred at 25 °C for 4 h. After completion, 1 N HCl was added to the mixture to adjust to pH = 5 in an ice bath. The reaction mixture was then extracted with EtOAc (25 mL × 3). The combined organic layer was dried, filtered, concentrated and purified by silica gel column chromatography (EtOAc:petroleum ether = 1:1) to afford the title product (471c) (275 mg, 73%) as a white solid. *m*/*z* (ESI, +ve ion) = 406.1 [M+H]⁺.

### Step D. Methyl (S)-4-(2-(4-((2-((4-Chloro-2-fluorophenoxy)methyl)pyridin-4-yl)oxy)-3-fluorophenyl)acetamido)-3-fluoro-5-((oxetan-2-ylmethyl)amino)benzoate (471d)

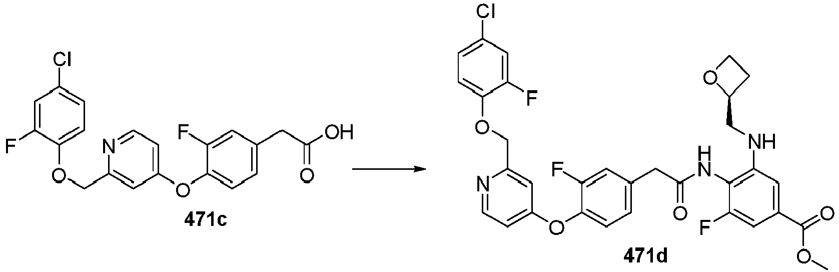

To a stirred solution of **471c** (30 mg, 0.07 mmol), **Ig** (21 mg, 0.08 mmol) in DCM (2 mL) at 25 °C was added T₃P (94 mg, 0.15 mmol, 50% in EtOAc), DIPEA (38 mg, 0.30 mmol). After stirring at 25 °C for 2 h, the reaction was diluted with H₂O (5 mL) and extracted with EtOAc (10 mL × 3). The combined organic layer was dried, filtered, concentrated and purified by silica gel column (DCM:MeOH = 20:1) to afford the title product (471d) (24 mg, 48%) as a white solid. *m*/*z* (ESI, +ve ion) = 642.2 [M+H]⁺.

### Step E. (S)-2-(4-((2-((4-Chloro-2-fluorophenoxy)methyl)pyridin-4-yl)oxy)-3-fluorobenzyl)-4-fluoro-1-(oxetan-2-ylmethyl)-1H-benzo[d]imidazole-6-carboxylic acid (471)

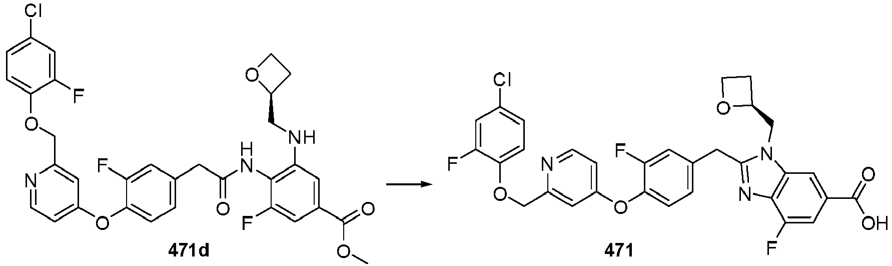

The title product **(471)** was synthesized from **471d** in similar procedures as described in Example **459,** Steps F and G. *m*/*z* (ESI, +ve ion) = 610.1 [M+H]⁺. ¹H NMR (400 MHz, CD₃OD) δ ppm 8.43 (d, *J* = 5.79 Hz, 1H), 8.11 (s, 1H), 7.67 (d, *J* = 11.31 Hz, 1H), 7.33 (d, *J* = 10.61 Hz, 1H), 7.22 - 7.29 (m, 2H), 7.02 - 7.19 (m, 4H), 6.97 (dd, *J* = 5.74, 2.43 Hz, 1H), 5.19 (s, 2H), 5.14 (dd, *J* = 9.59, 4.47 Hz, 1H), 4.59 - 4.71 (m, 2H), 4.55 (d, *J* = 5.63 Hz, 2H), 4.52 (dd, *J=* 15.72, 2.39 Hz, 1H), 4.43 (dt, *J* = 9.16, 5.92 Hz, 1H), 2.77 (dt, *J=* 14.10, 7.95 Hz, 1H), 2.47 (dt, *J* = 11.37, 7.36 Hz, 1H).

### Example 472. (S)-2-((4-((2-((4-Chloro-2-fluorophenoxy)methyl)pyrimidin-4-yl)amino)piperidin-1-yl)methyl)-1-(oxetan-2-ylmethyl)-1H-benzo[d]imidazole-6-carboxylic acid (472)

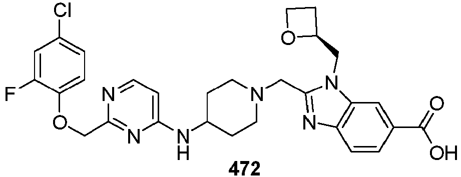

### Step A. tert-Butyl 4-((2-((4-chloro-2-fluorophenoxy)methyl)pyrimidin-4-yl)amino)piperidine-1-carboxylate (472b)

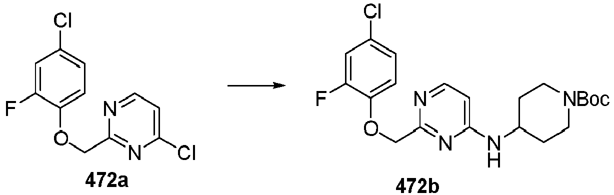

A solution of **472a** (synthesized as **460a,** 27.3 mg, 0.1 mmol), tert-butyl 4-aminopiperidine-1-carboxylate (22 mg, 110 µmol), and DIPEA (34.8 µL, 0.2 mmol) in DMF (0.5 mL) was heated to 40 °C for 24 h. Upon completion, the reaction mixture was diluted with H₂O (5 mL) and stirred 30 min. The aqueous layer was decanted and the residue was rinsed with H₂O (1 mL) and dried to provide the title product **(472b)** (8 mg), which was used directly in the next step without further purification. *m*/*z* (ESI, +ve ion) = 437.4 [M+H]⁺.

### Step B. 2-((4-Chloro-2-fluorophenoxy)methyl)-N-(piperidin-4-yl)pyrimidin-4-amine (472c)

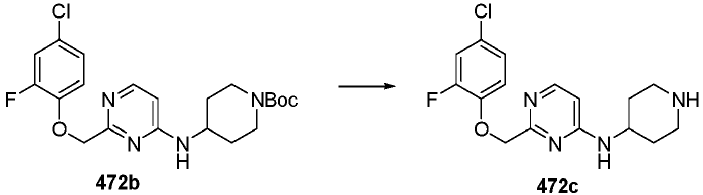

A solution of **472b** (8 mg, 18.3 µmol) and trifluoroacetic acid (0.5 mL) in DCM (1 mL) was stirred at 21 °C for 2 h. Upon completion, the reaction was concentrated and co-evaporated with DCE (3 × 2 mL) to provide the title product **(472c)** (8 mg), which was used directly in the next step without further purification. *m*/*z* (ESI, +ve ion) = 337.3 [M+H]⁺.

### Step C. Methyl (S)-2-((4-((2-((4-chloro-2-fluorophenoxy)methyl)pyrimidin-4-yl)amino)piperidin-1-yl)methyl)-1-(oxetan-2-ylmethyl)-1H-benzo[d]imidazole-6-carboxylate (472d)

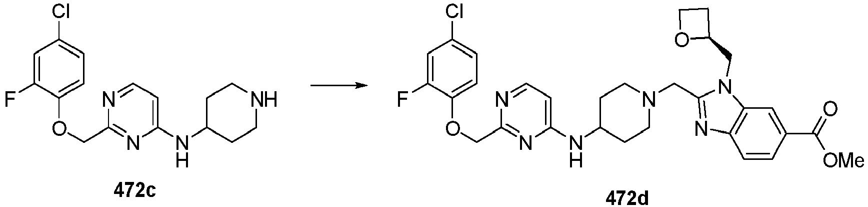

A solution of crude **472c** (8 mg), **Ih** (7 mg, 23.8 µmol) and DIPEA (41.4 µL, 238 µmol) in DMF (0.5 mL) was stirred at 21 °C for 24 h. Upon completion, the reaction mixture was diluted with H₂O (10 mL), stirred for 30 min and the aqueous layer was decanted. The residue was dried to provide the title product **(472d)** (14 mg), which was used directly in the next step without further purification. *m*/*z* (ESI, +ve ion) = 595.3 [M+H]⁺.

### Step D. (5)-2-((4-((2-((4-Chloro-2-fluorophenoxy)methyl)pyrimidin-4-yl)amino)piperidin-1-yl)methyl)-1-(oxetan-2-ylmethyl)-1H-benzo[d]imidazole-6-carboxylic acid (472)

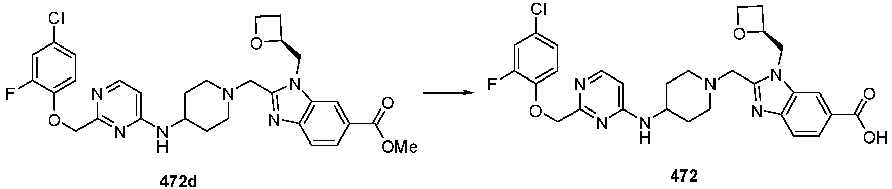

A mixture of **472d** (14 mg, 23.5 µmol) and LiOH (1 M) (118 µL, 118 µmol) in THF (235 µL), and MeOH (235 µL) were stirred at 21 °C for 4 h. Upon completion, the reaction mixture was diluted with H₂O (2 mL) and purified directly by reverse phase HPLC (Gemini 5 µm C18 110 Å, 250 × 21.2 mm, AXIA packed) (gradient elution, 10-40% CH₃CN in H₂O, with 0.05% NH₄OH as a modifier) to provide the title product **(472)** (7.1 mg, 52%) as a colorless solid. *m*/*z* (ESI, +ve ion) = 581.3 [M+H]⁺. ¹H NMR (400 MHz, CD₃OD) δ ppm 8.19 (s, 1H), 7.90 - 8.03 (m, 2H), 7.59 (d, J = 8.44 Hz, 1H), 7.19 - 7.23 (m, 1H), 7.02 - 7.08 (m, 2H), 6.36 (br s, 1H), 5.28 (qd, J = 7.03, 2.87 Hz, 1H), 5.11 (s, 2H), 4.91 - 4.93 (m, 1H), 4.84 - 4.87 (m, 1H), 4.68 - 4.74 (m, 1H), 4.61 - 4.68 (m, 1H), 4.48 (dt, *J* = 9.17, 5.99 Hz, 1H), 4.00 (d, *J* = 13.69 Hz, 1H), 3.85 - 3.97 (m, 1H), 2.88 - 2.95 (m, 1H), 2.74 - 2.86 (m, 2H), 2.48 - 2.60 (m, 1H), 2.15 - 2.31 (m, 2H), 1.85 (br s, 2H), 1.52 (br d, *J* = 12.84 Hz, 2H).

Example **473** was synthesized in similar procedures as described in Example **472.**

### Example 474. (S)-2-((3-(2-((2,4-Dichlorophenoxy)methyl)oxazol-5-yl)-2,5-dihydro-1H-pyrrol-1-yl)methyl)-1-(benzo[d]imidazole-6-carboxylic acid (474)

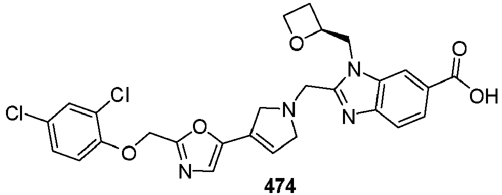

### Step A. Oxazol-2-ylmethanol (474a)

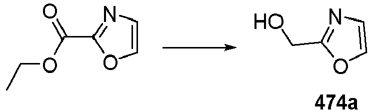

To a solution of ethyl oxazole-2-carboxylate (12.8 g, 90.8 mmol) in MeOH (100 mL) was added LiBH₄ (7.9 g, 363.1 mmol) at 0 °C. After stirring at 15 °C for 16 h, the reaction was diluted with H₂O (100 mL) and concentrated to remove MeOH. The resulting mixture was extracted with EtOAc (200 mL × 3). The combined organic layer was dried and concentrated to afford the crude title product **(474a)** (5.7 g, 63%) as a yellow oil, which used directly in the next step without further purification. *m*/*z* (ESI, +ve ion) = 100.1 [M+H]⁺.

### Step B. 2-(((tert-Butyldimethylsilyl)oxy)methyl)oxazole (474b)

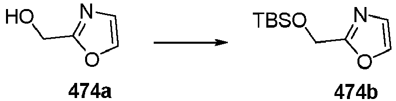

To a solution **of 474a** (5.7 g, 57.0 mmol) and imidazole (5.81 g, 85.5 mmol) in THF (50 mL) was added TBSCl (12.9 g, 85.5 mmol). After stirring at 15 °C for 2 h, the reaction was diluted with H₂O (100 mL) and extracted with EtOAc (100 mL × 3). The organic layer was dried, concentrated and purified by silica gel column chromatography (petroleum ether:EtOAc = 20:1) to afford the title product **(474b)** (11.2 g, 92.2%) as a colorless oil. *m*/*z* (ESI, +ve ion) = 214.2 [M+H]⁺.

### Step C. 5-Bromo-2-(((tert-butyldimethylsilyl)oxy)methyl)oxazole (474c)

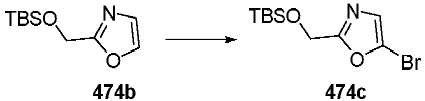

To a solution of **474b** (12.6 g, 59.2 mmol) in THF (100 mL) was added *n*-BuLi (2.5 M in hexanes) (30.8 mL, 77.0 mmol) and TMEDA (4.46 g, 38.5 mmol) dropwise at -60 °C. After stirring at -60 °C for 3 h, CBr₄ (29.4 g, 88.8 mmol) was added and the reaction was raised to room temperature and stirred at 17 °C for 4 h. After completion, the reaction was quenched with H₂O (100 mL) and extracted with EtOAc (100 mL × 3). The combined organic layer was dried, concentrated and purified by silica gel column chromatography (petroleum ether:EtOAc = 30:1) to afford the title product **(474c)** (10.8 g, 62.5%) as a yellow oil. *m*/*z* (ESI, +ve ion) = 292.1 [M+H]⁺.

### Step D. (5-Bromooxazol-2-yl)methanol (474d)

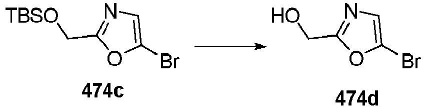

To a solution of **474c** (10.8 g, 37.1 mmol) in MeOH (50 mL) was added HCl (4 N in dioxane, 20 mL). After stirring at 17 °C for 1 h, the reaction was concentrated and purified by reverse phase HPLC (CH₃CN in H₂O, with 0.1% formic acid as a modifier) to afford the title product **(474d)** (3.9 g, 59%) as a yellow oil. *m*/*z* (ESI, +ve ion) = 178.1 [M+H]⁺.

### Step E. 5-Bromo-2-((2,4-dichlorophenoxy)methyl)oxazole (474e)

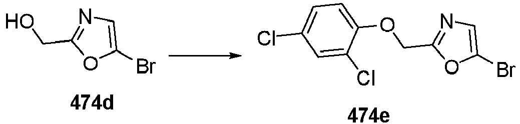

To a solution of **474d** (3.9 g, 21.9 mmol), 2,4-dichlorophenol (3.9 g, 24.1 mmol), PPh₃ (7.5 g, 28.5 mmol) in THF (20 mL) was added DEAD (5.0 g, 28.5 mmol). After stirring at 17 °C for 3 h, the reaction was diluted with H₂O (20 mL) and extracted EtOAc (20 mL × 3). The combined organic layer was dried, filtered and concentrated. The residue was purified by silica gel column chromatography (petroleum ether: EtOAc = 3:1) to afford the title product (**474e**) (4.2 g, 60%) as a light-yellow solid. *m*/*z* (ESI, +ve ion) = 323.9 [M+H]⁺.

### Step F. (S)-2-((3-(2-((2,4-Dichlorophenoxy)methyl)oxazol-5-yl)-2,5-dihydro-1H-pyrrol-1-yl)methyl)-1-(oxetan-2-ylmethyl)-1H-benzo[d]imidazole-6-carboxylic acid (474)

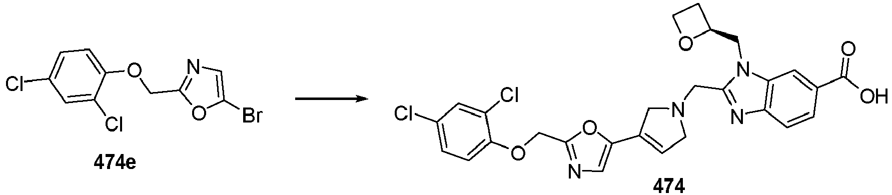

The title product **(474)** was synthesized from **474e** in similar procederes as described in Example **4,** Steps A, C, D and E. *m*/*z* (ESI, +ve ion) = 555.3 [M+H]⁺. ¹H NMR (600 MHz, CD₃OD) δ ppm 8.17 (s, 1H), 7.95 (dd, *J* = 8.44, 1.47 Hz, 1H), 7.59 (d, *J =* 8.44 Hz, 1H), 7.43 (d, *J =* 2.20 Hz, 1H), 7.27 (dd, *J =* 8.80, 2.57 Hz, 1H), 7.21 (d, *J =* 8.80 Hz, 1H), 7.07 (s, 1H) 6.23 - 6.28 (m, 1H), 5.21 - 5.29 (m, 3 H), 4.84 - 4.87 (m, 1H), 4.68 (br dd, *J* =15.41, 2.93 Hz, 1H), 4.58 - 4.64 (m, 1H), 4.44 (dt, *J* =9.17, 5.87 Hz, 1H), 4.33 (d, *J =* 13.94 Hz, 1H), 4.25 (d, *J =* 13.94 Hz, 1 H), 3.73 - 3.89 (m, 4H), 2.74 - 2.80 (m, 1 H), 2.49 - 2.61 (m, 1H).

Example **516** and Example **517** were synthesized in similar procedures as described in Example **449,** except the SFC chiral separation (CHIRALPAK AY-H, 0.46 cm I. D. × 25 cm, MeOH/CH₃CN/DEA=80/20/0.1 (V/V/V)) was performed at the second to last step before the ester hydrolysis. Example **516** was the first peak eluting from the separation and **517** the second peak. Stereochemistry is arbitrarily assigned.

Example **518** and Example **519** were synthesized in similar procedures as described in Examples **516** and **517.** SFC chiral separation condition: CHIRALPAK AY-3 (0.46 cm I. D. × 15 cm, hexanes/EtOH/DEA=50/50/0.1). Example **518** was the first peak eluting from the separation and **519** the second peak. Stereochemistry is arbitrarily assigned.

Examples **520** and **521** were synthesized in similar procedures as in Example **397.**

### Example 522. 2-((1-(6-((4-Cyano-2-fluorophenoxy)methyl)-5-fluoropyridin-2-yl)-3-azabicyclo[3.1.0]hexan-3-yl)methyl)-1-(((S)-oxetan-2-yl)methyl)-1H-benzo[d]imidazole-6-carboxylic acid (522)

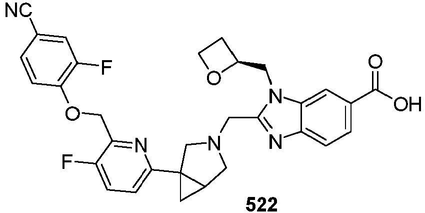

### Step A. (6-Chloro-3-fluoropyridin-2-yl)methanol (522a)

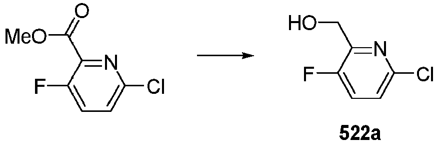

Methyl 6-chloro-3-fluoropyridine-2-carboxylate (2.16 g, 11.4 mmol) was dissolved in ethanol (57 mL) and cooled to 0 °C. CaCl₂ (1.9 g, 17.1 mmol) and NaBH₄ (647 mg, 17.1 mmol) were slowly added and the reaction mixture was warmed to 21 °C and stirred for 3 h. Upon completion, the reaction was slowly poured into H₂O (30 mL) and extracted with 30% IPA/CHCl₃ (30 mL × 3). The combined organic layer was dried with Na₂SO₄, filtered, and concentrated to afford the title product 522a (1.84 g, 100%) as a colorless solid which was used directly in the next step without further purification. *m*/*z* (ESI, +ve ion) = 162.1 [M+H]⁺.

### Step B: 4-((6-Chloro-3-fluoropyridin-2-yl)methoxy)-3-fluorobenzonitrile (522b)

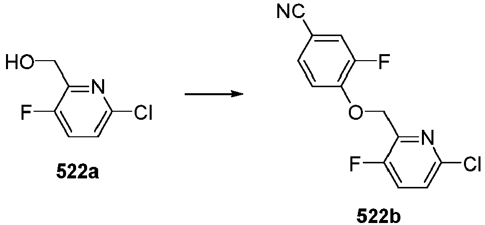

A mixture of **522a** (32.3 mg, 0.2 mmol), 3-chloro-4-hydroxybenzonitrile (30.7 mg, 0.2 mmol), PPh₃ (78.7 mg, 0.3 mmol), and DCM (1 mL) was cooled to 0 °C. N-({[(4-chlorophenyl)methoxy]carbonyl}imino)[(4-chlorophenyl)methoxy]formamide (110 mg, 0.3 mmol) was added slowly and the reaction was warmed to 21 °C and stirred for 1 h. Upon completion, the reaction mixture was filtered and directly purified by silica gel column chromatography (gradient elution, 0 to 75% EtOAc in hexanes) to give **522b** (50 mg, 84%) as a colorless solid. *m*/*z* (ESI, +ve ion) = 297.1 [M+H]⁺.

### Step C: tert-Butyl 1-(6-((4-cyano-2-fluorophenoxy)methyl)-5-fluoropyridin-2-yl)-3-azabicyclo[3.1.0]hexane-3-carboxylate (522c)

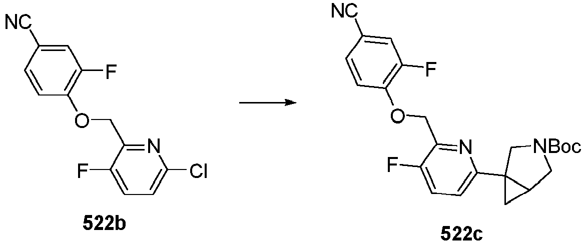

A mixture of **522b** (140 mg, 0.5 mmol), {3-[(tert-butoxy)carbonyl]-3-azabicyclo[3.1.0]hexan-1-yl}trifluoroboranium (144 mg, 0.5 mmol), cataCXium-A-Pd-G3 (18.2 mg, 25 µmol), Cs₂CO₃ (489 mg, 3 eq., 1.5 mmol), toluene (12 mL), and H₂O (1.2 mL) were thoroughly purged with Ar for 10 min. The reaction mixture was heated to 90 °C for 18 h. Upon completion, the reaction was concentrated and purified directly by silica gel column chromatography (0 to 30% EtOAc in DCM) to give **522c** (214 mg, 76%) as a colorless solid. *m*/*z* (ESI, +ve ion) = 450.3 [M+Na]⁺.

### Step D. 2-((1-(6-((4-Cyano-2-fluorophenoxy)methyl)-5-fluoropyridin-2-yl)-3-azabicyclo[3.1.0]hexan-3-yl)methyl)-1-(((S)-oxetan-2-yl)methyl)-1H-benzo[d]imidazole-6-carboxylic acid (522)

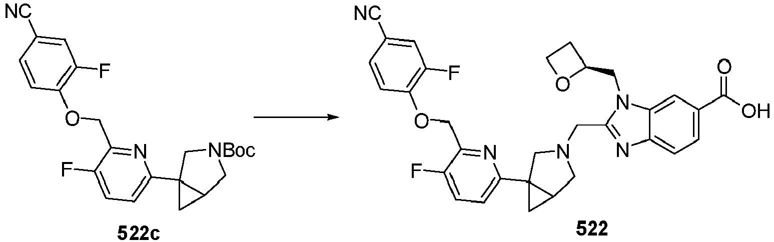

The title compound **(522)** was synthesized from **522c** in similar procedures as described in Example **384,** Steps C to E. *m*/*z* (ESI, +ve ion) = 572.4 [M+H]⁺. ¹H NMR (400 MHz, DMSO-*d₆*)) δ ppm 8.16 (s, 1H), 7.88 - 7.83 (m, 1H), 7.79 (dd, *J* = 1.2, 8.3 Hz, 1H), 7.65 - 7.73 (m, 2H), 7.50 - 7.57 (m, 2H), 7.22 - 7.28 (m, 1H), 5.40 (s, 2H), 5.04 (q, *J* = 7.2 Hz, 1H), 4.72 (ddd, *J* = 4.7, 7.1, 15.2 Hz, 1H), 4.57 (td, *J =* 2.9, 15.1 Hz, 1H), 4.45 - 4.53 (m, 1H), 4.28 - 4.44 (m, 1H), 4.09 (d, *J* = 13.3 Hz, 1H), 3.87 - 3.98 (m, 1H), 3.15 (br d, *J* = 8.8 Hz, 1H), 2.86 - 3.01 (m, 1H), 2.82 - 2.94 (m, 1H), 2.56 - 2.75 (m, 2H), 2.37 - 2.45 (m, 1H), 1.85 - 1.93 (m, 1H), 1.35 (t, *J* = 3.9 Hz, 1H), 1.00 - 1.06 (m, 1H).

Examples **523, 524, 525, 531,** and **532** were synthesized in similar procedures as described in Example **522.** Examples **531** and **532** resulted from the chiral separation of **522c.**

### Example 528. 2-(((2R)-4-(6-((4-Cyano-2-fluorophenoxy)methyl)-5-fluoropyridin-2-yl)-2-(fluoromethyl)pyrrolidin-1-yl)methyl)-1-(((S)-oxetan-2-yl)methyl)-1H-benzo[d]imidazole-6-carboxylic acid (528)

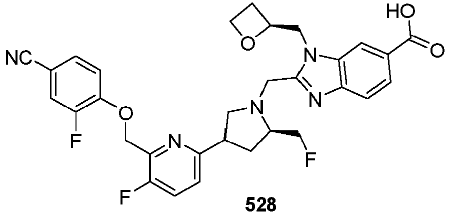

### Step A. 1-(tert-Butyl) 2-methyl (R)-4-(6-((4-cyano-2-fluorophenoxy)methyl)-5-fluoropyridin-2-yl)-2,5-dihydro-1H-pyrrole-1,2-dicarboxylate (528a)

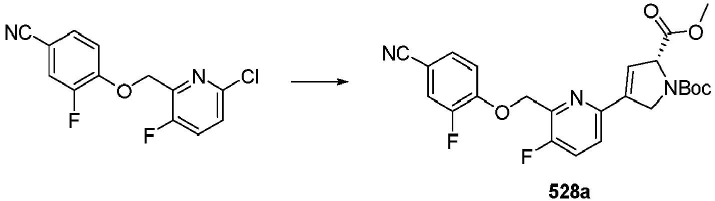

A mixture of 4-[(6-chloro-3-fluoropyridin-2-yl)methoxy]-3-fluorobenzonitrile (140 mg, 0.5 mmol), 1-*tert*-butyl 2-methyl (2*R*)-4-(4,4,5,5-tetramethyl-1,3,2-dioxaborolan-2-yl)-2,5-dihydro-1*H*-pyrrole-1,2-dicarboxylate (212 mg, 0.6 mmol), Pd(dppf)Cl₂ (36.6 mg, 50 µmol), K₂CO₃ (2 M) (0.5 mL, 1 mmol), and 1,4-dioxane (2 mL) was purged with Ar and heated to 100 °C for 3 h. Upon completion, the reaction mixture was diluted with EtOAc (5 mL), dried with Na₂SO₄, and concentrated. The crude residue was purified by silica gel column chromatography (gradient elution, 0 to 30% EtOAc in DCM) to afford the title compound **(528a)** (169 mg, 72%). *m*/*z* (ESI, +ve ion) = 472.4 [M+H]⁺.

### Step B. tert-Butyl (R)-4-(6-((4-cyano-2-fluorophenoxy)methyl)-5-fluoropyridin-2-yl)-2-(hydroxymethyl)-2,5-dihydro-1H-pyrrole-1-carboxylate (528b)

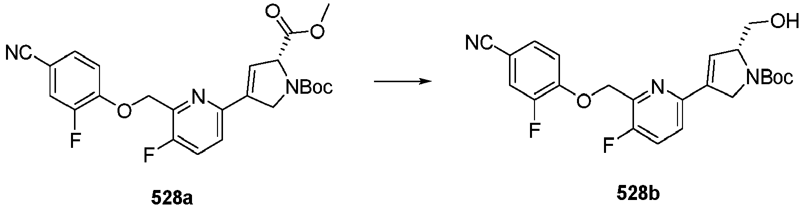

**528a** (165 mg, 0.35 mmol) and THF (3.5 mL) were cooled to 0 °C. LiBH₄ (15.2 mg, 0.7 mmol) was added slowly and the reaction mixture was warmed to 21 °C and stirred for 24 h. The reaction mixture was quenched with ice, diluted with EtOAc (30 mL) and H₂O (10 mL). The organic layer was washed with brine, dried with Na₂SO₄, filtered and concentrated. The residue was purified by silica gel column chromatography (gradient elution, 0 to 100% EtOAc in DCM) to give **528b** (103 mg, 66%) as a colorless oil. *m*/*z* (ESI, +ve ion) = 444.4 [M+H]⁺.

### Step C. tert-Butyl (2R)-4-(6-((4-cyano-2-fluorophenoxy)methyl)-5-fluoropyridin-2-yl)-2-(hydroxymethyl)pyrrolidine-1-carboxylate (528c)

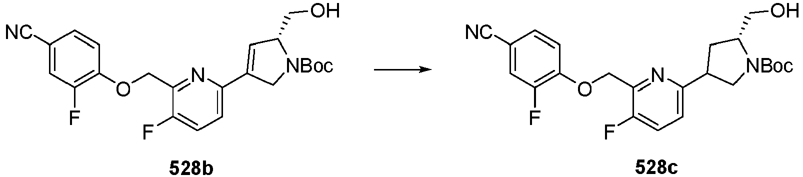

**528b** (22.2 mg, 50 µmol), MeOH (1 mL), and 10% Pd/C (0.5 mg, 5 µmol) were stirred under 1 atm H₂ for 3 h. The reaction mixture was filtered over Celite and rinsed with MeOH (5 mL). The filtrate was concentrated to afford the title product (528c), which was used directly in the next step without further purification. m/z (ESI, +ve ion) = 446.4 [M+H]⁺.

### Step D. 3-Fluoro-4-((3-fluoro-6-((5R)-5-(hydroxymethyl)pyrrolidin-3-yl)pyridin-2-yl)methoxy)benzonitrile (528d)

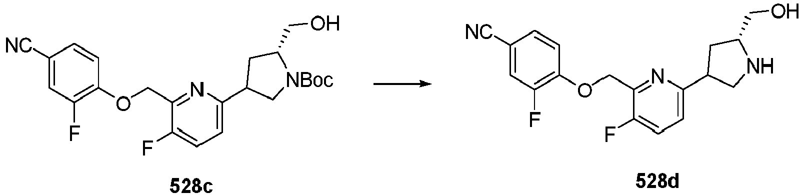

A solution of **528c** (crude from above), TFA (0.2 mL) in DCM (1 mL) was stirred at 21 °C for 2 h. Upon completion, the reaction mixture was concentrated and co-evaporated with DCE (2 mL × 3) to afford the title product **(528d),** which was used directly in the next step without further purification. *m*/*z* (ESI, +ve ion) = 346.4 [M+H]⁺.

### Step E. Methyl 2-(((2R)-4-(6-((4-cyano-2-fluorophenoxy)methyl)-5-fluoropyridin-2-yl)-2-(hydroxymethyl)pyrrolidin-1-yl)methyl)-1-(((5)-oxetan-2-yl)methyl)-1H-benzo[d]imidazole-6-carboxylate (528e)

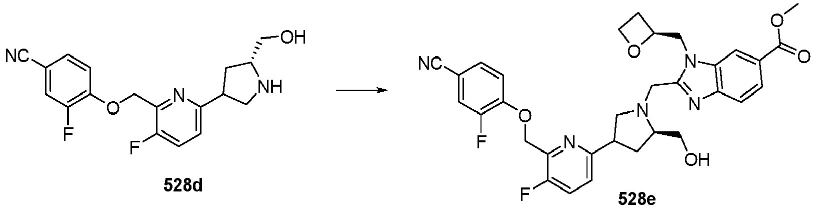

A mixture of **528d** (23 mg, 50 µmol), Ih, DMF (250 µL), and DIPEA (43.5 µL, 250 µmol) was stirred at 21 °C for 18 h. Upon completion, the reaction was diluted with H₂O (20 mL) and stirred for 1 h. The aqueous layer was decanted. The residual solid was rinsed with H₂O (5 mL) and dried to give **528e,** which was used directly in the next step without further purification. *m*/*z* (ESI, +ve ion) = 604.3 [M+H]⁺.

### Step F. Methyl 2-(((2R)-4-(6-((4-cyano-2-fluorophenoxy)methyl)-5-fluoropyridin-2-yl)-2-(fluoromethyl)pyrrolidin-1-yl)methyl)-1-(((S)-oxetan-2-yl)methyl)-1H-benzo[d]imidazole-6-carboxylate (528f)

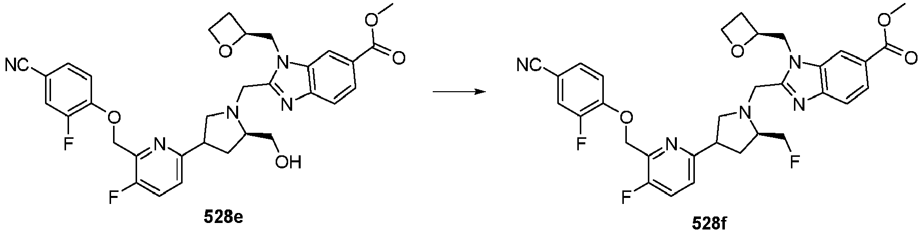

**528e** (15.1 mg, 25 µmol) was dissolved in DCM (3 mL) and cooled to -78 °C. DAST (6.61 µL, 50 µmol) in DCM (3 mL) was added dropwise and the reaction mixture was stirred at -78 °C for 3 h. Upon completion, the reaction mixture was quenched with sat. aq. NaHCO₃ (1 mL) and the organic layer was washed with H₂O (1 mL), brine (1 mL), dried with Na₂SO₄, filtered, and concentrated to afford the title compound **(528f),** which was used directly in the next step without further purification. LCMS indicated a 3:1 mixture of isomers. *m*/*z* (ESI, +ve ion) = 606.5 [M+H]⁺.

### Step G. 2-(((2R)-4-(6-((4-Cyano-2-fluorophenoxy)methyl)-5-fluoropyridin-2-yl)-2-(fluoromethyl)pyrrolidin-1-yl)methyl)-1-(((S)-oxetan-2-yl)methyl)-1H-benzo[d]imidazole-6-carboxylic acid (528)

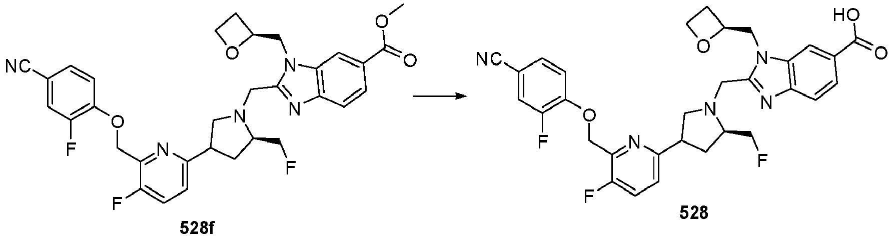

**528f** (30.3 mg, 50 µmol), THF (250 µL), and isopropanol (250 µL) were cooled to 0 °C. LiOH (1 M) (245 µL, 250 µmol) was added slowly and the reaction mixture was warmed to 21 °C and stirred for 7 h. Upon completion, the reaction mixture was diluted with 2 mL H₂O and purified directly by reverse phase HPLC (Gemini 5 um C18 110 A, 250 × 21.2 mm) at 15 ml/min (15-35% CH₃CN in H₂O, with 0.05% NH₄OH as a modifier), to provide the title compound **(528)** (1.1 mg, 4%, a 3:1 mixture of isomers) as a colorless solid. *m*/*z* (ESI, +ve ion) = 592.3 [M+H]⁺.

Example **526** and **527** were a pair of diastereomers and were synthesized in similar procedures as described in Example **528,** separated by reverse phase HPLC at the last step. **527** was the major isomer and later-eluting peak. The stereochemistry was arbitrarily assigned.

### Example 533. 2-(((S)-1-(6-((4-Cyano-2-fluorophenoxy)methyl)-5-fluoropyridin-2-yl)pyrrolidin-3-yl)methyl)-1-(((S)-oxetan-2-yl)methyl)-1H-benzo[d]imidazole-6-carboxylic acid (533)

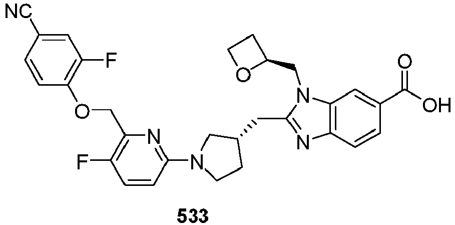

### Step A. tert-Butyl (S)-3-(2-((4-(methoxycarbonyl)-2-((((S)-oxetan-2-yl)methyl)amino)phenyl)amino)-2-oxoethyl)pyrrolidine-1-carboxylate (533a)

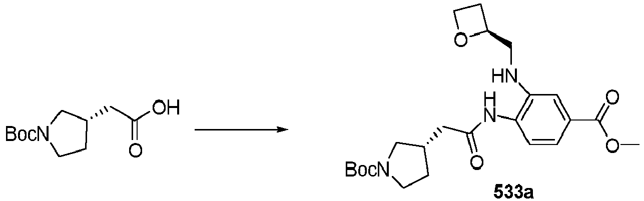

To a solution of (*S*)-2-(1-(*tert*-butoxycarbonyl)pyrrolidin-3-yl)acetic acid (28.0 mg, 0.122 mmol), **Ig** (28.9 mg, 0.122 mmol) and HATU (43.1 mg, 0.183 mmol) in THF (0.6 mL) was added DIPEA (42.5 µL, 0.244 mmol) at room temperature. Reaction mixture was stirred for 1 h. After completion, the reaction solvent was removed under reduced pressure to afford the title product **(533a)** (55.0 mg) as a crude material, which was used in the next step without further purification. *m*/*z* (ESI, +ve ion) = 448.3 [M+H]⁺

### Step B. Methyl 2-(((S)-1-(tert-butoxycarbonyl)pyrrolidin-3-yl)methyl)-1-(((S)-oxetan-2-yl)methyl)-1H-benzo[d]imidazole-6-carboxylate (533b)

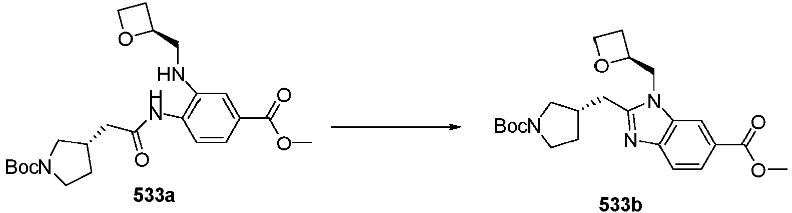

A solution of **533a** (55.0 mg, 0.123 mmol) in glacial acetic acid (1.0 mL) was stirred for 16 h. After completion, acetic acid was removed *in vacuo.* The resulting residue was diluted with EtOAc (20 mL). The organic layer was washed with sat. NaHCO₃ (20 mL × 2), brine (5 mL), dried over Na₂SO₄, filtered and concentrated and the resulting crude residue was purified by silica gel column chromatography (gradient elution, 50-90% EtOAc/hexanes) to afford the title product **(533b)** (31 mg, 59%) as a yellow oil. *m*/*z* (ESI, +ve ion) = 430.2 [M+H]⁺.

### Step C. Methyl 1-(((S)-oxetan-2-yl)methyl)-2-(((S)-pyrrolidin-3-yl)methyl)-1H-benzo[d]imidazole-6-carboxylate (533c)

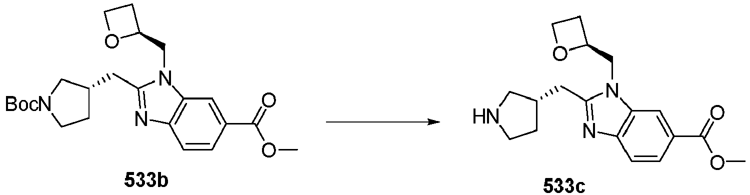

A solution of **533b** (31.0 mg, 0.072 mmol) in DCM (0.5 mL) and TFA (0.5 mL) was stirred at room temperature for 10 min. After completion, the reaction solvent was removed under reduced pressure and the resulting residue was diluted with EtOAc (3 mL). The organic layer was washed with sat. NaHCO₃ (5 mL), brine (3 mL), dried over Na₂SO₄, filtered and concentrated to afford the title product **(533c)** (26.5 mg) as a colorless oil, which was used in the next step without further purification. *m*/*z* (ESI, +ve ion) = 330.2 [M+H]⁺

### Step D. Methyl 2-(((S)-1-(6-((4-cyano-2-fluorophenoxy)methyl)-5-fluoropyridin-2-yl)pyrrolidin-3-yl)methyl)-1-(((S)-oxetan-2-yl)methyl)-1H-benzo[d]imidazole-6-carboxylate (533d)

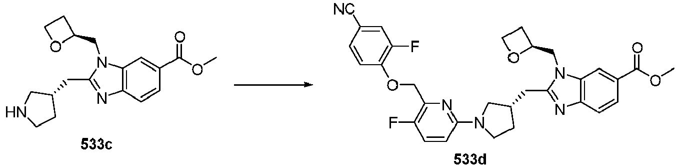

A solution of 4-((6-chloro-3-fluoropyridin-2-yl)methoxy)-3-fluorobenzonitrile (22.6 mg, 0.080 mmol), **533c** (26.5 mg, 0.080 mmol), Pd(OAc)₂ (1.81 mg, 0.008 mmol), BINAP (10.0 mg, 0.016 mmol) and Cs₂CO₃ (52.4 mg, 0.161 mmol) in toluene (0.8 mL) was purged with argon for 5 min. It was stirred at 110 °C for 16 h. Upon completion, the reaction mixture was diluted with water and extracted with EtOAc (x 3). The combined organic layer was washed with brine, dried over Na₂SO₄, filtered and concentrated and the resulting crude residue was purified by silica gel column chromatography (50-90% EtOAc/hexanes) to afford the title product **(533d)** (12.4 mg, 27%) as a colorless oil. *m*/*z* (ESI, +ve ion) = 574.2 [M+1]⁺.

### Step E. 2-(((S)-1-(6-((4-Cyano-2-fluorophenoxy)methyl)-5-fluoropyridin-2-yl)pyrrolidin-3-yl)methyl)-1-(((S)-oxetan-2-yl)methyl)-1H-benzo[d]imidazole-6-carboxylic acid (533)

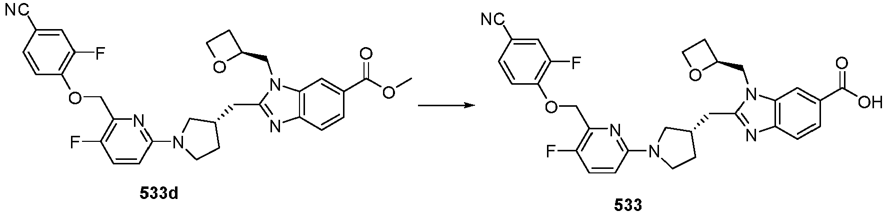

A solution of **533d** (12.4 mg, 0.022 mmol) and LiOH (2.6 mg, 0.11 mmol) in THF:*i*PrOH:H₂O = 1:1:1 (0.6 mL) was stirred at room temperature for 16 h. After completion, the reaction solvent was removed under reduced pressure and the resulting residue was diluted with water/acetonitrile (1:1) to 2 mL and purified by reverse phase HPLC (gradient elution, 20-60% CH₃CN in H₂O, with 20 mM NH₄HCO₃ as a modifier) to afford the title product **(533)** (4.3 mg, 36%) as a white solid. *m*/*z* (ESI, +ve ion) = 560.3 [M+H]⁺. ¹H NMR (400 MHz, DMSO-d6) δ ppm 8.18 (s, 1H), 7.76 -7.84 (m, 2H), 7.46 - 7.68 (m, 4H), 6.48 (dd, *J =* 9.2, 2.8 Hz, 1H), 5.25 (d, *J =* 1.7 Hz, 2H), 4.97 - 5.06 (m, 1H), 4.57 - 4.66 (m, 1H), 4.38 - 4.54 (m, 2H), 4.29 (dt, *J* = 9.2, 5.9 Hz, 1H), 3.69 (dd, *J =* 10.3 Hz, 1H), 3.45 - 3.53 (m, 1H), 3.35 - 3.42 (m, 1H), 3.04 - 3.18 (m, 3H), 2.91 - 3.02 (m, 1H), 2.62 - 2.72 (m, 1H), 2.28 - 2.40 (m, 1H), 2.16 - 2.27 (m, 1H), 1.73 - 1.86 (m, 1H).

Examples **529, 530,** and **534** were synthesized in similar procedures as described in Example **533.**

### Example 535. (S)-2-((3-(6-((4-Cyano-2-fluorophenoxy)methyl)-5-fluoropyridin-2-yl)-2-oxoimidazolidin-1-yl)methyl)-1-(oxetan-2-ylmethyl)-1H-benzo[d]imidazole-6-carboxylic acid (535)

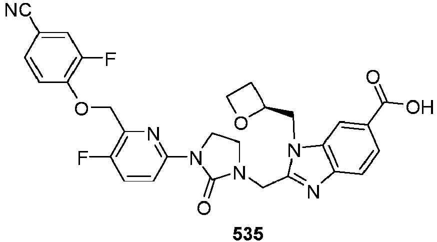

### Step A. 4-((6-Chloro-3-fluoropyridin-2-yl)methoxy)-3-fluorobenzonitrile (535a)

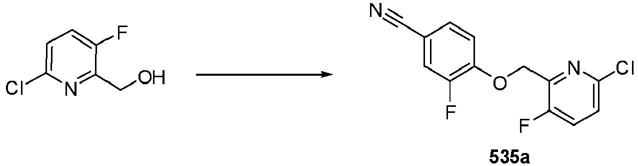

To a stirred solution (6-chloro-3-fluoropyridin-2-yl)methanol (0.75 g, 4.64 mmol), 3-fluoro-4-hydroxybenzonitrile (0.643 g, 4.64 mmol) and triphenylphosphine (1.83 g, 4.64 mmol) in anhydrous DCM (10 mL) was added di-(4-chlorobenzyl)azodicarboxylate (2.56 g, 4.64 mmol) at 0 °C. The reaction mixture was slowly raised to room temperature and stirred for 2 h. Then the reaction mixture was filtered and the filtrate was concentrated under reduced pressure and purified by silica gel column chromatography to afford the title compound **(535a)** as a white solid (910 mg, 70%). *m*/*z* (ESI, +ve ion) = 281.3 [M+H]⁺.

### Step B. 3-Fluoro-4-((3-fluoro-6-(2-oxoimidazolidin-1-yl)pyridin-2-yl)methoxy)benzonitrile (535b)

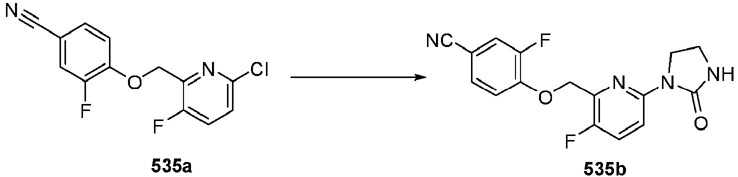

**535a** (30 mg, 0.107 mmol) was dissolved in anhydrous dioxane (2 mL) and degassed for 10 min using an argon balloon. Imidazolidin-2-one (0.046 mg, 0.534 mmol), 4,5-bis(diphenylphosphino)-9,9-dimethylxanthene (6 mg, 10 mol%), Pd(OAc)₂ (5 mol%) and Cs₂CO₃ (52 mg, 0.16 mmol) were added to the degassed solution and stirred at 100 °C overnight. The reaction mixture was quenched with sat. NH₄Cl and the solvent was removed under reduced pressure. The crude residue was diluted with brine and extracted with ethyl acetate (5 mL × 3). The combined organic layer was dried over anhydrous Na₂SO₄, filtered and concentrated under reduced pressure, which was purified by silica gel column chromatography to afford the title product **(535b)** as a colorless gummy liquid (14 mg, 40%). *m*/*z* (ESI, +ve ion) = 331.3 [M+H]⁺.

### Step C. Methyl (S)-2-((3-(6-((4-cyano-2-fluorophenoxy)methyl)-5-fluoropyridin-2-yl)-2-oxoimidazolidin-1-yl)methyl)-1-(oxetan-2-ylmethyl)-1H-benzo[d]imidazole-6-carboxylate (535c)

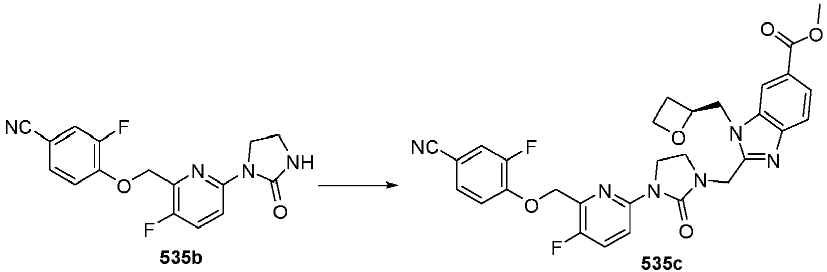

To a stirred solution of **535b** (14 mg, 0.042 mmol) in anhydrous DMF (2 mL) was added **Ih** (16 mg, 0.055 mmol) and DIPEA (0.022 mL, 0.127 mmol). After stirring at 60 °C overnight, the reaction mixture was quenched with water and diluted with brine. The aq. layer was extracted with ethyl acetate (5 mL × 3) and the combined organic layer was dried over anhydrous Na₂SO₄, filtered and concentrated under reduced pressure. The crude residue was purified by silica gel column chromatography using methanol and DCM to afford the title product (535c) (12 mg, 48%). *m*/*z* (ESI, +ve ion) = 589.3 [M+H]⁺.

### Step D. (5)-2-((3-(6-((4-Cyano-2-fluorophenoxy)methyl)-5-fluoropyridin-2-yl)-2-oxoimidazolidin-1-yl)methyl)-1-(oxetan-2-ylmethyl)-1H-benzo[d]imidazole-6-carboxylic acid (535)

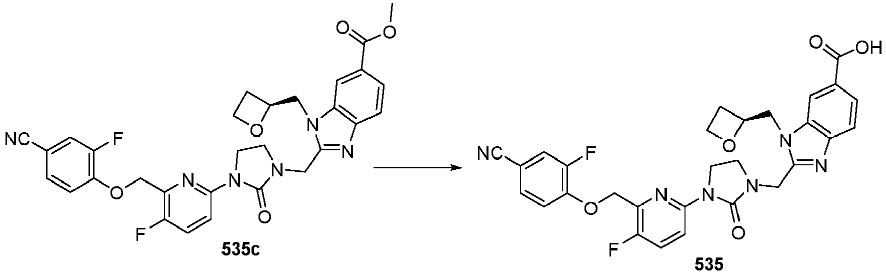

To a stirred solution of **535c** (12 mg, 0.02 mmol) in THF/methanol/water (6:1:1, 2 mL) was added lithium hydroxide (4 mg, 0.102 mmol). After stirring overnight at room temperature, the reaction mixture was quenched with acetic acid and the solvent was removed under reduced pressure. The crude mixture was diluted with brine and extracted with ethyl acetate (5 mL × 3). The combined organic layer was dried over anhydrous Na₂SO₄, concentrated, and purified by silica gel column chromatography using methanol/DCM to afford the title product (535) as a white solid (4 mg, 34%). *m*/*z* (ESI, +ve ion) = 575.3 [M+H]⁺. ¹H NMR (400 MHz, CDCl₃) δ ppm 8.25 (dd, *J =* 9.2, 3.3 Hz, 1H), 8.09 (s, 1H), 7.96 (d, *J =* 8.4 Hz, 1H), 7.73 (d, *J* = 8.6 Hz, 1H), 7.36 (d, *J* = 8.1 Hz, 1H), 7.26 - 7.31 (m, 2H), 7.13 (t, *J* = 8.4 Hz, 1H), 5.20 (s, 2H), 5.07 - 5.10 (m, 1H), 4.94 (d, *J* = 15.6 Hz, 1H), 4.81 (d, *J* = 15.6 Hz, 1H), 4.66 - 4.70 (m, 1H), 4.42 - 4.52 (m, 2H), 4.31 - 4.36 (m, 1H), 3.86 - 3.90 (m, 2H), 3.55 - 3.60 (m, 2H), 2.62 - 2.71 (m, 1H), 2.32 - 2.40 (m, 1H).

### Example 536. (S)-2-(4-((2-((4-Chloro-2-fluorophenoxy)methyl)pyridin-4-yl)oxy)-2,5-difluorobenzyl)-1-(oxetan-2-ylmethyl)-1H-benzo[d]imidazole-6-carboxylic acid (536)

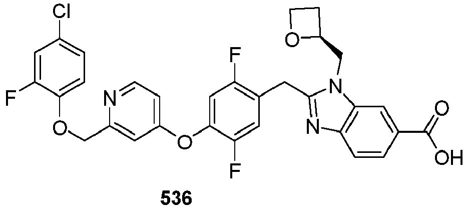

### Step A. tert-Butyl 2-(4-((2-((4-chloro-2-fluorophenoxy)methyl)pyridin-4-yl)oxy)-2,5-difluorophenyl)acetate (536a)

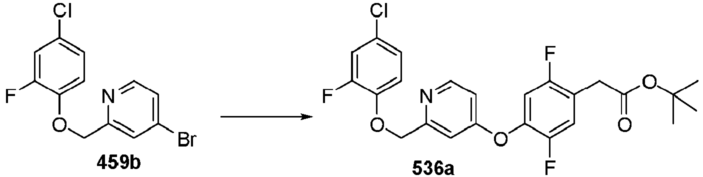

To a solution of **459b** (250 mg, 0.79 mmol), **IIIf** (212 mg, 0.87 mmol), tBuXphos (67 mg, 0.16 mmol) and Pd(OAc)₂ (35 mg, 0.16 mmol) in toluene (8 mL) was added K₃PO₄ (218 mg, 1.58 mmol). After heating at 100 °C under N₂ for 8 h, the reaction was filtered and concentrated. The crude residue was purified by Prep-TLC (EtOAc:petroleum ether = 1:1) to afford the title product **(536a)** (200 mg, 48%). *m*/*z* (ESI, +ve ion) = 480.1 [M+H]⁺.

### Step B. 2-(4-((2-((4-Chloro-2-fluorophenoxy)methyl)pyridin-4-yl)oxy)-2,5-difluorophenyl)acetic acid (536b)

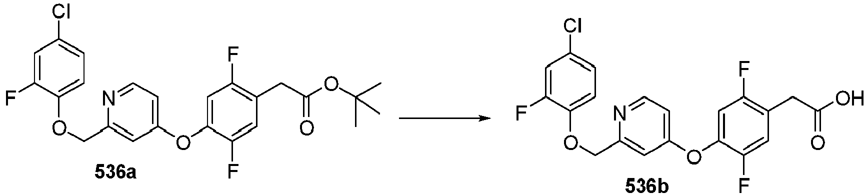

To a stirred solution of **536a** (100 mg, 0.21 mmol) in DCM (2 mL) at 0 °C was added 0.5 mL TFA. After stirring at 25°C for 2 h, the reaction mixture was concentrated under reduced pressure to afford the crude product **(536b)** (70 mg) as a yellow oil. *m*/*z* (ESI, +ve ion) = 424.1 [M+H]⁺.

### Step C. Methyl (S)-4-(2-(4-((2-((4-chloro-2-fluorophenoxy)methyl)pyridin-4-yl)oxy)-2,5-difluorophenyl)acetamido)-3-((oxetan-2-ylmethyl)amino)benzoate (536c)

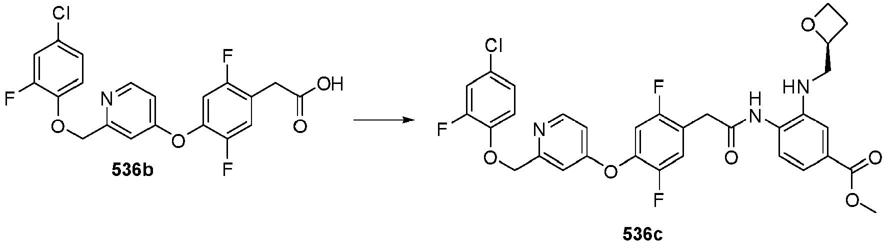

To a stirred solution of **536b** (109 mg, 0.21 mmol), **Ig** (49 mg, 0.21 mmol) and DIPEA (134 mg, 1.04 mmol) in DCM (2 mL) at 0 °C was added T₃P (198 mg, 0.31 mmol, 50% in EtOAc). After stirring at 25 °C for 5 h, the reaction was quenched by adding aq. sodium bicarbonate (10 mL) and extracted with DCM (10 mL × 3). The combined organic layer was dried over Na₂SO₄, filtered, concentrated, and purified by reverse phase HPLC (45% CH₃CN in H₂O) to afford the title product **(536c)** (42 mg, 30%) as a yellow oil. *m*/*z* (ESI, +ve ion) = 643.1 [M+H]⁺.

### Step D. Methyl 2-(4-((2-((4-chloro-2-fluorophenoxy)methyl)pyridin-4-yl)oxy)-2,5-difluorobenzyl)-1-(oxetan-2-ylmethyl)-1H-benzo[d]imidazole-6-carboxylate (536d)

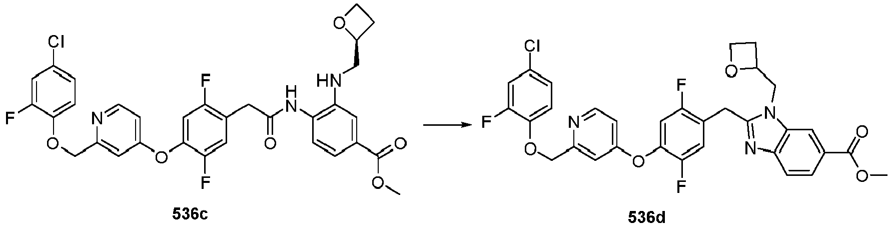

A solution of **536c** (42 mg, 0.07 mmol) in HOAc was heated at 100 °C for 3 h. The reaction mixture was then poured into water (5 mL), and extracted with EtOAc (15 mL × 2). The organic layer was dried, filtered and concentrated *in vacuo.* The crude residue was purified by Prep-TLC (10% MeOH in DCM) to afford the title product **(536d)** (16 mg, 38%) as ayellow oil. *m*/*z* (ESI, +ve ion) = 625.1 [M+H]⁺.

### Step E. (S)-2-(4-((2-((4-Chloro-2-fluorophenoxy)methyl)pyridin-4-yl)oxy)-2,5-difluorobenzyl)-1-(oxetan-2-ylmethyl)-1H-benzo[d]imidazole-6-carboxylic acid (536)

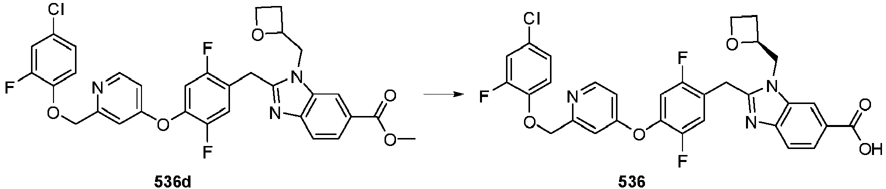

To a stirred solution of **536d** (16 mg, 0.03 mmol) in THF (1 mL) at 25°C was added a solution of lithium hydroxide (6 mg, 0.26 mmol) in H₂O (1 mL) dropwise. The reaction mixture was stirred at 25 °C for 3 h. After completion, 1 N HCl (about 2.0 mL) was added to the mixture to adjust to pH = 6 in an ice bath. The reaction mixture was then extracted with EtOAc (15 mL × 3). The combined organic layer was dried, filtered, concentrated and purified by reverse phase HPLC (CH₃CN in H₂O with 0.1% NH₄OH as a modifier) to afford the title product **(536)** (1.7 mg, 10%) as a white solid. *m*/*z* (ESI, +ve ion) = 610.1 [M+H]⁺. ¹H NMR (400 MHz, CD₃OD) δ ppm 8.47 (d, *J =* 5.80 Hz, 1H), 8.18 (s, 1H), 7.99 (d, *J =* 8.40 Hz, 1H), 7.58 (d, *J =* 8.43 Hz, 1H), 7.32 (dd, *J =* 10.59, 6.74 Hz, 1H), 7.21 - 7.26 (m, 1H), 7.20 (d, *J =* 2.49 Hz, 1H), 7.14 - 7.18 (m, 1H), 7.12 (d, J = 7.45 Hz, 1H), 7.07 - 7.11 (m, 1H), 7.05 (dd, *J* = 5.79, 2.53 Hz, 1H), 5.18 - 5.29 (m, 3H), 4.69 (dd, *J=* 15.63, 6.72 Hz, 1H), 4.62 - 4.66 (m, 1H), 4.61 (d, *J =* 3.54 Hz, 1H), 4.56 - 4.58 (m, 1H), 4.50 (s, 1H), 4.41 - 4.47 (m, 1H), 2.75 - 2.84 (m, 1H), 2.52 (dt, *J =* 16.39, 7.14 Hz, 1H).

Example **537** and **538** were synthesized in similar procedures as described in Example 536. For Example **537,** methyl 2-(2-fluoro-4-hydroxyphenyl)acetate was used instead of **IIIf.**

### Example 539. (S)-2-(4-((2-((4-cyano-2-fluorophenoxy)methyl)pyrimidin-4-yl)oxy)-3-fluorobenzyl)-4-fluoro-1-(oxetan-2-ylmethyl)-1H-benzo[d]imidazole-6-carboxylic acid (539)

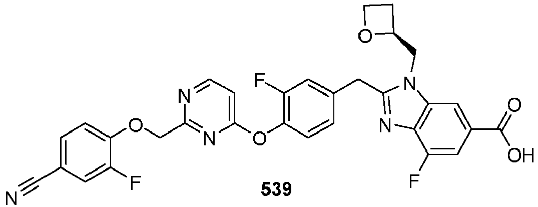

### Step A. 2-(4-((2-((4-Cyano-2-fluorophenoxy)methyl)pyrimidin-4-yl)oxy)-3-fluorophenyl)acetic acid (539a)

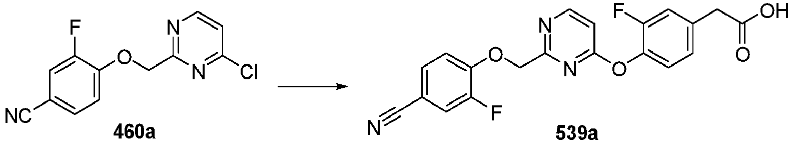

A solution of **460a** (100 mg, 0.38 mmol), 2-(3-fluoro-4-hydroxyphenyl)acetic acid (64.5 mg, 0.38 mmol) and K₂CO₃ (105 mg, 0.76 mmol) were stirred at room temperature for 2 h. The reaction was diluted with EtOAc and washed with brine. The organic layer was dried with Na₂SO₄, filtered, concentrated and purified by silica gel column chromatography (gradient elution, 50-90% EtOAc in hexanes) to afford the title compound **(539a)** (151 mg, 36.5%) as a yellow oil. *m*/*z* (ESI, +ve ion) = 398.3 [M+H]⁺.

### Step B. (S)-2-(4-((2-((4-Cyano-2-fluorophenoxy)methyl)pyrimidin-4-yl)oxy)-3-fluorobenzyl)-4-fluoro-1-(oxetan-2-ylmethyl)-1H-benzo[d]imidazole-6-carboxylic acid (539)

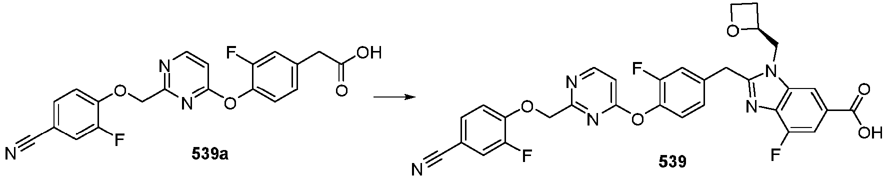

The title product (539) was synthesized in similar procedures from 539a as described in Example 536, Steps C to E. HATU was used in Step C. *m*/*z* (ESI, +ve ion) = 602.3 [M+H]⁺.

### Example 540. (S)-2-(4-((2-((4-Cyano-2-fluorophenoxy)methyl)pyrimidin-4-yl)oxy)-2-methylbenzyl)-4-fluoro-1-(oxetan-2-ylmethyl)-1H-benzo[d]imidazole-6-carboxylic acid (540)

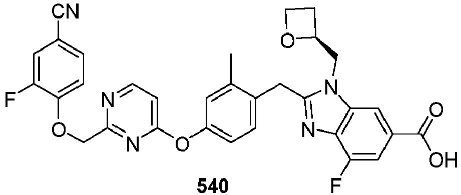

### Step A. tert-Butyl 2-(2-bromo-4-hydroxyphenyl)acetate (540b)

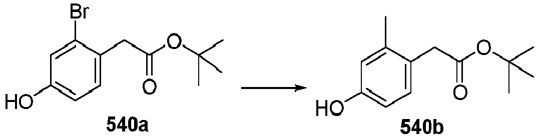

To a solution of **540a** (synthesized from 2-(2-bromo-4-hydroxyphenyl)acetic acid in similar procedures as described in **IIIf,** Steps D to F, 420 mg, 1.46 mmol), methylboronic acid (262.67 mg, 4.39 mmol) and K₂CO₃ (303 mg, 2.19 mmol) in dioxane/H₂O (4:1, 10 mL) was added Pd(dppf)Cl₂ (107 mg, 0.15 mmol). The mixture was stirred at 95 °C under N₂ for 16 h. Then the mixture was diluted with EtOAc (50 mL) and washed with brine (50 mL × 2). The combined organic layer was dried, evaporated to dryness and purified by silica gel column chromatography (20% EtOAc in petroleum ether) to afford the title product **(540b)** (290 mg, 85%) as a white solid. *m*/*z* (ESI, +ve ion) = 245.1 [M+Na]⁺.

### Step B. tert-Butyl 2-(4-((2-((4-cyano-2-fluorophenoxy)methyl)pyrimidin-4-yl)oxy)-2-methylphenyl)acetate (540c)

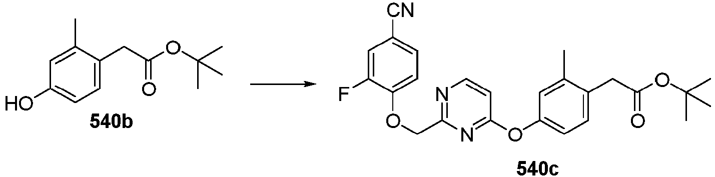

To a solution of **460a** (264 mg, 1 mmol) and **540b** (222 mg,1 mmol) in DMF (5 mL) was added K₂CO₃ (138 mg, 1 mmol). The mixture was stirred at 50 °C for 16 h, diluted with EtOAc (30 mL) and washed with brine (30 mL × 2). The combined organic layer was dried, evaporated to dryness and the residue was purified by silica gel column chromatography (25% EtOAc in petroleum ether) to afford the title product (540c) (290 mg, 61%) as a white solid. *m*/*z* (ESI, +ve ion) = 450.1 [M+H]⁺.

### Step C. (5)-2-(4-((2-((4-Cyano-2-fluorophenoxy)methyl)pyrimidin-4-yl)oxy)-2-methylbenzyl)-4-fluoro-1-(oxetan-2-ylmethyl)-1H-benzo[d]imidazole-6-carboxylic acid (540)

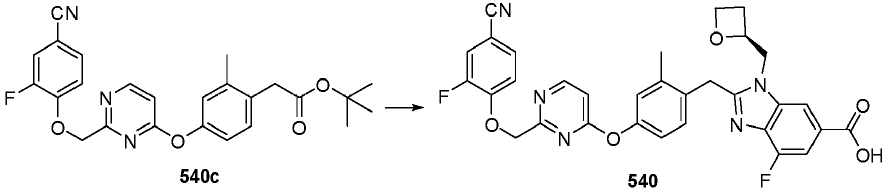

The title product **(540)** was synthesized in similar procedures as described in Example 536, Steps B to E. *m*/*z* (ESI, +ve ion) = 598.1 [M+H]⁺. ¹H NMR (400 MHz, CD₃OD) δ ppm 8.60 (d, *J* = 5.83 Hz, 1H), 8.11 (d, *J* = 1.09 Hz, 1H), 7.65 (d, *J* = 11.37 Hz, 1H), 7.33 - 7.51 (m, 2H), 7.11 (t, *J =* 8.47 Hz, 1H), 6.99 (d, *J =* 6.01 Hz, 3H), 6.84 (dd, *J =* 8.36, 2.47 Hz, 1H), 5.31 (s, 2H), 5.11 (td, *J =* 6.99, 4.80 Hz, 1H), 4.62 (ddd, *J =* 15.59, 12.18, 7.54 Hz, 2H), 4.51 (s, 2H), 4.39 - 4.48 (m, 2H), 2.67 - 2.81 (m, 1H), 2.45 (ddd, *J =* 16.48, 11.56, 7.43 Hz, 1H), 2.33 (s, 3H).

Example 541 was synthesized in similar procedures as described in Example 540.

### Example 542. (S)-2-((5-((2-((4-Cyano-2-fluorophenoxy)methyl)pyridin-4-yl)oxy)-3-fluoropyridin-2-yl)methyl)-4-fluoro-1-(oxetan-2-ylmethyl)-1H-benzo[d]imidazole-6-carboxylic acid (542)

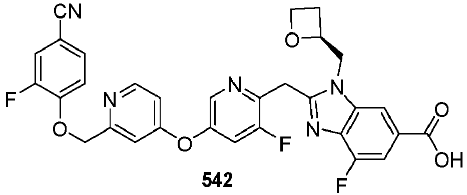

### Step A. Diethyl 2-(5-(benzyloxy)-3-fluoropyridin-2-yl)malonate (542a)

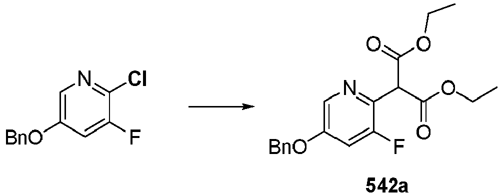

A mixture of 5-(benzyloxy)-2-chloro-3-fluoropyridine (2.0 g, 8.4 mmol), diethyl malonate (1.61 g, 10.0 mmol), K₃PO₄ (5.35 g, 25.2 mmol), BINAP (520 mg, 0.8 mmol) and Pd₂(dba)₃ (230 mg, 0.2 mmol) in toluene (20 mL) was stirred at 90 °C for 18 h under N₂. The mixture was filtered, concentrated, and purified by silica gel column chromatography (EtOAc:petroleum ether = 1:4) to afford the title compound **(542a)** (0.67 g, 25%) as a colorless oil. *m*/*z* (ESI, +ve ion) = 362.1 [M+H]⁺.

### Step B. 2-(5-(Benzyloxy)-3-fluoropyridin-2-yl)acetic acid (542b)

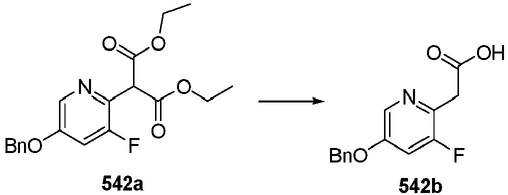

A mixture of **542a** (610 mg, 1.688 mmol) in EtOH/H₂O (2:1, 10 mL) was added NaOH (202 mg, 5.064 mmol). The reaction was heated at 80 °C for 2 h. After cooling down, the reaction was adjusted to pH = 6 with 6 N HCl and extracted with EtOAc (25 mL × 2). The combined organic layer was washed with H₂O (20 mL), brine (20 mL), dried over Na₂SO₄, filtered and concentrated to afford the title product **(542b)** (400 mg, 81.6%). m/z (ESI, +ve ion) = 262.0 [M+H]⁺.

### Step C. Methyl 2-(5-(benzyloxy)-3-fluoropyridin-2-yl)acetate (542c)

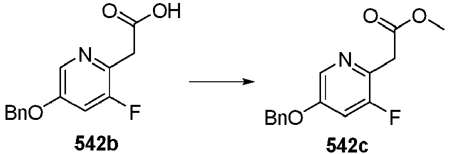

A mixture of **542b** (400 mg, 1.53 mmol), thionyl chloride (363 mg, 3.06 mmol) in CH₃OH (5 mL) was stirred at 60 °C for 2 h. The mixture was concentrated, diluted with EtOAc (80 mL), washed with aq. NaHCO₃ (30 mL × 2), H₂O (20 mL), and brine (20 mL). The organic layer was dried over Na₂SO₄, filtered and concentrated *in vacuo* to afford the title product (**542c**) (410 mg, 86.4%) as a colorless oil. *m*/*z* (ESI, +ve ion) = 276.1 [M+H]⁺.

### Step D. Methyl 2-(3-fluoro-5-hydroxypyridin-2-yl)acetate (542d)

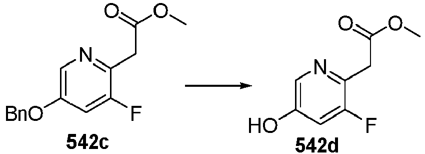

A mixture of **542c** (410 mg, 1.50 mmol) in CH₃OH (6 mL) was added 10% Pd/C (100 mg). The reaction was stirred at 20 °C for 2 h under H₂. Then it was filtered and concentrated *in vacuo* to afford the title product **(542d)** (220 mg, 71.8%) as a white solid, which was used in the next step without further purification. *m*/*z* (ESI, +ve ion) = 186.1 [M+H]⁺.

### Step E. (S)-2-((5-((2-((4-Cyano-2-fluorophenoxy)methyl)pyridin-4-yl)oxy)-3-fluoropyridin-2-yl)methyl)-4-fluoro-1-(oxetan-2-ylmethyl)-1H-benzo[d]imidazole-6-carboxylic acid (542)

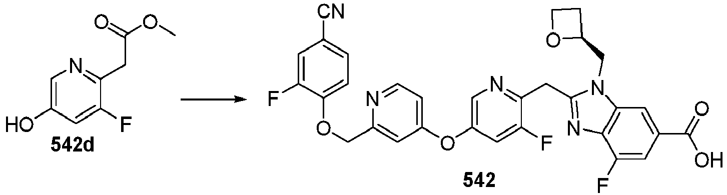

The title compound \**(542)** was synthesized from **542d** and 4-((4-bromopyridin-2-yl)methoxy)-3-fluorobenzonitrile (synthesized in similar procedures as **536a)** in similar procedures as described in Example 471, Steps B to E. *m*/*z* (ESI, +ve ion) = 602.1 [M+H]⁺. ¹H NMR (400 MHz, CD₃OD) δ ppm 8.50 (d, *J =* 5.75 Hz, 1H), 8.27 (d, *J =* 2.00 Hz, 1H) 8.03 (s, 1H), 7.51 - 7.65 (m, 4H), 7.27 - 7.35 (m, 2H), 7.08 (dd, *J =* 5.88, 2.38 Hz, 1H), 5.31 (s, 2H), 5.19 - 5.27 (m, 1H), 4.69 - 4.82 (m, 3H), 4.59 - 4.65 (m, 2H), 4.43 (dt, *J =* 9.19, 5.91 Hz, 1 H), 2.74 - 2.83 (m, 1 H), 2.45 - 2.54 (m, 1 H).

Example **543** was synthesized from **540a** and 4-((4-bromopyridin-2-yl)methoxy)-3-fluorobenzonitrile in similar procedures as described in Example **536,** Steps A to D, followed by bromo to cyano conversion with Zn(CN)₂, tBuXphosPdG₃ and then follow Step E in Example **536.**

### Example 544. (S)-2-((6-((2-((2-Fluoro-4-methylphenoxy)methyl)pyridin-4-yl)oxy)pyridin-3-yl)methyl)-1-(oxetan-2-ylmethyl)-1H-benzo[d]imidazole-6-carboxylic acid (544)

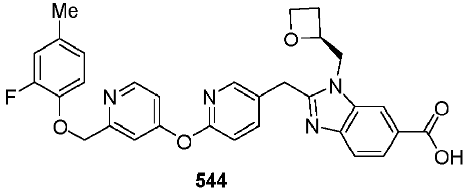

### Step A. 2-((2-Fluoro-4-methylphenoxy)methyl)pyridin-4-ol (544b)

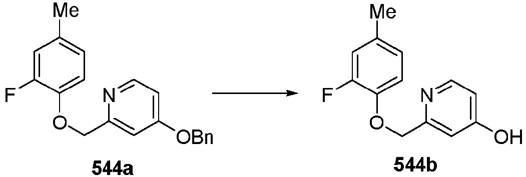

A solution of **544a** ((prepared from (4-(benzyloxy)pyridin-2-yl)methanol in similar procedures as **459b,** 1.02 g, 3.2 mmol) in TFA (20 mL) was stirred at 80 °C for 12 h. After completion, the solvent was removed under reduced pressure. The reaction was diluted with H₂O (30 mL) and extracted with EtOAc (30 mL × 3). The organic layer was dried, filtered, concentrated and purified by silica gel column chromatography (petroleum ether: EtOAc = 7:3) to afford the title product **(544b)** (0.7 g, 90.6%) as a white solid. *m*/*z* (ESI, +ve ion) = 234.1 [M+H]⁺.

### Step B. Methyl 2-(6-((2-((2-fluoro-4-methylphenoxy)methyl)pyridin-4-yl)oxy)pyridin-3-yl)acetate (544c)

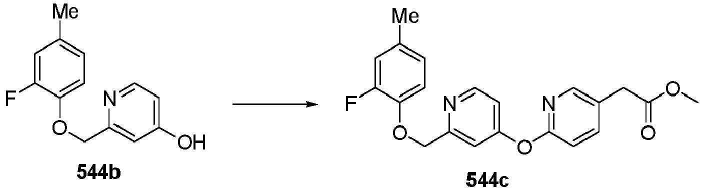

A solution of **544b** (200 mg, 0.86 mmol), methyl 2-(6-chloropyridin-3-yl)acetate (175 mg, 0.94 mmol), Pd₂(dba)₃ (157 mg, 0.17 mmol), BINAP (213 mg, 0.34 mmol) and Cs₂CO₃ (838 mg, 2.57 mmol) in toluene (10 mL) was reacted under microwave at 120 °C for 3 h. After completion, the mixture was quenched with H₂O (50 mL) and extracted with EtOAc (50 mL × 3). The organic layer was washed with H₂O (50 mL), brine (50 mL), dried over anhydrous Na₂SO₄, filtered and concentrated *in vacuo* to give a crude residue, which was purified by silica gel column chromatography (petroleum ether:EtOAc = 1:1) to afford the title product **(544c)** (195 mg, 59%) as a yellow oil. *m*/*z* (ESI, +ve ion) = 383.3 [M+H]⁺.

### Step C. (S)-2-((6-((2-((2-Fluoro-4-methylphenoxy)methyl)pyridin-4-yl)oxy)pyridin-3-yl)methyl)-1-(oxetan-2-ylmethyl)-1H-benzo[d]imidazole-6-carboxylic acid (544)

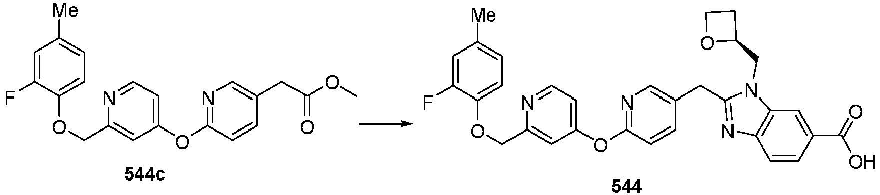

The title compound **(544)** was synthesized from **544c** in similar procedures as described in Example 471, Steps C to E. *m*/*z* (ESI, +ve ion) = 555.2 [M+H]⁺. ¹H NMR (400 MHz, CD₃OD) δ ppm 8.46 (d, *J =* 5.69 Hz, 1H), 8.24 (s, 1H), 8.12 (s, 1H), 7.94 (d, *J =* 8.40 Hz, 1H), 7.87 (dd, *J* = 8.39, 2.03 Hz, 1H), 7.55 (d, *J* = 8.42 Hz, 1H), 7.30 (s, 1H), 7.09 - 7.19 (m, 2H), 6.81 - 7.01 (m, 3H), 5.13 -5.25 (m, 3H), 4.57 - 4.69 (m, 2H), 4.45 - 4.56 (m, 3H), 4.40 (dd, *J =* 5.92, 3.12 Hz, 1H), 2.72 - 2.82 (m, 1H), 2.46 - 2.54 (m, 1H), 2.24 (s, 3H).

Example **545, 546, 547, 548, 549,** and **550** were synthesized in similar procedures as described in Example **360,** with the Boc group removed by TFA.

Example **551** was synthesized from **460a** and methyl 2-((1*S*,3*S*)-3-hydroxycyclobutyl)acetate in similar procedures as described in Example **471,** Steps B to E. In Step B, Pd₂(dba)₃, BINAP and K₃PO₄ were used for the coupling.

### Biological Example 1

### GLP-1R cAMP assay

cAMP accumulation was measured in Chinese hamster ovary (CHO) cells stably overexpressing the human GLP-1 receptor using the HitHunter cAMP Assay for Small Molecules Kit (Eurofins). Briefly, cells were grown in Ham's F12 Nutrient Mix with 10% FBS, and lifted with Cell Dissociation Buffer, Enzyme free, PBS based (ThermoFisher). Cells were pelleted, and resuspended in Hank's buffered saline solution with 10 mM HEPES, and 625 µM 3-isobutyl-1-methylxanthine. The anti-cAMP antibody reagent was then added to cells at a 1:2 ratio and 5 µL of mixture was seeded in 384-well small volume white assay plates, at 10,000 cells/well. Cells were then treated with 50 nL compound using an ECHO 550 acoustic dispenser (Labcyte) in a 20-point dose response format in triplicate for 30 min. Cells were then lysed, and detection reagents added according to the manufacturer's protocol. After overnight incubation, luminescence was measured using a Perkin Elmer Envision plate reader. Dose response curves were analyzed using GraphPad Prism 9.0.

Results are reported in Table 1 below. In the Table, EC₅₀ values are +++ ≤ 50 nM< ++ ≤ 500 nM < +. Molecular weights are calculated by standard techniques, and mass spectrometry results are reported according to the Examples above.

| **Example Compound** | **EC₅₀** | **MW Calc.** | **[M+H]⁺ Found** |
|---|---|---|---|
| **119** | ++ | 483.568 | 484.3 |
| **127** | +++ | 584.5 | 584.2 |
| **134** | ++ | 521.621 | 522.3 |
| **147** | +++ | 595.48 | 595.1 |
| **167** | +++ | 586.05 | 586.3 |
| **172** | + | 561.04 | 561.3 |
| **173** | ++ | 589.09 | 589.3 |
| **175** | +++ | 603.5 | 603.3 |
| **176** | +++ | 605.52 | 605.3 |
| **177** | +++ | 567.47 | 567.3 |
| **180** | +++ | 581.49 | 581 |
| **181** | +++ | 578.45 | 578 |
| **184** | +++ | 627.14 | 627.3 |
| **185** | ++ | 599.09 | 599.3 |
| **190** | +++ | 565.45 | 565.3 |
| **191** | +++ | 557.43 | 557.3 |
| **192** | +++ | 568.45 | 568.3 |
| **193** | +++ | 560.01 | 560.3 |
| **194** | +++ | 559.02 | 559.3 |
| **195** | +++ | 564.46 | 564.3 |
| **196** | +++ | 602.52 | 602.3 |
| **197** | +++ | 604.53 | 604.3 |
| **201** | ++ | 606.09 | 606.2 |
| **209** | +++ | 644.14 | 644.2 |
| **210** | +++ | 606.5 | 606.3 |
| **211** | +++ | 598.06 | 598.3 |
| **212** | +++ | 597.07 | 597.3 |
| **213** | +++ | 559.4 | 559.2 |
| **218** | +++ | 607.49 | 607.2 |
| **219** | +++ | 580.42 | 580.2 |
| **222** | +++ | 644.14 | 644.2 |
| **223** | +++ | 644.14 | 644.2 |
| **225** | +++ | 584.03 | 584.3 |
| **226** | +++ | 594.07 | 594.3 |
| **228** | +++ | 579.43 | 579.3 |
| **229** | +++ | 570 | 570.3 |
| **233** | +++ | 620.53 | 620.3 |
| **234** | +++ | 620.53 | 621.3 |
| **235** | ++ | 646.57 | 647.3 |
| **236** | +++ | 580.42 | 580.2 |
| **237** | +++ | 579.43 | 579.3 |
| **238** | +++ | 579.43 | 579.3 |
| **244** | +++ | 621.52 | 621.3 |
| **245** | +++ | 621.52 | 621.3 |
| **273** | +++ | 621.52 | 621.1 |
| **281** | +++ | 622.55 | 622.1 |
| **282** | +++ | 613.12 | 613.2 |
| **289** | +++ | 567.01 | 567.3 |
| **293** | +++ | 621.52 | 621.2 |
| **298** | +++ | 582.58 | 583.3 |
| **350** | +++ | 556.594 | 557.2 |
| **351** | +++ | 584.02 | 584.2 |
| **360** | +++ | 568 | 568.1 |
| **361** | +++ | 585.99 | 586.1 |
| **383** | +++ | 566.48 | 566.1 |
| **384** | +++ | 565.45 | 565.2 |
| **385** | +++ | 603.5 | 603.3 |
| **386** | +++ | 567.47 | 567.3 |
| **387** | +++ | 593.44 | 593.3 |
| **388** | +++ | 582.45 | 582.2 |
| **389** | +++ | 584.47 | 584.3 |
| **390** | +++ | 576.43 | 576.3 |
| **391** | +++ | 567.47 | 565.3 |
| **392** | +++ | 576.43 | 576.3 |
| **393** | ++ | 549 | 549.3 |
| **394** | ++ | 559.98 | 560.3 |
| **395** | +++ | 540.555 | 541.3 |
| **396** | +++ | 549.99 | 550.2 |
| **397** | +++ | 551.02 | 551.3 |
| **398** | +++ | 562 | 562.3 |
| **399** | +++ | 567.47 | 567.3 |
| **400** | +++ | 538.579 | 539.2 |
| **401** | +++ | 564.46 | 564 |
| **402** | +++ | 565.45 | 565.2 |
| **403** | +++ | 549 | 549.3 |
| **404** | +++ | 559.98 | 560.3 |
| **405** | +++ | 566.48 | 566.1 |
| **406** | +++ | 566.48 | 566.2 |
| **407** | +++ | 566.99 | 567.3 |
| **408** | +++ | 557.558 | 558.3 |
| **409** | +++ | 569.01 | 569.3 |
| **410** | +++ | 559.574 | 560.4 |
| **411** | +++ | 550.03 | 550.2 |
| **412** | ++ | 550.03 | 550.2 |
| **413** | +++ | 584.98 | 585.3 |
| **414** | +++ | 540.595 | 541.2 |
| **415** | ++ | 540.595 | 541.3 |
| **416** | +++ | 591.567 | 592.3 |
| **417** | +++ | 566 | 566.3 |
| **418** | +++ | 566 | 566.3 |
| **419** | +++ | 568.02 | 568.3 |
| **420** | +++ | 568.02 | 568.3 |
| **421** | +++ | 575.548 | 576.3 |
| **422** | +++ | 580.596 | 581.3 |
| **423** | ++ | 598.611 | 599.3 |
| **424** | +++ | 598.586 | 599.3 |
| **425** | ++ | 616.601 | 617.3 |
| **426** | ++ | 566 | 566.3 |
| **427** | +++ | 569.01 | 569.2 |
| **428** | +++ | 569.01 | 569.1 |
| **429** | +++ | 590.03 | 590.3 |
| **430** | +++ | 608.02 | 608.3 |
| **431** | +++ | 574.05 | 574.3 |
| **432** | +++ | 592.04 | 592.3 |
| **433** | ++ | 574.05 | 574.3 |
| **434** | ++ | 592.04 | 592.3 |
| **435** | +++ | 549.99 | 550.2 |
| **436** | +++ | 566.99 | 567.3 |
| **437** | +++ | 584.98 | 585.3 |
| **438** | +++ | 590.03 | 590.3 |
| **439** | ++ | 558.586 | 559.2 |
| **440** | +++ | 566.99 | 567.3 |
| **441** | ++ | 569.01 | 569.3 |
| **442** | +++ | 569.01 | 569.3 |
| **443** | +++ | 558.586 | 559.2 |
| **444** | + | 558.586 | 559.1 |
| **445** | +++ | 558.586 | 559.2 |
| **446** | +++ | 568.02 | 568.1 |
| **447** | +++ | 558.586 | 559.1 |
| **448** | ++ | 568.02 | 568.1 |
| **449** | +++ | 576.576 | 577.3 |
| **450** | +++ | 576.576 | 577.3 |
| **451** | +++ | 601.43 | 601.2 |
| **452** | +++ | 584.98 | 585.3 |
| **453** | +++ | 587.00 | 587.1 |
| **454** | +++ | 577.564 | 578.1 |
| **455** | ++ | 577.564 | 578.2 |
| **456** | +++ | 569.01 | 569.3 |
| **457** | +++ | 587.00 | 587.3 |
| **458** | +++ | 574.01 | 574.2 |
| **459** | +++ | 609.99 | 610.2 |
| **460** | +++ | 621.605 | 622.3 |
| **461** | +++ | 620.617 | 621.3 |
| **462** | +++ | 630.05 | 630.3 |
| **463** | +++ | 630.05 | 630.3 |
| **464** | +++ | 592.00 | 592.2 |
| **465** | +++ | 583.552 | 584.3 |
| **466** | +++ | 592.00 | 592.2 |
| **467** | +++ | 601.542 | 602.3 |
| **468** | +++ | 606.590 | 607.3 |
| **469** | +++ | 623.592 | 624.3 |
| **470** | +++ | 619.533 | 619.2 |
| **471** | +++ | 609.99 | 610.2 |
| **472** | +++ | 581.05 | 581.3 |
| **473** | +++ | 595.07 | 595.3 |
| **474** | +++ | 555.41 | 555.3 |
| **516** | +++ | 569.01 | 569.2 |
| **517** | +++ | 569.01 | 569.2 |
| **518** | +++ | 559.574 | 560 |
| **519** | +++ | 559.574 | 560.2 |
| **520** | +++ | 559.574 | 560.4 |
| **521** | +++ | 577.564 | 578.3 |
| **522** | +++ | 571.585 | 572.4 |
| **523** | +++ | 589.575 | 590.3 |
| **524** | +++ | 588.04 | 588.3 |
| **525** | +++ | 606.03 | 606.3 |
| **526** | ++ | 591.591 | 592.3 |
| **527** | +++ | 591.591 | 592.3 |
| **528** | +++ | 591.591 | 592.3 |
| **529** | +++ | 577.564 | 578.2 |
| **530** | +++ | 559.574 | 560.2 |
| **531** | +++ | 589.575 | 590.3 |
| **532** | +++ | 589.575 | 590.3 |
| **533** | +++ | 559.574 | 560.3 |
| **534** | +++ | 577.564 | 578.3 |
| **535** | + | 574.545 | 575.3 |
| **536** | +++ | 609.99 | 610 |
| **537** | +++ | 600.554 | 601.2 |
| **538** | +++ | 627.98 | 628 |
| **539** | +++ | 601.542 | 602.3 |
| **540** | +++ | 597.579 | 598.1 |
| **541** | +++ | 579.588 | 580.2 |
| **542** | +++ | 601.542 | 602.1 |
| **543** | +++ | 607.574 | 608.3 |
| **544** | +++ | 554.578 | 555.2 |
| **545** | +++ | 558.57 | 559.2 |
| **546** | +++ | 576.561 | 577.2 |
| **547** | +++ | 568 | 568.2 |
| **548** | +++ | 551.551 | 552.2 |
| **549** | + | 558.57 | 559.2 |
| **550** | +++ | 537.524 | 538.1 |
| **551** | +++ | 543.555 | 544.2 |

### Equivalents

The disclosure set forth above may encompass multiple distinct embodiments with independent utility. Although each of these embodiments has been disclosed, the specific embodiments thereof as disclosed and illustrated herein are not to be considered in a limiting sense, because numerous variations are possible. The subject matter of the embodiments includes all novel and nonobvious combinations and subcombinations of the various elements, features, functions, and/or properties disclosed herein. The following claims particularly point out certain combinations and subcombinations regarded as novel and nonobvious. Alternative embodiments as in other combinations and subcombinations of features, functions, elements, and/or properties may be claimed in this application, in applications claiming priority from this application, or in related applications. Such claims, whether directed to a different embodiment or to the same embodiment, and whether broader, narrower, equal, or different in scope in comparison to the original claims, also are regarded as included within the subject matter of this disclosure.

One or more features from any embodiments described herein or in the figures may be combined with one or more features of any other embodiments described herein or in the figures without departing from the scope of this disclosure.

All publications, patents and patent applications cited in this specification are herein incorporated by reference as if each individual publication or patent application were specifically and individually indicated to be incorporated by reference. Although the foregoing disclosure has been described in some detail by way of illustration and example for purposes of clarity of understanding, it will be readily apparent to those of ordinary skill in the art in light of the teachings of this disclosure that certain changes and modifications may be made thereto without departing from the spirit or scope of the appended claims.

Preferred embodiments of the present invention are described below and are referred to as embodiments E1 to E38.
E 1. A compound of Formula I, or a pharmaceutically acceptable salt or stereoisomer thereof: 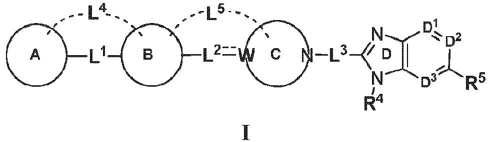
wherein, A is substituted or unsubstituted aryl, or substituted or unsubstituted heteroaryl;
Ring B is substituted or unsubstituted aryl, substituted or unsubstituted heteroaryl, or substituted or unsubsuted bicyclic;
Ring C is substituted or unsubstituted C₄-C₆ heterocycloalkyl, substituted or unsubstituted C₅ heterocycloalkenyl, or substituted or unsubstituted phenyl, wherein heterocycloalkyl and heterocycloalkenyl each comprises at least one N, linked to **L³** as depicted;
zero, one, or two of **D¹**, **D²**, and **D³** are N, and the rest are CH, or **CR⁶**;
**W** is **CR¹⁴** or **C;** when **W** is **CR¹⁴**, the adjacent dashed line indicates a single bond; when **W** is C, the adjacent dashed line indicates a double bond or **L²** is -C(H)=;
**L¹** is selected from the group consisting of a bond, -O-, -CH₂-, and -OCH₂-;
**L²** is selected from the group consisting of a bond, -CH₂-, -C(H)=, and -O-, ;
**L³** is -CH₂-;
**L⁴** is absent, or **L⁴** together with **L¹** and Rings **A** and **B** form a fused tricycle, and **L⁵** is absent;
**L⁵** is absent, or **L⁵** together with **L²** and Rings **B** and **C** form a fused tricycle, and **L⁴** is absent;
**R⁴** is unsubstituted alkyl, substituted alkyl, unsubstituted arylalkylene, substituted arylalkylene, unsubstituted heteroalkyl, substituted heteroalkyl, unsubstituted heteroarylalkylene, substituted heteroarylalkylene, unsubstituted heterocycloalkylalkylene, or substituted heterocycloalkylalkylene;
**R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, - SO₂NH₂, -SO₂NHMe, -B(OH)₂, 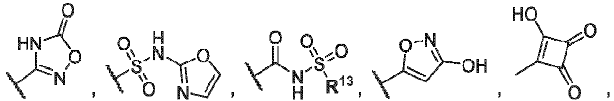 and tetrazolyl;
each **R⁶** is independently selected from the group consisting of alkyl, substituted alkyl, halo, hydroxyl, alkoxyl, **R¹¹R¹²**NCO-, and **R¹¹R¹²**N-;
**R¹¹** is hydrogen, or alkyl;
**R¹²** is hydrogen, or alkyl;
**R¹³** is hydrogen, or alkyl;
**R¹⁴** is hydrogen, cyano, halo, hydroxyl, alkyl, haloalkyl, or methyl;
wherein when the C ring is C₆ heterocycloalkyl, the B ring is pyrazole or 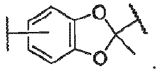
E 2. The compound of E1 according to Formula **la, lb,** or **Ic,** or a pharmaceutically acceptable salt or stereoisomer thereof: 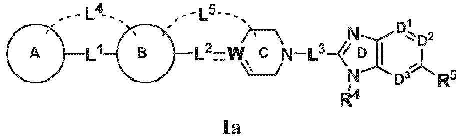 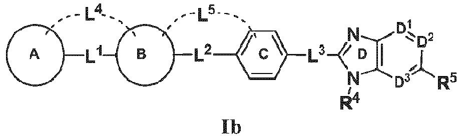 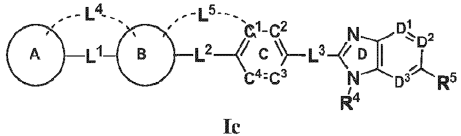
Wherein, **A** is substituted or unsubstituted aryl, substituted or unsubstituted heteroaryl;
Ring **B is** substituted or unsubstituted aryl, or substituted or unsubstituted heteroaryl;
Ring **C** is further unsubstituted or further substituted, and/or bridged, and/or fused;
zero, one, or two of **C¹**, **C²**, **C³**, and **C⁴** are N, and the rest are CH, or **CR⁶**;
zero, one, or two of **D¹**, **D²**, and **D³** are N, and the rest are CH, or **CR⁶**;
**W** is CH or C; when **W** is CH, the adjacent dashed lines indicate single bonds; when
**W is** C, one adjacent dashed line indicates a double bond or **L²** is -C(H)=;
**L¹** is selected from the group consisting of -O-, -CH₂-, and OCH₂-;
**L²** is selected from the group consisting of a bond, -CH₂-, -C(H)=, -CF₂-, and -O-;
**L³** is -CH₂-;
**L⁴** is absent, or **L⁴** together with **L¹** and Rings **A** and **B** form a fused tricycle, and **L⁵** is absent;
**L⁵** is absent, or **L⁵** together with **L²** and Rings **B** and **C** form a fused tricycle, and **L⁴** is absent;
**R⁴** is unsubstituted alkyl, substituted alkyl, unsubstituted arylalkylene, substituted arylalkylene, unsubstituted heteroalkyl, substituted heteroalkyl, unsubstituted heteroarylalkylene, substituted heteroarylalkylene, unsubstituted heterocycloalkylalkylene, or substituted heterocycloalkylalkylene;
**R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, - SO₂NH₂, -SO₂NHMe, -B(OH)₂, 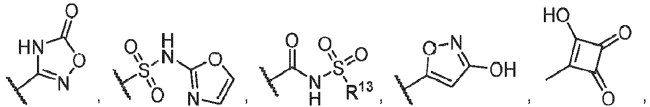 and tetrazolyl;
each **R⁶** is independently selected from the group consisting of alkyl, substituted alkyl, halo, hydroxyl, alkoxyl, **R¹¹R¹²**NCO-, and **R¹¹R¹²**N-;
**R¹¹** is hydrogen, or alkyl;
**R¹²** is hydrogen, or alkyl; and
**R¹³** is hydrogen, or alkyl.
E3. The compound of E 1 according to Formula **IIe, IIf,** or **IIi-IIn,** or a pharmaceutically acceptable salt, or stereoisomer thereof: 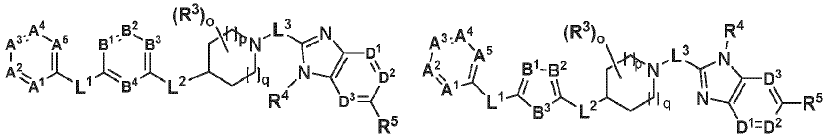 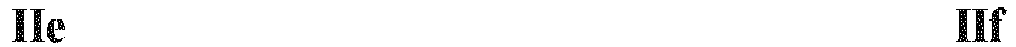 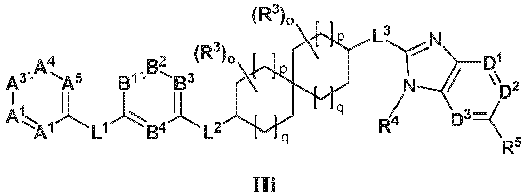 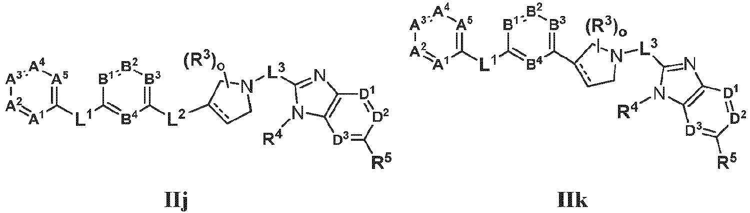 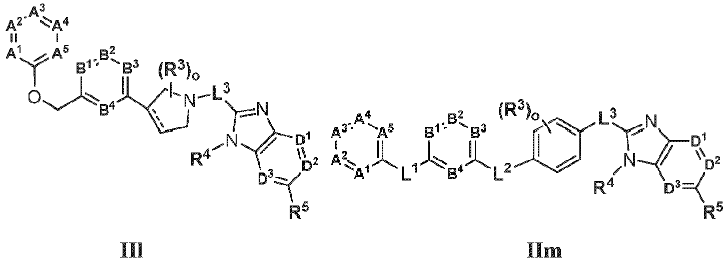 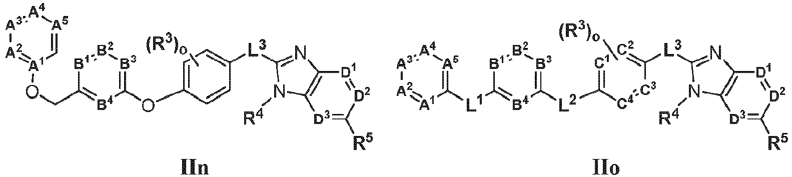
wherein zero, one, or two of **A¹**, **A²**, **A³**, **A⁴**, and **A⁵** are N, and the rest are CH, or C**R¹**; zero, one, or two of **B¹**, **B²**, **B³**, and **B⁴** are N, and the rest are CH, or C**R²**, wherein, in Formula **IIb**, **B⁴** is absent;
zero, one, or two of **C¹**, **C²**, **C³**, and **C⁴** are N, and the rest are CH, or **CR⁶**;
zero, one, or two of **D¹**, **D²**, and **D³** are N, and the rest are CH, or **CR⁶**;
each **R¹** is independently selected from the group consisting of alkyl, substituted alkyl, halo, cyano, azido, alkoxy, haloalkoxy, and **R¹¹R¹²**NCO-;
each **R²** is independently selected from the group consisting of alkyl, substituted alkyl, halo, cyano, azido, alkoxy, haloalkoxy, and **R¹¹R¹²**NCO-;
each **R³** is independently selected from the group consisting of alkyl, substituted alkyl, halo, cyano, azido, alkoxy, haloalkyl, haloalkoxy, and **R¹¹R¹²**NCO-;
**R⁴** is unsubstituted alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene;
**R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, - SO₂NH₂, -SO₂NHMe, -B(OH)₂, 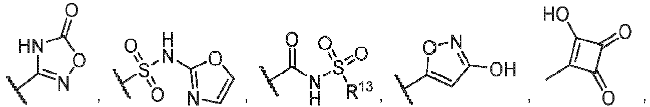 and tetrazolyl;
each **R⁶** is independently selected from the group consisting of F, and methyl;
**R¹¹** is hydrogen, or alkyl;
**R¹²** is hydrogen, or alkyl;
**R¹³** is hydrogen, or alkyl;
**o** is 1, 2, 3, or 4;
**p** is 0 or 1; and
**q** is 0 or 1.
E4. The compound of E 1 according to any of the following Formulas, or a pharmaceutically acceptable salt, or stereoisomer thereof: 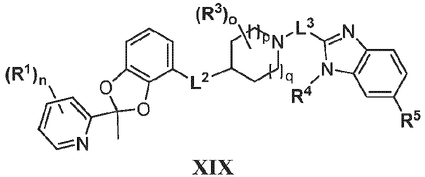 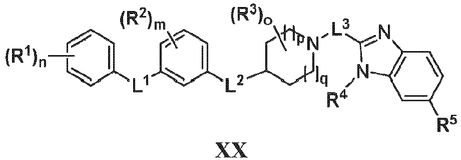 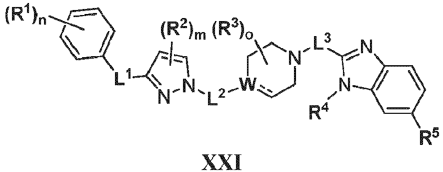 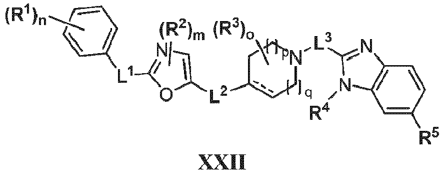 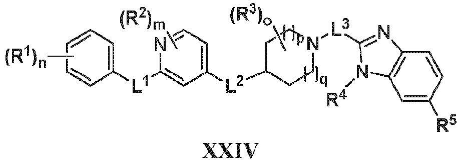 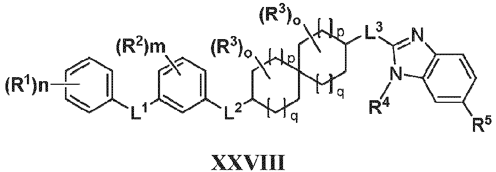 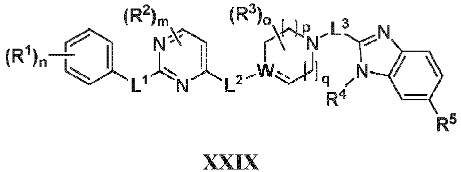 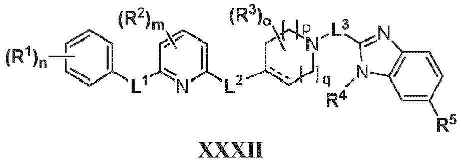 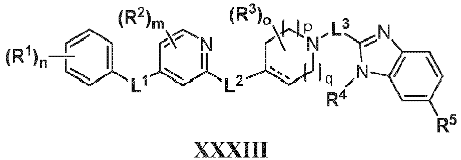 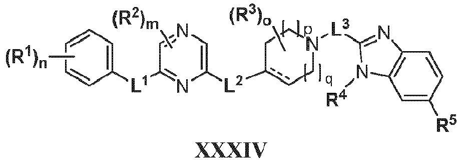 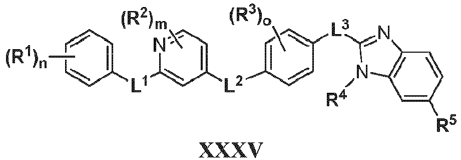 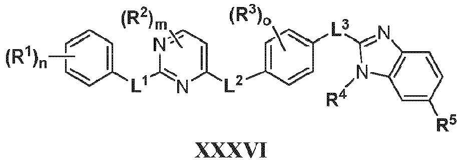 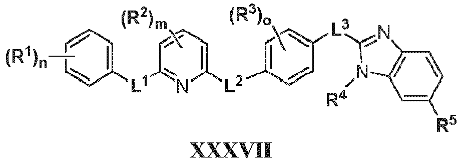 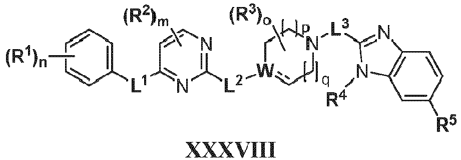 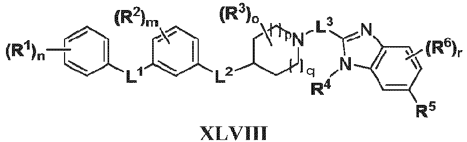 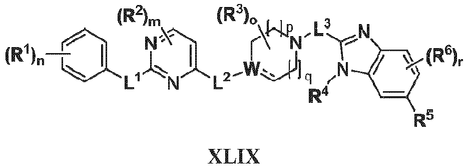 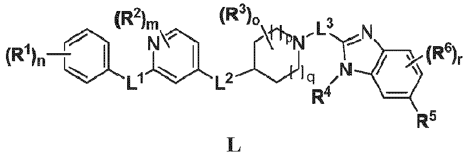 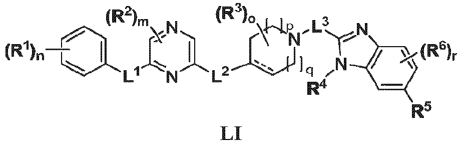 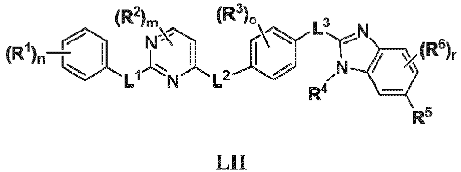 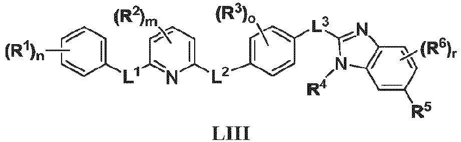 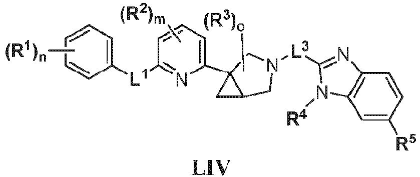 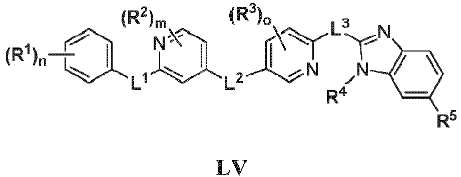 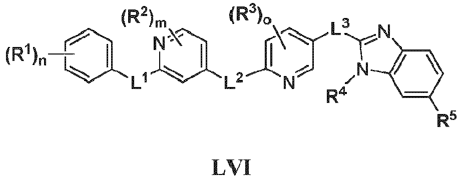
wherein each **R¹** is independently selected from the group consisting of alkyl, substituted alkyl, halo, cyano, azido, alkoxy, haloalkoxy, and **R¹¹R¹²**NCO-;
each **R³** is independently selected from the group consisting of alkyl, substituted alkyl, halo, cyano, azido, alkoxy, haloalkyl, haloalkoxy, and **R¹¹R¹²**NCO-;
**R⁴** is alkyl, substituted alkyl, unsubstituted arylalkylene, substituted arylalkylene, heteroalkyl, substituted heteroalkyl, unsubstituted heteroarylalkylene, substituted heteroarylalkylene, unsubstituted heterocycloalkylalkylene, or substituted heterocycloalkylalkylene;
**R⁵** is selected from the group consisting of -COOH, -COCF₃, -C(OH)CF₃, -CONHCN, -CONHOH, CONHOMe, -CONHSO₂N(Me)₂, -PO₃H₂, -PO(Me)(OH), -SO₃H, - SO₂NH₂, -SO₂NHMe, -B(OH)₂, 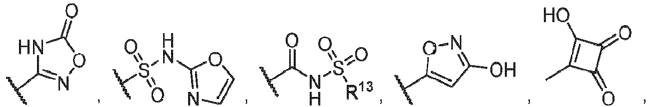 and tetrazolyl;
each **R¹¹** is hydrogen, or alkyl;
each **R¹²** is hydrogen, or alkyl;
each **R¹³** is hydrogen, or alkyl;
**R¹⁴** is hydrogen, cyano, halo, hydroxyl, or methyl; and
n is an integer from 0 to 5; **o** is an integer from 0 to 4; **p is** 0 or 1; **q** is 0 or 1.
E 5. The compound of any of E1-E4 wherein each **R²**, and **R³** is H, or F.
E 6. The compound of any of E1-E5 wherein **m** is 0, and o is 0.
E 7. The compound of any of E1-E6 wherein **W** is N, or CH.
E 8. The compound of any of E1-E7 wherein **W** is C.
E 9. The compound of any of E1-E8 wherein **W** is CH
E 10. The compound of any of E1-E9 wherein **W** is N.
E 11. The compound of any of E1-E10 wherein **R⁴** is selected from the group consisting of 1-(cyanomethyl)-cycloprop-1-yl-methyl, (1-ethyl-1H-imidazol-5-yl)-methyl, 1-(Fluoromethyl)-cycloprop-1-yl-methyl, isoxazol-5-yl-methyl, oxetan-2-yl-methyl, (2*S*)-oxetan-2-yl-methyl, oxelan-3-yl-methyl, (3*R*)-oxelan-3-yl-methyl, 1,3-oxazol-2-yl-methyl, and tetrahydrofura-2-yl-methyl.
E 12. The compound of any of E1-E11 wherein **R⁴** is selected from the group consisting of (1-ethyl-1H-imidazol-5-yl)-methyl, oxetan-2-yl-methyl, (2*S*)-oxetan-2-yl-methyl, oxelan-3-yl-methyl, (3*R*)-oxelan-3-yl-methyl, and 1,3-oxazol-2-yl-methyl.
E 13. The compound of any of E1-E12 wherein **R⁴** is selected from the group consisting of 1-(cyanomethyl)-cycloprop-1-yl-methyl, 1-(Fluoromethyl)-cycloprop-1-yl-methyl, isoxazol-5-yl-methyl, and tetrahydrofura-2-yl-methyl.
E14. The compound of any of E1-E13 wherein **R⁴** is selected from the group consisting of oxetan-2-yl-methyl, and (1-ethyl-1H-imidazol-5-yl)-methyl.
E 15. The compound of any of E1-E14 wherein **R⁴** is oxetan-2-yl-methyl.
E 16. The compound of any of E1-E15 wherein **R⁴** is (2*S*)-oxetan-2-yl-methyl.
E 17. The compound of any of E1-E16 wherein **R⁴** is (1-ethyl-1H-imidazol-5-yl)-methyl.
E 18. The compound of any of E1-E17 wherein **R⁴** is selected from the group consisting of oxelan-3-yl-methyl and 1,3-oxazol-2-yl-methyl.
E 19. The compound of any of E1-E18 wherein **R⁴** is (3*R*)-oxelan-3-yl-methyl.
E 20. The compound of any of E1-E19 wherein **R⁴** is 1,3-oxazol-2-yl-methyl.
E 21. The compound of any of E1-E20 wherein **R⁵** is -COOH, or -COOMe.
E 22. The compound of any of E1-E21 wherein **R⁵** is -COOH
E 23. The compound of any of E1-E22 wherein **R⁵** is tetrazolyl.
E 24. The compound of any of E1-E23 wherein **R⁵** is 1H-1,2,3,4-tetrazol-5-yl.
E25. The compound of any of E1-E24 wherein **L²** is -CH₂-.
E 26. The compound of any of E1-E25 wherein **L²** is -CH= or -O-.
E 27. The compound of any of E1-E26 wherein **L²** is -CH=-.
E 28. The compound of any of E1-E27 wherein **L²** is -O-.
E 29. The compound of any of E1-E28 wherein **L³** is -CH₂-.
E 30. The compound of any of E1-E29 wherein **L¹** is -OCHz-; and **L²** is -CH₂- or -O -.
E 31. The compound of any of E1-E30 wherein **L¹** is -O-.
E 32. The compound of any of E1-E31 selected from the following, or a pharmaceutically acceptable salt or solvate thereof:

| **Compound** | **Structure** |
|---|---|
| 119 | 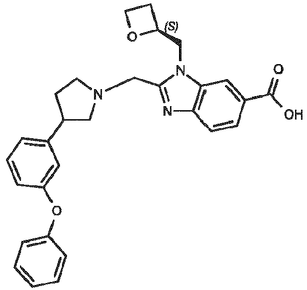 |
| 127 | 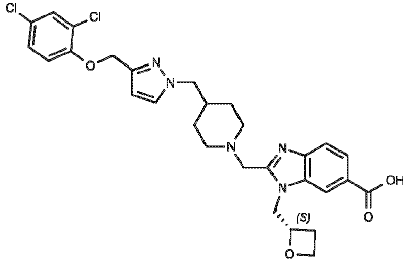 |
| 134 | 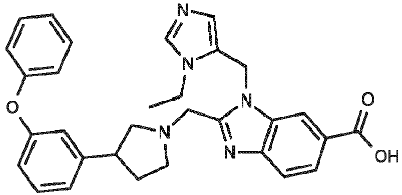 |
| 147 | 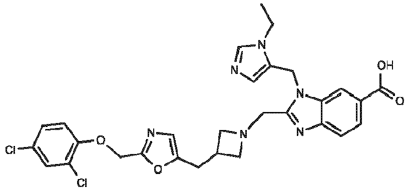 |
| 167 | 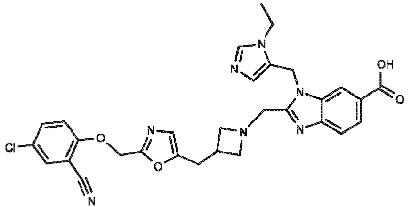 |
| 172 | 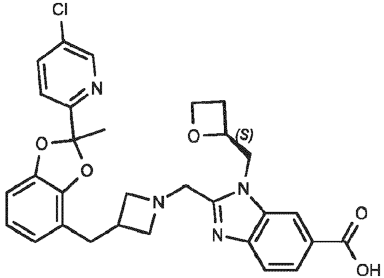 |
| 173 | 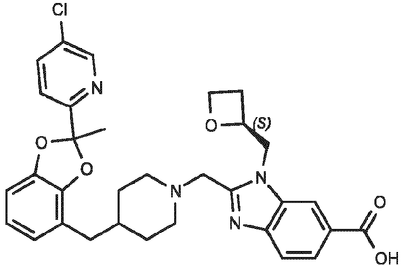 |
| 175 | 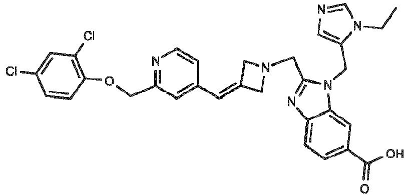 |
| 176 | 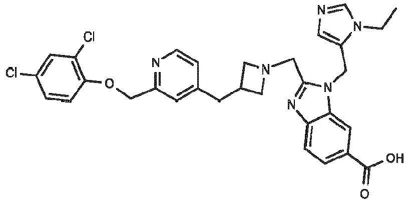 |
| 177 | 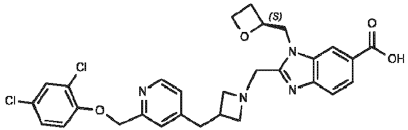 |
| 180 | 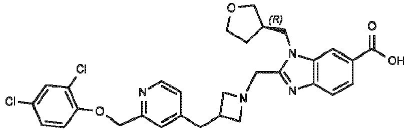 |
| 181 | 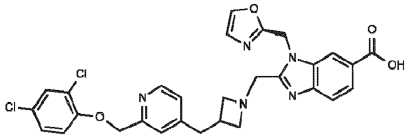 |
| 184 | 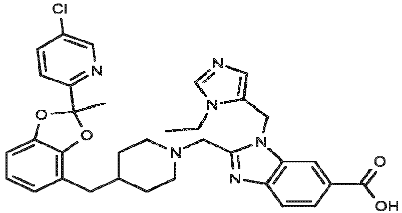 |
| 185 | 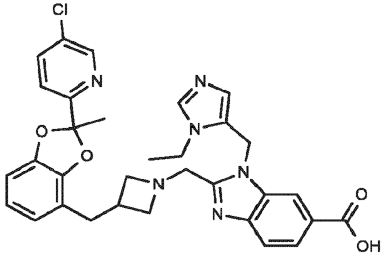 |
| 190 | 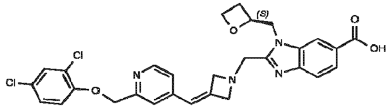 |
| 191 | 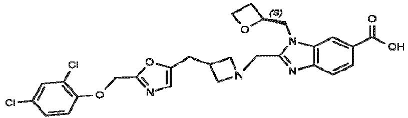 |
| 192 | 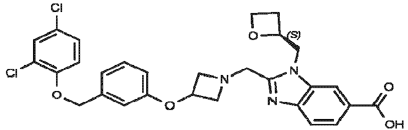 |
| 193 | 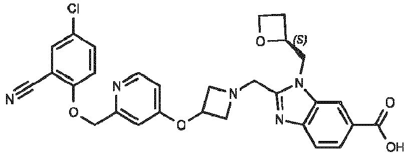 |
| 194 | 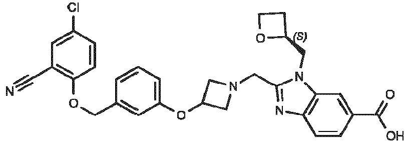 |
| 195 | 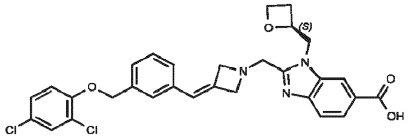 |
| 196 | 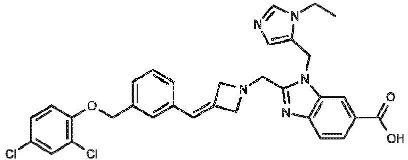 |
| 197 | 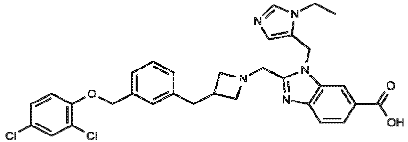 |
| 201 | 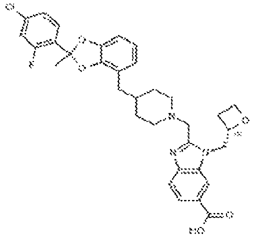 |
| 209 | 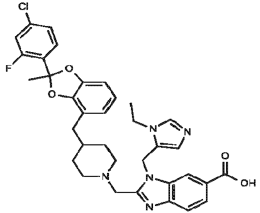 |
| 210 | 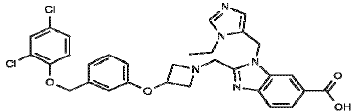 |
| 211 | 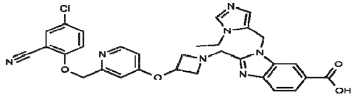 |
| 212 | 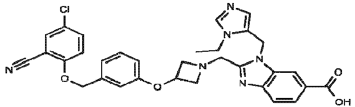 |
| 213 | 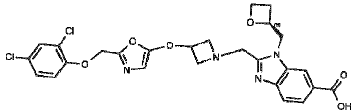 |
| 218 | 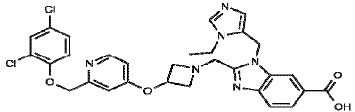 |
| 219 | 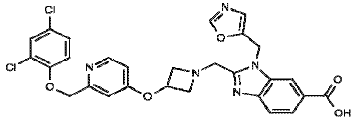 |
| 222 | 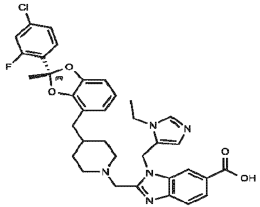 |
| 223 | 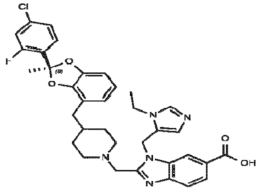 |
| 225 | 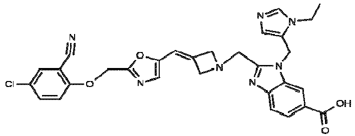 |
| 226 | 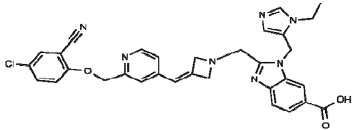 |
| 228 | 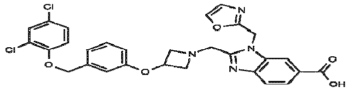 |
| 229 | 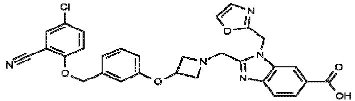 |
| 233 | 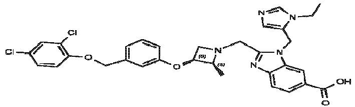 |
| 234 | 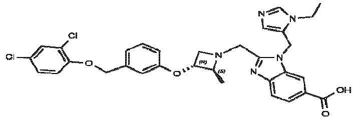 |
| 235 | 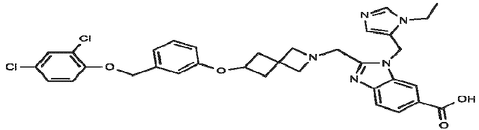 |
| 236 | 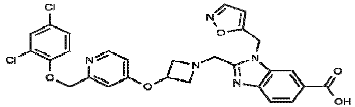 |
| 237 | 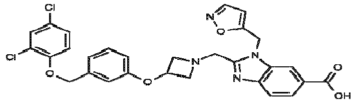 |
| 238 | 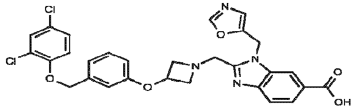 |
| 244 | 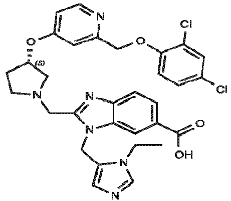 |
| 245 | 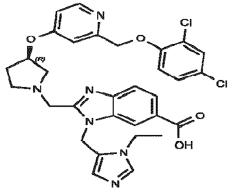 |
| 273 | 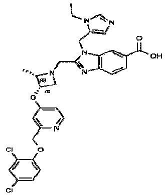 |
| 281 | 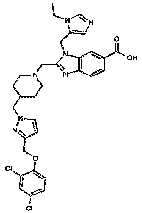 |
| 282 | 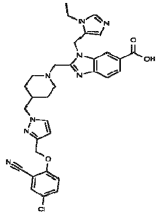 |
| 289 | 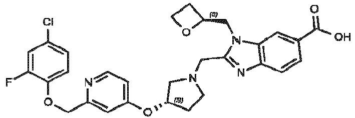 |
| 293 | 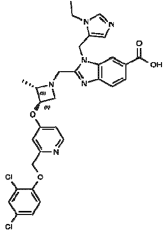 |
| 298 | 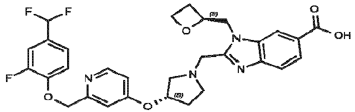 |
| 350 | 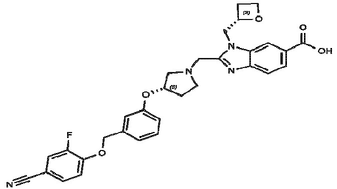 |
| 351 | 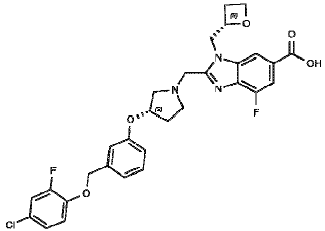 |
| 360 | 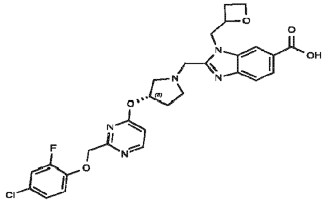 |
| 361 | 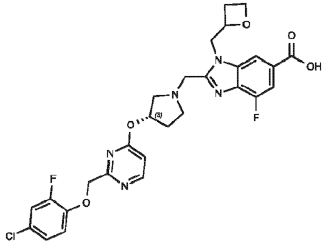 |
| 383 | 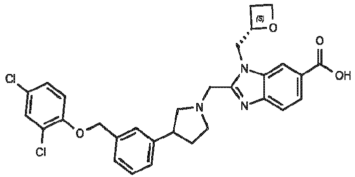 |
| 384 | 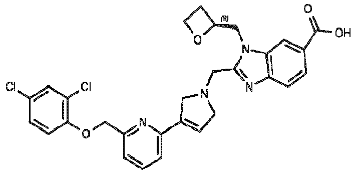 |
| 385 | 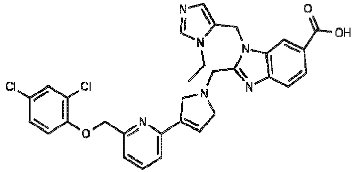 |
| 386 | 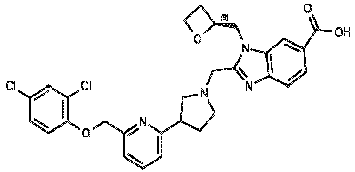 |
| 387 | 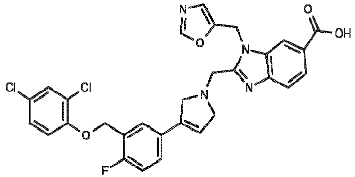 |
| 388 | 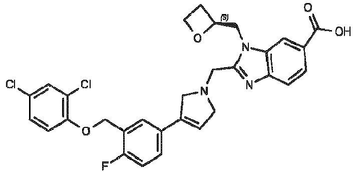 |
| 389 | 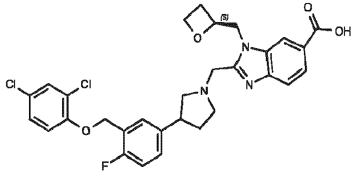 |
| 390 | 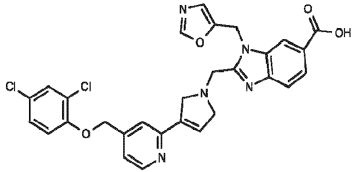 |
| 391 | 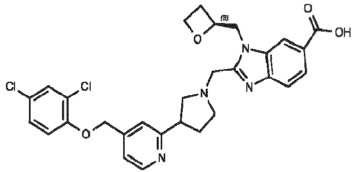 |
| 392 | 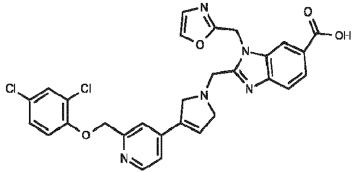 |
| 393 | 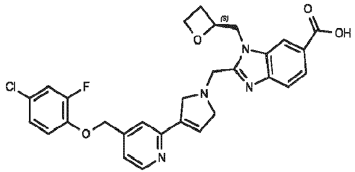 |
| 394 | 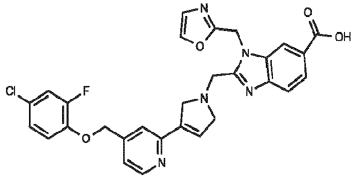 |
| 395 | 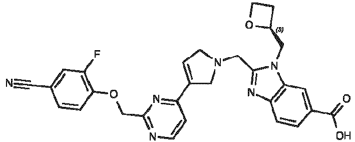 |
| 396 | 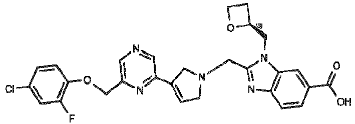 |
| 397 | 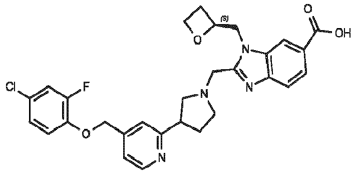 |
| 398 | 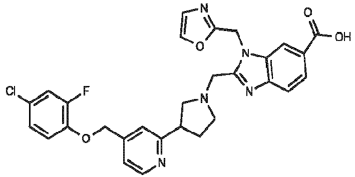 |
| 399 | 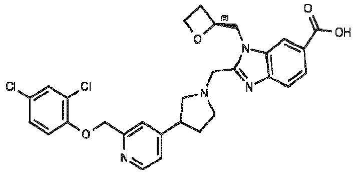 |
| 400 | 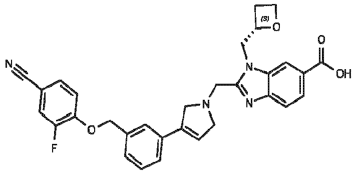 |
| 401 | 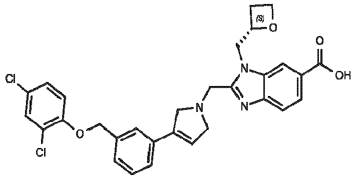 |
| 402 | 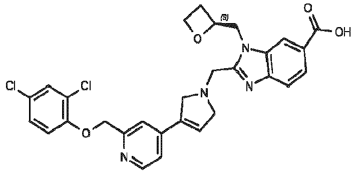 |
| 403 | 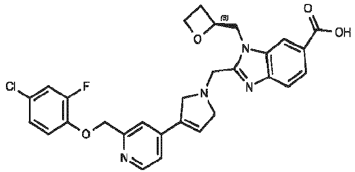 |
| 404 | 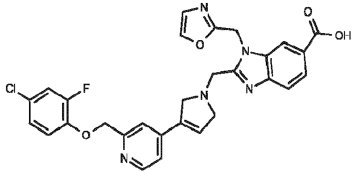 |
| 405 | 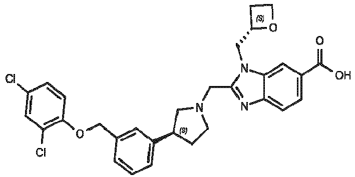 |
| 406 | 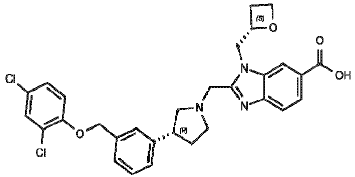 |
| 407 | 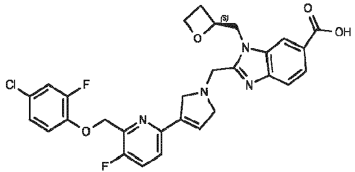 |
| 408 | 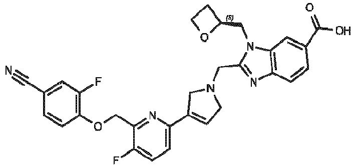 |
| 409 | 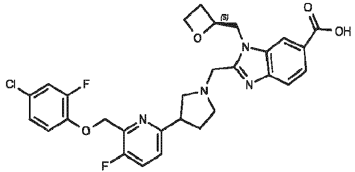 |
| 410 | 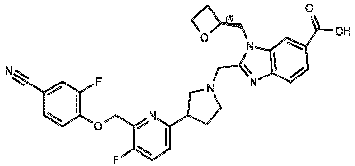 |
| 411 | 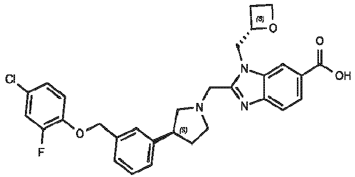 |
| 412 | 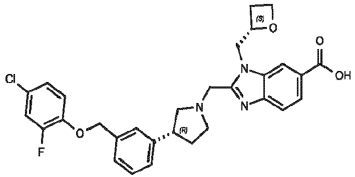 |
| 413 | 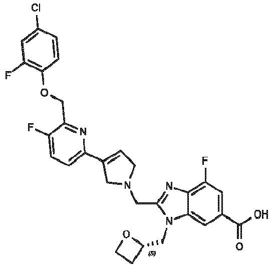 |
| 414 | 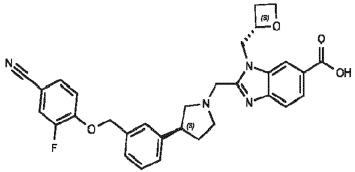 |
| 415 | 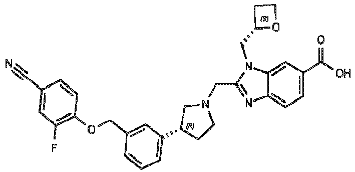 |
| 416 | 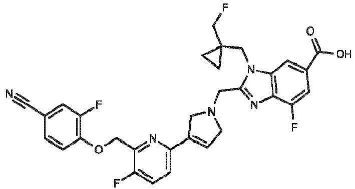 |
| 417 | 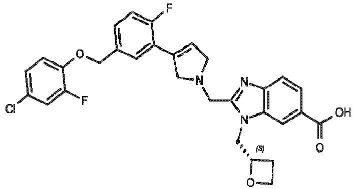 |
| 418 | 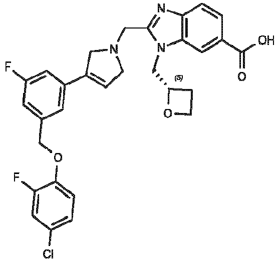 |
| 419 | 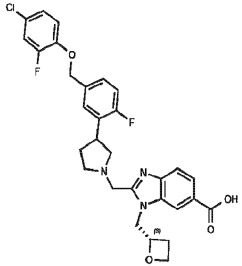 |
| 420 | 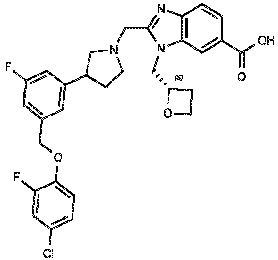 |
| 421 | 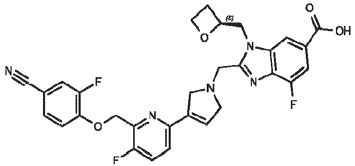 |
| 422 | 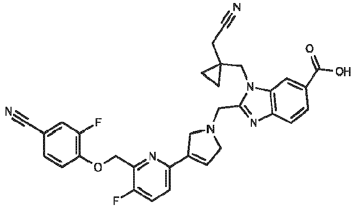 |
| 423 | 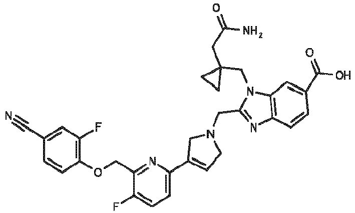 |
| 424 | 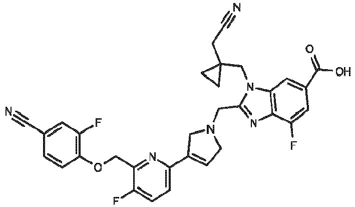 |
| 425 | 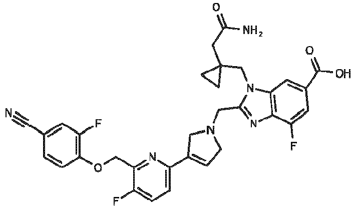 |
| 426 | 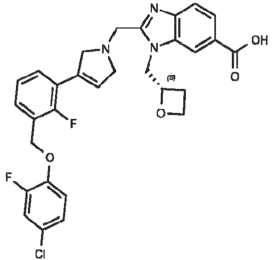 |
| 427 | 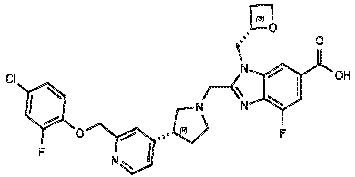 |
| 428 | 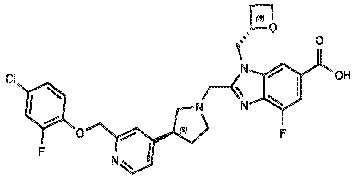 |
| 429 | 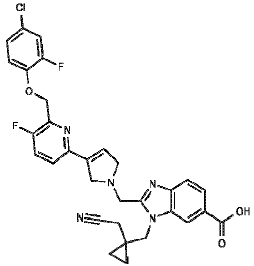 |
| 430 | 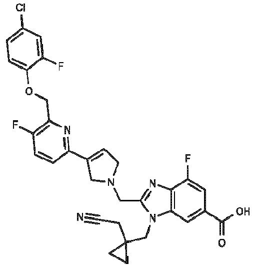 |
| 431 | 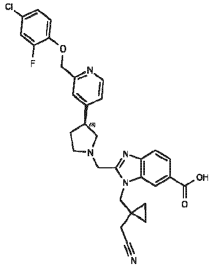 |
| 432 | 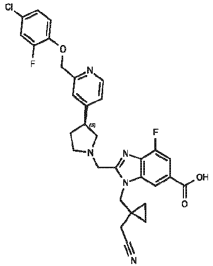 |
| 433 | 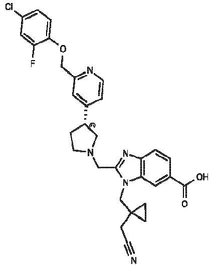 |
| 434 | 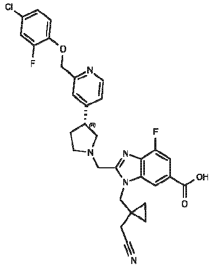 |
| 435 | 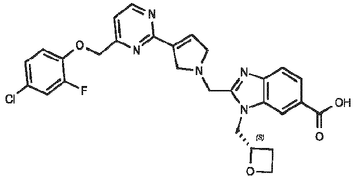 |
| 436 | 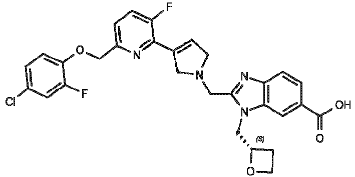 |
| 437 | 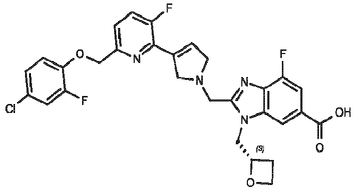 |
| 438 | 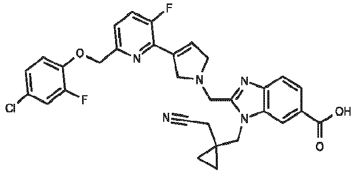 |
| 439 | 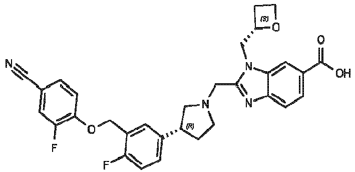 |
| 440 | 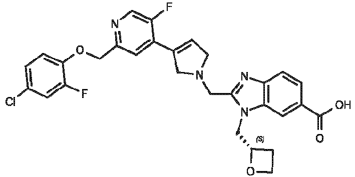 |
| 441 | 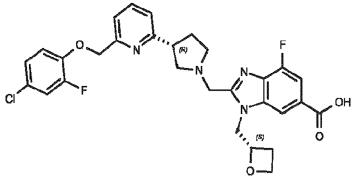 |
| 442 | 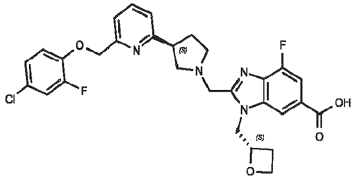 |
| 443 | 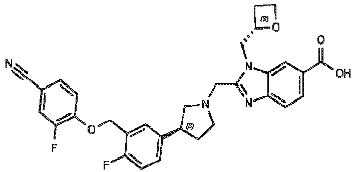 |
| 444 | 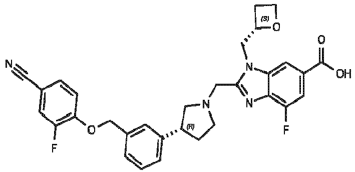 |
| 445 | 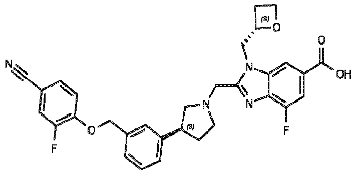 |
| 446 | 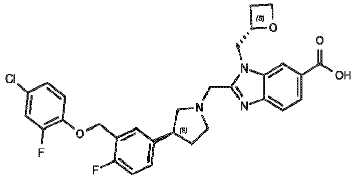 |
| 447 | 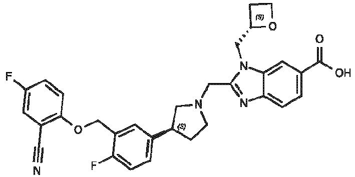 |
| 448 | 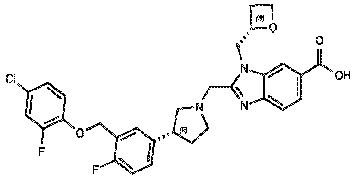 |
| 449 | 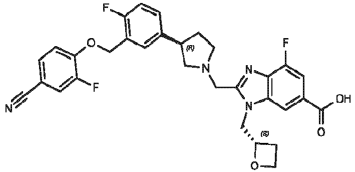 |
| 450 | 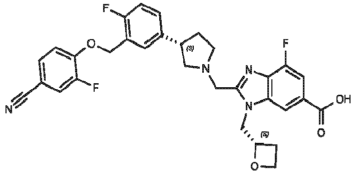 |
| 451 | 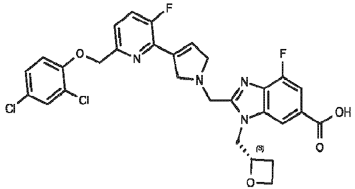 |
| 452 | 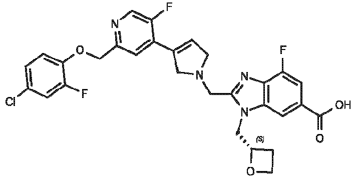 |
| 453 | 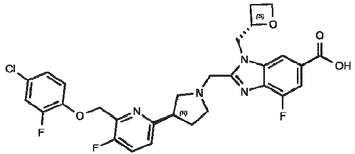 |
| 454 | 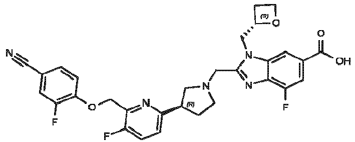 |
| 455 | 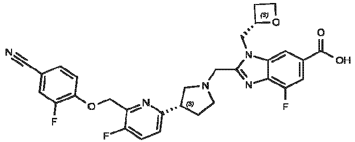 |
| 456 | 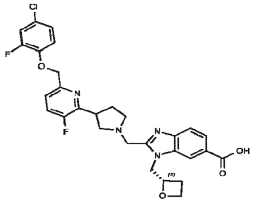 |
| 457 | 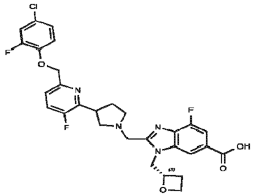 |
| 458 | 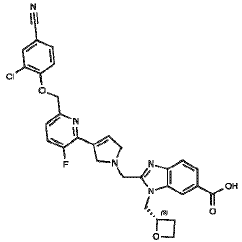 |
| 459 | 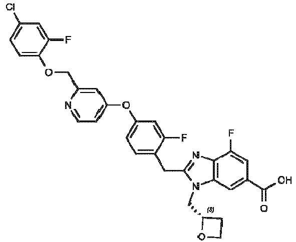 |
| 460 | 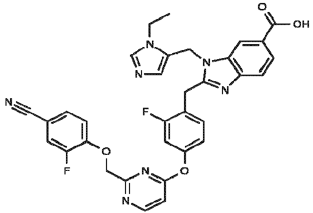 |
| 461 | 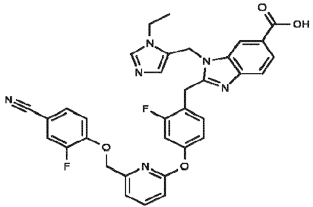 |
| 462 | 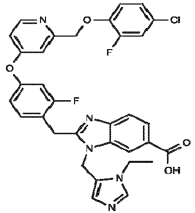 |
| 463 | 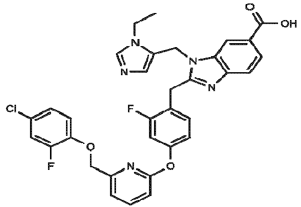 |
| 464 | 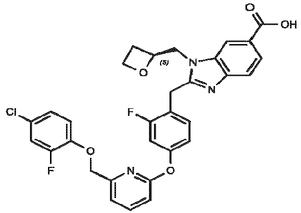 |
| 465 | 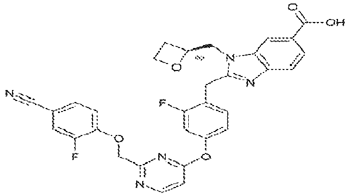 |
| 466 | 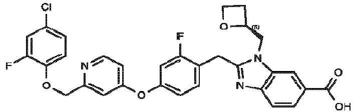 |
| 4G7 | 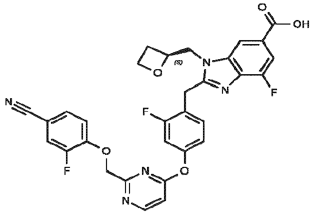 |
| 468 | 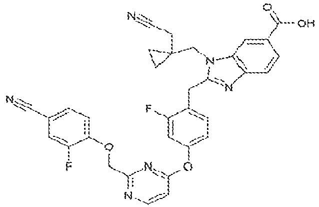 |
| 469 | 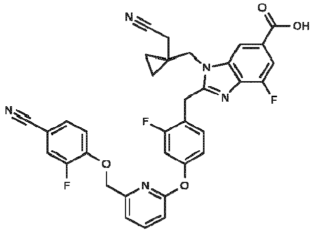 |
| 470 | 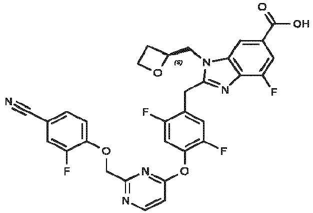 |
| 471 | 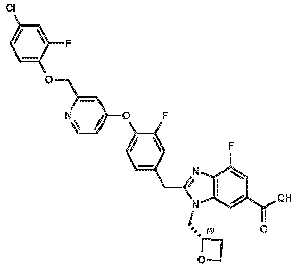 |
| 472 | 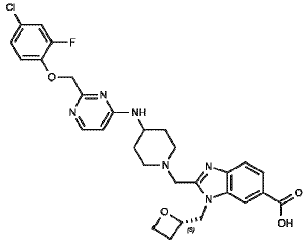 |
| 473 | 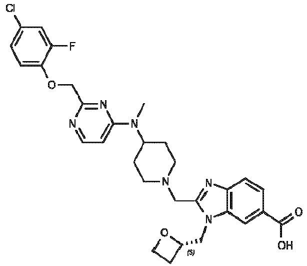 |
| 474 | 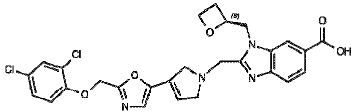 |
| 516 | 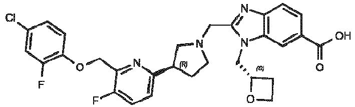 |
| 517 | 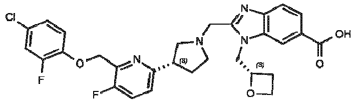 |
| 518 | 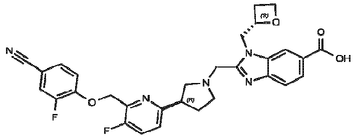 |
| 519 | 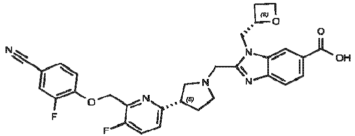 |
| 520 | 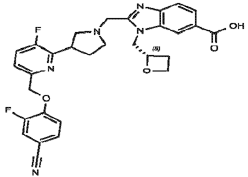 |
| 521 | 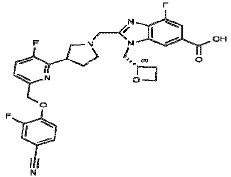 |
| 522 | 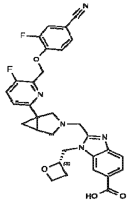 |
| 523 | 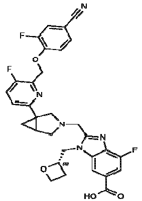 |
| 524 | 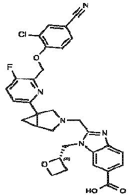 |
| 525 | 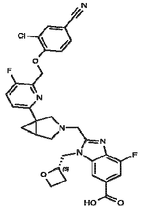 |
| 526 | 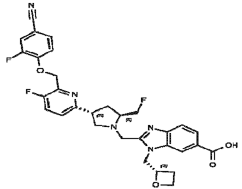 |
| 527 | 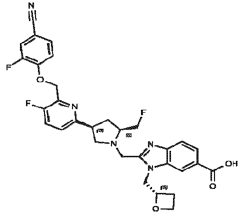 |
| 528 | 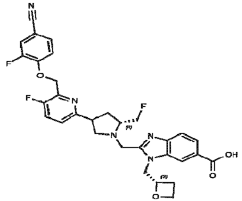 |
| 531 | 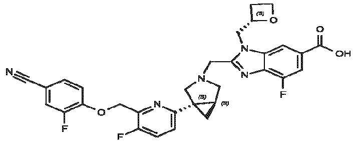 |
| 532 | 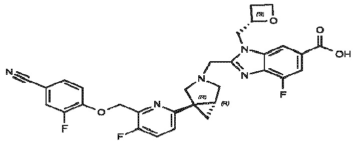 |
| 536 | 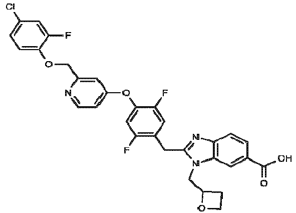 |
| 537 | 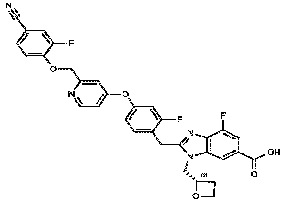 |
| 538 | 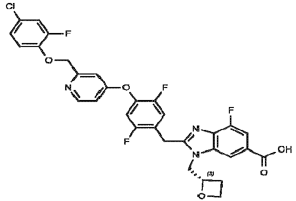 |
| 539 | 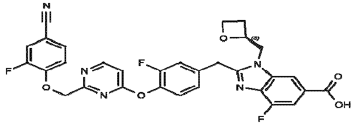 |
| 540 | 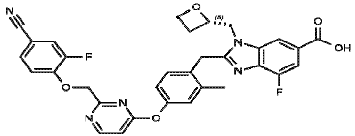 |
| 541 | 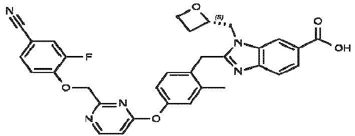 |
| 542 | 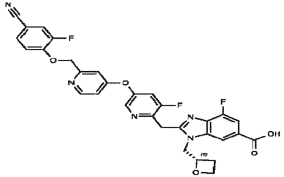 |
| 543 | 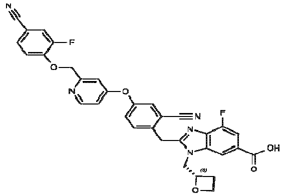 |
| 544 | 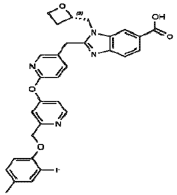 |
| 545 | 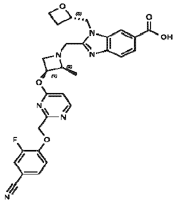 |
| 546 | 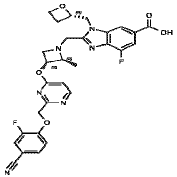 |
| 547 | 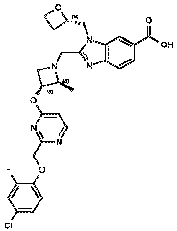 |
| 548 | 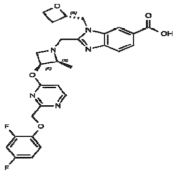 |
| 549 | 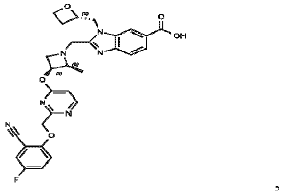 and |
| 550 | 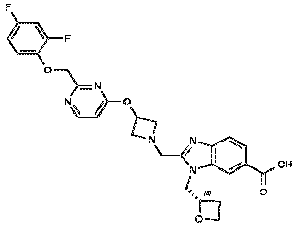 |

E 33. A compound selected from Compound 529, 530, 533, 534, 535, and 551, or a pharmaceutically acceptable salt or solvate thereof: 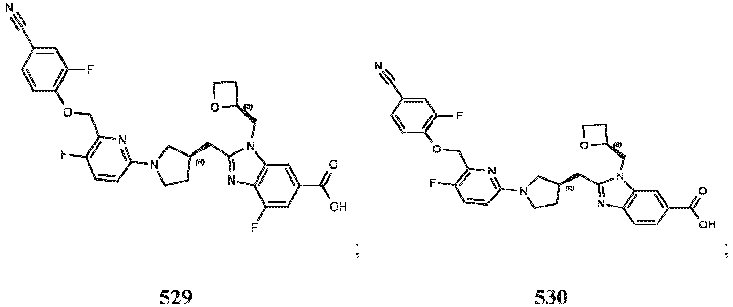 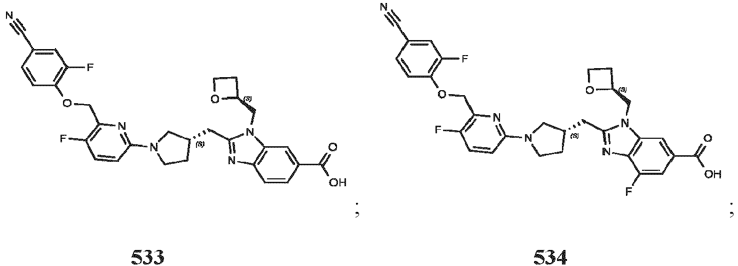 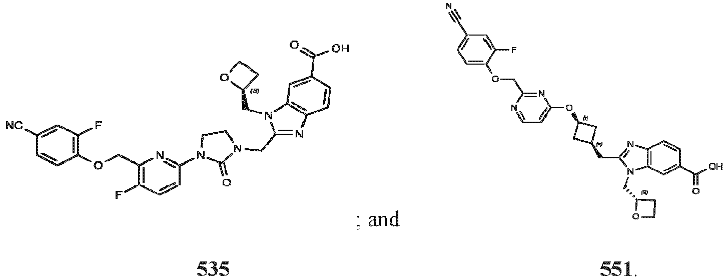 E 34. A pharmaceutical composition comprising the compound of any of E1-E33,
or a pharmaceutically acceptable salt thereof, and one, or more pharmaceutically acceptable carriers, excipients, or diluents.
E 35. A method of treating a metabolic disease or disorder comprising the step of administering to a patient in need thereof a therapeutically effective amount of a compound of any one of E 1-33, or a pharmaceutically acceptable salt thereof, or a therapeutically effective amount of the pharmaceutical composition of E34.
E36. The compound of any one of E1-33, or the pharmaceutical composition of E35 for use in therapy.
E37. The compound of any of one E1-33, or the pharmaceutical composition of E35 for use in the treatment of a metabolic disease, or disorder.
E 38. Use of the compound of any one of E 1-35, or the pharmaceutical composition of E 36 in the manufacture of a medicament for the treatment of a metabolic disease, or disorder.

## Claims

1. A compound selected from Compounds 127, 191, 213, 397, 522, and 532, or a pharmaceutically acceptable salt or solvate thereof:
| **Compound** | **Structure** |
|---|---|
| 127 | 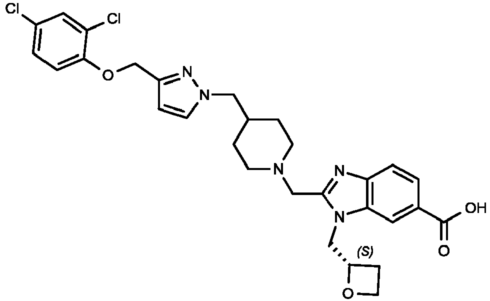 |
| 191 | 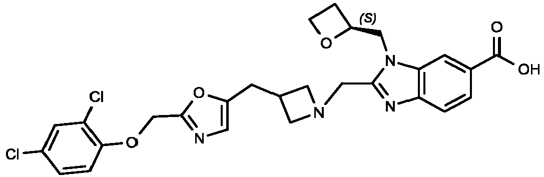 |
| 213 | 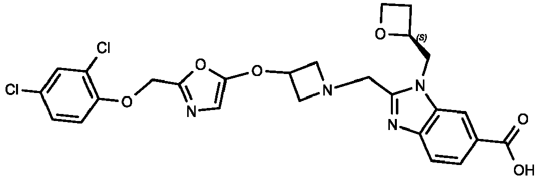 |
| 397 | 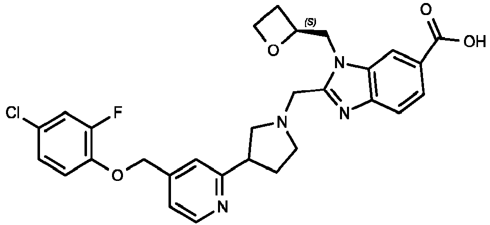 |
| 522 | 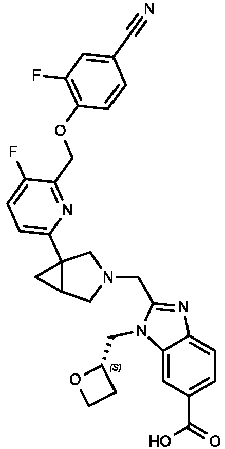 |
| 532 | 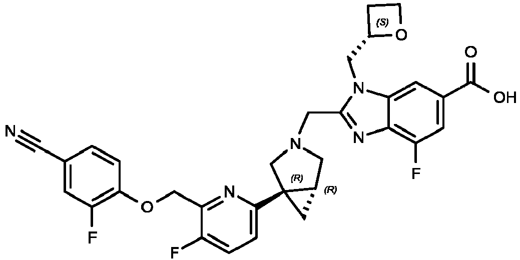 |

2. The compound of claim 1, wherein the compound is Compound 127, or a pharmaceutically acceptable salt or solvate thereof:

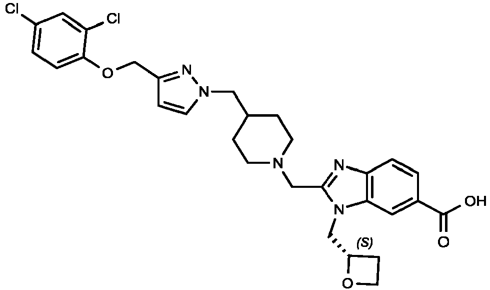

3. The compound of claim 1, wherein the compound is Compound 191, or a pharmaceutically acceptable salt or solvate thereof:

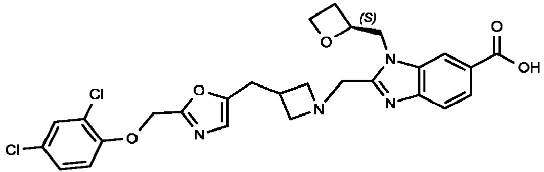

4. The compound of claim 1, wherein the compound is Compound 213, or a pharmaceutically acceptable salt or solvate thereof:

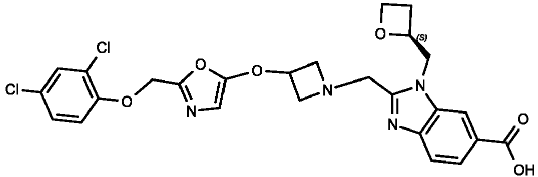

5. The compound of claim 1, wherein the compound is Compound 397, or a pharmaceutically acceptable salt or solvate thereof:

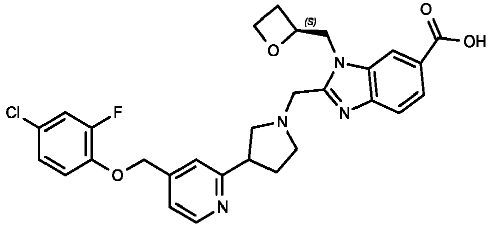

6. The compound of claim 1, wherein the compound is Compound 522, or a pharmaceutically acceptable salt or solvate thereof:

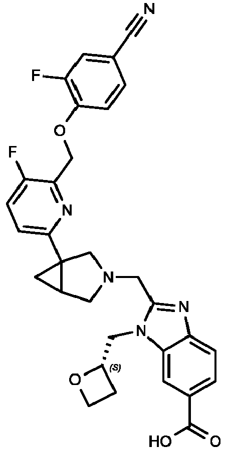

7. The compound of claim 1, wherein the compound is Compound 532, or a pharmaceutically acceptable salt or solvate thereof:

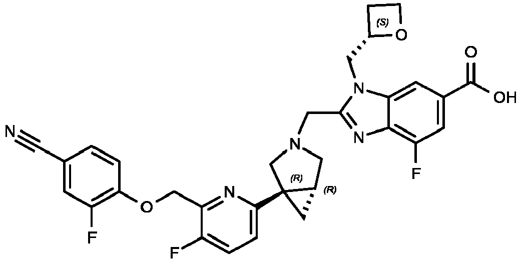

8. A pharmaceutical composition comprising a compound of any of claims 1-7, or a pharmaceutically acceptable salt or solvate thereof, and one or more pharmaceutically acceptable carriers, excipients, or diluents.

9. The compound of any of claims 1-7, or the pharmaceutical composition of claim 8 for use in therapy.

10. The compound of any of claims 1-7, or the pharmaceutical composition of claim 8 for use in the treatment of a metabolic disease, or disorder.
